# EUROPEAN PATENT APPLICATION

(11) **EP 3 412 306 A2**
(43) Date of publication of application: **12.12.2018**
(21) Application number: 18168155.2
(22) Date of filing: 18.08.2010
(51) Int. Cl.: A61K 39/015

(54) **MODIFICATION OF RECOMBINANT ADENOVIRUS WITH IMMUNOGENIC PLASMODIUM CIRCUMSPOROZOITE PROTEIN EPITOPES**

(30) Priority: 18.08.2009 WO PCT/US2009/054212
(62) Divisional of application: 10810576.8
(71) Applicant: Rockefeller University, New York, New York 10065 (US)
(72) Inventor: SHIRATSUCHI, Takayuki, Tokushima City, Tokushima 771-0804 (JP); TSUJI, Moriya, New York, NY 10065 (US)
(74) Representative: HGF Limited

(57) **Abstract**

The present disclosure relates to adenovirus protein modifications to augment immune response to a transgene of a recombinant adenovirus and to circumvent pre-existing anti-adenovirus immunity. Some embodiments are directed to a recombinant adenovirus derived from a recombinant adenovirus plasmid vector, wherein the recombinant adenovirus plasmid vector comprises a nucleotide sequence encoding a Plasmodium circumsporozoite protein, or antigenic portion thereof, operably linked to a heterologous promoter and a modified capsid or core protein, wherein an immunogenic epitope of Plasmodium circumsporozoite is inserted into or replaces at least part of a capsid or core protein. Other embodiments are directed to a pharmaceutical composition or a malaria vaccine composition comprising a recombinant adenovirus according to the above embodiments. Further embodiments include a method of treating, preventing, or diagnosing malaria, comprising administering a therapeutic amount of the pharmaceutical composition or malaria vaccine composition in accordance with the above embodiment.

SEQUENCE LISTING

<110>  Takayuki SHIRATSHUCHI
       Moriya TSUJI

<120>  MODIFICATION OF RECOMBINANT ADENOVIRUS WITH IMMUNOGENIC
       PLASMODIUM CIRCUMSPOROZOITE PROTEIN EPITOPES

<130>  71624.8001.WO01

<140>  PCT/US10/045952
<141>  2010-08-18

<150>  PCT/US09/054212
<151>  2009-08-18

<160>  66

<170>  PatentIn version 3.5

<210>  1
<211>  1127
<212>  DNA
<213>  Artificial Sequence

<220>
<223>  Codon-optimized Plasmodium yoelii circumsporozoite protein


<220>
<221>  CDS
<222>  (13)..(1119)

<400>  1
ggtaccgcca cc atg aag aag tgc acc atc ctg gtg gtg gcc agc ctg ctg        51
              Met Lys Lys Cys Thr Ile Leu Val Val Ala Ser Leu Leu
              1               5                   10

ctg gtg gac agc ctg ctg ccc ggc tac ggc cag aac aag agc gtg cag          99
Leu Val Asp Ser Leu Leu Pro Gly Tyr Gly Gln Asn Lys Ser Val Gln
    15                  20                  25

gcc cag agg aac ctg aac gag ctg tgc tac aac gag gag aac gac aac         147
Ala Gln Arg Asn Leu Asn Glu Leu Cys Tyr Asn Glu Glu Asn Asp Asn
30                  35                  40                  45

aag ctg tac cac gtg ctg aac agc aag aac ggc aag atc tac aac agg         195
Lys Leu Tyr His Val Leu Asn Ser Lys Asn Gly Lys Ile Tyr Asn Arg
                50                  55                  60

aac atc gtg aac agg ctg ctg ggc gac gcc ctg aac ggc aag ccc gag         243
Asn Ile Val Asn Arg Leu Leu Gly Asp Ala Leu Asn Gly Lys Pro Glu
            65                  70                  75

gag aag aag gac gac ccc ccc aag gac gac aac aag gac gac ctg ccc         291
Glu Lys Lys Asp Asp Pro Pro Lys Asp Asp Asn Lys Asp Asp Leu Pro
        80                  85                  90

aag gag gag aag aag gac gac ctg ccc aag gag gag aag aag gac gac         339
Lys Glu Glu Lys Lys Asp Asp Leu Pro Lys Glu Glu Lys Lys Asp Asp
    95                  100                 105

ccc ccc aag gac ccc aag aag gac gac ccc ccc aag gag gcc cag aac         387

Pro Pro Lys Asp Pro Lys Lys Asp Asp Pro Pro Lys Glu Ala Gln Asn
110                 115                 120                 125

aag ctg aac cag ccc gta gtg gca gat gaa aat gta gat caa ggg cca       435
Lys Leu Asn Gln Pro Val Val Ala Asp Glu Asn Val Asp Gln Gly Pro
                130                 135                 140

gga gca cca caa ggg cca gga gca cca caa ggg cca gga gca cca cag       483
Gly Ala Pro Gln Gly Pro Gly Ala Pro Gln Gly Pro Gly Ala Pro Gln
            145                 150                 155

ggg cca gga gca cca cag ggg cca gga gca cca caa ggg cca gga gca       531
Gly Pro Gly Ala Pro Gln Gly Pro Gly Ala Pro Gln Gly Pro Gly Ala
        160                 165                 170

cca caa gga cca gga gca cca caa ggg cca gga gca cca caa ggg cca       579
Pro Gln Gly Pro Gly Ala Pro Gln Gly Pro Gly Ala Pro Gln Gly Pro
    175                 180                 185

gga gca cca caa ggg cca gga gca ccg cag ggg cca gga gca cca caa       627
Gly Ala Pro Gln Gly Pro Gly Ala Pro Gln Gly Pro Gly Ala Pro Gln
190                 195                 200                 205

ggg cca gga gca cca caa gga cca gga gca cca cag ggt cca gga gca       675
Gly Pro Gly Ala Pro Gln Gly Pro Gly Ala Pro Gln Gly Pro Gly Ala
                210                 215                 220

cca caa gga cca gga gca cca caa gga cca gga gca cca caa ggt cca       723
Pro Gln Gly Pro Gly Ala Pro Gln Gly Pro Gly Ala Pro Gln Gly Pro
            225                 230                 235

gga gca cca cag ggg cca gga gca cca caa ggg cca gga gca cca caa       771
Gly Ala Pro Gln Gly Pro Gly Ala Pro Gln Gly Pro Gly Ala Pro Gln
        240                 245                 250

gaa cca ccc caa caa ccc cca caa cag cca cca caa cag cca cca caa       819
Glu Pro Pro Gln Gln Pro Pro Gln Gln Pro Pro Gln Gln Pro Pro Gln
    255                 260                 265

cag cca cca caa cag cca cca caa caa cca aac aac aac aac aac aac       867
Gln Pro Pro Gln Gln Pro Pro Gln Gln Pro Asn Asn Asn Asn Asn Asn
270                 275                 280                 285

aac ggc aac aac aac gag gac agc tac gtg ccc agc gcc gag cag atc       915
Asn Gly Asn Asn Asn Glu Asp Ser Tyr Val Pro Ser Ala Glu Gln Ile
                290                 295                 300

ctg gag ttc gtg aag cag atc agc agc cag ctg acc gag gag tgg agc       963
Leu Glu Phe Val Lys Gln Ile Ser Ser Gln Leu Thr Glu Glu Trp Ser
            305                 310                 315

cag tgc agc gtg acc tgc ggc agc ggc gtg agg gtg agg aag agg aag       1011
Gln Cys Ser Val Thr Cys Gly Ser Gly Val Arg Val Arg Lys Arg Lys
        320                 325                 330

aac gtg aac aag cag ccc gag aac ctg acc ctg gag gac atc gac acc       1059
Asn Val Asn Lys Gln Pro Glu Asn Leu Thr Leu Glu Asp Ile Asp Thr
    335                 340                 345

gag atc tgc aag atg gac aag tgc agc agc atc ttc aac atc gtg agc       1107
Glu Ile Cys Lys Met Asp Lys Cys Ser Ser Ile Phe Asn Ile Val Ser
350                 355                 360                 365

aac agc ctg ggc taaagctt                                          1127
Asn Ser Leu Gly

<210>  2
<211>  1145
<212>  DNA
<213>  Artificial Sequence

<220>
<223>  Codon-optimized Plasmodium falciparus circumsporozoite protein

<220>
<221>  CDS
<222>  (13)..(1134)

<400>  2
ggtaccgcca cc atg atg cgc aag ctc gcc ata ctg tct gtt agt agc ttt      51
              Met Met Arg Lys Leu Ala Ile Leu Ser Val Ser Ser Phe
              1               5                   10

ctc ttt gta gag gcc ctg ttt cag gaa tac cag tgc tat ggc agc agc      99
Leu Phe Val Glu Ala Leu Phe Gln Glu Tyr Gln Cys Tyr Gly Ser Ser
    15                  20                  25

agc aac act cgt gtg ctg aac gaa ctt aac tat gat aat gca gga aca      147
Ser Asn Thr Arg Val Leu Asn Glu Leu Asn Tyr Asp Asn Ala Gly Thr
30                  35                  40                  45

aat tta tat aac gaa ctg gag atg aat tac tat ggt aag cag gaa aat      195
Asn Leu Tyr Asn Glu Leu Glu Met Asn Tyr Tyr Gly Lys Gln Glu Asn
                50                  55                  60

tgg tac tct ctg aaa aag aac tct aga tct ctg ggc gag aac gac gac      243
Trp Tyr Ser Leu Lys Lys Asn Ser Arg Ser Leu Gly Glu Asn Asp Asp
            65                  70                  75

ggc aat aat gaa gac aat gaa aag ctg agg aag cca aag cac aaa aaa      291
Gly Asn Asn Glu Asp Asn Glu Lys Leu Arg Lys Pro Lys His Lys Lys
        80                  85                  90

cta aag cag ccc gca gac ggc aat cca gac ccc aat gct aac cca aac      339
Leu Lys Gln Pro Ala Asp Gly Asn Pro Asp Pro Asn Ala Asn Pro Asn
    95                  100                 105

gtg gac ccc aat gct aat cca aac gtg gat cct aac gct aac ccg aat      387
Val Asp Pro Asn Ala Asn Pro Asn Val Asp Pro Asn Ala Asn Pro Asn
110                 115                 120                 125

gtg gac cct aac gcc aat cca aat gcg aat ccc aac gct aat cct aac      435
Val Asp Pro Asn Ala Asn Pro Asn Ala Asn Pro Asn Ala Asn Pro Asn
                130                 135                 140

gca aac ccg aat gct aac cct aac gca aac ccc aac gct aac ccc aac      483
Ala Asn Pro Asn Ala Asn Pro Asn Ala Asn Pro Asn Ala Asn Pro Asn
            145                 150                 155

gcg aac ccc aat gcc aac ccc aac gcc aac ccg aac gcc aat cca aac      531
Ala Asn Pro Asn Ala Asn Pro Asn Ala Asn Pro Asn Ala Asn Pro Asn
        160                 165                 170

gct aac cct aat gcc aat cct aat gcc aat ccg aac gcc aat cca aat     579
Ala Asn Pro Asn Ala Asn Pro Asn Ala Asn Pro Asn Ala Asn Pro Asn
    175                 180                 185

gcc aat cca aat gct aat ccc aac gtg gac ccc aac gcg aac cct aat     627
Ala Asn Pro Asn Ala Asn Pro Asn Val Asp Pro Asn Ala Asn Pro Asn
190                 195                 200                 205

gcc aac ccc aac gct aat cca aat gcg aac cct aac gcc aac ccg aat     675
Ala Asn Pro Asn Ala Asn Pro Asn Ala Asn Pro Asn Ala Asn Pro Asn
                210                 215                 220

gct aat ccc aat gcc aac ccc aat gct aat ccc aat gcg aac cct aat     723
Ala Asn Pro Asn Ala Asn Pro Asn Ala Asn Pro Asn Ala Asn Pro Asn
            225                 230                 235

gcc aat ccc aac gcc aac ccc aac gca aac cct aat gct aac ccg aac     771
Ala Asn Pro Asn Ala Asn Pro Asn Ala Asn Pro Asn Ala Asn Pro Asn
        240                 245                 250

gcc aat ccg aac gca aat cct aat gct aat cct aac gct aac ccc aac     819
Ala Asn Pro Asn Ala Asn Pro Asn Ala Asn Pro Asn Ala Asn Pro Asn
    255                 260                 265

gcc aat cca aat aag aac aat caa ggc aac ggg cag ggg cac aat atg     867
Ala Asn Pro Asn Lys Asn Asn Gln Gly Asn Gly Gln Gly His Asn Met
270                 275                 280                 285

cca aat gac cct aac cgg aat gtc gac gag aat gca aat gcc aat agc     915
Pro Asn Asp Pro Asn Arg Asn Val Asp Glu Asn Ala Asn Ala Asn Ser
                290                 295                 300

gcc gtg aaa aat aat aat aac gag gag cca agt gac aaa cac att aag     963
Ala Val Lys Asn Asn Asn Asn Glu Glu Pro Ser Asp Lys His Ile Lys
            305                 310                 315

gaa tat ttg aac aag att caa aac tcc ctc tca aca gaa tgg tct cct     1011
Glu Tyr Leu Asn Lys Ile Gln Asn Ser Leu Ser Thr Glu Trp Ser Pro
        320                 325                 330

tgc agc gtg act tgt gga aat ggc atc cag gtt cgt att aaa cca ggt     1059
Cys Ser Val Thr Cys Gly Asn Gly Ile Gln Val Arg Ile Lys Pro Gly
    335                 340                 345

agt gcc aac aag ccc aag gat gaa cta gac tat gcg aat gat ata gag     1107
Ser Ala Asn Lys Pro Lys Asp Glu Leu Asp Tyr Ala Asn Asp Ile Glu
350                 355                 360                 365

aaa aaa atc tgt aag atg gag aaa tgc tagcttctag a                   1145
Lys Lys Ile Cys Lys Met Glu Lys Cys
                370

<210>  3
<211>  2832
<212>  DNA
<213>  Artificial Sequence

<220>
<223>  (QGPGAP)3-HVR1 modified Hexon protein

<220>
<221>  CDS
<222>  (1)..(2829)

<400>  3
atg gct acc cct tcg atg atg ccg cag tgg tct tac atg cac atc tcg      48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa cag ggc cct gga gct cca cag      432
Ala Pro Asn Pro Cys Glu Trp Asp Glu Gln Gly Pro Gly Ala Pro Gln
    130                 135                 140

gga cca ggt gca cct caa ggg cct gga gcc cct aaa act cac gta ttt      480
Gly Pro Gly Ala Pro Gln Gly Pro Gly Ala Pro Lys Thr His Val Phe
145                 150                 155                 160

ggg cag gcg cct tat tct ggt ata aat att aca aag gag ggt att caa      528
Gly Gln Ala Pro Tyr Ser Gly Ile Asn Ile Thr Lys Glu Gly Ile Gln
                165                 170                 175

ata ggt gtc gaa ggt caa aca cct aaa tat gcc gat aaa aca ttt caa      576
Ile Gly Val Glu Gly Gln Thr Pro Lys Tyr Ala Asp Lys Thr Phe Gln
            180                 185                 190

cct gaa cct caa ata gga gaa tct cag tgg tac gaa aca gaa att aat      624
Pro Glu Pro Gln Ile Gly Glu Ser Gln Trp Tyr Glu Thr Glu Ile Asn
        195                 200                 205

cat gca gct ggg aga gtc cta aaa aag act acc cca atg aaa cca tgt      672
His Ala Ala Gly Arg Val Leu Lys Lys Thr Thr Pro Met Lys Pro Cys
    210                 215                 220

tac ggt tca tat gca aaa ccc aca aat gaa aat gga ggg caa ggc att      720
Tyr Gly Ser Tyr Ala Lys Pro Thr Asn Glu Asn Gly Gly Gln Gly Ile

225                 230                 235                 240

ctt gta aag caa caa aat gga aag cta gaa agt caa gtg gaa atg caa       768
Leu Val Lys Gln Gln Asn Gly Lys Leu Glu Ser Gln Val Glu Met Gln
                245                 250                 255

ttt ttc tca act act gag gca gcc gca ggc aat ggt gat aac ttg act       816
Phe Phe Ser Thr Thr Glu Ala Ala Ala Gly Asn Gly Asp Asn Leu Thr
            260                 265                 270

cct aaa gtg gta ttg tac agt gaa gat gta gat ata gaa acc cca gac       864
Pro Lys Val Val Leu Tyr Ser Glu Asp Val Asp Ile Glu Thr Pro Asp
        275                 280                 285

act cat att tct tac atg ccc act att aag gaa ggt aac tca cga gaa       912
Thr His Ile Ser Tyr Met Pro Thr Ile Lys Glu Gly Asn Ser Arg Glu
    290                 295                 300

cta atg ggc caa caa tct atg ccc aac agg cct aat tac att gct ttt       960
Leu Met Gly Gln Gln Ser Met Pro Asn Arg Pro Asn Tyr Ile Ala Phe
305                 310                 315                 320

agg gac aat ttt att ggt cta atg tat tac aac agc acg ggt aat atg      1008
Arg Asp Asn Phe Ile Gly Leu Met Tyr Tyr Asn Ser Thr Gly Asn Met
                325                 330                 335

ggt gtt ctg gcg ggc caa gca tcg cag ttg aat gct gtt gta gat ttg      1056
Gly Val Leu Ala Gly Gln Ala Ser Gln Leu Asn Ala Val Val Asp Leu
            340                 345                 350

caa gac aga aac aca gag ctt tca tac cag ctt ttg ctt gat tcc att      1104
Gln Asp Arg Asn Thr Glu Leu Ser Tyr Gln Leu Leu Leu Asp Ser Ile
        355                 360                 365

ggt gat aga acc agg tac ttt tct atg tgg aat cag gct gtt gac agc      1152
Gly Asp Arg Thr Arg Tyr Phe Ser Met Trp Asn Gln Ala Val Asp Ser
    370                 375                 380

tat gat cca gat gtt aga att att gaa aat cat gga act gaa gat gaa      1200
Tyr Asp Pro Asp Val Arg Ile Ile Glu Asn His Gly Thr Glu Asp Glu
385                 390                 395                 400

ctt cca aat tac tgc ttt cca ctg gga ggt gtg att aat aca gag act      1248
Leu Pro Asn Tyr Cys Phe Pro Leu Gly Gly Val Ile Asn Thr Glu Thr
                405                 410                 415

ctt acc aag gta aaa cct aaa aca ggt cag gaa aat gga tgg gaa aaa      1296
Leu Thr Lys Val Lys Pro Lys Thr Gly Gln Glu Asn Gly Trp Glu Lys
            420                 425                 430

gat gct aca gaa ttt tca gat aaa aat gaa ata aga gtt gga aat aat      1344
Asp Ala Thr Glu Phe Ser Asp Lys Asn Glu Ile Arg Val Gly Asn Asn
        435                 440                 445

ttt gcc atg gaa atc aat cta aat gcc aac ctg tgg aga aat ttc ctg      1392
Phe Ala Met Glu Ile Asn Leu Asn Ala Asn Leu Trp Arg Asn Phe Leu
    450                 455                 460

tac tcc aac ata gcg ctg tat ttg ccc gac aag cta aag tac agt cct      1440
Tyr Ser Asn Ile Ala Leu Tyr Leu Pro Asp Lys Leu Lys Tyr Ser Pro
465                 470                 475                 480

tcc aac gta aaa att tct gat aac cca aac acc tac gac tac atg aac      1488

Ser Asn Val Lys Ile Ser Asp Asn Pro Asn Thr Tyr Asp Tyr Met Asn
                485                 490                 495

aag cga gtg gtg gct ccc ggg cta gtg gac tgc tac att aac ctt gga      1536
Lys Arg Val Val Ala Pro Gly Leu Val Asp Cys Tyr Ile Asn Leu Gly
            500                 505                 510

gca cgc tgg tcc ctt gac tat atg gac aac gtc aac cca ttt aac cac      1584
Ala Arg Trp Ser Leu Asp Tyr Met Asp Asn Val Asn Pro Phe Asn His
        515                 520                 525

cac cgc aat gct ggc ctg cgc tac cgc tca atg ttg ctg ggc aat ggt      1632
His Arg Asn Ala Gly Leu Arg Tyr Arg Ser Met Leu Leu Gly Asn Gly
    530                 535                 540

cgc tat gtg ccc ttc cac atc cag gtg cct cag aag ttc ttt gcc att      1680
Arg Tyr Val Pro Phe His Ile Gln Val Pro Gln Lys Phe Phe Ala Ile
545                 550                 555                 560

aaa aac ctc ctt ctc ctg ccg ggc tca tac acc tac gag tgg aac ttc      1728
Lys Asn Leu Leu Leu Leu Pro Gly Ser Tyr Thr Tyr Glu Trp Asn Phe
                565                 570                 575

agg aag gat gtt aac atg gtt ctg cag agc tcc cta gga aat gac cta      1776
Arg Lys Asp Val Asn Met Val Leu Gln Ser Ser Leu Gly Asn Asp Leu
            580                 585                 590

agg gtt gac gga gcc agc att aag ttt gat agc att tgc ctt tac gcc      1824
Arg Val Asp Gly Ala Ser Ile Lys Phe Asp Ser Ile Cys Leu Tyr Ala
        595                 600                 605

acc ttc ttc ccc atg gcc cac aac acc gcc tcc acg ctt gag gcc atg      1872
Thr Phe Phe Pro Met Ala His Asn Thr Ala Ser Thr Leu Glu Ala Met
    610                 615                 620

ctt aga aac gac acc aac gac cag tcc ttt aac gac tat ctc tcc gcc      1920
Leu Arg Asn Asp Thr Asn Asp Gln Ser Phe Asn Asp Tyr Leu Ser Ala
625                 630                 635                 640

gcc aac atg ctc tac cct ata ccc gcc aac gct acc aac gtg ccc ata      1968
Ala Asn Met Leu Tyr Pro Ile Pro Ala Asn Ala Thr Asn Val Pro Ile
                645                 650                 655

tcc atc ccc tcc cgc aac tgg gcg gct ttc cgc ggc tgg gcc ttc acg      2016
Ser Ile Pro Ser Arg Asn Trp Ala Ala Phe Arg Gly Trp Ala Phe Thr
            660                 665                 670

cgc ctt aag act aag gaa acc cca tca ctg ggc tcg ggc tac gac cct      2064
Arg Leu Lys Thr Lys Glu Thr Pro Ser Leu Gly Ser Gly Tyr Asp Pro
        675                 680                 685

tat tac acc tac tct ggc tct ata ccc tac cta gat gga acc ttt tac      2112
Tyr Tyr Thr Tyr Ser Gly Ser Ile Pro Tyr Leu Asp Gly Thr Phe Tyr
    690                 695                 700

ctc aac cac acc ttt aag aag gtg gcc att acc ttt gac tct tct gtc      2160
Leu Asn His Thr Phe Lys Lys Val Ala Ile Thr Phe Asp Ser Ser Val
705                 710                 715                 720

agc tgg cct ggc aat gac cgc ctg ctt acc ccc aac gag ttt gaa att      2208
Ser Trp Pro Gly Asn Asp Arg Leu Leu Thr Pro Asn Glu Phe Glu Ile
                725                 730                 735

aag cgc tca gtt gac ggg gag ggt tac aac gtt gcc cag tgt aac atg          2256
Lys Arg Ser Val Asp Gly Glu Gly Tyr Asn Val Ala Gln Cys Asn Met
            740                 745                 750

acc aaa gac tgg ttc ctg gta caa atg cta gct aac tat aac att ggc          2304
Thr Lys Asp Trp Phe Leu Val Gln Met Leu Ala Asn Tyr Asn Ile Gly
        755                 760                 765

tac cag ggc ttc tat atc cca gag agc tac aag gac cgc atg tac tcc          2352
Tyr Gln Gly Phe Tyr Ile Pro Glu Ser Tyr Lys Asp Arg Met Tyr Ser
    770                 775                 780

ttc ttt aga aac ttc cag ccc atg agc cgt cag gtg gtg gat gat act          2400
Phe Phe Arg Asn Phe Gln Pro Met Ser Arg Gln Val Val Asp Asp Thr
785                 790                 795                 800

aaa tac aag gac tac caa cag gtg ggc atc cta cac caa cac aac aac          2448
Lys Tyr Lys Asp Tyr Gln Gln Val Gly Ile Leu His Gln His Asn Asn
                805                 810                 815

tct gga ttt gtt ggc tac ctt gcc ccc acc atg cgc gaa gga cag gcc          2496
Ser Gly Phe Val Gly Tyr Leu Ala Pro Thr Met Arg Glu Gly Gln Ala
            820                 825                 830

tac cct gct aac ttc ccc tat ccg ctt ata ggc aag acc gca gtt gac          2544
Tyr Pro Ala Asn Phe Pro Tyr Pro Leu Ile Gly Lys Thr Ala Val Asp
        835                 840                 845

agc att acc cag aaa aag ttt ctt tgc gat cgc acc ctt tgg cgc atc          2592
Ser Ile Thr Gln Lys Lys Phe Leu Cys Asp Arg Thr Leu Trp Arg Ile
    850                 855                 860

cca ttc tcc agt aac ttt atg tcc atg ggc gca ctc aca gac ctg ggc          2640
Pro Phe Ser Ser Asn Phe Met Ser Met Gly Ala Leu Thr Asp Leu Gly
865                 870                 875                 880

caa aac ctt ctc tac gcc aac tcc gcc cac gcg cta gac atg act ttt          2688
Gln Asn Leu Leu Tyr Ala Asn Ser Ala His Ala Leu Asp Met Thr Phe
                885                 890                 895

gag gtg gat ccc atg gac gag ccc acc ctt ctt tat gtt ttg ttt gaa          2736
Glu Val Asp Pro Met Asp Glu Pro Thr Leu Leu Tyr Val Leu Phe Glu
            900                 905                 910

gtc ttt gac gtg gtc cgt gtg cac cag ccg cac cgc ggc gtc atc gaa          2784
Val Phe Asp Val Val Arg Val His Gln Pro His Arg Gly Val Ile Glu
        915                 920                 925

acc gtg tac ctg cgc acg ccc ttc tcg gcc ggc aac gcc aca aca taa          2832
Thr Val Tyr Leu Arg Thr Pro Phe Ser Ala Gly Asn Ala Thr Thr
    930                 935                 940

<210>  4
<211>  2790
<212>  DNA
<213>  Artificial Sequence

<220>
<223>  (QGPGAP)4-HVR1 modified Hexon protein

<220>
<221>  CDS
<222>  (1)..(2787)

<400>  4
atg gct acc cct tcg atg atg ccg cag tgg tct tac atg cac atc tcg      48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa cag gga cca gga gca cca cag      432
Ala Pro Asn Pro Cys Glu Trp Asp Glu Gln Gly Pro Gly Ala Pro Gln
    130                 135                 140

gga cca gga gca cca cag gga cca gga gca ccg caa ggt cct ggt gct      480
Gly Pro Gly Ala Pro Gln Gly Pro Gly Ala Pro Gln Gly Pro Gly Ala
145                 150                 155                 160

cct aaa act cac gta ttt ggg cag gcg cct tat tct ggt ata aat att      528
Pro Lys Thr His Val Phe Gly Gln Ala Pro Tyr Ser Gly Ile Asn Ile
                165                 170                 175

aca aag gag ggt att caa ata ggt gtc gaa ggt caa aca cct aaa tat      576
Thr Lys Glu Gly Ile Gln Ile Gly Val Glu Gly Gln Thr Pro Lys Tyr
            180                 185                 190

gcc gat aaa aca ttt caa cct gaa cct caa ata gga gaa tct cag tgg      624
Ala Asp Lys Thr Phe Gln Pro Glu Pro Gln Ile Gly Glu Ser Gln Trp
        195                 200                 205

tac gaa aca gaa att aat cat gca gct ggg aga gtc cta aaa aag act      672
Tyr Glu Thr Glu Ile Asn His Ala Ala Gly Arg Val Leu Lys Lys Thr
    210                 215                 220

acc cca atg aaa cca tgt tac ggt tca tat gca aaa ccc aca aat gaa      720

Thr Pro Met Lys Pro Cys Tyr Gly Ser Tyr Ala Lys Pro Thr Asn Glu
225                 230                 235                 240

aat gga ggg caa ggc att ctt gta aag caa caa aat gga aag cta gaa      768
Asn Gly Gly Gln Gly Ile Leu Val Lys Gln Gln Asn Gly Lys Leu Glu
                245                 250                 255

agt caa gtg gaa atg caa ttt ttc tca act act gag gca gcc gca ggc      816
Ser Gln Val Glu Met Gln Phe Phe Ser Thr Thr Glu Ala Ala Ala Gly
            260                 265                 270

aat ggt gat aac ttg act cct aaa gtg gta ttg tac agt gaa gat gta      864
Asn Gly Asp Asn Leu Thr Pro Lys Val Val Leu Tyr Ser Glu Asp Val
        275                 280                 285

gat ata gaa acc cca gac act cat att tct tac atg ccc act att aag      912
Asp Ile Glu Thr Pro Asp Thr His Ile Ser Tyr Met Pro Thr Ile Lys
    290                 295                 300

gaa ggt aac tca cga gaa cta atg ggc caa caa tct atg ccc aac agg      960
Glu Gly Asn Ser Arg Glu Leu Met Gly Gln Gln Ser Met Pro Asn Arg
305                 310                 315                 320

cct aat tac att gct ttt agg gac aat ttt att ggt cta atg tat tac     1008
Pro Asn Tyr Ile Ala Phe Arg Asp Asn Phe Ile Gly Leu Met Tyr Tyr
                325                 330                 335

aac agc acg ggt aat atg ggt gtt ctg gcg ggc caa gca tcg cag ttg     1056
Asn Ser Thr Gly Asn Met Gly Val Leu Ala Gly Gln Ala Ser Gln Leu
            340                 345                 350

aat gct gtt gta gat ttg caa gac aga aac aca gag ctt tca tac cag     1104
Asn Ala Val Val Asp Leu Gln Asp Arg Asn Thr Glu Leu Ser Tyr Gln
        355                 360                 365

ctt ttg ctt gat tcc att ggt gat aga acc agg tac ttt tct atg tgg     1152
Leu Leu Leu Asp Ser Ile Gly Asp Arg Thr Arg Tyr Phe Ser Met Trp
    370                 375                 380

aat cag ctt cca aat tac tgc ttt cca ctg gga ggt gtg att aat aca     1200
Asn Gln Leu Pro Asn Tyr Cys Phe Pro Leu Gly Gly Val Ile Asn Thr
385                 390                 395                 400

gag act ctt acc aag gta aaa cct aaa aca ggt cag gaa aat gga tgg     1248
Glu Thr Leu Thr Lys Val Lys Pro Lys Thr Gly Gln Glu Asn Gly Trp
                405                 410                 415

gaa aaa gat gct aca gaa ttt tca gat aaa aat gaa ata aga gtt gga     1296
Glu Lys Asp Ala Thr Glu Phe Ser Asp Lys Asn Glu Ile Arg Val Gly
            420                 425                 430

aat aat ttt gcc atg gaa atc aat cta aat gcc aac ctg tgg aga aat     1344
Asn Asn Phe Ala Met Glu Ile Asn Leu Asn Ala Asn Leu Trp Arg Asn
        435                 440                 445

ttc ctg tac tcc aac ata gcg ctg tat ttg ccc gac aag cta aag tac     1392
Phe Leu Tyr Ser Asn Ile Ala Leu Tyr Leu Pro Asp Lys Leu Lys Tyr
    450                 455                 460

agt cct tcc aac gta aaa att tct gat aac cca aac acc tac gac tac     1440
Ser Pro Ser Asn Val Lys Ile Ser Asp Asn Pro Asn Thr Tyr Asp Tyr
465                 470                 475                 480

atg aac aag cga gtg gtg gct ccc ggg cta gtg gac tgc tac att aac     1488
Met Asn Lys Arg Val Val Ala Pro Gly Leu Val Asp Cys Tyr Ile Asn
                485                 490                 495

ctt gga gca cgc tgg tcc ctt gac tat atg gac aac gtc aac cca ttt     1536
Leu Gly Ala Arg Trp Ser Leu Asp Tyr Met Asp Asn Val Asn Pro Phe
            500                 505                 510

aac cac cac cgc aat gct ggc ctg cgc tac cgc tca atg ttg ctg ggc     1584
Asn His His Arg Asn Ala Gly Leu Arg Tyr Arg Ser Met Leu Leu Gly
        515                 520                 525

aat ggt cgc tat gtg ccc ttc cac atc cag gtg cct cag aag ttc ttt     1632
Asn Gly Arg Tyr Val Pro Phe His Ile Gln Val Pro Gln Lys Phe Phe
    530                 535                 540

gcc att aaa aac ctc ctt ctc ctg ccg ggc tca tac acc tac gag tgg     1680
Ala Ile Lys Asn Leu Leu Leu Leu Pro Gly Ser Tyr Thr Tyr Glu Trp
545                 550                 555                 560

aac ttc agg aag gat gtt aac atg gtt ctg cag agc tcc cta gga aat     1728
Asn Phe Arg Lys Asp Val Asn Met Val Leu Gln Ser Ser Leu Gly Asn
                565                 570                 575

gac cta agg gtt gac gga gcc agc att aag ttt gat agc att tgc ctt     1776
Asp Leu Arg Val Asp Gly Ala Ser Ile Lys Phe Asp Ser Ile Cys Leu
            580                 585                 590

tac gcc acc ttc ttc ccc atg gcc cac aac acc gcc tcc acg ctt gag     1824
Tyr Ala Thr Phe Phe Pro Met Ala His Asn Thr Ala Ser Thr Leu Glu
        595                 600                 605

gcc atg ctt aga aac gac acc aac gac cag tcc ttt aac gac tat ctc     1872
Ala Met Leu Arg Asn Asp Thr Asn Asp Gln Ser Phe Asn Asp Tyr Leu
    610                 615                 620

tcc gcc gcc aac atg ctc tac cct ata ccc gcc aac gct acc aac gtg     1920
Ser Ala Ala Asn Met Leu Tyr Pro Ile Pro Ala Asn Ala Thr Asn Val
625                 630                 635                 640

ccc ata tcc atc ccc tcc cgc aac tgg gcg gct ttc cgc ggc tgg gcc     1968
Pro Ile Ser Ile Pro Ser Arg Asn Trp Ala Ala Phe Arg Gly Trp Ala
                645                 650                 655

ttc acg cgc ctt aag act aag gaa acc cca tca ctg ggc tcg ggc tac     2016
Phe Thr Arg Leu Lys Thr Lys Glu Thr Pro Ser Leu Gly Ser Gly Tyr
            660                 665                 670

gac cct tat tac acc tac tct ggc tct ata ccc tac cta gat gga acc     2064
Asp Pro Tyr Tyr Thr Tyr Ser Gly Ser Ile Pro Tyr Leu Asp Gly Thr
        675                 680                 685

ttt tac ctc aac cac acc ttt aag aag gtg gcc att acc ttt gac tct     2112
Phe Tyr Leu Asn His Thr Phe Lys Lys Val Ala Ile Thr Phe Asp Ser
    690                 695                 700

tct gtc agc tgg cct ggc aat gac cgc ctg ctt acc ccc aac gag ttt     2160
Ser Val Ser Trp Pro Gly Asn Asp Arg Leu Leu Thr Pro Asn Glu Phe
705                 710                 715                 720

gaa att aag cgc tca gtt gac ggg gag ggt tac aac gtt gcc cag tgt     2208
Glu Ile Lys Arg Ser Val Asp Gly Glu Gly Tyr Asn Val Ala Gln Cys
                725                 730                 735

aac atg acc aaa gac tgg ttc ctg gta caa atg cta gct aac tat aac     2256
Asn Met Thr Lys Asp Trp Phe Leu Val Gln Met Leu Ala Asn Tyr Asn
            740                 745                 750

att ggc tac cag ggc ttc tat atc cca gag agc tac aag gac cgc atg     2304
Ile Gly Tyr Gln Gly Phe Tyr Ile Pro Glu Ser Tyr Lys Asp Arg Met
        755                 760                 765

tac tcc ttc ttt aga aac ttc cag ccc atg agc cgt cag gtg gtg gat     2352
Tyr Ser Phe Phe Arg Asn Phe Gln Pro Met Ser Arg Gln Val Val Asp
    770                 775                 780

gat act aaa tac aag gac tac caa cag gtg ggc atc cta cac caa cac     2400
Asp Thr Lys Tyr Lys Asp Tyr Gln Gln Val Gly Ile Leu His Gln His
785                 790                 795                 800

aac aac tct gga ttt gtt ggc tac ctt gcc ccc acc atg cgc gaa gga     2448
Asn Asn Ser Gly Phe Val Gly Tyr Leu Ala Pro Thr Met Arg Glu Gly
                805                 810                 815

cag gcc tac cct gct aac ttc ccc tat ccg ctt ata ggc aag acc gca     2496
Gln Ala Tyr Pro Ala Asn Phe Pro Tyr Pro Leu Ile Gly Lys Thr Ala
            820                 825                 830

gtt gac agc att acc cag aaa aag ttt ctt tgc gat cgc acc ctt tgg     2544
Val Asp Ser Ile Thr Gln Lys Lys Phe Leu Cys Asp Arg Thr Leu Trp
        835                 840                 845

cgc atc cca ttc tcc agt aac ttt atg tcc atg ggc gca ctc aca gac     2592
Arg Ile Pro Phe Ser Ser Asn Phe Met Ser Met Gly Ala Leu Thr Asp
    850                 855                 860

ctg ggc caa aac ctt ctc tac gcc aac tcc gcc cac gcg cta gac atg     2640
Leu Gly Gln Asn Leu Leu Tyr Ala Asn Ser Ala His Ala Leu Asp Met
865                 870                 875                 880

act ttt gag gtg gat ccc atg gac gag ccc acc ctt ctt tat gtt ttg     2688
Thr Phe Glu Val Asp Pro Met Asp Glu Pro Thr Leu Leu Tyr Val Leu
                885                 890                 895

ttt gaa gtc ttt gac gtg gtc cgt gtg cac cag ccg cac cgc ggc gtc     2736
Phe Glu Val Phe Asp Val Val Arg Val His Gln Pro His Arg Gly Val
            900                 905                 910

atc gaa acc gtg tac ctg cgc acg ccc ttc tcg gcc ggc aac gcc aca     2784
Ile Glu Thr Val Tyr Leu Arg Thr Pro Phe Ser Ala Gly Asn Ala Thr
        915                 920                 925

aca taa                                                             2790
Thr

<210>  5
<211>  2808
<212>  DNA
<213>  Artificial Sequence

<220>
<223>  (QPGGAP)5-HVR1 modified Hexon protein

<220>
<221>  CDS
<222>  (1)..(2805)

<400>  5
atg gct acc cct tcg atg atg ccg cag tgg tct tac atg cac atc tcg      48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa cag gga cca gga gca cca cag      432
Ala Pro Asn Pro Cys Glu Trp Asp Glu Gln Gly Pro Gly Ala Pro Gln
    130                 135                 140

gga cca gga gca cca cag gga cca gga gca ccg caa ggt cct ggt gct      480
Gly Pro Gly Ala Pro Gln Gly Pro Gly Ala Pro Gln Gly Pro Gly Ala
145                 150                 155                 160

cct cag gga cca gga gca cca aaa act cac gta ttt ggg cag gcg cct      528
Pro Gln Gly Pro Gly Ala Pro Lys Thr His Val Phe Gly Gln Ala Pro
                165                 170                 175

tat tct ggt ata aat att aca aag gag ggt att caa ata ggt gtc gaa      576
Tyr Ser Gly Ile Asn Ile Thr Lys Glu Gly Ile Gln Ile Gly Val Glu
            180                 185                 190

ggt caa aca cct aaa tat gcc gat aaa aca ttt caa cct gaa cct caa      624
Gly Gln Thr Pro Lys Tyr Ala Asp Lys Thr Phe Gln Pro Glu Pro Gln
        195                 200                 205

ata gga gaa tct cag tgg tac gaa aca gaa att aat cat gca gct ggg      672
Ile Gly Glu Ser Gln Trp Tyr Glu Thr Glu Ile Asn His Ala Ala Gly
    210                 215                 220

aga gtc cta aaa aag act acc cca atg aaa cca tgt tac ggt tca tat      720

Arg Val Leu Lys Lys Thr Thr Pro Met Lys Pro Cys Tyr Gly Ser Tyr
225                 230                 235                 240

gca aaa ccc aca aat gaa aat gga ggg caa ggc att ctt gta aag caa     768
Ala Lys Pro Thr Asn Glu Asn Gly Gly Gln Gly Ile Leu Val Lys Gln
                245                 250                 255

caa aat gga aag cta gaa agt caa gtg gaa atg caa ttt ttc tca act     816
Gln Asn Gly Lys Leu Glu Ser Gln Val Glu Met Gln Phe Phe Ser Thr
            260                 265                 270

act gag gca gcc gca ggc aat ggt gat aac ttg act cct aaa gtg gta     864
Thr Glu Ala Ala Ala Gly Asn Gly Asp Asn Leu Thr Pro Lys Val Val
        275                 280                 285

ttg tac agt gaa gat gta gat ata gaa acc cca gac act cat att tct     912
Leu Tyr Ser Glu Asp Val Asp Ile Glu Thr Pro Asp Thr His Ile Ser
    290                 295                 300

tac atg ccc act att aag gaa ggt aac tca cga gaa cta atg ggc caa     960
Tyr Met Pro Thr Ile Lys Glu Gly Asn Ser Arg Glu Leu Met Gly Gln
305                 310                 315                 320

caa tct atg ccc aac agg cct aat tac att gct ttt agg gac aat ttt     1008
Gln Ser Met Pro Asn Arg Pro Asn Tyr Ile Ala Phe Arg Asp Asn Phe
                325                 330                 335

att ggt cta atg tat tac aac agc acg ggt aat atg ggt gtt ctg gcg     1056
Ile Gly Leu Met Tyr Tyr Asn Ser Thr Gly Asn Met Gly Val Leu Ala
            340                 345                 350

ggc caa gca tcg cag ttg aat gct gtt gta gat ttg caa gac aga aac     1104
Gly Gln Ala Ser Gln Leu Asn Ala Val Val Asp Leu Gln Asp Arg Asn
        355                 360                 365

aca gag ctt tca tac cag ctt ttg ctt gat tcc att ggt gat aga acc     1152
Thr Glu Leu Ser Tyr Gln Leu Leu Leu Asp Ser Ile Gly Asp Arg Thr
    370                 375                 380

agg tac ttt tct atg tgg aat cag ctt cca aat tac tgc ttt cca ctg     1200
Arg Tyr Phe Ser Met Trp Asn Gln Leu Pro Asn Tyr Cys Phe Pro Leu
385                 390                 395                 400

gga ggt gtg att aat aca gag act ctt acc aag gta aaa cct aaa aca     1248
Gly Gly Val Ile Asn Thr Glu Thr Leu Thr Lys Val Lys Pro Lys Thr
                405                 410                 415

ggt cag gaa aat gga tgg gaa aaa gat gct aca gaa ttt tca gat aaa     1296
Gly Gln Glu Asn Gly Trp Glu Lys Asp Ala Thr Glu Phe Ser Asp Lys
            420                 425                 430

aat gaa ata aga gtt gga aat aat ttt gcc atg gaa atc aat cta aat     1344
Asn Glu Ile Arg Val Gly Asn Asn Phe Ala Met Glu Ile Asn Leu Asn
        435                 440                 445

gcc aac ctg tgg aga aat ttc ctg tac tcc aac ata gcg ctg tat ttg     1392
Ala Asn Leu Trp Arg Asn Phe Leu Tyr Ser Asn Ile Ala Leu Tyr Leu
    450                 455                 460

ccc gac aag cta aag tac agt cct tcc aac gta aaa att tct gat aac     1440
Pro Asp Lys Leu Lys Tyr Ser Pro Ser Asn Val Lys Ile Ser Asp Asn
465                 470                 475                 480

cca aac acc tac gac tac atg aac aag cga gtg gtg gct ccc ggg cta     1488
Pro Asn Thr Tyr Asp Tyr Met Asn Lys Arg Val Val Ala Pro Gly Leu
                485                 490                 495

gtg gac tgc tac att aac ctt gga gca cgc tgg tcc ctt gac tat atg     1536
Val Asp Cys Tyr Ile Asn Leu Gly Ala Arg Trp Ser Leu Asp Tyr Met
            500                 505                 510

gac aac gtc aac cca ttt aac cac cac cgc aat gct ggc ctg cgc tac     1584
Asp Asn Val Asn Pro Phe Asn His His Arg Asn Ala Gly Leu Arg Tyr
        515                 520                 525

cgc tca atg ttg ctg ggc aat ggt cgc tat gtg ccc ttc cac atc cag     1632
Arg Ser Met Leu Leu Gly Asn Gly Arg Tyr Val Pro Phe His Ile Gln
    530                 535                 540

gtg cct cag aag ttc ttt gcc att aaa aac ctc ctt ctc ctg ccg ggc     1680
Val Pro Gln Lys Phe Phe Ala Ile Lys Asn Leu Leu Leu Leu Pro Gly
545                 550                 555                 560

tca tac acc tac gag tgg aac ttc agg aag gat gtt aac atg gtt ctg     1728
Ser Tyr Thr Tyr Glu Trp Asn Phe Arg Lys Asp Val Asn Met Val Leu
                565                 570                 575

cag agc tcc cta gga aat gac cta agg gtt gac gga gcc agc att aag     1776
Gln Ser Ser Leu Gly Asn Asp Leu Arg Val Asp Gly Ala Ser Ile Lys
            580                 585                 590

ttt gat agc att tgc ctt tac gcc acc ttc ttc ccc atg gcc cac aac     1824
Phe Asp Ser Ile Cys Leu Tyr Ala Thr Phe Phe Pro Met Ala His Asn
        595                 600                 605

acc gcc tcc acg ctt gag gcc atg ctt aga aac gac acc aac gac cag     1872
Thr Ala Ser Thr Leu Glu Ala Met Leu Arg Asn Asp Thr Asn Asp Gln
    610                 615                 620

tcc ttt aac gac tat ctc tcc gcc gcc aac atg ctc tac cct ata ccc     1920
Ser Phe Asn Asp Tyr Leu Ser Ala Ala Asn Met Leu Tyr Pro Ile Pro
625                 630                 635                 640

gcc aac gct acc aac gtg ccc ata tcc atc ccc tcc cgc aac tgg gcg     1968
Ala Asn Ala Thr Asn Val Pro Ile Ser Ile Pro Ser Arg Asn Trp Ala
                645                 650                 655

gct ttc cgc ggc tgg gcc ttc acg cgc ctt aag act aag gaa acc cca     2016
Ala Phe Arg Gly Trp Ala Phe Thr Arg Leu Lys Thr Lys Glu Thr Pro
            660                 665                 670

tca ctg ggc tcg ggc tac gac cct tat tac acc tac tct ggc tct ata     2064
Ser Leu Gly Ser Gly Tyr Asp Pro Tyr Tyr Thr Tyr Ser Gly Ser Ile
        675                 680                 685

ccc tac cta gat gga acc ttt tac ctc aac cac acc ttt aag aag gtg     2112
Pro Tyr Leu Asp Gly Thr Phe Tyr Leu Asn His Thr Phe Lys Lys Val
    690                 695                 700

gcc att acc ttt gac tct tct gtc agc tgg cct ggc aat gac cgc ctg     2160
Ala Ile Thr Phe Asp Ser Ser Val Ser Trp Pro Gly Asn Asp Arg Leu
705                 710                 715                 720

ctt acc ccc aac gag ttt gaa att aag cgc tca gtt gac ggg gag ggt     2208
Leu Thr Pro Asn Glu Phe Glu Ile Lys Arg Ser Val Asp Gly Glu Gly
                725                 730                 735

tac aac gtt gcc cag tgt aac atg acc aaa gac tgg ttc ctg gta caa 2256
Tyr Asn Val Ala Gln Cys Asn Met Thr Lys Asp Trp Phe Leu Val Gln
            740                 745                 750

atg cta gct aac tat aac att ggc tac cag ggc ttc tat atc cca gag 2304
Met Leu Ala Asn Tyr Asn Ile Gly Tyr Gln Gly Phe Tyr Ile Pro Glu
        755                 760                 765

agc tac aag gac cgc atg tac tcc ttc ttt aga aac ttc cag ccc atg 2352
Ser Tyr Lys Asp Arg Met Tyr Ser Phe Phe Arg Asn Phe Gln Pro Met
    770                 775                 780

agc cgt cag gtg gtg gat gat act aaa tac aag gac tac caa cag gtg 2400
Ser Arg Gln Val Val Asp Asp Thr Lys Tyr Lys Asp Tyr Gln Gln Val
785                 790                 795                 800

ggc atc cta cac caa cac aac aac tct gga ttt gtt ggc tac ctt gcc 2448
Gly Ile Leu His Gln His Asn Asn Ser Gly Phe Val Gly Tyr Leu Ala
                805                 810                 815

ccc acc atg cgc gaa gga cag gcc tac cct gct aac ttc ccc tat ccg 2496
Pro Thr Met Arg Glu Gly Gln Ala Tyr Pro Ala Asn Phe Pro Tyr Pro
            820                 825                 830

ctt ata ggc aag acc gca gtt gac agc att acc cag aaa aag ttt ctt 2544
Leu Ile Gly Lys Thr Ala Val Asp Ser Ile Thr Gln Lys Lys Phe Leu
        835                 840                 845

tgc gat cgc acc ctt tgg cgc atc cca ttc tcc agt aac ttt atg tcc 2592
Cys Asp Arg Thr Leu Trp Arg Ile Pro Phe Ser Ser Asn Phe Met Ser
    850                 855                 860

atg ggc gca ctc aca gac ctg ggc caa aac ctt ctc tac gcc aac tcc 2640
Met Gly Ala Leu Thr Asp Leu Gly Gln Asn Leu Leu Tyr Ala Asn Ser
865                 870                 875                 880

gcc cac gcg cta gac atg act ttt gag gtg gat ccc atg gac gag ccc 2688
Ala His Ala Leu Asp Met Thr Phe Glu Val Asp Pro Met Asp Glu Pro
                885                 890                 895

acc ctt ctt tat gtt ttg ttt gaa gtc ttt gac gtg gtc cgt gtg cac 2736
Thr Leu Leu Tyr Val Leu Phe Glu Val Phe Asp Val Val Arg Val His
            900                 905                 910

cag ccg cac cgc ggc gtc atc gaa acc gtg tac ctg cgc acg ccc ttc 2784
Gln Pro His Arg Gly Val Ile Glu Thr Val Tyr Leu Arg Thr Pro Phe
        915                 920                 925

tcg gcc ggc aac gcc aca aca taa 2808
Ser Ala Gly Asn Ala Thr Thr
    930                 935

<210> 6
<211> 2826
<212> DNA
<213> Artificial Sequence

<220>
<223> (QGPGAP)6-HVR1 modified Hexon protein

<220>
<221>  CDS
<222>  (1)..(2823)

<400>  6
atg gct acc cct tcg atg atg ccg cag tgg tct tac atg cac atc tcg      48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa cag gga cca gga gca cca cag      432
Ala Pro Asn Pro Cys Glu Trp Asp Glu Gln Gly Pro Gly Ala Pro Gln
    130                 135                 140

gga cca gga gca cca cag gga cca gga gca ccg caa ggt cct ggt gct      480
Gly Pro Gly Ala Pro Gln Gly Pro Gly Ala Pro Gln Gly Pro Gly Ala
145                 150                 155                 160

cct cag gga cca gga gca cca cag gga cca gga gca cca aaa act cac      528
Pro Gln Gly Pro Gly Ala Pro Gln Gly Pro Gly Ala Pro Lys Thr His
                165                 170                 175

gta ttt ggg cag gcg cct tat tct ggt ata aat att aca aag gag ggt      576
Val Phe Gly Gln Ala Pro Tyr Ser Gly Ile Asn Ile Thr Lys Glu Gly
            180                 185                 190

att caa ata ggt gtc gaa ggt caa aca cct aaa tat gcc gat aaa aca      624
Ile Gln Ile Gly Val Glu Gly Gln Thr Pro Lys Tyr Ala Asp Lys Thr
        195                 200                 205

ttt caa cct gaa cct caa ata gga gaa tct cag tgg tac gaa aca gaa      672
Phe Gln Pro Glu Pro Gln Ile Gly Glu Ser Gln Trp Tyr Glu Thr Glu
    210                 215                 220

att aat cat gca gct ggg aga gtc cta aaa aag act acc cca atg aaa 720
Ile Asn His Ala Ala Gly Arg Val Leu Lys Lys Thr Thr Pro Met Lys
225                 230                 235                 240

cca tgt tac ggt tca tat gca aaa ccc aca aat gaa aat gga ggg caa 768
Pro Cys Tyr Gly Ser Tyr Ala Lys Pro Thr Asn Glu Asn Gly Gly Gln
                245                 250                 255

ggc att ctt gta aag caa caa aat gga aag cta gaa agt caa gtg gaa 816
Gly Ile Leu Val Lys Gln Gln Asn Gly Lys Leu Glu Ser Gln Val Glu
            260                 265                 270

atg caa ttt ttc tca act act gag gca gcc gca ggc aat ggt gat aac 864
Met Gln Phe Phe Ser Thr Thr Glu Ala Ala Ala Gly Asn Gly Asp Asn
        275                 280                 285

ttg act cct aaa gtg gta ttg tac agt gaa gat gta gat ata gaa acc 912
Leu Thr Pro Lys Val Val Leu Tyr Ser Glu Asp Val Asp Ile Glu Thr
    290                 295                 300

cca gac act cat att tct tac atg ccc act att aag gaa ggt aac tca 960
Pro Asp Thr His Ile Ser Tyr Met Pro Thr Ile Lys Glu Gly Asn Ser
305                 310                 315                 320

cga gaa cta atg ggc caa caa tct atg ccc aac agg cct aat tac att 1008
Arg Glu Leu Met Gly Gln Gln Ser Met Pro Asn Arg Pro Asn Tyr Ile
                325                 330                 335

gct ttt agg gac aat ttt att ggt cta atg tat tac aac agc acg ggt 1056
Ala Phe Arg Asp Asn Phe Ile Gly Leu Met Tyr Tyr Asn Ser Thr Gly
            340                 345                 350

aat atg ggt gtt ctg gcg ggc caa gca tcg cag ttg aat gct gtt gta 1104
Asn Met Gly Val Leu Ala Gly Gln Ala Ser Gln Leu Asn Ala Val Val
        355                 360                 365

gat ttg caa gac aga aac aca gag ctt tca tac cag ctt ttg ctt gat 1152
Asp Leu Gln Asp Arg Asn Thr Glu Leu Ser Tyr Gln Leu Leu Leu Asp
    370                 375                 380

tcc att ggt gat aga acc agg tac ttt tct atg tgg aat cag ctt cca 1200
Ser Ile Gly Asp Arg Thr Arg Tyr Phe Ser Met Trp Asn Gln Leu Pro
385                 390                 395                 400

aat tac tgc ttt cca ctg gga ggt gtg att aat aca gag act ctt acc 1248
Asn Tyr Cys Phe Pro Leu Gly Gly Val Ile Asn Thr Glu Thr Leu Thr
                405                 410                 415

aag gta aaa cct aaa aca ggt cag gaa aat gga tgg gaa aaa gat gct 1296
Lys Val Lys Pro Lys Thr Gly Gln Glu Asn Gly Trp Glu Lys Asp Ala
            420                 425                 430

aca gaa ttt tca gat aaa aat gaa ata aga gtt gga aat aat ttt gcc 1344
Thr Glu Phe Ser Asp Lys Asn Glu Ile Arg Val Gly Asn Asn Phe Ala
        435                 440                 445

atg gaa atc aat cta aat gcc aac ctg tgg aga aat ttc ctg tac tcc 1392
Met Glu Ile Asn Leu Asn Ala Asn Leu Trp Arg Asn Phe Leu Tyr Ser
    450                 455                 460

aac ata gcg ctg tat ttg ccc gac aag cta aag tac agt cct tcc aac 1440
Asn Ile Ala Leu Tyr Leu Pro Asp Lys Leu Lys Tyr Ser Pro Ser Asn
465                 470                 475                 480

gta aaa att tct gat aac cca aac acc tac gac tac atg aac aag cga     1488
Val Lys Ile Ser Asp Asn Pro Asn Thr Tyr Asp Tyr Met Asn Lys Arg
                485                 490                 495

gtg gtg gct ccc ggg cta gtg gac tgc tac att aac ctt gga gca cgc     1536
Val Val Ala Pro Gly Leu Val Asp Cys Tyr Ile Asn Leu Gly Ala Arg
            500                 505                 510

tgg tcc ctt gac tat atg gac aac gtc aac cca ttt aac cac cac cgc     1584
Trp Ser Leu Asp Tyr Met Asp Asn Val Asn Pro Phe Asn His His Arg
        515                 520                 525

aat gct ggc ctg cgc tac cgc tca atg ttg ctg ggc aat ggt cgc tat     1632
Asn Ala Gly Leu Arg Tyr Arg Ser Met Leu Leu Gly Asn Gly Arg Tyr
    530                 535                 540

gtg ccc ttc cac atc cag gtg cct cag aag ttc ttt gcc att aaa aac     1680
Val Pro Phe His Ile Gln Val Pro Gln Lys Phe Phe Ala Ile Lys Asn
545                 550                 555                 560

ctc ctt ctc ctg ccg ggc tca tac acc tac gag tgg aac ttc agg aag     1728
Leu Leu Leu Leu Pro Gly Ser Tyr Thr Tyr Glu Trp Asn Phe Arg Lys
                565                 570                 575

gat gtt aac atg gtt ctg cag agc tcc cta gga aat gac cta agg gtt     1776
Asp Val Asn Met Val Leu Gln Ser Ser Leu Gly Asn Asp Leu Arg Val
            580                 585                 590

gac gga gcc agc att aag ttt gat agc att tgc ctt tac gcc acc ttc     1824
Asp Gly Ala Ser Ile Lys Phe Asp Ser Ile Cys Leu Tyr Ala Thr Phe
        595                 600                 605

ttc ccc atg gcc cac aac acc gcc tcc acg ctt gag gcc atg ctt aga     1872
Phe Pro Met Ala His Asn Thr Ala Ser Thr Leu Glu Ala Met Leu Arg
    610                 615                 620

aac gac acc aac gac cag tcc ttt aac gac tat ctc tcc gcc gcc aac     1920
Asn Asp Thr Asn Asp Gln Ser Phe Asn Asp Tyr Leu Ser Ala Ala Asn
625                 630                 635                 640

atg ctc tac cct ata ccc gcc aac gct acc aac gtg ccc ata tcc atc     1968
Met Leu Tyr Pro Ile Pro Ala Asn Ala Thr Asn Val Pro Ile Ser Ile
                645                 650                 655

ccc tcc cgc aac tgg gcg gct ttc cgc ggc tgg gcc ttc acg cgc ctt     2016
Pro Ser Arg Asn Trp Ala Ala Phe Arg Gly Trp Ala Phe Thr Arg Leu
            660                 665                 670

aag act aag gaa acc cca tca ctg ggc tcg ggc tac gac cct tat tac     2064
Lys Thr Lys Glu Thr Pro Ser Leu Gly Ser Gly Tyr Asp Pro Tyr Tyr
        675                 680                 685

acc tac tct ggc tct ata ccc tac cta gat gga acc ttt tac ctc aac     2112
Thr Tyr Ser Gly Ser Ile Pro Tyr Leu Asp Gly Thr Phe Tyr Leu Asn
    690                 695                 700

cac acc ttt aag aag gtg gcc att acc ttt gac tct tct gtc agc tgg     2160
His Thr Phe Lys Lys Val Ala Ile Thr Phe Asp Ser Ser Val Ser Trp
705                 710                 715                 720

cct ggc aat gac cgc ctg ctt acc ccc aac gag ttt gaa att aag cgc     2208
Pro Gly Asn Asp Arg Leu Leu Thr Pro Asn Glu Phe Glu Ile Lys Arg

725                 730                 735

tca gtt gac ggg gag ggt tac aac gtt gcc cag tgt aac atg acc aaa      2256
Ser Val Asp Gly Glu Gly Tyr Asn Val Ala Gln Cys Asn Met Thr Lys
            740                 745                 750

gac tgg ttc ctg gta caa atg cta gct aac tat aac att ggc tac cag      2304
Asp Trp Phe Leu Val Gln Met Leu Ala Asn Tyr Asn Ile Gly Tyr Gln
        755                 760                 765

ggc ttc tat atc cca gag agc tac aag gac cgc atg tac tcc ttc ttt      2352
Gly Phe Tyr Ile Pro Glu Ser Tyr Lys Asp Arg Met Tyr Ser Phe Phe
    770                 775                 780

aga aac ttc cag ccc atg agc cgt cag gtg gtg gat gat act aaa tac      2400
Arg Asn Phe Gln Pro Met Ser Arg Gln Val Val Asp Asp Thr Lys Tyr
785                 790                 795                 800

aag gac tac caa cag gtg ggc atc cta cac caa cac aac aac tct gga      2448
Lys Asp Tyr Gln Gln Val Gly Ile Leu His Gln His Asn Asn Ser Gly
                805                 810                 815

ttt gtt ggc tac ctt gcc ccc acc atg cgc gaa gga cag gcc tac cct      2496
Phe Val Gly Tyr Leu Ala Pro Thr Met Arg Glu Gly Gln Ala Tyr Pro
            820                 825                 830

gct aac ttc ccc tat ccg ctt ata ggc aag acc gca gtt gac agc att      2544
Ala Asn Phe Pro Tyr Pro Leu Ile Gly Lys Thr Ala Val Asp Ser Ile
        835                 840                 845

acc cag aaa aag ttt ctt tgc gat cgc acc ctt tgg cgc atc cca ttc      2592
Thr Gln Lys Lys Phe Leu Cys Asp Arg Thr Leu Trp Arg Ile Pro Phe
    850                 855                 860

tcc agt aac ttt atg tcc atg ggc gca ctc aca gac ctg ggc caa aac      2640
Ser Ser Asn Phe Met Ser Met Gly Ala Leu Thr Asp Leu Gly Gln Asn
865                 870                 875                 880

ctt ctc tac gcc aac tcc gcc cac gcg cta gac atg act ttt gag gtg      2688
Leu Leu Tyr Ala Asn Ser Ala His Ala Leu Asp Met Thr Phe Glu Val
                885                 890                 895

gat ccc atg gac gag ccc acc ctt ctt tat gtt ttg ttt gaa gtc ttt      2736
Asp Pro Met Asp Glu Pro Thr Leu Leu Tyr Val Leu Phe Glu Val Phe
            900                 905                 910

gac gtg gtc cgt gtg cac cag ccg cac cgc ggc gtc atc gaa acc gtg      2784
Asp Val Val Arg Val His Gln Pro His Arg Gly Val Ile Glu Thr Val
        915                 920                 925

tac ctg cgc acg ccc ttc tcg gcc ggc aac gcc aca aca taa              2826
Tyr Leu Arg Thr Pro Phe Ser Ala Gly Asn Ala Thr Thr
    930                 935                 940

<210>  7
<211>  2844
<212>  DNA
<213>  Artificial Sequence

<220>

<223>  (QGPGAP)7-HVR1 modified Hexon protein

<220>
<221>  CDS
<222>  (1)..(2841)

<400>  7
atg gct acc cct tcg atg atg ccg cag tgg tct tac atg cac atc tcg      48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa cag gga cca gga gca cca cag      432
Ala Pro Asn Pro Cys Glu Trp Asp Glu Gln Gly Pro Gly Ala Pro Gln
    130                 135                 140

gga cca gga gca cca cag gga cca gga gca cca cag gga cca gga gca      480
Gly Pro Gly Ala Pro Gln Gly Pro Gly Ala Pro Gln Gly Pro Gly Ala
145                 150                 155                 160

cca cag gga cca gga gca cca cag gga cca gga gca ccg caa ggt cct      528
Pro Gln Gly Pro Gly Ala Pro Gln Gly Pro Gly Ala Pro Gln Gly Pro
                165                 170                 175

ggt gct cct aaa act cac gta ttt ggg cag gcg cct tat tct ggt ata      576
Gly Ala Pro Lys Thr His Val Phe Gly Gln Ala Pro Tyr Ser Gly Ile
            180                 185                 190

aat att aca aag gag ggt att caa ata ggt gtc gaa ggt caa aca cct      624
Asn Ile Thr Lys Glu Gly Ile Gln Ile Gly Val Glu Gly Gln Thr Pro
        195                 200                 205

aaa tat gcc gat aaa aca ttt caa cct gaa cct caa ata gga gaa tct      672
Lys Tyr Ala Asp Lys Thr Phe Gln Pro Glu Pro Gln Ile Gly Glu Ser
    210                 215                 220

cag tgg tac gaa aca gaa att aat cat gca gct ggg aga gtc cta aaa 720
Gln Trp Tyr Glu Thr Glu Ile Asn His Ala Ala Gly Arg Val Leu Lys
225                 230                 235                 240

aag act acc cca atg aaa cca tgt tac ggt tca tat gca aaa ccc aca 768
Lys Thr Thr Pro Met Lys Pro Cys Tyr Gly Ser Tyr Ala Lys Pro Thr
                245                 250                 255

aat gaa aat gga ggg caa ggc att ctt gta aag caa caa aat gga aag 816
Asn Glu Asn Gly Gly Gln Gly Ile Leu Val Lys Gln Gln Asn Gly Lys
            260                 265                 270

cta gaa agt caa gtg gaa atg caa ttt ttc tca act act gag gca gcc 864
Leu Glu Ser Gln Val Glu Met Gln Phe Phe Ser Thr Thr Glu Ala Ala
        275                 280                 285

gca ggc aat ggt gat aac ttg act cct aaa gtg gta ttg tac agt gaa 912
Ala Gly Asn Gly Asp Asn Leu Thr Pro Lys Val Val Leu Tyr Ser Glu
    290                 295                 300

gat gta gat ata gaa acc cca gac act cat att tct tac atg ccc act 960
Asp Val Asp Ile Glu Thr Pro Asp Thr His Ile Ser Tyr Met Pro Thr
305                 310                 315                 320

att aag gaa ggt aac tca cga gaa cta atg ggc caa caa tct atg ccc 1008
Ile Lys Glu Gly Asn Ser Arg Glu Leu Met Gly Gln Gln Ser Met Pro
                325                 330                 335

aac agg cct aat tac att gct ttt agg gac aat ttt att ggt cta atg 1056
Asn Arg Pro Asn Tyr Ile Ala Phe Arg Asp Asn Phe Ile Gly Leu Met
            340                 345                 350

tat tac aac agc acg ggt aat atg ggt gtt ctg gcg ggc caa gca tcg 1104
Tyr Tyr Asn Ser Thr Gly Asn Met Gly Val Leu Ala Gly Gln Ala Ser
        355                 360                 365

cag ttg aat gct gtt gta gat ttg caa gac aga aac aca gag ctt tca 1152
Gln Leu Asn Ala Val Val Asp Leu Gln Asp Arg Asn Thr Glu Leu Ser
    370                 375                 380

tac cag ctt ttg ctt gat tcc att ggt gat aga acc agg tac ttt tct 1200
Tyr Gln Leu Leu Leu Asp Ser Ile Gly Asp Arg Thr Arg Tyr Phe Ser
385                 390                 395                 400

atg tgg aat cag ctt cca aat tac tgc ttt cca ctg gga ggt gtg att 1248
Met Trp Asn Gln Leu Pro Asn Tyr Cys Phe Pro Leu Gly Gly Val Ile
                405                 410                 415

aat aca gag act ctt acc aag gta aaa cct aaa aca ggt cag gaa aat 1296
Asn Thr Glu Thr Leu Thr Lys Val Lys Pro Lys Thr Gly Gln Glu Asn
            420                 425                 430

gga tgg gaa aaa gat gct aca gaa ttt tca gat aaa aat gaa ata aga 1344
Gly Trp Glu Lys Asp Ala Thr Glu Phe Ser Asp Lys Asn Glu Ile Arg
        435                 440                 445

gtt gga aat aat ttt gcc atg gaa atc aat cta aat gcc aac ctg tgg 1392
Val Gly Asn Asn Phe Ala Met Glu Ile Asn Leu Asn Ala Asn Leu Trp
    450                 455                 460

aga aat ttc ctg tac tcc aac ata gcg ctg tat ttg ccc gac aag cta 1440
Arg Asn Phe Leu Tyr Ser Asn Ile Ala Leu Tyr Leu Pro Asp Lys Leu

465                 470                 475                 480

aag tac agt cct tcc aac gta aaa att tct gat aac cca aac acc tac      1488
Lys Tyr Ser Pro Ser Asn Val Lys Ile Ser Asp Asn Pro Asn Thr Tyr
                485                 490                 495

gac tac atg aac aag cga gtg gtg gct ccc ggg cta gtg gac tgc tac      1536
Asp Tyr Met Asn Lys Arg Val Val Ala Pro Gly Leu Val Asp Cys Tyr
            500                 505                 510

att aac ctt gga gca cgc tgg tcc ctt gac tat atg gac aac gtc aac      1584
Ile Asn Leu Gly Ala Arg Trp Ser Leu Asp Tyr Met Asp Asn Val Asn
        515                 520                 525

cca ttt aac cac cac cgc aat gct ggc ctg cgc tac cgc tca atg ttg      1632
Pro Phe Asn His His Arg Asn Ala Gly Leu Arg Tyr Arg Ser Met Leu
    530                 535                 540

ctg ggc aat ggt cgc tat gtg ccc ttc cac atc cag gtg cct cag aag      1680
Leu Gly Asn Gly Arg Tyr Val Pro Phe His Ile Gln Val Pro Gln Lys
545                 550                 555                 560

ttc ttt gcc att aaa aac ctc ctt ctc ctg ccg ggc tca tac acc tac      1728
Phe Phe Ala Ile Lys Asn Leu Leu Leu Leu Pro Gly Ser Tyr Thr Tyr
                565                 570                 575

gag tgg aac ttc agg aag gat gtt aac atg gtt ctg cag agc tcc cta      1776
Glu Trp Asn Phe Arg Lys Asp Val Asn Met Val Leu Gln Ser Ser Leu
            580                 585                 590

gga aat gac cta agg gtt gac gga gcc agc att aag ttt gat agc att      1824
Gly Asn Asp Leu Arg Val Asp Gly Ala Ser Ile Lys Phe Asp Ser Ile
        595                 600                 605

tgc ctt tac gcc acc ttc ttc ccc atg gcc cac aac acc gcc tcc acg      1872
Cys Leu Tyr Ala Thr Phe Phe Pro Met Ala His Asn Thr Ala Ser Thr
    610                 615                 620

ctt gag gcc atg ctt aga aac gac acc aac gac cag tcc ttt aac gac      1920
Leu Glu Ala Met Leu Arg Asn Asp Thr Asn Asp Gln Ser Phe Asn Asp
625                 630                 635                 640

tat ctc tcc gcc gcc aac atg ctc tac cct ata ccc gcc aac gct acc      1968
Tyr Leu Ser Ala Ala Asn Met Leu Tyr Pro Ile Pro Ala Asn Ala Thr
                645                 650                 655

aac gtg ccc ata tcc atc ccc tcc cgc aac tgg gcg gct ttc cgc ggc      2016
Asn Val Pro Ile Ser Ile Pro Ser Arg Asn Trp Ala Ala Phe Arg Gly
            660                 665                 670

tgg gcc ttc acg cgc ctt aag act aag gaa acc cca tca ctg ggc tcg      2064
Trp Ala Phe Thr Arg Leu Lys Thr Lys Glu Thr Pro Ser Leu Gly Ser
        675                 680                 685

ggc tac gac cct tat tac acc tac tct ggc tct ata ccc tac cta gat      2112
Gly Tyr Asp Pro Tyr Tyr Thr Tyr Ser Gly Ser Ile Pro Tyr Leu Asp
    690                 695                 700

gga acc ttt tac ctc aac cac acc ttt aag aag gtg gcc att acc ttt      2160
Gly Thr Phe Tyr Leu Asn His Thr Phe Lys Lys Val Ala Ile Thr Phe
705                 710                 715                 720

gac tct tct gtc agc tgg cct ggc aat gac cgc ctg ctt acc ccc aac      2208

Asp Ser Ser Val Ser Trp Pro Gly Asn Asp Arg Leu Leu Thr Pro Asn
                725                 730                 735

gag ttt gaa att aag cgc tca gtt gac ggg gag ggt tac aac gtt gcc 2256
Glu Phe Glu Ile Lys Arg Ser Val Asp Gly Glu Gly Tyr Asn Val Ala
            740                 745                 750

cag tgt aac atg acc aaa gac tgg ttc ctg gta caa atg cta gct aac 2304
Gln Cys Asn Met Thr Lys Asp Trp Phe Leu Val Gln Met Leu Ala Asn
        755                 760                 765

tat aac att ggc tac cag ggc ttc tat atc cca gag agc tac aag gac 2352
Tyr Asn Ile Gly Tyr Gln Gly Phe Tyr Ile Pro Glu Ser Tyr Lys Asp
    770                 775                 780

cgc atg tac tcc ttc ttt aga aac ttc cag ccc atg agc cgt cag gtg 2400
Arg Met Tyr Ser Phe Phe Arg Asn Phe Gln Pro Met Ser Arg Gln Val
785                 790                 795                 800

gtg gat gat act aaa tac aag gac tac caa cag gtg ggc atc cta cac 2448
Val Asp Asp Thr Lys Tyr Lys Asp Tyr Gln Gln Val Gly Ile Leu His
                805                 810                 815

caa cac aac aac tct gga ttt gtt ggc tac ctt gcc ccc acc atg cgc 2496
Gln His Asn Asn Ser Gly Phe Val Gly Tyr Leu Ala Pro Thr Met Arg
            820                 825                 830

gaa gga cag gcc tac cct gct aac ttc ccc tat ccg ctt ata ggc aag 2544
Glu Gly Gln Ala Tyr Pro Ala Asn Phe Pro Tyr Pro Leu Ile Gly Lys
        835                 840                 845

acc gca gtt gac agc att acc cag aaa aag ttt ctt tgc gat cgc acc 2592
Thr Ala Val Asp Ser Ile Thr Gln Lys Lys Phe Leu Cys Asp Arg Thr
    850                 855                 860

ctt tgg cgc atc cca ttc tcc agt aac ttt atg tcc atg ggc gca ctc 2640
Leu Trp Arg Ile Pro Phe Ser Ser Asn Phe Met Ser Met Gly Ala Leu
865                 870                 875                 880

aca gac ctg ggc caa aac ctt ctc tac gcc aac tcc gcc cac gcg cta 2688
Thr Asp Leu Gly Gln Asn Leu Leu Tyr Ala Asn Ser Ala His Ala Leu
                885                 890                 895

gac atg act ttt gag gtg gat ccc atg gac gag ccc acc ctt ctt tat 2736
Asp Met Thr Phe Glu Val Asp Pro Met Asp Glu Pro Thr Leu Leu Tyr
            900                 905                 910

gtt ttg ttt gaa gtc ttt gac gtg gtc cgt gtg cac cag ccg cac cgc 2784
Val Leu Phe Glu Val Phe Asp Val Val Arg Val His Gln Pro His Arg
        915                 920                 925

ggc gtc atc gaa acc gtg tac ctg cgc acg ccc ttc tcg gcc ggc aac 2832
Gly Val Ile Glu Thr Val Tyr Leu Arg Thr Pro Phe Ser Ala Gly Asn
    930                 935                 940

gcc aca aca taa 2844
Ala Thr Thr
945

<210>  8
<211>  2862
<212>  DNA
<213>  Artificial Sequence

<220>
<223>  (QGPGAP)8-HVR1 modified Hexon protein


<220>
<221>  CDS
<222>  (1)..(2859)

<400>  8
atg gct acc cct tcg atg atg ccg cag tgg tct tac atg cac atc tcg      48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa cag gga cca gga gca cca cag      432
Ala Pro Asn Pro Cys Glu Trp Asp Glu Gln Gly Pro Gly Ala Pro Gln
    130                 135                 140

gga cca gga gca cca cag gga cca gga gca cca cag gga cca gga gca      480
Gly Pro Gly Ala Pro Gln Gly Pro Gly Ala Pro Gln Gly Pro Gly Ala
145                 150                 155                 160

cca cag gga cca gga gca cca cag gga cca gga gca ccg caa ggt cct      528
Pro Gln Gly Pro Gly Ala Pro Gln Gly Pro Gly Ala Pro Gln Gly Pro
                165                 170                 175

ggt gct cct cag gga cca gga gca cca aaa act cac gta ttt ggg cag      576
Gly Ala Pro Gln Gly Pro Gly Ala Pro Lys Thr His Val Phe Gly Gln
            180                 185                 190

gcg cct tat tct ggt ata aat att aca aag gag ggt att caa ata ggt      624

Ala Pro Tyr Ser Gly Ile Asn Ile Thr Lys Glu Gly Ile Gln Ile Gly
        195                 200                 205

gtc gaa ggt caa aca cct aaa tat gcc gat aaa aca ttt caa cct gaa      672
Val Glu Gly Gln Thr Pro Lys Tyr Ala Asp Lys Thr Phe Gln Pro Glu
    210                 215                 220

cct caa ata gga gaa tct cag tgg tac gaa aca gaa att aat cat gca      720
Pro Gln Ile Gly Glu Ser Gln Trp Tyr Glu Thr Glu Ile Asn His Ala
225                 230                 235                 240

gct ggg aga gtc cta aaa aag act acc cca atg aaa cca tgt tac ggt      768
Ala Gly Arg Val Leu Lys Lys Thr Thr Pro Met Lys Pro Cys Tyr Gly
                245                 250                 255

tca tat gca aaa ccc aca aat gaa aat gga ggg caa ggc att ctt gta      816
Ser Tyr Ala Lys Pro Thr Asn Glu Asn Gly Gly Gln Gly Ile Leu Val
            260                 265                 270

aag caa caa aat gga aag cta gaa agt caa gtg gaa atg caa ttt ttc      864
Lys Gln Gln Asn Gly Lys Leu Glu Ser Gln Val Glu Met Gln Phe Phe
        275                 280                 285

tca act act gag gca gcc gca ggc aat ggt gat aac ttg act cct aaa      912
Ser Thr Thr Glu Ala Ala Ala Gly Asn Gly Asp Asn Leu Thr Pro Lys
    290                 295                 300

gtg gta ttg tac agt gaa gat gta gat ata gaa acc cca gac act cat      960
Val Val Leu Tyr Ser Glu Asp Val Asp Ile Glu Thr Pro Asp Thr His
305                 310                 315                 320

att tct tac atg ccc act att aag gaa ggt aac tca cga gaa cta atg     1008
Ile Ser Tyr Met Pro Thr Ile Lys Glu Gly Asn Ser Arg Glu Leu Met
                325                 330                 335

ggc caa caa tct atg ccc aac agg cct aat tac att gct ttt agg gac     1056
Gly Gln Gln Ser Met Pro Asn Arg Pro Asn Tyr Ile Ala Phe Arg Asp
            340                 345                 350

aat ttt att ggt cta atg tat tac aac agc acg ggt aat atg ggt gtt     1104
Asn Phe Ile Gly Leu Met Tyr Tyr Asn Ser Thr Gly Asn Met Gly Val
        355                 360                 365

ctg gcg ggc caa gca tcg cag ttg aat gct gtt gta gat ttg caa gac     1152
Leu Ala Gly Gln Ala Ser Gln Leu Asn Ala Val Val Asp Leu Gln Asp
    370                 375                 380

aga aac aca gag ctt tca tac cag ctt ttg ctt gat tcc att ggt gat     1200
Arg Asn Thr Glu Leu Ser Tyr Gln Leu Leu Leu Asp Ser Ile Gly Asp
385                 390                 395                 400

aga acc agg tac ttt tct atg tgg aat cag ctt cca aat tac tgc ttt     1248
Arg Thr Arg Tyr Phe Ser Met Trp Asn Gln Leu Pro Asn Tyr Cys Phe
                405                 410                 415

cca ctg gga ggt gtg att aat aca gag act ctt acc aag gta aaa cct     1296
Pro Leu Gly Gly Val Ile Asn Thr Glu Thr Leu Thr Lys Val Lys Pro
            420                 425                 430

aaa aca ggt cag gaa aat gga tgg gaa aaa gat gct aca gaa ttt tca     1344
Lys Thr Gly Gln Glu Asn Gly Trp Glu Lys Asp Ala Thr Glu Phe Ser
        435                 440                 445

gat aaa aat gaa ata aga gtt gga aat aat ttt gcc atg gaa atc aat     1392
Asp Lys Asn Glu Ile Arg Val Gly Asn Asn Phe Ala Met Glu Ile Asn
    450                 455                 460

cta aat gcc aac ctg tgg aga aat ttc ctg tac tcc aac ata gcg ctg     1440
Leu Asn Ala Asn Leu Trp Arg Asn Phe Leu Tyr Ser Asn Ile Ala Leu
465                 470                 475                 480

tat ttg ccc gac aag cta aag tac agt cct tcc aac gta aaa att tct     1488
Tyr Leu Pro Asp Lys Leu Lys Tyr Ser Pro Ser Asn Val Lys Ile Ser
                485                 490                 495

gat aac cca aac acc tac gac tac atg aac aag cga gtg gtg gct ccc     1536
Asp Asn Pro Asn Thr Tyr Asp Tyr Met Asn Lys Arg Val Val Ala Pro
            500                 505                 510

ggg cta gtg gac tgc tac att aac ctt gga gca cgc tgg tcc ctt gac     1584
Gly Leu Val Asp Cys Tyr Ile Asn Leu Gly Ala Arg Trp Ser Leu Asp
        515                 520                 525

tat atg gac aac gtc aac cca ttt aac cac cac cgc aat gct ggc ctg     1632
Tyr Met Asp Asn Val Asn Pro Phe Asn His His Arg Asn Ala Gly Leu
    530                 535                 540

cgc tac cgc tca atg ttg ctg ggc aat ggt cgc tat gtg ccc ttc cac     1680
Arg Tyr Arg Ser Met Leu Leu Gly Asn Gly Arg Tyr Val Pro Phe His
545                 550                 555                 560

atc cag gtg cct cag aag ttc ttt gcc att aaa aac ctc ctt ctc ctg     1728
Ile Gln Val Pro Gln Lys Phe Phe Ala Ile Lys Asn Leu Leu Leu Leu
                565                 570                 575

ccg ggc tca tac acc tac gag tgg aac ttc agg aag gat gtt aac atg     1776
Pro Gly Ser Tyr Thr Tyr Glu Trp Asn Phe Arg Lys Asp Val Asn Met
            580                 585                 590

gtt ctg cag agc tcc cta gga aat gac cta agg gtt gac gga gcc agc     1824
Val Leu Gln Ser Ser Leu Gly Asn Asp Leu Arg Val Asp Gly Ala Ser
        595                 600                 605

att aag ttt gat agc att tgc ctt tac gcc acc ttc ttc ccc atg gcc     1872
Ile Lys Phe Asp Ser Ile Cys Leu Tyr Ala Thr Phe Phe Pro Met Ala
    610                 615                 620

cac aac acc gcc tcc acg ctt gag gcc atg ctt aga aac gac acc aac     1920
His Asn Thr Ala Ser Thr Leu Glu Ala Met Leu Arg Asn Asp Thr Asn
625                 630                 635                 640

gac cag tcc ttt aac gac tat ctc tcc gcc gcc aac atg ctc tac cct     1968
Asp Gln Ser Phe Asn Asp Tyr Leu Ser Ala Ala Asn Met Leu Tyr Pro
                645                 650                 655

ata ccc gcc aac gct acc aac gtg ccc ata tcc atc ccc tcc cgc aac     2016
Ile Pro Ala Asn Ala Thr Asn Val Pro Ile Ser Ile Pro Ser Arg Asn
            660                 665                 670

tgg gcg gct ttc cgc ggc tgg gcc ttc acg cgc ctt aag act aag gaa     2064
Trp Ala Ala Phe Arg Gly Trp Ala Phe Thr Arg Leu Lys Thr Lys Glu
        675                 680                 685

acc cca tca ctg ggc tcg ggc tac gac cct tat tac acc tac tct ggc     2112
Thr Pro Ser Leu Gly Ser Gly Tyr Asp Pro Tyr Tyr Thr Tyr Ser Gly
    690                 695                 700

tct ata ccc tac cta gat gga acc ttt tac ctc aac cac acc ttt aag 2160
Ser Ile Pro Tyr Leu Asp Gly Thr Phe Tyr Leu Asn His Thr Phe Lys
705                 710                 715                 720

aag gtg gcc att acc ttt gac tct tct gtc agc tgg cct ggc aat gac 2208
Lys Val Ala Ile Thr Phe Asp Ser Ser Val Ser Trp Pro Gly Asn Asp
                725                 730                 735

cgc ctg ctt acc ccc aac gag ttt gaa att aag cgc tca gtt gac ggg 2256
Arg Leu Leu Thr Pro Asn Glu Phe Glu Ile Lys Arg Ser Val Asp Gly
            740                 745                 750

gag ggt tac aac gtt gcc cag tgt aac atg acc aaa gac tgg ttc ctg 2304
Glu Gly Tyr Asn Val Ala Gln Cys Asn Met Thr Lys Asp Trp Phe Leu
        755                 760                 765

gta caa atg cta gct aac tat aac att ggc tac cag ggc ttc tat atc 2352
Val Gln Met Leu Ala Asn Tyr Asn Ile Gly Tyr Gln Gly Phe Tyr Ile
    770                 775                 780

cca gag agc tac aag gac cgc atg tac tcc ttc ttt aga aac ttc cag 2400
Pro Glu Ser Tyr Lys Asp Arg Met Tyr Ser Phe Phe Arg Asn Phe Gln
785                 790                 795                 800

ccc atg agc cgt cag gtg gtg gat gat act aaa tac aag gac tac caa 2448
Pro Met Ser Arg Gln Val Val Asp Asp Thr Lys Tyr Lys Asp Tyr Gln
                805                 810                 815

cag gtg ggc atc cta cac caa cac aac aac tct gga ttt gtt ggc tac 2496
Gln Val Gly Ile Leu His Gln His Asn Asn Ser Gly Phe Val Gly Tyr
            820                 825                 830

ctt gcc ccc acc atg cgc gaa gga cag gcc tac cct gct aac ttc ccc 2544
Leu Ala Pro Thr Met Arg Glu Gly Gln Ala Tyr Pro Ala Asn Phe Pro
        835                 840                 845

tat ccg ctt ata ggc aag acc gca gtt gac agc att acc cag aaa aag 2592
Tyr Pro Leu Ile Gly Lys Thr Ala Val Asp Ser Ile Thr Gln Lys Lys
    850                 855                 860

ttt ctt tgc gat cgc acc ctt tgg cgc atc cca ttc tcc agt aac ttt 2640
Phe Leu Cys Asp Arg Thr Leu Trp Arg Ile Pro Phe Ser Ser Asn Phe
865                 870                 875                 880

atg tcc atg ggc gca ctc aca gac ctg ggc caa aac ctt ctc tac gcc 2688
Met Ser Met Gly Ala Leu Thr Asp Leu Gly Gln Asn Leu Leu Tyr Ala
                885                 890                 895

aac tcc gcc cac gcg cta gac atg act ttt gag gtg gat ccc atg gac 2736
Asn Ser Ala His Ala Leu Asp Met Thr Phe Glu Val Asp Pro Met Asp
            900                 905                 910

gag ccc acc ctt ctt tat gtt ttg ttt gaa gtc ttt gac gtg gtc cgt 2784
Glu Pro Thr Leu Leu Tyr Val Leu Phe Glu Val Phe Asp Val Val Arg
        915                 920                 925

gtg cac cag ccg cac cgc ggc gtc atc gaa acc gtg tac ctg cgc acg 2832
Val His Gln Pro His Arg Gly Val Ile Glu Thr Val Tyr Leu Arg Thr
    930                 935                 940

ccc ttc tcg gcc ggc aac gcc aca aca taa 2862
Pro Phe Ser Ala Gly Asn Ala Thr Thr

945                 950

<210>  9
<211>  2880
<212>  DNA
<213>  Artificial Sequence

<220>
<223>  (QGPGAP)9-HVR1 modified Hexon protein

<220>
<221>  CDS
<222>  (1)..(2877)

<400>  9
atg gct acc cct tcg atg atg ccg cag tgg tct tac atg cac atc tcg          48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc          96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc         144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg         192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac         240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg         288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act         336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt         384
Phe Lys Pro Tyr Ser Gly Thr Ala Tyr Asn Ala Leu Ala Pro Lys Gly
        115                 120                 125

gcc cca aat cct tgc gaa tgg gat gaa cag gga cca gga gca cca cag         432
Ala Pro Asn Pro Cys Glu Trp Asp Glu Gln Gly Pro Gly Ala Pro Gln
    130                 135                 140

gga cca gga gca cca cag gga cca gga gca cca cag gga cca gga gca         480
Gly Pro Gly Ala Pro Gln Gly Pro Gly Ala Pro Gln Gly Pro Gly Ala
145                 150                 155                 160

cca cag gga cca gga gca cca cag gga cca gga gca ccg caa ggt cct         528
Pro Gln Gly Pro Gly Ala Pro Gln Gly Pro Gly Ala Pro Gln Gly Pro
                165                 170                 175

ggt gct cct cag gga cca gga gca cca cag gga cca gga gca cca aaa 576
Gly Ala Pro Gln Gly Pro Gly Ala Pro Gln Gly Pro Gly Ala Pro Lys
            180                 185                 190

act cac gta ttt ggg cag gcg cct tat tct ggt ata aat att aca aag 624
Thr His Val Phe Gly Gln Ala Pro Tyr Ser Gly Ile Asn Ile Thr Lys
        195                 200                 205

gag ggt att caa ata ggt gtc gaa ggt caa aca cct aaa tat gcc gat 672
Glu Gly Ile Gln Ile Gly Val Glu Gly Gln Thr Pro Lys Tyr Ala Asp
    210                 215                 220

aaa aca ttt caa cct gaa cct caa ata gga gaa tct cag tgg tac gaa 720
Lys Thr Phe Gln Pro Glu Pro Gln Ile Gly Glu Ser Gln Trp Tyr Glu
225                 230                 235                 240

aca gaa att aat cat gca gct ggg aga gtc cta aaa aag act acc cca 768
Thr Glu Ile Asn His Ala Ala Gly Arg Val Leu Lys Lys Thr Thr Pro
                245                 250                 255

atg aaa cca tgt tac ggt tca tat gca aaa ccc aca aat gaa aat gga 816
Met Lys Pro Cys Tyr Gly Ser Tyr Ala Lys Pro Thr Asn Glu Asn Gly
            260                 265                 270

ggg caa ggc att ctt gta aag caa caa aat gga aag cta gaa agt caa 864
Gly Gln Gly Ile Leu Val Lys Gln Gln Asn Gly Lys Leu Glu Ser Gln
        275                 280                 285

gtg gaa atg caa ttt ttc tca act act gag gca gcc gca ggc aat ggt 912
Val Glu Met Gln Phe Phe Ser Thr Thr Glu Ala Ala Ala Gly Asn Gly
    290                 295                 300

gat aac ttg act cct aaa gtg gta ttg tac agt gaa gat gta gat ata 960
Asp Asn Leu Thr Pro Lys Val Val Leu Tyr Ser Glu Asp Val Asp Ile
305                 310                 315                 320

gaa acc cca gac act cat att tct tac atg ccc act att aag gaa ggt 1008
Glu Thr Pro Asp Thr His Ile Ser Tyr Met Pro Thr Ile Lys Glu Gly
                325                 330                 335

aac tca cga gaa cta atg ggc caa caa tct atg ccc aac agg cct aat 1056
Asn Ser Arg Glu Leu Met Gly Gln Gln Ser Met Pro Asn Arg Pro Asn
            340                 345                 350

tac att gct ttt agg gac aat ttt att ggt cta atg tat tac aac agc 1104
Tyr Ile Ala Phe Arg Asp Asn Phe Ile Gly Leu Met Tyr Tyr Asn Ser
        355                 360                 365

acg ggt aat atg ggt gtt ctg gcg ggc caa gca tcg cag ttg aat gct 1152
Thr Gly Asn Met Gly Val Leu Ala Gly Gln Ala Ser Gln Leu Asn Ala
    370                 375                 380

gtt gta gat ttg caa gac aga aac aca gag ctt tca tac cag ctt ttg 1200
Val Val Asp Leu Gln Asp Arg Asn Thr Glu Leu Ser Tyr Gln Leu Leu
385                 390                 395                 400

ctt gat tcc att ggt gat aga acc agg tac ttt tct atg tgg aat cag 1248
Leu Asp Ser Ile Gly Asp Arg Thr Arg Tyr Phe Ser Met Trp Asn Gln
                405                 410                 415

ctt cca aat tac tgc ttt cca ctg gga ggt gtg att aat aca gag act 1296
Leu Pro Asn Tyr Cys Phe Pro Leu Gly Gly Val Ile Asn Thr Glu Thr

420                 425                 430

ctt acc aag gta aaa cct aaa aca ggt cag gaa aat gga tgg gaa aaa      1344
Leu Thr Lys Val Lys Pro Lys Thr Gly Gln Glu Asn Gly Trp Glu Lys
        435                 440                 445

gat gct aca gaa ttt tca gat aaa aat gaa ata aga gtt gga aat aat      1392
Asp Ala Thr Glu Phe Ser Asp Lys Asn Glu Ile Arg Val Gly Asn Asn
    450                 455                 460

ttt gcc atg gaa atc aat cta aat gcc aac ctg tgg aga aat ttc ctg      1440
Phe Ala Met Glu Ile Asn Leu Asn Ala Asn Leu Trp Arg Asn Phe Leu
465                 470                 475                 480

tac tcc aac ata gcg ctg tat ttg ccc gac aag cta aag tac agt cct      1488
Tyr Ser Asn Ile Ala Leu Tyr Leu Pro Asp Lys Leu Lys Tyr Ser Pro
                485                 490                 495

tcc aac gta aaa att tct gat aac cca aac acc tac gac tac atg aac      1536
Ser Asn Val Lys Ile Ser Asp Asn Pro Asn Thr Tyr Asp Tyr Met Asn
            500                 505                 510

aag cga gtg gtg gct ccc ggg cta gtg gac tgc tac att aac ctt gga      1584
Lys Arg Val Val Ala Pro Gly Leu Val Asp Cys Tyr Ile Asn Leu Gly
        515                 520                 525

gca cgc tgg tcc ctt gac tat atg gac aac gtc aac cca ttt aac cac      1632
Ala Arg Trp Ser Leu Asp Tyr Met Asp Asn Val Asn Pro Phe Asn His
    530                 535                 540

cac cgc aat gct ggc ctg cgc tac cgc tca atg ttg ctg ggc aat ggt      1680
His Arg Asn Ala Gly Leu Arg Tyr Arg Ser Met Leu Leu Gly Asn Gly
545                 550                 555                 560

cgc tat gtg ccc ttc cac atc cag gtg cct cag aag ttc ttt gcc att      1728
Arg Tyr Val Pro Phe His Ile Gln Val Pro Gln Lys Phe Phe Ala Ile
                565                 570                 575

aaa aac ctc ctt ctc ctg ccg ggc tca tac acc tac gag tgg aac ttc      1776
Lys Asn Leu Leu Leu Leu Pro Gly Ser Tyr Thr Tyr Glu Trp Asn Phe
            580                 585                 590

agg aag gat gtt aac atg gtt ctg cag agc tcc cta gga aat gac cta      1824
Arg Lys Asp Val Asn Met Val Leu Gln Ser Ser Leu Gly Asn Asp Leu
        595                 600                 605

agg gtt gac gga gcc agc att aag ttt gat agc att tgc ctt tac gcc      1872
Arg Val Asp Gly Ala Ser Ile Lys Phe Asp Ser Ile Cys Leu Tyr Ala
    610                 615                 620

acc ttc ttc ccc atg gcc cac aac acc gcc tcc acg ctt gag gcc atg      1920
Thr Phe Phe Pro Met Ala His Asn Thr Ala Ser Thr Leu Glu Ala Met
625                 630                 635                 640

ctt aga aac gac acc aac gac cag tcc ttt aac gac tat ctc tcc gcc      1968
Leu Arg Asn Asp Thr Asn Asp Gln Ser Phe Asn Asp Tyr Leu Ser Ala
                645                 650                 655

gcc aac atg ctc tac cct ata ccc gcc aac gct acc aac gtg ccc ata      2016
Ala Asn Met Leu Tyr Pro Ile Pro Ala Asn Ala Thr Asn Val Pro Ile
            660                 665                 670

tcc atc ccc tcc cgc aac tgg gcg gct ttc cgc ggc tgg gcc ttc acg      2064

Ser Ile Pro Ser Arg Asn Trp Ala Ala Phe Arg Gly Trp Ala Phe Thr
        675                 680                 685

cgc ctt aag act aag gaa acc cca tca ctg ggc tcg ggc tac gac cct      2112
Arg Leu Lys Thr Lys Glu Thr Pro Ser Leu Gly Ser Gly Tyr Asp Pro
    690                 695                 700

tat tac acc tac tct ggc tct ata ccc tac cta gat gga acc ttt tac      2160
Tyr Tyr Thr Tyr Ser Gly Ser Ile Pro Tyr Leu Asp Gly Thr Phe Tyr
705                 710                 715                 720

ctc aac cac acc ttt aag aag gtg gcc att acc ttt gac tct tct gtc      2208
Leu Asn His Thr Phe Lys Lys Val Ala Ile Thr Phe Asp Ser Ser Val
                725                 730                 735

agc tgg cct ggc aat gac cgc ctg ctt acc ccc aac gag ttt gaa att      2256
Ser Trp Pro Gly Asn Asp Arg Leu Leu Thr Pro Asn Glu Phe Glu Ile
            740                 745                 750

aag cgc tca gtt gac ggg gag ggt tac aac gtt gcc cag tgt aac atg      2304
Lys Arg Ser Val Asp Gly Glu Gly Tyr Asn Val Ala Gln Cys Asn Met
        755                 760                 765

acc aaa gac tgg ttc ctg gta caa atg cta gct aac tat aac att ggc      2352
Thr Lys Asp Trp Phe Leu Val Gln Met Leu Ala Asn Tyr Asn Ile Gly
    770                 775                 780

tac cag ggc ttc tat atc cca gag agc tac aag gac cgc atg tac tcc      2400
Tyr Gln Gly Phe Tyr Ile Pro Glu Ser Tyr Lys Asp Arg Met Tyr Ser
785                 790                 795                 800

ttc ttt aga aac ttc cag ccc atg agc cgt cag gtg gtg gat gat act      2448
Phe Phe Arg Asn Phe Gln Pro Met Ser Arg Gln Val Val Asp Asp Thr
                805                 810                 815

aaa tac aag gac tac caa cag gtg ggc atc cta cac caa cac aac aac      2496
Lys Tyr Lys Asp Tyr Gln Gln Val Gly Ile Leu His Gln His Asn Asn
            820                 825                 830

tct gga ttt gtt ggc tac ctt gcc ccc acc atg cgc gaa gga cag gcc      2544
Ser Gly Phe Val Gly Tyr Leu Ala Pro Thr Met Arg Glu Gly Gln Ala
        835                 840                 845

tac cct gct aac ttc ccc tat ccg ctt ata ggc aag acc gca gtt gac      2592
Tyr Pro Ala Asn Phe Pro Tyr Pro Leu Ile Gly Lys Thr Ala Val Asp
    850                 855                 860

agc att acc cag aaa aag ttt ctt tgc gat cgc acc ctt tgg cgc atc      2640
Ser Ile Thr Gln Lys Lys Phe Leu Cys Asp Arg Thr Leu Trp Arg Ile
865                 870                 875                 880

cca ttc tcc agt aac ttt atg tcc atg ggc gca ctc aca gac ctg ggc      2688
Pro Phe Ser Ser Asn Phe Met Ser Met Gly Ala Leu Thr Asp Leu Gly
                885                 890                 895

caa aac ctt ctc tac gcc aac tcc gcc cac gcg cta gac atg act ttt      2736
Gln Asn Leu Leu Tyr Ala Asn Ser Ala His Ala Leu Asp Met Thr Phe
            900                 905                 910

gag gtg gat ccc atg gac gag ccc acc ctt ctt tat gtt ttg ttt gaa      2784
Glu Val Asp Pro Met Asp Glu Pro Thr Leu Leu Tyr Val Leu Phe Glu
        915                 920                 925

gtc ttt gac gtg gtc cgt gtg cac cag ccg cac cgc ggc gtc atc gaa          2832
Val Phe Asp Val Val Arg Val His Gln Pro His Arg Gly Val Ile Glu
    930                 935                 940

acc gtg tac ctg cgc acg ccc ttc tcg gcc ggc aac gcc aca aca taa          2880
Thr Val Tyr Leu Arg Thr Pro Phe Ser Ala Gly Asn Ala Thr Thr
945                 950                 955

<210>  10
<211>  2916
<212>  DNA
<213>  Artificial Sequence

<220>
<223>  (QGPGAP)11-HVR1 modified Hexon protein

<220>
<221>  CDS
<222>  (1)..(2913)

<400>  10
atg gct acc cct tcg atg atg ccg cag tgg tct tac atg cac atc tcg          48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc          96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc          144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg          192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac          240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg          288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act          336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt          384
Phe Lys Pro Tyr Ser Gly Thr Ala Tyr Asn Ala Leu Ala Pro Lys Gly
        115                 120                 125

gcc cca aat cct tgc gaa tgg gat gaa cag gga cca gga gca cca cag          432
Ala Pro Asn Pro Cys Glu Trp Asp Glu Gln Gly Pro Gly Ala Pro Gln
    130                 135                 140

gga cca gga gca cca cag gga cca gga gca cca cag gga cca gga gca          480
Gly Pro Gly Ala Pro Gln Gly Pro Gly Ala Pro Gln Gly Pro Gly Ala

145                 150                 155                 160

cca cag gga cca gga gca cca cag gga cca gga gca cca cag gga cca       528
Pro Gln Gly Pro Gly Ala Pro Gln Gly Pro Gly Ala Pro Gln Gly Pro
                165                 170                 175

gga gca cca cag gga cca gga gca ccg caa ggt cct ggt gct cct cag       576
Gly Ala Pro Gln Gly Pro Gly Ala Pro Gln Gly Pro Gly Ala Pro Gln
            180                 185                 190

gga cca gga gca cca cag gga cca gga gca cca aaa act cac gta ttt       624
Gly Pro Gly Ala Pro Gln Gly Pro Gly Ala Pro Lys Thr His Val Phe
        195                 200                 205

ggg cag gcg cct tat tct ggt ata aat att aca aag gag ggt att caa       672
Gly Gln Ala Pro Tyr Ser Gly Ile Asn Ile Thr Lys Glu Gly Ile Gln
    210                 215                 220

ata ggt gtc gaa ggt caa aca cct aaa tat gcc gat aaa aca ttt caa       720
Ile Gly Val Glu Gly Gln Thr Pro Lys Tyr Ala Asp Lys Thr Phe Gln
225                 230                 235                 240

cct gaa cct caa ata gga gaa tct cag tgg tac gaa aca gaa att aat       768
Pro Glu Pro Gln Ile Gly Glu Ser Gln Trp Tyr Glu Thr Glu Ile Asn
                245                 250                 255

cat gca gct ggg aga gtc cta aaa aag act acc cca atg aaa cca tgt       816
His Ala Ala Gly Arg Val Leu Lys Lys Thr Thr Pro Met Lys Pro Cys
            260                 265                 270

tac ggt tca tat gca aaa ccc aca aat gaa aat gga ggg caa ggc att       864
Tyr Gly Ser Tyr Ala Lys Pro Thr Asn Glu Asn Gly Gly Gln Gly Ile
        275                 280                 285

ctt gta aag caa caa aat gga aag cta gaa agt caa gtg gaa atg caa       912
Leu Val Lys Gln Gln Asn Gly Lys Leu Glu Ser Gln Val Glu Met Gln
    290                 295                 300

ttt ttc tca act act gag gca gcc gca ggc aat ggt gat aac ttg act       960
Phe Phe Ser Thr Thr Glu Ala Ala Ala Gly Asn Gly Asp Asn Leu Thr
305                 310                 315                 320

cct aaa gtg gta ttg tac agt gaa gat gta gat ata gaa acc cca gac      1008
Pro Lys Val Val Leu Tyr Ser Glu Asp Val Asp Ile Glu Thr Pro Asp
                325                 330                 335

act cat att tct tac atg ccc act att aag gaa ggt aac tca cga gaa      1056
Thr His Ile Ser Tyr Met Pro Thr Ile Lys Glu Gly Asn Ser Arg Glu
            340                 345                 350

cta atg ggc caa caa tct atg ccc aac agg cct aat tac att gct ttt      1104
Leu Met Gly Gln Gln Ser Met Pro Asn Arg Pro Asn Tyr Ile Ala Phe
        355                 360                 365

agg gac aat ttt att ggt cta atg tat tac aac agc acg ggt aat atg      1152
Arg Asp Asn Phe Ile Gly Leu Met Tyr Tyr Asn Ser Thr Gly Asn Met
    370                 375                 380

ggt gtt ctg gcg ggc caa gca tcg cag ttg aat gct gtt gta gat ttg      1200
Gly Val Leu Ala Gly Gln Ala Ser Gln Leu Asn Ala Val Val Asp Leu
385                 390                 395                 400

caa gac aga aac aca gag ctt tca tac cag ctt ttg ctt gat tcc att      1248

Gln Asp Arg Asn Thr Glu Leu Ser Tyr Gln Leu Leu Leu Asp Ser Ile
                405                 410                 415

ggt gat aga acc agg tac ttt tct atg tgg aat cag ctt cca aat tac      1296
Gly Asp Arg Thr Arg Tyr Phe Ser Met Trp Asn Gln Leu Pro Asn Tyr
            420                 425                 430

tgc ttt cca ctg gga ggt gtg att aat aca gag act ctt acc aag gta      1344
Cys Phe Pro Leu Gly Gly Val Ile Asn Thr Glu Thr Leu Thr Lys Val
        435                 440                 445

aaa cct aaa aca ggt cag gaa aat gga tgg gaa aaa gat gct aca gaa      1392
Lys Pro Lys Thr Gly Gln Glu Asn Gly Trp Glu Lys Asp Ala Thr Glu
    450                 455                 460

ttt tca gat aaa aat gaa ata aga gtt gga aat aat ttt gcc atg gaa      1440
Phe Ser Asp Lys Asn Glu Ile Arg Val Gly Asn Asn Phe Ala Met Glu
465                 470                 475                 480

atc aat cta aat gcc aac ctg tgg aga aat ttc ctg tac tcc aac ata      1488
Ile Asn Leu Asn Ala Asn Leu Trp Arg Asn Phe Leu Tyr Ser Asn Ile
                485                 490                 495

gcg ctg tat ttg ccc gac aag cta aag tac agt cct tcc aac gta aaa      1536
Ala Leu Tyr Leu Pro Asp Lys Leu Lys Tyr Ser Pro Ser Asn Val Lys
            500                 505                 510

att tct gat aac cca aac acc tac gac tac atg aac aag cga gtg gtg      1584
Ile Ser Asp Asn Pro Asn Thr Tyr Asp Tyr Met Asn Lys Arg Val Val
        515                 520                 525

gct ccc ggg cta gtg gac tgc tac att aac ctt gga gca cgc tgg tcc      1632
Ala Pro Gly Leu Val Asp Cys Tyr Ile Asn Leu Gly Ala Arg Trp Ser
    530                 535                 540

ctt gac tat atg gac aac gtc aac cca ttt aac cac cac cgc aat gct      1680
Leu Asp Tyr Met Asp Asn Val Asn Pro Phe Asn His His Arg Asn Ala
545                 550                 555                 560

ggc ctg cgc tac cgc tca atg ttg ctg ggc aat ggt cgc tat gtg ccc      1728
Gly Leu Arg Tyr Arg Ser Met Leu Leu Gly Asn Gly Arg Tyr Val Pro
                565                 570                 575

ttc cac atc cag gtg cct cag aag ttc ttt gcc att aaa aac ctc ctt      1776
Phe His Ile Gln Val Pro Gln Lys Phe Phe Ala Ile Lys Asn Leu Leu
            580                 585                 590

ctc ctg ccg ggc tca tac acc tac gag tgg aac ttc agg aag gat gtt      1824
Leu Leu Pro Gly Ser Tyr Thr Tyr Glu Trp Asn Phe Arg Lys Asp Val
        595                 600                 605

aac atg gtt ctg cag agc tcc cta gga aat gac cta agg gtt gac gga      1872
Asn Met Val Leu Gln Ser Ser Leu Gly Asn Asp Leu Arg Val Asp Gly
    610                 615                 620

gcc agc att aag ttt gat agc att tgc ctt tac gcc acc ttc ttc ccc      1920
Ala Ser Ile Lys Phe Asp Ser Ile Cys Leu Tyr Ala Thr Phe Phe Pro
625                 630                 635                 640

atg gcc cac aac acc gcc tcc acg ctt gag gcc atg ctt aga aac gac      1968
Met Ala His Asn Thr Ala Ser Thr Leu Glu Ala Met Leu Arg Asn Asp
                645                 650                 655

acc aac gac cag tcc ttt aac gac tat ctc tcc gcc gcc aac atg ctc     2016
Thr Asn Asp Gln Ser Phe Asn Asp Tyr Leu Ser Ala Ala Asn Met Leu
        660                 665                 670

tac cct ata ccc gcc aac gct acc aac gtg ccc ata tcc atc ccc tcc     2064
Tyr Pro Ile Pro Ala Asn Ala Thr Asn Val Pro Ile Ser Ile Pro Ser
        675                 680                 685

cgc aac tgg gcg gct ttc cgc ggc tgg gcc ttc acg cgc ctt aag act     2112
Arg Asn Trp Ala Ala Phe Arg Gly Trp Ala Phe Thr Arg Leu Lys Thr
    690                 695                 700

aag gaa acc cca tca ctg ggc tcg ggc tac gac cct tat tac acc tac     2160
Lys Glu Thr Pro Ser Leu Gly Ser Gly Tyr Asp Pro Tyr Tyr Thr Tyr
705                 710                 715                 720

tct ggc tct ata ccc tac cta gat gga acc ttt tac ctc aac cac acc     2208
Ser Gly Ser Ile Pro Tyr Leu Asp Gly Thr Phe Tyr Leu Asn His Thr
                725                 730                 735

ttt aag aag gtg gcc att acc ttt gac tct tct gtc agc tgg cct ggc     2256
Phe Lys Lys Val Ala Ile Thr Phe Asp Ser Ser Val Ser Trp Pro Gly
        740                 745                 750

aat gac cgc ctg ctt acc ccc aac gag ttt gaa att aag cgc tca gtt     2304
Asn Asp Arg Leu Leu Thr Pro Asn Glu Phe Glu Ile Lys Arg Ser Val
        755                 760                 765

gac ggg gag ggt tac aac gtt gcc cag tgt aac atg acc aaa gac tgg     2352
Asp Gly Glu Gly Tyr Asn Val Ala Gln Cys Asn Met Thr Lys Asp Trp
    770                 775                 780

ttc ctg gta caa atg cta gct aac tat aac att ggc tac cag ggc ttc     2400
Phe Leu Val Gln Met Leu Ala Asn Tyr Asn Ile Gly Tyr Gln Gly Phe
785                 790                 795                 800

tat atc cca gag agc tac aag gac cgc atg tac tcc ttc ttt aga aac     2448
Tyr Ile Pro Glu Ser Tyr Lys Asp Arg Met Tyr Ser Phe Phe Arg Asn
                805                 810                 815

ttc cag ccc atg agc cgt cag gtg gtg gat gat act aaa tac aag gac     2496
Phe Gln Pro Met Ser Arg Gln Val Val Asp Asp Thr Lys Tyr Lys Asp
        820                 825                 830

tac caa cag gtg ggc atc cta cac caa cac aac aac tct gga ttt gtt     2544
Tyr Gln Gln Val Gly Ile Leu His Gln His Asn Asn Ser Gly Phe Val
        835                 840                 845

ggc tac ctt gcc ccc acc atg cgc gaa gga cag gcc tac cct gct aac     2592
Gly Tyr Leu Ala Pro Thr Met Arg Glu Gly Gln Ala Tyr Pro Ala Asn
    850                 855                 860

ttc ccc tat ccg ctt ata ggc aag acc gca gtt gac agc att acc cag     2640
Phe Pro Tyr Pro Leu Ile Gly Lys Thr Ala Val Asp Ser Ile Thr Gln
865                 870                 875                 880

aaa aag ttt ctt tgc gat cgc acc ctt tgg cgc atc cca ttc tcc agt     2688
Lys Lys Phe Leu Cys Asp Arg Thr Leu Trp Arg Ile Pro Phe Ser Ser
                885                 890                 895

aac ttt atg tcc atg ggc gca ctc aca gac ctg ggc caa aac ctt ctc     2736
Asn Phe Met Ser Met Gly Ala Leu Thr Asp Leu Gly Gln Asn Leu Leu
        900                 905                 910

tac gcc aac tcc gcc cac gcg cta gac atg act ttt gag gtg gat ccc 2784
Tyr Ala Asn Ser Ala His Ala Leu Asp Met Thr Phe Glu Val Asp Pro
        915                 920                 925

atg gac gag ccc acc ctt ctt tat gtt ttg ttt gaa gtc ttt gac gtg 2832
Met Asp Glu Pro Thr Leu Leu Tyr Val Leu Phe Glu Val Phe Asp Val
    930                 935                 940

gtc cgt gtg cac cag ccg cac cgc ggc gtc atc gaa acc gtg tac ctg 2880
Val Arg Val His Gln Pro His Arg Gly Val Ile Glu Thr Val Tyr Leu
945                 950                 955                 960

cgc acg ccc ttc tcg gcc ggc aac gcc aca aca taa 2916
Arg Thr Pro Phe Ser Ala Gly Asn Ala Thr Thr
                965                 970

<210> 11
<211> 2934
<212> DNA
<213> Artificial Sequence

<220>
<223> (QGPGAP)12-HVR1 modified Hexon protein

<220>
<221> CDS
<222> (1)..(2931)

<400> 11
atg gct acc cct tcg atg atg ccg cag tgg tct tac atg cac atc tcg 48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc 96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc 144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg 192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac 240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg 288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act 336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt 384

Phe Lys Pro Tyr Ser Gly Thr Ala Tyr Asn Ala Leu Ala Pro Lys Gly
        115                 120                 125

gcc cca aat cct tgc gaa tgg gat gaa cag gga cca gga gca cca cag     432
Ala Pro Asn Pro Cys Glu Trp Asp Glu Gln Gly Pro Gly Ala Pro Gln
    130                 135                 140

gga cca gga gca cca cag gga cca gga gca cca cag gga cca gga gca     480
Gly Pro Gly Ala Pro Gln Gly Pro Gly Ala Pro Gln Gly Pro Gly Ala
145                 150                 155                 160

cca cag gga cca gga gca cca cag gga cca gga gca cca cag gga cca     528
Pro Gln Gly Pro Gly Ala Pro Gln Gly Pro Gly Ala Pro Gln Gly Pro
                165                 170                 175

gga gca cca cag gga cca gga gca ccg caa ggt cct ggt gct cct cag     576
Gly Ala Pro Gln Gly Pro Gly Ala Pro Gln Gly Pro Gly Ala Pro Gln
            180                 185                 190

gga cca gga gca cca cag gga cca gga gca cca cag gga cca gga gca     624
Gly Pro Gly Ala Pro Gln Gly Pro Gly Ala Pro Gln Gly Pro Gly Ala
        195                 200                 205

cca aaa act cac gta ttt ggg cag gcg cct tat tct ggt ata aat att     672
Pro Lys Thr His Val Phe Gly Gln Ala Pro Tyr Ser Gly Ile Asn Ile
    210                 215                 220

aca aag gag ggt att caa ata ggt gtc gaa ggt caa aca cct aaa tat     720
Thr Lys Glu Gly Ile Gln Ile Gly Val Glu Gly Gln Thr Pro Lys Tyr
225                 230                 235                 240

gcc gat aaa aca ttt caa cct gaa cct caa ata gga gaa tct cag tgg     768
Ala Asp Lys Thr Phe Gln Pro Glu Pro Gln Ile Gly Glu Ser Gln Trp
                245                 250                 255

tac gaa aca gaa att aat cat gca gct ggg aga gtc cta aaa aag act     816
Tyr Glu Thr Glu Ile Asn His Ala Ala Gly Arg Val Leu Lys Lys Thr
            260                 265                 270

acc cca atg aaa cca tgt tac ggt tca tat gca aaa ccc aca aat gaa     864
Thr Pro Met Lys Pro Cys Tyr Gly Ser Tyr Ala Lys Pro Thr Asn Glu
        275                 280                 285

aat gga ggg caa ggc att ctt gta aag caa caa aat gga aag cta gaa     912
Asn Gly Gly Gln Gly Ile Leu Val Lys Gln Gln Asn Gly Lys Leu Glu
    290                 295                 300

agt caa gtg gaa atg caa ttt ttc tca act act gag gca gcc gca ggc     960
Ser Gln Val Glu Met Gln Phe Phe Ser Thr Thr Glu Ala Ala Ala Gly
305                 310                 315                 320

aat ggt gat aac ttg act cct aaa gtg gta ttg tac agt gaa gat gta    1008
Asn Gly Asp Asn Leu Thr Pro Lys Val Val Leu Tyr Ser Glu Asp Val
                325                 330                 335

gat ata gaa acc cca gac act cat att tct tac atg ccc act att aag    1056
Asp Ile Glu Thr Pro Asp Thr His Ile Ser Tyr Met Pro Thr Ile Lys
            340                 345                 350

gaa ggt aac tca cga gaa cta atg ggc caa caa tct atg ccc aac agg    1104
Glu Gly Asn Ser Arg Glu Leu Met Gly Gln Gln Ser Met Pro Asn Arg
        355                 360                 365

cct aat tac att gct ttt agg gac aat ttt att ggt cta atg tat tac      1152
Pro Asn Tyr Ile Ala Phe Arg Asp Asn Phe Ile Gly Leu Met Tyr Tyr
    370                 375                 380

aac agc acg ggt aat atg ggt gtt ctg gcg ggc caa gca tcg cag ttg      1200
Asn Ser Thr Gly Asn Met Gly Val Leu Ala Gly Gln Ala Ser Gln Leu
385                 390                 395                 400

aat gct gtt gta gat ttg caa gac aga aac aca gag ctt tca tac cag      1248
Asn Ala Val Val Asp Leu Gln Asp Arg Asn Thr Glu Leu Ser Tyr Gln
                405                 410                 415

ctt ttg ctt gat tcc att ggt gat aga acc agg tac ttt tct atg tgg      1296
Leu Leu Leu Asp Ser Ile Gly Asp Arg Thr Arg Tyr Phe Ser Met Trp
            420                 425                 430

aat cag ctt cca aat tac tgc ttt cca ctg gga ggt gtg att aat aca      1344
Asn Gln Leu Pro Asn Tyr Cys Phe Pro Leu Gly Gly Val Ile Asn Thr
        435                 440                 445

gag act ctt acc aag gta aaa cct aaa aca ggt cag gaa aat gga tgg      1392
Glu Thr Leu Thr Lys Val Lys Pro Lys Thr Gly Gln Glu Asn Gly Trp
    450                 455                 460

gaa aaa gat gct aca gaa ttt tca gat aaa aat gaa ata aga gtt gga      1440
Glu Lys Asp Ala Thr Glu Phe Ser Asp Lys Asn Glu Ile Arg Val Gly
465                 470                 475                 480

aat aat ttt gcc atg gaa atc aat cta aat gcc aac ctg tgg aga aat      1488
Asn Asn Phe Ala Met Glu Ile Asn Leu Asn Ala Asn Leu Trp Arg Asn
                485                 490                 495

ttc ctg tac tcc aac ata gcg ctg tat ttg ccc gac aag cta aag tac      1536
Phe Leu Tyr Ser Asn Ile Ala Leu Tyr Leu Pro Asp Lys Leu Lys Tyr
            500                 505                 510

agt cct tcc aac gta aaa att tct gat aac cca aac acc tac gac tac      1584
Ser Pro Ser Asn Val Lys Ile Ser Asp Asn Pro Asn Thr Tyr Asp Tyr
        515                 520                 525

atg aac aag cga gtg gtg gct ccc ggg cta gtg gac tgc tac att aac      1632
Met Asn Lys Arg Val Val Ala Pro Gly Leu Val Asp Cys Tyr Ile Asn
    530                 535                 540

ctt gga gca cgc tgg tcc ctt gac tat atg gac aac gtc aac cca ttt      1680
Leu Gly Ala Arg Trp Ser Leu Asp Tyr Met Asp Asn Val Asn Pro Phe
545                 550                 555                 560

aac cac cac cgc aat gct ggc ctg cgc tac cgc tca atg ttg ctg ggc      1728
Asn His His Arg Asn Ala Gly Leu Arg Tyr Arg Ser Met Leu Leu Gly
                565                 570                 575

aat ggt cgc tat gtg ccc ttc cac atc cag gtg cct cag aag ttc ttt      1776
Asn Gly Arg Tyr Val Pro Phe His Ile Gln Val Pro Gln Lys Phe Phe
            580                 585                 590

gcc att aaa aac ctc ctt ctc ctg ccg ggc tca tac acc tac gag tgg      1824
Ala Ile Lys Asn Leu Leu Leu Leu Pro Gly Ser Tyr Thr Tyr Glu Trp
        595                 600                 605

aac ttc agg aag gat gtt aac atg gtt ctg cag agc tcc cta gga aat      1872
Asn Phe Arg Lys Asp Val Asn Met Val Leu Gln Ser Ser Leu Gly Asn
    610                 615                 620

gac cta agg gtt gac gga gcc agc att aag ttt gat agc att tgc ctt 1920
Asp Leu Arg Val Asp Gly Ala Ser Ile Lys Phe Asp Ser Ile Cys Leu
625                 630                 635                 640

tac gcc acc ttc ttc ccc atg gcc cac aac acc gcc tcc acg ctt gag 1968
Tyr Ala Thr Phe Phe Pro Met Ala His Asn Thr Ala Ser Thr Leu Glu
                645                 650                 655

gcc atg ctt aga aac gac acc aac gac cag tcc ttt aac gac tat ctc 2016
Ala Met Leu Arg Asn Asp Thr Asn Asp Gln Ser Phe Asn Asp Tyr Leu
            660                 665                 670

tcc gcc gcc aac atg ctc tac cct ata ccc gcc aac gct acc aac gtg 2064
Ser Ala Ala Asn Met Leu Tyr Pro Ile Pro Ala Asn Ala Thr Asn Val
        675                 680                 685

ccc ata tcc atc ccc tcc cgc aac tgg gcg gct ttc cgc ctg ctt acc 2112
Pro Ile Ser Ile Pro Ser Arg Asn Trp Ala Ala Phe Arg Leu Leu Thr
    690                 695                 700

ccc aac gag ttt gaa att aag cgc tca gtt gac ggg gag ggt tac aac 2160
Pro Asn Glu Phe Glu Ile Lys Arg Ser Val Asp Gly Glu Gly Tyr Asn
705                 710                 715                 720

gac cct tat tac acc tac tct ggc tct ata ccc tac cta gat gga acc 2208
Asp Pro Tyr Tyr Thr Tyr Ser Gly Ser Ile Pro Tyr Leu Asp Gly Thr
                725                 730                 735

ttt tac ctc aac cac acc ttt aag aag gtg gcc att acc ttt gac tct 2256
Phe Tyr Leu Asn His Thr Phe Lys Lys Val Ala Ile Thr Phe Asp Ser
            740                 745                 750

tct gtc agc tgg cct ggc aat gac cgc ctg ctt acc ccc aac gag ttt 2304
Ser Val Ser Trp Pro Gly Asn Asp Arg Leu Leu Thr Pro Asn Glu Phe
        755                 760                 765

gaa att aag cgc tca gtt gac ggg gag ggt tac aac gtt gcc cag tgt 2352
Glu Ile Lys Arg Ser Val Asp Gly Glu Gly Tyr Asn Val Ala Gln Cys
    770                 775                 780

aac atg acc aaa gac tgg ttc ctg gta caa atg cta gct aac tat aac 2400
Asn Met Thr Lys Asp Trp Phe Leu Val Gln Met Leu Ala Asn Tyr Asn
785                 790                 795                 800

att ggc tac cag ggc ttc tat atc cca gag agc tac aag gac cgc atg 2448
Ile Gly Tyr Gln Gly Phe Tyr Ile Pro Glu Ser Tyr Lys Asp Arg Met
                805                 810                 815

tac tcc ttc ttt aga aac ttc cag ccc atg agc cgt cag gtg gtg gat 2496
Tyr Ser Phe Phe Arg Asn Phe Gln Pro Met Ser Arg Gln Val Val Asp
            820                 825                 830

gat act aaa tac aag gac tac caa cag gtg ggc atc cta cac caa cac 2544
Asp Thr Lys Tyr Lys Asp Tyr Gln Gln Val Gly Ile Leu His Gln His
        835                 840                 845

aac aac tct gga ttt gtt ggc tac ctt gcc ccc acc atg cgc gaa gga 2592
Asn Asn Ser Gly Phe Val Gly Tyr Leu Ala Pro Thr Met Arg Glu Gly
    850                 855                 860

cag gcc tac cct gct aac ttc ccc tat ccg ctt ata ggc aag acc gca 2640
Gln Ala Tyr Pro Ala Asn Phe Pro Tyr Pro Leu Ile Gly Lys Thr Ala

865                 870                 875                 880

gtt gac agc att acc cag aaa aag ttt ctt tgc gat cgc acc ctt tgg       2688
Val Asp Ser Ile Thr Gln Lys Lys Phe Leu Cys Asp Arg Thr Leu Trp
                885                 890                 895

cgc atc cca ttc tcc agt aac ttt atg tcc atg ggc gca ctc aca gac       2736
Arg Ile Pro Phe Ser Ser Asn Phe Met Ser Met Gly Ala Leu Thr Asp
            900                 905                 910

ctg ggc caa aac ctt ctc tac gcc aac tcc gcc cac gcg cta gac atg       2784
Leu Gly Gln Asn Leu Leu Tyr Ala Asn Ser Ala His Ala Leu Asp Met
        915                 920                 925

act ttt gag gtg gat ccc atg gac gag ccc acc ctt ctt tat gtt ttg       2832
Thr Phe Glu Val Asp Pro Met Asp Glu Pro Thr Leu Leu Tyr Val Leu
    930                 935                 940

ttt gaa gtc ttt gac gtg gtc cgt gtg cac cag ccg cac cgc ggc gtc       2880
Phe Glu Val Phe Asp Val Val Arg Val His Gln Pro His Arg Gly Val
945                 950                 955                 960

atc gaa acc gtg tac ctg cgc acg ccc ttc tcg gcc ggc aac gcc aca       2928
Ile Glu Thr Val Tyr Leu Arg Thr Pro Phe Ser Ala Gly Asn Ala Thr
                965                 970                 975

aca taa                                                               2934
Thr

<210>  12
<211>  2826
<212>  DNA
<213>  Artificial Sequence

<220>
<223>  (NANP)4-HVR1 modified Hexon protein

<220>
<221>  CDS
<222>  (1)..(2823)

<400>  12
atg gct acc cct tcg atg atg ccg cag tgg tct tac atg cac atc tcg         48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc         96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc        144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg        192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac        240
Thr Leu Arg Phe Ile Pro Val Asp Arg Glu Asp Thr Ala Tyr Ser Tyr

65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa aac gct aat ccc aac gct aac      432
Ala Pro Asn Pro Cys Glu Trp Asp Glu Asn Ala Asn Pro Asn Ala Asn
    130                 135                 140

cca aat gca aat cct aat gcc aac ccc aaa act cac gta ttt ggg cag      480
Pro Asn Ala Asn Pro Asn Ala Asn Pro Lys Thr His Val Phe Gly Gln
145                 150                 155                 160

gcg cct tat tct ggt ata aat att aca aag gag ggt att caa ata ggt      528
Ala Pro Tyr Ser Gly Ile Asn Ile Thr Lys Glu Gly Ile Gln Ile Gly
                165                 170                 175

gtc gaa ggt caa aca cct aaa tat gcc gat aaa aca ttt caa cct gaa      576
Val Glu Gly Gln Thr Pro Lys Tyr Ala Asp Lys Thr Phe Gln Pro Glu
            180                 185                 190

cct caa ata gga gaa tct cag tgg tac gaa aca gaa att aat cat gca      624
Pro Gln Ile Gly Glu Ser Gln Trp Tyr Glu Thr Glu Ile Asn His Ala
        195                 200                 205

gct ggg aga gtc cta aaa aag act acc cca atg aaa cca tgt tac ggt      672
Ala Gly Arg Val Leu Lys Lys Thr Thr Pro Met Lys Pro Cys Tyr Gly
    210                 215                 220

tca tat gca aaa ccc aca aat gaa aat gga ggg caa ggc att ctt gta      720
Ser Tyr Ala Lys Pro Thr Asn Glu Asn Gly Gly Gln Gly Ile Leu Val
225                 230                 235                 240

aag caa caa aat gga aag cta gaa agt caa gtg gaa atg caa ttt ttc      768
Lys Gln Gln Asn Gly Lys Leu Glu Ser Gln Val Glu Met Gln Phe Phe
                245                 250                 255

tca act act gag gca gcc gca ggc aat ggt gat aac ttg act cct aaa      816
Ser Thr Thr Glu Ala Ala Ala Gly Asn Gly Asp Asn Leu Thr Pro Lys
            260                 265                 270

gtg gta ttg tac agt gaa gat gta gat ata gaa acc cca gac act cat      864
Val Val Leu Tyr Ser Glu Asp Val Asp Ile Glu Thr Pro Asp Thr His
        275                 280                 285

att tct tac atg ccc act att aag gaa ggt aac tca cga gaa cta atg      912
Ile Ser Tyr Met Pro Thr Ile Lys Glu Gly Asn Ser Arg Glu Leu Met
    290                 295                 300

ggc caa caa tct atg ccc aac agg cct aat tac att gct ttt agg gac      960
Gly Gln Gln Ser Met Pro Asn Arg Pro Asn Tyr Ile Ala Phe Arg Asp
305                 310                 315                 320

aat ttt att ggt cta atg tat tac aac agc acg ggt aat atg ggt gtt     1008

Asn Phe Ile Gly Leu Met Tyr Tyr Asn Ser Thr Gly Asn Met Gly Val
                325                 330                 335

ctg gcg ggc caa gca tcg cag ttg aat gct gtt gta gat ttg caa gac      1056
Leu Ala Gly Gln Ala Ser Gln Leu Asn Ala Val Val Asp Leu Gln Asp
            340                 345                 350

aga aac aca gag ctt tca tac cag ctt ttg ctt gat tcc att ggt gat      1104
Arg Asn Thr Glu Leu Ser Tyr Gln Leu Leu Leu Asp Ser Ile Gly Asp
        355                 360                 365

aga acc agg tac ttt tct atg tgg aat cag gct gtt gac agc tat gat      1152
Arg Thr Arg Tyr Phe Ser Met Trp Asn Gln Ala Val Asp Ser Tyr Asp
    370                 375                 380

cca gat gtt aga att att gaa aat cat gga act gaa gat gaa ctt cca      1200
Pro Asp Val Arg Ile Ile Glu Asn His Gly Thr Glu Asp Glu Leu Pro
385                 390                 395                 400

aat tac tgc ttt cca ctg gga ggt gtg att aat aca gag act ctt acc      1248
Asn Tyr Cys Phe Pro Leu Gly Gly Val Ile Asn Thr Glu Thr Leu Thr
                405                 410                 415

aag gta aaa cct aaa aca ggt cag gaa aat gga tgg gaa aaa gat gct      1296
Lys Val Lys Pro Lys Thr Gly Gln Glu Asn Gly Trp Glu Lys Asp Ala
            420                 425                 430

aca gaa ttt tca gat aaa aat gaa ata aga gtt gga aat aat ttt gcc      1344
Thr Glu Phe Ser Asp Lys Asn Glu Ile Arg Val Gly Asn Asn Phe Ala
        435                 440                 445

atg gaa atc aat cta aat gcc aac ctg tgg aga aat ttc ctg tac tcc      1392
Met Glu Ile Asn Leu Asn Ala Asn Leu Trp Arg Asn Phe Leu Tyr Ser
    450                 455                 460

aac ata gcg ctg tat ttg ccc gac aag cta aag tac agt cct tcc aac      1440
Asn Ile Ala Leu Tyr Leu Pro Asp Lys Leu Lys Tyr Ser Pro Ser Asn
465                 470                 475                 480

gta aaa att tct gat aac cca aac acc tac gac tac atg aac aag cga      1488
Val Lys Ile Ser Asp Asn Pro Asn Thr Tyr Asp Tyr Met Asn Lys Arg
                485                 490                 495

gtg gtg gct ccc ggg cta gtg gac tgc tac att aac ctt gga gca cgc      1536
Val Val Ala Pro Gly Leu Val Asp Cys Tyr Ile Asn Leu Gly Ala Arg
            500                 505                 510

tgg tcc ctt gac tat atg gac aac gtc aac cca ttt aac cac cac cgc      1584
Trp Ser Leu Asp Tyr Met Asp Asn Val Asn Pro Phe Asn His His Arg
        515                 520                 525

aat gct ggc ctg cgc tac cgc tca atg ttg ctg ggc aat ggt cgc tat      1632
Asn Ala Gly Leu Arg Tyr Arg Ser Met Leu Leu Gly Asn Gly Arg Tyr
    530                 535                 540

gtg ccc ttc cac atc cag gtg cct cag aag ttc ttt gcc att aaa aac      1680
Val Pro Phe His Ile Gln Val Pro Gln Lys Phe Phe Ala Ile Lys Asn
545                 550                 555                 560

ctc ctt ctc ctg ccg ggc tca tac acc tac gag tgg aac ttc agg aag      1728
Leu Leu Leu Leu Pro Gly Ser Tyr Thr Tyr Glu Trp Asn Phe Arg Lys
                565                 570                 575

gat gtt aac atg gtt ctg cag agc tcc cta gga aat gac cta agg gtt          1776
Asp Val Asn Met Val Leu Gln Ser Ser Leu Gly Asn Asp Leu Arg Val
            580                 585                 590

gac gga gcc agc att aag ttt gat agc att tgc ctt tac gcc acc ttc          1824
Asp Gly Ala Ser Ile Lys Phe Asp Ser Ile Cys Leu Tyr Ala Thr Phe
        595                 600                 605

ttc ccc atg gcc cac aac acc gcc tcc acg ctt gag gcc atg ctt aga          1872
Phe Pro Met Ala His Asn Thr Ala Ser Thr Leu Glu Ala Met Leu Arg
    610                 615                 620

aac gac acc aac gac cag tcc ttt aac gac tat ctc tcc gcc gcc aac          1920
Asn Asp Thr Asn Asp Gln Ser Phe Asn Asp Tyr Leu Ser Ala Ala Asn
625                 630                 635                 640

atg ctc tac cct ata ccc gcc aac gct acc aac gtg ccc ata tcc atc          1968
Met Leu Tyr Pro Ile Pro Ala Asn Ala Thr Asn Val Pro Ile Ser Ile
                645                 650                 655

ccc tcc cgc aac tgg gcg gct ttc cgc ggc tgg gcc ttc acg cgc ctt          2016
Pro Ser Arg Asn Trp Ala Ala Phe Arg Gly Trp Ala Phe Thr Arg Leu
            660                 665                 670

aag act aag gaa acc cca tca ctg ggc tcg ggc tac gac cct tat tac          2064
Lys Thr Lys Glu Thr Pro Ser Leu Gly Ser Gly Tyr Asp Pro Tyr Tyr
        675                 680                 685

acc tac tct ggc tct ata ccc tac cta gat gga acc ttt tac ctc aac          2112
Thr Tyr Ser Gly Ser Ile Pro Tyr Leu Asp Gly Thr Phe Tyr Leu Asn
    690                 695                 700

cac acc ttt aag aag gtg gcc att acc ttt gac tct tct gtc agc tgg          2160
His Thr Phe Lys Lys Val Ala Ile Thr Phe Asp Ser Ser Val Ser Trp
705                 710                 715                 720

cct ggc aat gac cgc ctg ctt acc ccc aac gag ttt gaa att aag cgc          2208
Pro Gly Asn Asp Arg Leu Leu Thr Pro Asn Glu Phe Glu Ile Lys Arg
                725                 730                 735

tca gtt gac ggg gag ggt tac aac gtt gcc cag tgt aac atg acc aaa          2256
Ser Val Asp Gly Glu Gly Tyr Asn Val Ala Gln Cys Asn Met Thr Lys
            740                 745                 750

gac tgg ttc ctg gta caa atg cta gct aac tat aac att ggc tac cag          2304
Asp Trp Phe Leu Val Gln Met Leu Ala Asn Tyr Asn Ile Gly Tyr Gln
        755                 760                 765

ggc ttc tat atc cca gag agc tac aag gac cgc atg tac tcc ttc ttt          2352
Gly Phe Tyr Ile Pro Glu Ser Tyr Lys Asp Arg Met Tyr Ser Phe Phe
    770                 775                 780

aga aac ttc cag ccc atg agc cgt cag gtg gtg gat gat act aaa tac          2400
Arg Asn Phe Gln Pro Met Ser Arg Gln Val Val Asp Asp Thr Lys Tyr
785                 790                 795                 800

aag gac tac caa cag gtg ggc atc cta cac caa cac aac aac tct gga          2448
Lys Asp Tyr Gln Gln Val Gly Ile Leu His Gln His Asn Asn Ser Gly
                805                 810                 815

ttt gtt ggc tac ctt gcc ccc acc atg cgc gaa gga cag gcc tac cct          2496
Phe Val Gly Tyr Leu Ala Pro Thr Met Arg Glu Gly Gln Ala Tyr Pro
            820                 825                 830

gct aac ttc ccc tat ccg ctt ata ggc aag acc gca gtt gac agc att     2544
Ala Asn Phe Pro Tyr Pro Leu Ile Gly Lys Thr Ala Val Asp Ser Ile
        835                 840                 845

acc cag aaa aag ttt ctt tgc gat cgc acc ctt tgg cgc atc cca ttc     2592
Thr Gln Lys Lys Phe Leu Cys Asp Arg Thr Leu Trp Arg Ile Pro Phe
    850                 855                 860

tcc agt aac ttt atg tcc atg ggc gca ctc aca gac ctg ggc caa aac     2640
Ser Ser Asn Phe Met Ser Met Gly Ala Leu Thr Asp Leu Gly Gln Asn
865                 870                 875                 880

ctt ctc tac gcc aac tcc gcc cac gcg cta gac atg act ttt gag gtg     2688
Leu Leu Tyr Ala Asn Ser Ala His Ala Leu Asp Met Thr Phe Glu Val
                885                 890                 895

gat ccc atg gac gag ccc acc ctt ctt tat gtt ttg ttt gaa gtc ttt     2736
Asp Pro Met Asp Glu Pro Thr Leu Leu Tyr Val Leu Phe Glu Val Phe
            900                 905                 910

gac gtg gtc cgt gtg cac cag ccg cac cgc ggc gtc atc gaa acc gtg     2784
Asp Val Val Arg Val His Gln Pro His Arg Gly Val Ile Glu Thr Val
        915                 920                 925

tac ctg cgc acg ccc ttc tcg gcc ggc aac gcc aca aca taa             2826
Tyr Leu Arg Thr Pro Phe Ser Ala Gly Asn Ala Thr Thr
    930                 935                 940

<210>  13
<211>  2790
<212>  DNA
<213>  Artificial Sequence

<220>
<223>  (NANP)6-HVR1 modified Hexon protein

<220>
<221>  CDS
<222>  (1)..(2787)

<400>  13
atg gct acc cct tcg atg atg ccg cag tgg tct tac atg cac atc tcg     48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc     96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc     144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg     192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac     240

Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat      432
Ala Pro Asn Pro Cys Glu Trp Asp Glu Asn Ala Asn Pro Asn Ala Asn
    130                 135                 140

cca aat gca aat cca aat gca aat cca aac gcg aac ccg aat gct aat      480
Pro Asn Ala Asn Pro Asn Ala Asn Pro Asn Ala Asn Pro Asn Ala Asn
145                 150                 155                 160

cct aaa act cac gta ttt ggg cag gcg cct tat tct ggt ata aat att      528
Pro Lys Thr His Val Phe Gly Gln Ala Pro Tyr Ser Gly Ile Asn Ile
                165                 170                 175

aca aag gag ggt att caa ata ggt gtc gaa ggt caa aca cct aaa tat      576
Thr Lys Glu Gly Ile Gln Ile Gly Val Glu Gly Gln Thr Pro Lys Tyr
            180                 185                 190

gcc gat aaa aca ttt caa cct gaa cct caa ata gga gaa tct cag tgg      624
Ala Asp Lys Thr Phe Gln Pro Glu Pro Gln Ile Gly Glu Ser Gln Trp
        195                 200                 205

tac gaa aca gaa att aat cat gca gct ggg aga gtc cta aaa aag act      672
Tyr Glu Thr Glu Ile Asn His Ala Ala Gly Arg Val Leu Lys Lys Thr
    210                 215                 220

acc cca atg aaa cca tgt tac ggt tca tat gca aaa ccc aca aat gaa      720
Thr Pro Met Lys Pro Cys Tyr Gly Ser Tyr Ala Lys Pro Thr Asn Glu
225                 230                 235                 240

aat gga ggg caa ggc att ctt gta aag caa caa aat gga aag cta gaa      768
Asn Gly Gly Gln Gly Ile Leu Val Lys Gln Gln Asn Gly Lys Leu Glu
                245                 250                 255

agt caa gtg gaa atg caa ttt ttc tca act act gag gca gcc gca ggc      816
Ser Gln Val Glu Met Gln Phe Phe Ser Thr Thr Glu Ala Ala Ala Gly
            260                 265                 270

aat ggt gat aac ttg act cct aaa gtg gta ttg tac agt gaa gat gta      864
Asn Gly Asp Asn Leu Thr Pro Lys Val Val Leu Tyr Ser Glu Asp Val
        275                 280                 285

gat ata gaa acc cca gac act cat att tct tac atg ccc act att aag      912
Asp Ile Glu Thr Pro Asp Thr His Ile Ser Tyr Met Pro Thr Ile Lys
    290                 295                 300

gaa ggt aac tca cga gaa cta atg ggc caa caa tct atg ccc aac agg      960
Glu Gly Asn Ser Arg Glu Leu Met Gly Gln Gln Ser Met Pro Asn Arg
305                 310                 315                 320

cct aat tac att gct ttt agg gac aat ttt att ggt cta atg tat tac     1008
Pro Asn Tyr Ile Ala Phe Arg Asp Asn Phe Ile Gly Leu Met Tyr Tyr
                325                 330                 335

aac agc acg ggt aat atg ggt gtt ctg gcg ggc caa gca tcg cag ttg     1056
Asn Ser Thr Gly Asn Met Gly Val Leu Ala Gly Gln Ala Ser Gln Leu
            340                 345                 350

aat gct gtt gta gat ttg caa gac aga aac aca gag ctt tca tac cag     1104
Asn Ala Val Val Asp Leu Gln Asp Arg Asn Thr Glu Leu Ser Tyr Gln
        355                 360                 365

ctt ttg ctt gat tcc att ggt gat aga acc agg tac ttt tct atg tgg     1152
Leu Leu Leu Asp Ser Ile Gly Asp Arg Thr Arg Tyr Phe Ser Met Trp
    370                 375                 380

aat cag ctt cca aat tac tgc ttt cca ctg gga ggt gtg att aat aca     1200
Asn Gln Leu Pro Asn Tyr Cys Phe Pro Leu Gly Gly Val Ile Asn Thr
385                 390                 395                 400

gag act ctt acc aag gta aaa cct aaa aca ggt cag gaa aat gga tgg     1248
Glu Thr Leu Thr Lys Val Lys Pro Lys Thr Gly Gln Glu Asn Gly Trp
                405                 410                 415

gaa aaa gat gct aca gaa ttt tca gat aaa aat gaa ata aga gtt gga     1296
Glu Lys Asp Ala Thr Glu Phe Ser Asp Lys Asn Glu Ile Arg Val Gly
            420                 425                 430

aat aat ttt gcc atg gaa atc aat cta aat gcc aac ctg tgg aga aat     1344
Asn Asn Phe Ala Met Glu Ile Asn Leu Asn Ala Asn Leu Trp Arg Asn
        435                 440                 445

ttc ctg tac tcc aac ata gcg ctg tat ttg ccc gac aag cta aag tac     1392
Phe Leu Tyr Ser Asn Ile Ala Leu Tyr Leu Pro Asp Lys Leu Lys Tyr
    450                 455                 460

agt cct tcc aac gta aaa att tct gat aac cca aac acc tac gac tac     1440
Ser Pro Ser Asn Val Lys Ile Ser Asp Asn Pro Asn Thr Tyr Asp Tyr
465                 470                 475                 480

atg aac aag cga gtg gtg gct ccc ggg cta gtg gac tgc tac att aac     1488
Met Asn Lys Arg Val Val Ala Pro Gly Leu Val Asp Cys Tyr Ile Asn
                485                 490                 495

ctt gga gca cgc tgg tcc ctt gac tat atg gac aac gtc aac cca ttt     1536
Leu Gly Ala Arg Trp Ser Leu Asp Tyr Met Asp Asn Val Asn Pro Phe
            500                 505                 510

aac cac cac cgc aat gct ggc ctg cgc tac cgc tca atg ttg ctg ggc     1584
Asn His His Arg Asn Ala Gly Leu Arg Tyr Arg Ser Met Leu Leu Gly
        515                 520                 525

aat ggt cgc tat gtg ccc ttc cac atc cag gtg cct cag aag ttc ttt     1632
Asn Gly Arg Tyr Val Pro Phe His Ile Gln Val Pro Gln Lys Phe Phe
    530                 535                 540

gcc att aaa aac ctc ctt ctc ctg ccg ggc tca tac acc tac gag tgg     1680
Ala Ile Lys Asn Leu Leu Leu Leu Pro Gly Ser Tyr Thr Tyr Glu Trp
545                 550                 555                 560

aac ttc agg aag gat gtt aac atg gtt ctg cag agc tcc cta gga aat     1728
Asn Phe Arg Lys Asp Val Asn Met Val Leu Gln Ser Ser Leu Gly Asn
                565                 570                 575

gac cta agg gtt gac gga gcc agc att aag ttt gat agc att tgc ctt 1776
Asp Leu Arg Val Asp Gly Ala Ser Ile Lys Phe Asp Ser Ile Cys Leu
            580                 585                 590

tac gcc acc ttc ttc ccc atg gcc cac aac acc gcc tcc acg ctt gag 1824
Tyr Ala Thr Phe Phe Pro Met Ala His Asn Thr Ala Ser Thr Leu Glu
        595                 600                 605

gcc atg ctt aga aac gac acc aac gac cag tcc ttt aac gac tat ctc 1872
Ala Met Leu Arg Asn Asp Thr Asn Asp Gln Ser Phe Asn Asp Tyr Leu
    610                 615                 620

tcc gcc gcc aac atg ctc tac cct ata ccc gcc aac gct acc aac gtg 1920
Ser Ala Ala Asn Met Leu Tyr Pro Ile Pro Ala Asn Ala Thr Asn Val
625                 630                 635                 640

ccc ata tcc atc ccc tcc cgc aac tgg gcg gct ttc cgc ggc tgg gcc 1968
Pro Ile Ser Ile Pro Ser Arg Asn Trp Ala Ala Phe Arg Gly Trp Ala
                645                 650                 655

ttc acg cgc ctt aag act aag gaa acc cca tca ctg ggc tcg ggc tac 2016
Phe Thr Arg Leu Lys Thr Lys Glu Thr Pro Ser Leu Gly Ser Gly Tyr
            660                 665                 670

gac cct tat tac acc tac tct ggc tct ata ccc tac cta gat gga acc 2064
Asp Pro Tyr Tyr Thr Tyr Ser Gly Ser Ile Pro Tyr Leu Asp Gly Thr
        675                 680                 685

ttt tac ctc aac cac acc ttt aag aag gtg gcc att acc ttt gac tct 2112
Phe Tyr Leu Asn His Thr Phe Lys Lys Val Ala Ile Thr Phe Asp Ser
    690                 695                 700

tct gtc agc tgg cct ggc aat gac cgc ctg ctt acc ccc aac gag ttt 2160
Ser Val Ser Trp Pro Gly Asn Asp Arg Leu Leu Thr Pro Asn Glu Phe
705                 710                 715                 720

gaa att aag cgc tca gtt gac ggg gag ggt tac aac gtt gcc cag tgt 2208
Glu Ile Lys Arg Ser Val Asp Gly Glu Gly Tyr Asn Val Ala Gln Cys
                725                 730                 735

aac atg acc aaa gac tgg ttc ctg gta caa atg cta gct aac tat aac 2256
Asn Met Thr Lys Asp Trp Phe Leu Val Gln Met Leu Ala Asn Tyr Asn
            740                 745                 750

att ggc tac cag ggc ttc tat atc cca gag agc tac aag gac cgc atg 2304
Ile Gly Tyr Gln Gly Phe Tyr Ile Pro Glu Ser Tyr Lys Asp Arg Met
        755                 760                 765

tac tcc ttc ttt aga aac ttc cag ccc atg agc cgt cag gtg gtg gat 2352
Tyr Ser Phe Phe Arg Asn Phe Gln Pro Met Ser Arg Gln Val Val Asp
    770                 775                 780

gat act aaa tac aag gac tac caa cag gtg ggc atc cta cac caa cac 2400
Asp Thr Lys Tyr Lys Asp Tyr Gln Gln Val Gly Ile Leu His Gln His
785                 790                 795                 800

aac aac tct gga ttt gtt ggc tac ctt gcc ccc acc atg cgc gaa gga 2448
Asn Asn Ser Gly Phe Val Gly Tyr Leu Ala Pro Thr Met Arg Glu Gly
                805                 810                 815

cag gcc tac cct gct aac ttc ccc tat ccg ctt ata ggc aag acc gca 2496
Gln Ala Tyr Pro Ala Asn Phe Pro Tyr Pro Leu Ile Gly Lys Thr Ala

820                 825                 830

gtt gac agc att acc cag aaa aag ttt ctt tgc gat cgc acc ctt tgg          2544
Val Asp Ser Ile Thr Gln Lys Lys Phe Leu Cys Asp Arg Thr Leu Trp
        835                 840                 845

cgc atc cca ttc tcc agt aac ttt atg tcc atg ggc gca ctc aca gac          2592
Arg Ile Pro Phe Ser Ser Asn Phe Met Ser Met Gly Ala Leu Thr Asp
    850                 855                 860

ctg ggc caa aac ctt ctc tac gcc aac tcc gcc cac gcg cta gac atg          2640
Leu Gly Gln Asn Leu Leu Tyr Ala Asn Ser Ala His Ala Leu Asp Met
865                 870                 875                 880

act ttt gag gtg gat ccc atg gac gag ccc acc ctt ctt tat gtt ttg          2688
Thr Phe Glu Val Asp Pro Met Asp Glu Pro Thr Leu Leu Tyr Val Leu
                885                 890                 895

ttt gaa gtc ttt gac gtg gtc cgt gtg cac cag ccg cac cgc ggc gtc          2736
Phe Glu Val Phe Asp Val Val Arg Val His Gln Pro His Arg Gly Val
            900                 905                 910

atc gaa acc gtg tac ctg cgc acg ccc ttc tcg gcc ggc aac gcc aca          2784
Ile Glu Thr Val Tyr Leu Arg Thr Pro Phe Ser Ala Gly Asn Ala Thr
        915                 920                 925

aca taa                                                                  2790
Thr

<210>  14
<211>  2814
<212>  DNA
<213>  Artificial Sequence

<220>
<223>  (NANP)8-HVR1 modified Hexon protein

<220>
<221>  CDS
<222>  (1)..(2811)

<400>  14
atg gct acc cct tcg atg atg ccg cag tgg tct tac atg cac atc tcg          48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc          96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc          144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg          192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac          240

Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat      432
Ala Pro Asn Pro Cys Glu Trp Asp Glu Asn Ala Asn Pro Asn Ala Asn
    130                 135                 140

cca aat gca aat cca aat gca aat cca aac gcg aac ccg aat gct aat      480
Pro Asn Ala Asn Pro Asn Ala Asn Pro Asn Ala Asn Pro Asn Ala Asn
145                 150                 155                 160

cct aat gca aat cca aat gca aat cca aaa act cac gta ttt ggg cag      528
Pro Asn Ala Asn Pro Asn Ala Asn Pro Lys Thr His Val Phe Gly Gln
                165                 170                 175

gcg cct tat tct ggt ata aat att aca aag gag ggt att caa ata ggt      576
Ala Pro Tyr Ser Gly Ile Asn Ile Thr Lys Glu Gly Ile Gln Ile Gly
            180                 185                 190

gtc gaa ggt caa aca cct aaa tat gcc gat aaa aca ttt caa cct gaa      624
Val Glu Gly Gln Thr Pro Lys Tyr Ala Asp Lys Thr Phe Gln Pro Glu
        195                 200                 205

cct caa ata gga gaa tct cag tgg tac gaa aca gaa att aat cat gca      672
Pro Gln Ile Gly Glu Ser Gln Trp Tyr Glu Thr Glu Ile Asn His Ala
    210                 215                 220

gct ggg aga gtc cta aaa aag act acc cca atg aaa cca tgt tac ggt      720
Ala Gly Arg Val Leu Lys Lys Thr Thr Pro Met Lys Pro Cys Tyr Gly
225                 230                 235                 240

tca tat gca aaa ccc aca aat gaa aat gga ggg caa ggc att ctt gta      768
Ser Tyr Ala Lys Pro Thr Asn Glu Asn Gly Gly Gln Gly Ile Leu Val
                245                 250                 255

aag caa caa aat gga aag cta gaa agt caa gtg gaa atg caa ttt ttc      816
Lys Gln Gln Asn Gly Lys Leu Glu Ser Gln Val Glu Met Gln Phe Phe
            260                 265                 270

tca act act gag gca gcc gca ggc aat ggt gat aac ttg act cct aaa      864
Ser Thr Thr Glu Ala Ala Ala Gly Asn Gly Asp Asn Leu Thr Pro Lys
        275                 280                 285

gtg gta ttg tac agt gaa gat gta gat ata gaa acc cca gac act cat      912
Val Val Leu Tyr Ser Glu Asp Val Asp Ile Glu Thr Pro Asp Thr His
    290                 295                 300

att tct tac atg ccc act att aag gaa ggt aac tca cga gaa cta atg      960
Ile Ser Tyr Met Pro Thr Ile Lys Glu Gly Asn Ser Arg Glu Leu Met
305                 310                 315                 320

ggc caa caa tct atg ccc aac agg cct aat tac att gct ttt agg gac 1008
Gly Gln Gln Ser Met Pro Asn Arg Pro Asn Tyr Ile Ala Phe Arg Asp
                325                 330                 335

aat ttt att ggt cta atg tat tac aac agc acg ggt aat atg ggt gtt 1056
Asn Phe Ile Gly Leu Met Tyr Tyr Asn Ser Thr Gly Asn Met Gly Val
            340                 345                 350

ctg gcg ggc caa gca tcg cag ttg aat gct gtt gta gat ttg caa gac 1104
Leu Ala Gly Gln Ala Ser Gln Leu Asn Ala Val Val Asp Leu Gln Asp
        355                 360                 365

aga aac aca gag ctt tca tac cag ctt ttg ctt gat tcc att ggt gat 1152
Arg Asn Thr Glu Leu Ser Tyr Gln Leu Leu Leu Asp Ser Ile Gly Asp
    370                 375                 380

aga acc agg tac ttt tct atg tgg aat cag ctt cca aat tac tgc ttt 1200
Arg Thr Arg Tyr Phe Ser Met Trp Asn Gln Leu Pro Asn Tyr Cys Phe
385                 390                 395                 400

cca ctg gga ggt gtg att aat aca gag act ctt acc aag gta aaa cct 1248
Pro Leu Gly Gly Val Ile Asn Thr Glu Thr Leu Thr Lys Val Lys Pro
                405                 410                 415

aaa aca ggt cag gaa aat gga tgg gaa aaa gat gct aca gaa ttt tca 1296
Lys Thr Gly Gln Glu Asn Gly Trp Glu Lys Asp Ala Thr Glu Phe Ser
            420                 425                 430

gat aaa aat gaa ata aga gtt gga aat aat ttt gcc atg gaa atc aat 1344
Asp Lys Asn Glu Ile Arg Val Gly Asn Asn Phe Ala Met Glu Ile Asn
        435                 440                 445

cta aat gcc aac ctg tgg aga aat ttc ctg tac tcc aac ata gcg ctg 1392
Leu Asn Ala Asn Leu Trp Arg Asn Phe Leu Tyr Ser Asn Ile Ala Leu
    450                 455                 460

tat ttg ccc gac aag cta aag tac agt cct tcc aac gta aaa att tct 1440
Tyr Leu Pro Asp Lys Leu Lys Tyr Ser Pro Ser Asn Val Lys Ile Ser
465                 470                 475                 480

gat aac cca aac acc tac gac tac atg aac aag cga gtg gtg gct ccc 1488
Asp Asn Pro Asn Thr Tyr Asp Tyr Met Asn Lys Arg Val Val Ala Pro
                485                 490                 495

ggg cta gtg gac tgc tac att aac ctt gga gca cgc tgg tcc ctt gac 1536
Gly Leu Val Asp Cys Tyr Ile Asn Leu Gly Ala Arg Trp Ser Leu Asp
            500                 505                 510

tat atg gac aac gtc aac cca ttt aac cac cac cgc aat gct ggc ctg 1584
Tyr Met Asp Asn Val Asn Pro Phe Asn His His Arg Asn Ala Gly Leu
        515                 520                 525

cgc tac cgc tca atg ttg ctg ggc aat ggt cgc tat gtg ccc ttc cac 1632
Arg Tyr Arg Ser Met Leu Leu Gly Asn Gly Arg Tyr Val Pro Phe His
    530                 535                 540

atc cag gtg cct cag aag ttc ttt gcc att aaa aac ctc ctt ctc ctg 1680
Ile Gln Val Pro Gln Lys Phe Phe Ala Ile Lys Asn Leu Leu Leu Leu
545                 550                 555                 560

ccg ggc tca tac acc tac gag tgg aac ttc agg aag gat gtt aac atg 1728
Pro Gly Ser Tyr Thr Tyr Glu Trp Asn Phe Arg Lys Asp Val Asn Met
                565                 570                 575

gtt ctg cag agc tcc cta gga aat gac cta agg gtt gac gga gcc agc 1776
Val Leu Gln Ser Ser Leu Gly Asn Asp Leu Arg Val Asp Gly Ala Ser
            580                 585                 590

att aag ttt gat agc att tgc ctt tac gcc acc ttc ttc ccc atg gcc 1824
Ile Lys Phe Asp Ser Ile Cys Leu Tyr Ala Thr Phe Phe Pro Met Ala
        595                 600                 605

cac aac acc gcc tcc acg ctt gag gcc atg ctt aga aac gac acc aac 1872
His Asn Thr Ala Ser Thr Leu Glu Ala Met Leu Arg Asn Asp Thr Asn
    610                 615                 620

gac cag tcc ttt aac gac tat ctc tcc gcc gcc aac atg ctc tac cct 1920
Asp Gln Ser Phe Asn Asp Tyr Leu Ser Ala Ala Asn Met Leu Tyr Pro
625                 630                 635                 640

ata ccc gcc aac gct acc aac gtg ccc ata tcc atc ccc tcc cgc aac 1968
Ile Pro Ala Asn Ala Thr Asn Val Pro Ile Ser Ile Pro Ser Arg Asn
                645                 650                 655

tgg gcg gct ttc cgc ggc tgg gcc ttc acg cgc ctt aag act aag gaa 2016
Trp Ala Ala Phe Arg Gly Trp Ala Phe Thr Arg Leu Lys Thr Lys Glu
            660                 665                 670

acc cca tca ctg ggc tcg ggc tac gac cct tat tac acc tac tct ggc 2064
Thr Pro Ser Leu Gly Ser Gly Tyr Asp Pro Tyr Tyr Thr Tyr Ser Gly
        675                 680                 685

tct ata ccc tac cta gat gga acc ttt tac ctc aac cac acc ttt aag 2112
Ser Ile Pro Tyr Leu Asp Gly Thr Phe Tyr Leu Asn His Thr Phe Lys
    690                 695                 700

aag gtg gcc att acc ttt gac tct tct gtc agc tgg cct ggc aat gac 2160
Lys Val Ala Ile Thr Phe Asp Ser Ser Val Ser Trp Pro Gly Asn Asp
705                 710                 715                 720

cgc ctg ctt acc ccc aac gag ttt gaa att aag cgc tca gtt gac ggg 2208
Arg Leu Leu Thr Pro Asn Glu Phe Glu Ile Lys Arg Ser Val Asp Gly
                725                 730                 735

gag ggt tac aac gtt gcc cag tgt aac atg acc aaa gac tgg ttc ctg 2256
Glu Gly Tyr Asn Val Ala Gln Cys Asn Met Thr Lys Asp Trp Phe Leu
            740                 745                 750

gta caa atg cta gct aac tat aac att ggc tac cag ggc ttc tat atc 2304
Val Gln Met Leu Ala Asn Tyr Asn Ile Gly Tyr Gln Gly Phe Tyr Ile
        755                 760                 765

cca gag agc tac aag gac cgc atg tac tcc ttc ttt aga aac ttc cag 2352
Pro Glu Ser Tyr Lys Asp Arg Met Tyr Ser Phe Phe Arg Asn Phe Gln
    770                 775                 780

ccc atg agc cgt cag gtg gtg gat gat act aaa tac aag gac tac caa 2400
Pro Met Ser Arg Gln Val Val Asp Asp Thr Lys Tyr Lys Asp Tyr Gln
785                 790                 795                 800

cag gtg ggc atc cta cac caa cac aac aac tct gga ttt gtt ggc tac 2448
Gln Val Gly Ile Leu His Gln His Asn Asn Ser Gly Phe Val Gly Tyr
                805                 810                 815

ctt gcc ccc acc atg cgc gaa gga cag gcc tac cct gct aac ttc ccc 2496
Leu Ala Pro Thr Met Arg Glu Gly Gln Ala Tyr Pro Ala Asn Phe Pro

820                 825                 830

tat ccg ctt ata ggc aag acc gca gtt gac agc att acc cag aaa aag        2544
Tyr Pro Leu Ile Gly Lys Thr Ala Val Asp Ser Ile Thr Gln Lys Lys
        835                 840                 845

ttt ctt tgc gat cgc acc ctt tgg cgc atc cca ttc tcc agt aac ttt        2592
Phe Leu Cys Asp Arg Thr Leu Trp Arg Ile Pro Phe Ser Ser Asn Phe
    850                 855                 860

atg tcc atg ggc gca ctc aca gac ctg ggc caa aac ctt ctc tac gcc        2640
Met Ser Met Gly Ala Leu Thr Asp Leu Gly Gln Asn Leu Leu Tyr Ala
865                 870                 875                 880

aac tcc gcc cac gcg cta gac atg act ttt gag gtg gat ccc atg gac        2688
Asn Ser Ala His Ala Leu Asp Met Thr Phe Glu Val Asp Pro Met Asp
                885                 890                 895

gag ccc acc ctt ctt tat gtt ttg ttt gaa gtc ttt gac gtg gtc cgt        2736
Glu Pro Thr Leu Leu Tyr Val Leu Phe Glu Val Phe Asp Val Val Arg
            900                 905                 910

gtg cac cag ccg cac cgc ggc gtc atc gaa acc gtg tac ctg cgc acg        2784
Val His Gln Pro His Arg Gly Val Ile Glu Thr Val Tyr Leu Arg Thr
        915                 920                 925

ccc ttc tcg gcc ggc aac gcc aca aca taa                                2814
Pro Phe Ser Ala Gly Asn Ala Thr Thr
    930                 935

<210>  15
<211>  2838
<212>  DNA
<213>  Artificial Sequence

<220>
<223>  (NANP)10-HVR1 modified Hexon protein

<220>
<221>  CDS
<222>  (1)..(2835)

<400>  15
atg gct acc cct tcg atg atg ccg cag tgg tct tac atg cac atc tcg          48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc          96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc         144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg         192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac 240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg 288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act 336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt 384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat 432
Ala Pro Asn Pro Cys Glu Trp Asp Glu Asn Ala Asn Pro Asn Ala Asn
    130                 135                 140

cca aat gca aat cca aat gca aat cca aac gcg aac ccg aat gct aat 480
Pro Asn Ala Asn Pro Asn Ala Asn Pro Asn Ala Asn Pro Asn Ala Asn
145                 150                 155                 160

cct aat gca aat cca aat gca aat cca aat gca aat cca aat gca aat 528
Pro Asn Ala Asn Pro Asn Ala Asn Pro Asn Ala Asn Pro Asn Ala Asn
                165                 170                 175

cca aaa act cac gta ttt ggg cag gcg cct tat tct ggt ata aat att 576
Pro Lys Thr His Val Phe Gly Gln Ala Pro Tyr Ser Gly Ile Asn Ile
            180                 185                 190

aca aag gag ggt att caa ata ggt gtc gaa ggt caa aca cct aaa tat 624
Thr Lys Glu Gly Ile Gln Ile Gly Val Glu Gly Gln Thr Pro Lys Tyr
        195                 200                 205

gcc gat aaa aca ttt caa cct gaa cct caa ata gga gaa tct cag tgg 672
Ala Asp Lys Thr Phe Gln Pro Glu Pro Gln Ile Gly Glu Ser Gln Trp
    210                 215                 220

tac gaa aca gaa att aat cat gca gct ggg aga gtc cta aaa aag act 720
Tyr Glu Thr Glu Ile Asn His Ala Ala Gly Arg Val Leu Lys Lys Thr
225                 230                 235                 240

acc cca atg aaa cca tgt tac ggt tca tat gca aaa ccc aca aat gaa 768
Thr Pro Met Lys Pro Cys Tyr Gly Ser Tyr Ala Lys Pro Thr Asn Glu
                245                 250                 255

aat gga ggg caa ggc att ctt gta aag caa caa aat gga aag cta gaa 816
Asn Gly Gly Gln Gly Ile Leu Val Lys Gln Gln Asn Gly Lys Leu Glu
            260                 265                 270

agt caa gtg gaa atg caa ttt ttc tca act act gag gca gcc gca ggc 864
Ser Gln Val Glu Met Gln Phe Phe Ser Thr Thr Glu Ala Ala Ala Gly
        275                 280                 285

aat ggt gat aac ttg act cct aaa gtg gta ttg tac agt gaa gat gta 912
Asn Gly Asp Asn Leu Thr Pro Lys Val Val Leu Tyr Ser Glu Asp Val
    290                 295                 300

gat ata gaa acc cca gac act cat att tct tac atg ccc act att aag 960
Asp Ile Glu Thr Pro Asp Thr His Ile Ser Tyr Met Pro Thr Ile Lys
305                 310                 315                 320

gaa ggt aac tca cga gaa cta atg ggc caa caa tct atg ccc aac agg 1008
Glu Gly Asn Ser Arg Glu Leu Met Gly Gln Gln Ser Met Pro Asn Arg
                325                 330                 335

cct aat tac att gct ttt agg gac aat ttt att ggt cta atg tat tac 1056
Pro Asn Tyr Ile Ala Phe Arg Asp Asn Phe Ile Gly Leu Met Tyr Tyr
            340                 345                 350

aac agc acg ggt aat atg ggt gtt ctg gcg ggc caa gca tcg cag ttg 1104
Asn Ser Thr Gly Asn Met Gly Val Leu Ala Gly Gln Ala Ser Gln Leu
        355                 360                 365

aat gct gtt gta gat ttg caa gac aga aac aca gag ctt tca tac cag 1152
Asn Ala Val Val Asp Leu Gln Asp Arg Asn Thr Glu Leu Ser Tyr Gln
    370                 375                 380

ctt ttg ctt gat tcc att ggt gat aga acc agg tac ttt tct atg tgg 1200
Leu Leu Leu Asp Ser Ile Gly Asp Arg Thr Arg Tyr Phe Ser Met Trp
385                 390                 395                 400

aat cag ctt cca aat tac tgc ttt cca ctg gga ggt gtg att aat aca 1248
Asn Gln Leu Pro Asn Tyr Cys Phe Pro Leu Gly Gly Val Ile Asn Thr
                405                 410                 415

gag act ctt acc aag gta aaa cct aaa aca ggt cag gaa aat gga tgg 1296
Glu Thr Leu Thr Lys Val Lys Pro Lys Thr Gly Gln Glu Asn Gly Trp
            420                 425                 430

gaa aaa gat gct aca gaa ttt tca gat aaa aat gaa ata aga gtt gga 1344
Glu Lys Asp Ala Thr Glu Phe Ser Asp Lys Asn Glu Ile Arg Val Gly
        435                 440                 445

aat aat ttt gcc atg gaa atc aat cta aat gcc aac ctg tgg aga aat 1392
Asn Asn Phe Ala Met Glu Ile Asn Leu Asn Ala Asn Leu Trp Arg Asn
    450                 455                 460

ttc ctg tac tcc aac ata gcg ctg tat ttg ccc gac aag cta aag tac 1440
Phe Leu Tyr Ser Asn Ile Ala Leu Tyr Leu Pro Asp Lys Leu Lys Tyr
465                 470                 475                 480

agt cct tcc aac gta aaa att tct gat aac cca aac acc tac gac tac 1488
Ser Pro Ser Asn Val Lys Ile Ser Asp Asn Pro Asn Thr Tyr Asp Tyr
                485                 490                 495

atg aac aag cga gtg gtg gct ccc ggg cta gtg gac tgc tac att aac 1536
Met Asn Lys Arg Val Val Ala Pro Gly Leu Val Asp Cys Tyr Ile Asn
            500                 505                 510

ctt gga gca cgc tgg tcc ctt gac tat atg gac aac gtc aac cca ttt 1584
Leu Gly Ala Arg Trp Ser Leu Asp Tyr Met Asp Asn Val Asn Pro Phe
        515                 520                 525

aac cac cac cgc aat gct ggc ctg cgc tac cgc tca atg ttg ctg ggc 1632
Asn His His Arg Asn Ala Gly Leu Arg Tyr Arg Ser Met Leu Leu Gly
    530                 535                 540

aat ggt cgc tat gtg ccc ttc cac atc cag gtg cct cag aag ttc ttt 1680
Asn Gly Arg Tyr Val Pro Phe His Ile Gln Val Pro Gln Lys Phe Phe
545                 550                 555                 560

gcc att aaa aac ctc ctt ctc ctg ccg ggc tca tac acc tac gag tgg 1728
Ala Ile Lys Asn Leu Leu Leu Leu Pro Gly Ser Tyr Thr Tyr Glu Trp

565                 570                 575

aac ttc agg aag gat gtt aac atg gtt ctg cag agc tcc cta gga aat     1776
Asn Phe Arg Lys Asp Val Asn Met Val Leu Gln Ser Ser Leu Gly Asn
            580                 585                 590

gac cta agg gtt gac gga gcc agc att aag ttt gat agc att tgc ctt     1824
Asp Leu Arg Val Asp Gly Ala Ser Ile Lys Phe Asp Ser Ile Cys Leu
        595                 600                 605

tac gcc acc ttc ttc ccc atg gcc cac aac acc gcc tcc acg ctt gag     1872
Tyr Ala Thr Phe Phe Pro Met Ala His Asn Thr Ala Ser Thr Leu Glu
    610                 615                 620

gcc atg ctt aga aac gac acc aac gac cag tcc ttt aac gac tat ctc     1920
Ala Met Leu Arg Asn Asp Thr Asn Asp Gln Ser Phe Asn Asp Tyr Leu
625                 630                 635                 640

tcc gcc gcc aac atg ctc tac cct ata ccc gcc aac gct acc aac gtg     1968
Ser Ala Ala Asn Met Leu Tyr Pro Ile Pro Ala Asn Ala Thr Asn Val
                645                 650                 655

ccc ata tcc atc ccc tcc cgc aac tgg gcg gct ttc cgc ggc tgg gcc     2016
Pro Ile Ser Ile Pro Ser Arg Asn Trp Ala Ala Phe Arg Gly Trp Ala
            660                 665                 670

ttc acg cgc ctt aag act aag gaa acc cca tca ctg ggc tcg ggc tac     2064
Phe Thr Arg Leu Lys Thr Lys Glu Thr Pro Ser Leu Gly Ser Gly Tyr
        675                 680                 685

gac cct tat tac acc tac tct ggc tct ata ccc tac cta gat gga acc     2112
Asp Pro Tyr Tyr Thr Tyr Ser Gly Ser Ile Pro Tyr Leu Asp Gly Thr
    690                 695                 700

ttt tac ctc aac cac acc ttt aag aag gtg gcc att acc ttt gac tct     2160
Phe Tyr Leu Asn His Thr Phe Lys Lys Val Ala Ile Thr Phe Asp Ser
705                 710                 715                 720

tct gtc agc tgg cct ggc aat gac cgc ctg ctt acc ccc aac gag ttt     2208
Ser Val Ser Trp Pro Gly Asn Asp Arg Leu Leu Thr Pro Asn Glu Phe
                725                 730                 735

gaa att aag cgc tca gtt gac ggg gag ggt tac aac gtt gcc cag tgt     2256
Glu Ile Lys Arg Ser Val Asp Gly Glu Gly Tyr Asn Val Ala Gln Cys
            740                 745                 750

aac atg acc aaa gac tgg ttc ctg gta caa atg cta gct aac tat aac     2304
Asn Met Thr Lys Asp Trp Phe Leu Val Gln Met Leu Ala Asn Tyr Asn
        755                 760                 765

att ggc tac cag ggc ttc tat atc cca gag agc tac aag gac cgc atg     2352
Ile Gly Tyr Gln Gly Phe Tyr Ile Pro Glu Ser Tyr Lys Asp Arg Met
    770                 775                 780

tac tcc ttc ttt aga aac ttc cag ccc atg agc cgt cag gtg gtg gat     2400
Tyr Ser Phe Phe Arg Asn Phe Gln Pro Met Ser Arg Gln Val Val Asp
785                 790                 795                 800

gat act aaa tac aag gac tac caa cag gtg ggc atc cta cac caa cac     2448
Asp Thr Lys Tyr Lys Asp Tyr Gln Gln Val Gly Ile Leu His Gln His
                805                 810                 815

aac aac tct gga ttt gtt ggc tac ctt gcc ccc acc atg cgc gaa gga     2496

Asn Asn Ser Gly Phe Val Gly Tyr Leu Ala Pro Thr Met Arg Glu Gly
            820                 825                 830

cag gcc tac cct gct aac ttc ccc tat ccg ctt ata ggc aag acc gca      2544
Gln Ala Tyr Pro Ala Asn Phe Pro Tyr Pro Leu Ile Gly Lys Thr Ala
        835                 840                 845

gtt gac agc att acc cag aaa aag ttt ctt tgc gat cgc acc ctt tgg      2592
Val Asp Ser Ile Thr Gln Lys Lys Phe Leu Cys Asp Arg Thr Leu Trp
    850                 855                 860

cgc atc cca ttc tcc agt aac ttt atg tcc atg ggc gca ctc aca gac      2640
Arg Ile Pro Phe Ser Ser Asn Phe Met Ser Met Gly Ala Leu Thr Asp
865                 870                 875                 880

ctg ggc caa aac ctt ctc tac gcc aac tcc gcc cac gcg cta gac atg      2688
Leu Gly Gln Asn Leu Leu Tyr Ala Asn Ser Ala His Ala Leu Asp Met
                885                 890                 895

act ttt gag gtg gat ccc atg gac gag ccc acc ctt ctt tat gtt ttg      2736
Thr Phe Glu Val Asp Pro Met Asp Glu Pro Thr Leu Leu Tyr Val Leu
            900                 905                 910

ttt gaa gtc ttt gac gtg gtc cgt gtg cac cag ccg cac cgc ggc gtc      2784
Phe Glu Val Phe Asp Val Val Arg Val His Gln Pro His Arg Gly Val
        915                 920                 925

atc gaa acc gtg tac ctg cgc acg ccc ttc tcg gcc ggc aac gcc aca      2832
Ile Glu Thr Val Tyr Leu Arg Thr Pro Phe Ser Ala Gly Asn Ala Thr
    930                 935                 940

aca taa                                                              2838
Thr
945



<210>  16
<211>  2862
<212>  DNA
<213>  Artificial Sequence

<220>
<223>  (NANP)12-HVR1 modified Hexon protein


<220>
<221>  CDS
<222>  (1)..(2859)

<400>  16
atg gct acc cct tcg atg atg ccg cag tgg tct tac atg cac atc tcg        48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc        96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc       144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg     192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac     240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg     288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act     336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt     384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat     432
Ala Pro Asn Pro Cys Glu Trp Asp Glu Asn Ala Asn Pro Asn Ala Asn
    130                 135                 140

cca aat gca aat cca aat gca aat cca aat gca aat cca aat gca aat     480
Pro Asn Ala Asn Pro Asn Ala Asn Pro Asn Ala Asn Pro Asn Ala Asn
145                 150                 155                 160

cca aat gca aat cca aat gca aat cca aac gcg aac ccg aat gct aat     528
Pro Asn Ala Asn Pro Asn Ala Asn Pro Asn Ala Asn Pro Asn Ala Asn
                165                 170                 175

cct aat gca aat cca aat gca aat cca aaa act cac gta ttt ggg cag     576
Pro Asn Ala Asn Pro Asn Ala Asn Pro Lys Thr His Val Phe Gly Gln
            180                 185                 190

gcg cct tat tct ggt ata aat att aca aag gag ggt att caa ata ggt     624
Ala Pro Tyr Ser Gly Ile Asn Ile Thr Lys Glu Gly Ile Gln Ile Gly
        195                 200                 205

gtc gaa ggt caa aca cct aaa tat gcc gat aaa aca ttt caa cct gaa     672
Val Glu Gly Gln Thr Pro Lys Tyr Ala Asp Lys Thr Phe Gln Pro Glu
    210                 215                 220

cct caa ata gga gaa tct cag tgg tac gaa aca gaa att aat cat gca     720
Pro Gln Ile Gly Glu Ser Gln Trp Tyr Glu Thr Glu Ile Asn His Ala
225                 230                 235                 240

gct ggg aga gtc cta aaa aag act acc cca atg aaa cca tgt tac ggt     768
Ala Gly Arg Val Leu Lys Lys Thr Thr Pro Met Lys Pro Cys Tyr Gly
                245                 250                 255

tca tat gca aaa ccc aca aat gaa aat gga ggg caa ggc att ctt gta     816
Ser Tyr Ala Lys Pro Thr Asn Glu Asn Gly Gly Gln Gly Ile Leu Val
            260                 265                 270

aag caa caa aat gga aag cta gaa agt caa gtg gaa atg caa ttt ttc     864
Lys Gln Gln Asn Gly Lys Leu Glu Ser Gln Val Glu Met Gln Phe Phe
        275                 280                 285

tca act act gag gca gcc gca ggc aat ggt gat aac ttg act cct aaa     912
Ser Thr Thr Glu Ala Ala Ala Gly Asn Gly Asp Asn Leu Thr Pro Lys
    290                 295                 300

gtg gta ttg tac agt gaa gat gta gat ata gaa acc cca gac act cat     960
Val Val Leu Tyr Ser Glu Asp Val Asp Ile Glu Thr Pro Asp Thr His
305                 310                 315                 320

att tct tac atg ccc act att aag gaa ggt aac tca cga gaa cta atg     1008
Ile Ser Tyr Met Pro Thr Ile Lys Glu Gly Asn Ser Arg Glu Leu Met
                325                 330                 335

ggc caa caa tct atg ccc aac agg cct aat tac att gct ttt agg gac     1056
Gly Gln Gln Ser Met Pro Asn Arg Pro Asn Tyr Ile Ala Phe Arg Asp
            340                 345                 350

aat ttt att ggt cta atg tat tac aac agc acg ggt aat atg ggt gtt     1104
Asn Phe Ile Gly Leu Met Tyr Tyr Asn Ser Thr Gly Asn Met Gly Val
        355                 360                 365

ctg gcg ggc caa gca tcg cag ttg aat gct gtt gta gat ttg caa gac     1152
Leu Ala Gly Gln Ala Ser Gln Leu Asn Ala Val Val Asp Leu Gln Asp
    370                 375                 380

aga aac aca gag ctt tca tac cag ctt ttg ctt gat tcc att ggt gat     1200
Arg Asn Thr Glu Leu Ser Tyr Gln Leu Leu Leu Asp Ser Ile Gly Asp
385                 390                 395                 400

aga acc agg tac ttt tct atg tgg aat cag ctt cca aat tac tgc ttt     1248
Arg Thr Arg Tyr Phe Ser Met Trp Asn Gln Leu Pro Asn Tyr Cys Phe
                405                 410                 415

cca ctg gga ggt gtg att aat aca gag act ctt acc aag gta aaa cct     1296
Pro Leu Gly Gly Val Ile Asn Thr Glu Thr Leu Thr Lys Val Lys Pro
            420                 425                 430

aaa aca ggt cag gaa aat gga tgg gaa aaa gat gct aca gaa ttt tca     1344
Lys Thr Gly Gln Glu Asn Gly Trp Glu Lys Asp Ala Thr Glu Phe Ser
        435                 440                 445

gat aaa aat gaa ata aga gtt gga aat aat ttt gcc atg gaa atc aat     1392
Asp Lys Asn Glu Ile Arg Val Gly Asn Asn Phe Ala Met Glu Ile Asn
    450                 455                 460

cta aat gcc aac ctg tgg aga aat ttc ctg tac tcc aac ata gcg ctg     1440
Leu Asn Ala Asn Leu Trp Arg Asn Phe Leu Tyr Ser Asn Ile Ala Leu
465                 470                 475                 480

tat ttg ccc gac aag cta aag tac agt cct tcc aac gta aaa att tct     1488
Tyr Leu Pro Asp Lys Leu Lys Tyr Ser Pro Ser Asn Val Lys Ile Ser
                485                 490                 495

gat aac cca aac acc tac gac tac atg aac aag cga gtg gtg gct ccc     1536
Asp Asn Pro Asn Thr Tyr Asp Tyr Met Asn Lys Arg Val Val Ala Pro
            500                 505                 510

ggg cta gtg gac tgc tac att aac ctt gga gca cgc tgg tcc ctt gac     1584
Gly Leu Val Asp Cys Tyr Ile Asn Leu Gly Ala Arg Trp Ser Leu Asp
        515                 520                 525

tat atg gac aac gtc aac cca ttt aac cac cac cgc aat gct ggc ctg     1632
Tyr Met Asp Asn Val Asn Pro Phe Asn His His Arg Asn Ala Gly Leu
    530                 535                 540

cgc tac cgc tca atg ttg ctg ggc aat ggt cgc tat gtg ccc ttc cac     1680
Arg Tyr Arg Ser Met Leu Leu Gly Asn Gly Arg Tyr Val Pro Phe His

545                 550                 555                 560

atc cag gtg cct cag aag ttc ttt gcc att aaa aac ctc ctt ctc ctg     1728
Ile Gln Val Pro Gln Lys Phe Phe Ala Ile Lys Asn Leu Leu Leu Leu
                565                 570                 575

ccg ggc tca tac acc tac gag tgg aac ttc agg aag gat gtt aac atg     1776
Pro Gly Ser Tyr Thr Tyr Glu Trp Asn Phe Arg Lys Asp Val Asn Met
            580                 585                 590

gtt ctg cag agc tcc cta gga aat gac cta agg gtt gac gga gcc agc     1824
Val Leu Gln Ser Ser Leu Gly Asn Asp Leu Arg Val Asp Gly Ala Ser
        595                 600                 605

att aag ttt gat agc att tgc ctt tac gcc acc ttc ttc ccc atg gcc     1872
Ile Lys Phe Asp Ser Ile Cys Leu Tyr Ala Thr Phe Phe Pro Met Ala
    610                 615                 620

cac aac acc gcc tcc acg ctt gag gcc atg ctt aga aac gac acc aac     1920
His Asn Thr Ala Ser Thr Leu Glu Ala Met Leu Arg Asn Asp Thr Asn
625                 630                 635                 640

gac cag tcc ttt aac gac tat ctc tcc gcc gcc aac atg ctc tac cct     1968
Asp Gln Ser Phe Asn Asp Tyr Leu Ser Ala Ala Asn Met Leu Tyr Pro
                645                 650                 655

ata ccc gcc aac gct acc aac gtg ccc ata tcc atc ccc tcc cgc aac     2016
Ile Pro Ala Asn Ala Thr Asn Val Pro Ile Ser Ile Pro Ser Arg Asn
            660                 665                 670

tgg gcg gct ttc cgc ggc tgg gcc ttc acg cgc ctt aag act aag gaa     2064
Trp Ala Ala Phe Arg Gly Trp Ala Phe Thr Arg Leu Lys Thr Lys Glu
        675                 680                 685

acc cca tca ctg ggc tcg ggc tac gac cct tat tac acc tac tct ggc     2112
Thr Pro Ser Leu Gly Ser Gly Tyr Asp Pro Tyr Tyr Thr Tyr Ser Gly
    690                 695                 700

tct ata ccc tac cta gat gga acc ttt tac ctc aac cac acc ttt aag     2160
Ser Ile Pro Tyr Leu Asp Gly Thr Phe Tyr Leu Asn His Thr Phe Lys
705                 710                 715                 720

aag gtg gcc att acc ttt gac tct tct gtc agc tgg cct ggc aat gac     2208
Lys Val Ala Ile Thr Phe Asp Ser Ser Val Ser Trp Pro Gly Asn Asp
                725                 730                 735

cgc ctg ctt acc ccc aac gag ttt gaa att aag cgc tca gtt gac ggg     2256
Arg Leu Leu Thr Pro Asn Glu Phe Glu Ile Lys Arg Ser Val Asp Gly
            740                 745                 750

gag ggt tac aac gtt gcc cag tgt aac atg acc aaa gac tgg ttc ctg     2304
Glu Gly Tyr Asn Val Ala Gln Cys Asn Met Thr Lys Asp Trp Phe Leu
        755                 760                 765

gta caa atg cta gct aac tat aac att ggc tac cag ggc ttc tat atc     2352
Val Gln Met Leu Ala Asn Tyr Asn Ile Gly Tyr Gln Gly Phe Tyr Ile
    770                 775                 780

cca gag agc tac aag gac cgc atg tac tcc ttc ttt aga aac ttc cag     2400
Pro Glu Ser Tyr Lys Asp Arg Met Tyr Ser Phe Phe Arg Asn Phe Gln
785                 790                 795                 800

ccc atg agc cgt cag gtg gtg gat gat act aaa tac aag gac tac caa     2448

Pro Met Ser Arg Gln Val Val Asp Asp Thr Lys Tyr Lys Asp Tyr Gln
                805                 810                 815

cag gtg ggc atc cta cac caa cac aac aac tct gga ttt gtt ggc tac      2496
Gln Val Gly Ile Leu His Gln His Asn Asn Ser Gly Phe Val Gly Tyr
            820                 825                 830

ctt gcc ccc acc atg cgc gaa gga cag gcc tac cct gct aac ttc ccc      2544
Leu Ala Pro Thr Met Arg Glu Gly Gln Ala Tyr Pro Ala Asn Phe Pro
        835                 840                 845

tat ccg ctt ata ggc aag acc gca gtt gac agc att acc cag aaa aag      2592
Tyr Pro Leu Ile Gly Lys Thr Ala Val Asp Ser Ile Thr Gln Lys Lys
    850                 855                 860

ttt ctt tgc gat cgc acc ctt tgg cgc atc cca ttc tcc agt aac ttt      2640
Phe Leu Cys Asp Arg Thr Leu Trp Arg Ile Pro Phe Ser Ser Asn Phe
865                 870                 875                 880

atg tcc atg ggc gca ctc aca gac ctg ggc caa aac ctt ctc tac gcc      2688
Met Ser Met Gly Ala Leu Thr Asp Leu Gly Gln Asn Leu Leu Tyr Ala
                885                 890                 895

aac tcc gcc cac gcg cta gac atg act ttt gag gtg gat ccc atg gac      2736
Asn Ser Ala His Ala Leu Asp Met Thr Phe Glu Val Asp Pro Met Asp
            900                 905                 910

gag ccc acc ctt ctt tat gtt ttg ttt gaa gtc ttt gac gtg gtc cgt      2784
Glu Pro Thr Leu Leu Tyr Val Leu Phe Glu Val Phe Asp Val Val Arg
        915                 920                 925

gtg cac cag ccg cac cgc ggc gtc atc gaa acc gtg tac ctg cgc acg      2832
Val His Gln Pro His Arg Gly Val Ile Glu Thr Val Tyr Leu Arg Thr
    930                 935                 940

ccc ttc tcg gcc ggc aac gcc aca aca taa                              2862
Pro Phe Ser Ala Gly Asn Ala Thr Thr
945                 950

<210>  17
<211>  2886
<212>  DNA
<213>  Artificial Sequence

<220>
<223>  (NANP)14-HVR1 modified Hexon protein

<220>
<221>  CDS
<222>  (1)..(2883)

<400>  17
atg gct acc cct tcg atg atg ccg cag tgg tct tac atg cac atc tcg        48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc        96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc     144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg     192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac     240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg     288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act     336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt     384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat     432
Ala Pro Asn Pro Cys Glu Trp Asp Glu Asn Ala Asn Pro Asn Ala Asn
    130                 135                 140

cca aat gca aat cca aat gca aat cca aat gca aat cca aat gca aat     480
Pro Asn Ala Asn Pro Asn Ala Asn Pro Asn Ala Asn Pro Asn Ala Asn
145                 150                 155                 160

cca aat gca aat cca aat gca aat cca aac gcg aac ccg aat gct aat     528
Pro Asn Ala Asn Pro Asn Ala Asn Pro Asn Ala Asn Pro Asn Ala Asn
                165                 170                 175

cct aat gca aat cca aat gca aat cca aat gca aat cca aat gca aat     576
Pro Asn Ala Asn Pro Asn Ala Asn Pro Asn Ala Asn Pro Asn Ala Asn
            180                 185                 190

cca aaa act cac gta ttt ggg cag gcg cct tat tct ggt ata aat att     624
Pro Lys Thr His Val Phe Gly Gln Ala Pro Tyr Ser Gly Ile Asn Ile
        195                 200                 205

aca aag gag ggt att caa ata ggt gtc gaa ggt caa aca cct aaa tat     672
Thr Lys Glu Gly Ile Gln Ile Gly Val Glu Gly Gln Thr Pro Lys Tyr
    210                 215                 220

gcc gat aaa aca ttt caa cct gaa cct caa ata gga gaa tct cag tgg     720
Ala Asp Lys Thr Phe Gln Pro Glu Pro Gln Ile Gly Glu Ser Gln Trp
225                 230                 235                 240

tac gaa aca gaa att aat cat gca gct ggg aga gtc cta aaa aag act     768
Tyr Glu Thr Glu Ile Asn His Ala Ala Gly Arg Val Leu Lys Lys Thr
                245                 250                 255

acc cca atg aaa cca tgt tac ggt tca tat gca aaa ccc aca aat gaa     816
Thr Pro Met Lys Pro Cys Tyr Gly Ser Tyr Ala Lys Pro Thr Asn Glu
            260                 265                 270

aat gga ggg caa ggc att ctt gta aag caa caa aat gga aag cta gaa     864
Asn Gly Gly Gln Gly Ile Leu Val Lys Gln Gln Asn Gly Lys Leu Glu

275                 280                 285

agt caa gtg gaa atg caa ttt ttc tca act act gag gca gcc gca ggc      912
Ser Gln Val Glu Met Gln Phe Phe Ser Thr Thr Glu Ala Ala Ala Gly
    290                 295                 300

aat ggt gat aac ttg act cct aaa gtg gta ttg tac agt gaa gat gta      960
Asn Gly Asp Asn Leu Thr Pro Lys Val Val Leu Tyr Ser Glu Asp Val
305                 310                 315                 320

gat ata gaa acc cca gac act cat att tct tac atg ccc act att aag     1008
Asp Ile Glu Thr Pro Asp Thr His Ile Ser Tyr Met Pro Thr Ile Lys
                325                 330                 335

gaa ggt aac tca cga gaa cta atg ggc caa caa tct atg ccc aac agg     1056
Glu Gly Asn Ser Arg Glu Leu Met Gly Gln Gln Ser Met Pro Asn Arg
            340                 345                 350

cct aat tac att gct ttt agg gac aat ttt att ggt cta atg tat tac     1104
Pro Asn Tyr Ile Ala Phe Arg Asp Asn Phe Ile Gly Leu Met Tyr Tyr
        355                 360                 365

aac agc acg ggt aat atg ggt gtt ctg gcg ggc caa gca tcg cag ttg     1152
Asn Ser Thr Gly Asn Met Gly Val Leu Ala Gly Gln Ala Ser Gln Leu
    370                 375                 380

aat gct gtt gta gat ttg caa gac aga aac aca gag ctt tca tac cag     1200
Asn Ala Val Val Asp Leu Gln Asp Arg Asn Thr Glu Leu Ser Tyr Gln
385                 390                 395                 400

ctt ttg ctt gat tcc att ggt gat aga acc agg tac ttt tct atg tgg     1248
Leu Leu Leu Asp Ser Ile Gly Asp Arg Thr Arg Tyr Phe Ser Met Trp
                405                 410                 415

aat cag ctt cca aat tac tgc ttt cca ctg gga ggt gtg att aat aca     1296
Asn Gln Leu Pro Asn Tyr Cys Phe Pro Leu Gly Gly Val Ile Asn Thr
            420                 425                 430

gag act ctt acc aag gta aaa cct aaa aca ggt cag gaa aat gga tgg     1344
Glu Thr Leu Thr Lys Val Lys Pro Lys Thr Gly Gln Glu Asn Gly Trp
        435                 440                 445

gaa aaa gat gct aca gaa ttt tca gat aaa aat gaa ata aga gtt gga     1392
Glu Lys Asp Ala Thr Glu Phe Ser Asp Lys Asn Glu Ile Arg Val Gly
    450                 455                 460

aat aat ttt gcc atg gaa atc aat cta aat gcc aac ctg tgg aga aat     1440
Asn Asn Phe Ala Met Glu Ile Asn Leu Asn Ala Asn Leu Trp Arg Asn
465                 470                 475                 480

ttc ctg tac tcc aac ata gcg ctg tat ttg ccc gac aag cta aag tac     1488
Phe Leu Tyr Ser Asn Ile Ala Leu Tyr Leu Pro Asp Lys Leu Lys Tyr
                485                 490                 495

agt cct tcc aac gta aaa att tct gat aac cca aac acc tac gac tac     1536
Ser Pro Ser Asn Val Lys Ile Ser Asp Asn Pro Asn Thr Tyr Asp Tyr
            500                 505                 510

atg aac aag cga gtg gtg gct ccc ggg cta gtg gac tgc tac att aac     1584
Met Asn Lys Arg Val Val Ala Pro Gly Leu Val Asp Cys Tyr Ile Asn
        515                 520                 525

ctt gga gca cgc tgg tcc ctt gac tat atg gac aac gtc aac cca ttt     1632

Leu Gly Ala Arg Trp Ser Leu Asp Tyr Met Asp Asn Val Asn Pro Phe
    530                 535                 540

aac cac cac cgc aat gct ggc ctg cgc tac cgc tca atg ttg ctg ggc 1680
Asn His His Arg Asn Ala Gly Leu Arg Tyr Arg Ser Met Leu Leu Gly
545                 550                 555                 560

aat ggt cgc tat gtg ccc ttc cac atc cag gtg cct cag aag ttc ttt 1728
Asn Gly Arg Tyr Val Pro Phe His Ile Gln Val Pro Gln Lys Phe Phe
                565                 570                 575

gcc att aaa aac ctc ctt ctc ctg ccg ggc tca tac acc tac gag tgg 1776
Ala Ile Lys Asn Leu Leu Leu Leu Pro Gly Ser Tyr Thr Tyr Glu Trp
            580                 585                 590

aac ttc agg aag gat gtt aac atg gtt ctg cag agc tcc cta gga aat 1824
Asn Phe Arg Lys Asp Val Asn Met Val Leu Gln Ser Ser Leu Gly Asn
        595                 600                 605

gac cta agg gtt gac gga gcc agc att aag ttt gat agc att tgc ctt 1872
Asp Leu Arg Val Asp Gly Ala Ser Ile Lys Phe Asp Ser Ile Cys Leu
    610                 615                 620

tac gcc acc ttc ttc ccc atg gcc cac aac acc gcc tcc acg ctt gag 1920
Tyr Ala Thr Phe Phe Pro Met Ala His Asn Thr Ala Ser Thr Leu Glu
625                 630                 635                 640

gcc atg ctt aga aac gac acc aac gac cag tcc ttt aac gac tat ctc 1968
Ala Met Leu Arg Asn Asp Thr Asn Asp Gln Ser Phe Asn Asp Tyr Leu
                645                 650                 655

tcc gcc gcc aac atg ctc tac cct ata ccc gcc aac gct acc aac gtg 2016
Ser Ala Ala Asn Met Leu Tyr Pro Ile Pro Ala Asn Ala Thr Asn Val
            660                 665                 670

ccc ata tcc atc ccc tcc cgc aac tgg gcg gct ttc cgc ggc tgg gcc 2064
Pro Ile Ser Ile Pro Ser Arg Asn Trp Ala Ala Phe Arg Gly Trp Ala
        675                 680                 685

ttc acg cgc ctt aag act aag gaa acc cca tca ctg ggc tcg ggc tac 2112
Phe Thr Arg Leu Lys Thr Lys Glu Thr Pro Ser Leu Gly Ser Gly Tyr
    690                 695                 700

gac cct tat tac acc tac tct ggc tct ata ccc tac cta gat gga acc 2160
Asp Pro Tyr Tyr Thr Tyr Ser Gly Ser Ile Pro Tyr Leu Asp Gly Thr
705                 710                 715                 720

ttt tac ctc aac cac acc ttt aag aag gtg gcc att acc ttt gac tct 2208
Phe Tyr Leu Asn His Thr Phe Lys Lys Val Ala Ile Thr Phe Asp Ser
                725                 730                 735

tct gtc agc tgg cct ggc aat gac cgc ctg ctt acc ccc aac gag ttt 2256
Ser Val Ser Trp Pro Gly Asn Asp Arg Leu Leu Thr Pro Asn Glu Phe
            740                 745                 750

gaa att aag cgc tca gtt gac ggg gag ggt tac aac gtt gcc cag tgt 2304
Glu Ile Lys Arg Ser Val Asp Gly Glu Gly Tyr Asn Val Ala Gln Cys
        755                 760                 765

aac atg acc aaa gac tgg ttc ctg gta caa atg cta gct aac tat aac 2352
Asn Met Thr Lys Asp Trp Phe Leu Val Gln Met Leu Ala Asn Tyr Asn
    770                 775                 780

att ggc tac cag ggc ttc tat atc cca gag agc tac aag gac cgc atg          2400
Ile Gly Tyr Gln Gly Phe Tyr Ile Pro Glu Ser Tyr Lys Asp Arg Met
785                 790                 795                 800

tac tcc ttc ttt aga aac ttc cag ccc atg agc cgt cag gtg gtg gat          2448
Tyr Ser Phe Phe Arg Asn Phe Gln Pro Met Ser Arg Gln Val Val Asp
                805                 810                 815

gat act aaa tac aag gac tac caa cag gtg ggc atc cta cac caa cac          2496
Asp Thr Lys Tyr Lys Asp Tyr Gln Gln Val Gly Ile Leu His Gln His
            820                 825                 830

aac aac tct gga ttt gtt ggc tac ctt gcc ccc acc atg cgc gaa gga          2544
Asn Asn Ser Gly Phe Val Gly Tyr Leu Ala Pro Thr Met Arg Glu Gly
        835                 840                 845

cag gcc tac cct gct aac ttc ccc tat ccg ctt ata ggc aag acc gca          2592
Gln Ala Tyr Pro Ala Asn Phe Pro Tyr Pro Leu Ile Gly Lys Thr Ala
    850                 855                 860

gtt gac agc att acc cag aaa aag ttt ctt tgc gat cgc acc ctt tgg          2640
Val Asp Ser Ile Thr Gln Lys Lys Phe Leu Cys Asp Arg Thr Leu Trp
865                 870                 875                 880

cgc atc cca ttc tcc agt aac ttt atg tcc atg ggc gca ctc aca gac          2688
Arg Ile Pro Phe Ser Ser Asn Phe Met Ser Met Gly Ala Leu Thr Asp
                885                 890                 895

ctg ggc caa aac ctt ctc tac gcc aac tcc gcc cac gcg cta gac atg          2736
Leu Gly Gln Asn Leu Leu Tyr Ala Asn Ser Ala His Ala Leu Asp Met
            900                 905                 910

act ttt gag gtg gat ccc atg gac gag ccc acc ctt ctt tat gtt ttg          2784
Thr Phe Glu Val Asp Pro Met Asp Glu Pro Thr Leu Leu Tyr Val Leu
        915                 920                 925

ttt gaa gtc ttt gac gtg gtc cgt gtg cac cag ccg cac cgc ggc gtc          2832
Phe Glu Val Phe Asp Val Val Arg Val His Gln Pro His Arg Gly Val
    930                 935                 940

atc gaa acc gtg tac ctg cgc acg ccc ttc tcg gcc ggc aac gcc aca          2880
Ile Glu Thr Val Tyr Leu Arg Thr Pro Phe Ser Ala Gly Asn Ala Thr
945                 950                 955                 960

aca taa                                                                  2886
Thr


<210>  18
<211>  2910
<212>  DNA
<213>  Artificial Sequence

<220>
<223>  (NANP)16-HVR1 modified Hexon protein


<220>
<221>  CDS
<222>  (1)..(2907)

<400>  18
atg gct acc cct tcg atg atg ccg cag tgg tct tac atg cac atc tcg          48

Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat      432
Ala Pro Asn Pro Cys Glu Trp Asp Glu Asn Ala Asn Pro Asn Ala Asn
    130                 135                 140

cca aat gca aat cca aat gca aat cca aat gca aat cca aat gca aat      480
Pro Asn Ala Asn Pro Asn Ala Asn Pro Asn Ala Asn Pro Asn Ala Asn
145                 150                 155                 160

cca aat gca aat cca aat gca aat cca aac gcg aac ccg aat gct aat      528
Pro Asn Ala Asn Pro Asn Ala Asn Pro Asn Ala Asn Pro Asn Ala Asn
                165                 170                 175

cct aat gca aat cca aat gca aat cca aat gca aat cca aat gca aat      576
Pro Asn Ala Asn Pro Asn Ala Asn Pro Asn Ala Asn Pro Asn Ala Asn
            180                 185                 190

cca aat gca aat cca aat gca aat cca aaa act cac gta ttt ggg cag      624
Pro Asn Ala Asn Pro Asn Ala Asn Pro Lys Thr His Val Phe Gly Gln
        195                 200                 205

gcg cct tat tct ggt ata aat att aca aag gag ggt att caa ata ggt      672
Ala Pro Tyr Ser Gly Ile Asn Ile Thr Lys Glu Gly Ile Gln Ile Gly
    210                 215                 220

gtc gaa ggt caa aca cct aaa tat gcc gat aaa aca ttt caa cct gaa      720
Val Glu Gly Gln Thr Pro Lys Tyr Ala Asp Lys Thr Phe Gln Pro Glu
225                 230                 235                 240

cct caa ata gga gaa tct cag tgg tac gaa aca gaa att aat cat gca      768
Pro Gln Ile Gly Glu Ser Gln Trp Tyr Glu Thr Glu Ile Asn His Ala
                245                 250                 255

gct ggg aga gtc cta aaa aag act acc cca atg aaa cca tgt tac ggt 816
Ala Gly Arg Val Leu Lys Lys Thr Thr Pro Met Lys Pro Cys Tyr Gly
            260                 265                 270

tca tat gca aaa ccc aca aat gaa aat gga ggg caa ggc att ctt gta 864
Ser Tyr Ala Lys Pro Thr Asn Glu Asn Gly Gly Gln Gly Ile Leu Val
        275                 280                 285

aag caa caa aat gga aag cta gaa agt caa gtg gaa atg caa ttt ttc 912
Lys Gln Gln Asn Gly Lys Leu Glu Ser Gln Val Glu Met Gln Phe Phe
    290                 295                 300

tca act act gag gca gcc gca ggc aat ggt gat aac ttg act cct aaa 960
Ser Thr Thr Glu Ala Ala Ala Gly Asn Gly Asp Asn Leu Thr Pro Lys
305                 310                 315                 320

gtg gta ttg tac agt gaa gat gta gat ata gaa acc cca gac act cat 1008
Val Val Leu Tyr Ser Glu Asp Val Asp Ile Glu Thr Pro Asp Thr His
                325                 330                 335

att tct tac atg ccc act att aag gaa ggt aac tca cga gaa cta atg 1056
Ile Ser Tyr Met Pro Thr Ile Lys Glu Gly Asn Ser Arg Glu Leu Met
            340                 345                 350

ggc caa caa tct atg ccc aac agg cct aat tac att gct ttt agg gac 1104
Gly Gln Gln Ser Met Pro Asn Arg Pro Asn Tyr Ile Ala Phe Arg Asp
        355                 360                 365

aat ttt att ggt cta atg tat tac aac agc acg ggt aat atg ggt gtt 1152
Asn Phe Ile Gly Leu Met Tyr Tyr Asn Ser Thr Gly Asn Met Gly Val
    370                 375                 380

ctg gcg ggc caa gca tcg cag ttg aat gct gtt gta gat ttg caa gac 1200
Leu Ala Gly Gln Ala Ser Gln Leu Asn Ala Val Val Asp Leu Gln Asp
385                 390                 395                 400

aga aac aca gag ctt tca tac cag ctt ttg ctt gat tcc att ggt gat 1248
Arg Asn Thr Glu Leu Ser Tyr Gln Leu Leu Leu Asp Ser Ile Gly Asp
                405                 410                 415

aga acc agg tac ttt tct atg tgg aat cag ctt cca aat tac tgc ttt 1296
Arg Thr Arg Tyr Phe Ser Met Trp Asn Gln Leu Pro Asn Tyr Cys Phe
            420                 425                 430

cca ctg gga ggt gtg att aat aca gag act ctt acc aag gta aaa cct 1344
Pro Leu Gly Gly Val Ile Asn Thr Glu Thr Leu Thr Lys Val Lys Pro
        435                 440                 445

aaa aca ggt cag gaa aat gga tgg gaa aaa gat gct aca gaa ttt tca 1392
Lys Thr Gly Gln Glu Asn Gly Trp Glu Lys Asp Ala Thr Glu Phe Ser
    450                 455                 460

gat aaa aat gaa ata aga gtt gga aat aat ttt gcc atg gaa atc aat 1440
Asp Lys Asn Glu Ile Arg Val Gly Asn Asn Phe Ala Met Glu Ile Asn
465                 470                 475                 480

cta aat gcc aac ctg tgg aga aat ttc ctg tac tcc aac ata gcg ctg 1488
Leu Asn Ala Asn Leu Trp Arg Asn Phe Leu Tyr Ser Asn Ile Ala Leu
                485                 490                 495

tat ttg ccc gac aag cta aag tac agt cct tcc aac gta aaa att tct 1536
Tyr Leu Pro Asp Lys Leu Lys Tyr Ser Pro Ser Asn Val Lys Ile Ser
            500                 505                 510

gat aac cca aac acc tac gac tac atg aac aag cga gtg gtg gct ccc 1584
Asp Asn Pro Asn Thr Tyr Asp Tyr Met Asn Lys Arg Val Val Ala Pro
        515                 520                 525

ggg cta gtg gac tgc tac att aac ctt gga gca cgc tgg tcc ctt gac 1632
Gly Leu Val Asp Cys Tyr Ile Asn Leu Gly Ala Arg Trp Ser Leu Asp
    530                 535                 540

tat atg gac aac gtc aac cca ttt aac cac cac cgc aat gct ggc ctg 1680
Tyr Met Asp Asn Val Asn Pro Phe Asn His His Arg Asn Ala Gly Leu
545                 550                 555                 560

cgc tac cgc tca atg ttg ctg ggc aat ggt cgc tat gtg ccc ttc cac 1728
Arg Tyr Arg Ser Met Leu Leu Gly Asn Gly Arg Tyr Val Pro Phe His
                565                 570                 575

atc cag gtg cct cag aag ttc ttt gcc att aaa aac ctc ctt ctc ctg 1776
Ile Gln Val Pro Gln Lys Phe Phe Ala Ile Lys Asn Leu Leu Leu Leu
            580                 585                 590

ccg ggc tca tac acc tac gag tgg aac ttc agg aag gat gtt aac atg 1824
Pro Gly Ser Tyr Thr Tyr Glu Trp Asn Phe Arg Lys Asp Val Asn Met
        595                 600                 605

gtt ctg cag agc tcc cta gga aat gac cta agg gtt gac gga gcc agc 1872
Val Leu Gln Ser Ser Leu Gly Asn Asp Leu Arg Val Asp Gly Ala Ser
    610                 615                 620

att aag ttt gat agc att tgc ctt tac gcc acc ttc ttc ccc atg gcc 1920
Ile Lys Phe Asp Ser Ile Cys Leu Tyr Ala Thr Phe Phe Pro Met Ala
625                 630                 635                 640

cac aac acc gcc tcc acg ctt gag gcc atg ctt aga aac gac acc aac 1968
His Asn Thr Ala Ser Thr Leu Glu Ala Met Leu Arg Asn Asp Thr Asn
                645                 650                 655

gac cag tcc ttt aac gac tat ctc tcc gcc gcc aac atg ctc tac cct 2016
Asp Gln Ser Phe Asn Asp Tyr Leu Ser Ala Ala Asn Met Leu Tyr Pro
            660                 665                 670

ata ccc gcc aac gct acc aac gtg ccc ata tcc atc ccc tcc cgc aac 2064
Ile Pro Ala Asn Ala Thr Asn Val Pro Ile Ser Ile Pro Ser Arg Asn
        675                 680                 685

tgg gcg gct ttc cgc ggc tgg gcc ttc acg cgc ctt aag act aag gaa 2112
Trp Ala Ala Phe Arg Gly Trp Ala Phe Thr Arg Leu Lys Thr Lys Glu
    690                 695                 700

acc cca tca ctg ggc tcg ggc tac gac cct tat tac acc tac tct ggc 2160
Thr Pro Ser Leu Gly Ser Gly Tyr Asp Pro Tyr Tyr Thr Tyr Ser Gly
705                 710                 715                 720

tct ata ccc tac cta gat gga acc ttt tac ctc aac cac acc ttt aag 2208
Ser Ile Pro Tyr Leu Asp Gly Thr Phe Tyr Leu Asn His Thr Phe Lys
                725                 730                 735

aag gtg gcc att acc ttt gac tct tct gtc agc tgg cct ggc aat gac 2256
Lys Val Ala Ile Thr Phe Asp Ser Ser Val Ser Trp Pro Gly Asn Asp
            740                 745                 750

cgc ctg ctt acc ccc aac gag ttt gaa att aag cgc tca gtt gac ggg 2304
Arg Leu Leu Thr Pro Asn Glu Phe Glu Ile Lys Arg Ser Val Asp Gly

755                 760                 765

gag ggt tac aac gtt gcc cag tgt aac atg acc aaa gac tgg ttc ctg        2352
Glu Gly Tyr Asn Val Ala Gln Cys Asn Met Thr Lys Asp Trp Phe Leu
    770                 775                 780

gta caa atg cta gct aac tat aac att ggc tac cag ggc ttc tat atc        2400
Val Gln Met Leu Ala Asn Tyr Asn Ile Gly Tyr Gln Gly Phe Tyr Ile
785                 790                 795                 800

cca gag agc tac aag gac cgc atg tac tcc ttc ttt aga aac ttc cag        2448
Pro Glu Ser Tyr Lys Asp Arg Met Tyr Ser Phe Phe Arg Asn Phe Gln
                805                 810                 815

ccc atg agc cgt cag gtg gtg gat gat act aaa tac aag gac tac caa        2496
Pro Met Ser Arg Gln Val Val Asp Asp Thr Lys Tyr Lys Asp Tyr Gln
            820                 825                 830

cag gtg ggc atc cta cac caa cac aac aac tct gga ttt gtt ggc tac        2544
Gln Val Gly Ile Leu His Gln His Asn Asn Ser Gly Phe Val Gly Tyr
        835                 840                 845

ctt gcc ccc acc atg cgc gaa gga cag gcc tac cct gct aac ttc ccc        2592
Leu Ala Pro Thr Met Arg Glu Gly Gln Ala Tyr Pro Ala Asn Phe Pro
    850                 855                 860

tat ccg ctt ata ggc aag acc gca gtt gac agc att acc cag aaa aag        2640
Tyr Pro Leu Ile Gly Lys Thr Ala Val Asp Ser Ile Thr Gln Lys Lys
865                 870                 875                 880

ttt ctt tgc gat cgc acc ctt tgg cgc atc cca ttc tcc agt aac ttt        2688
Phe Leu Cys Asp Arg Thr Leu Trp Arg Ile Pro Phe Ser Ser Asn Phe
                885                 890                 895

atg tcc atg ggc gca ctc aca gac ctg ggc caa aac ctt ctc tac gcc        2736
Met Ser Met Gly Ala Leu Thr Asp Leu Gly Gln Asn Leu Leu Tyr Ala
            900                 905                 910

aac tcc gcc cac gcg cta gac atg act ttt gag gtg gat ccc atg gac        2784
Asn Ser Ala His Ala Leu Asp Met Thr Phe Glu Val Asp Pro Met Asp
        915                 920                 925

gag ccc acc ctt ctt tat gtt ttg ttt gaa gtc ttt gac gtg gtc cgt        2832
Glu Pro Thr Leu Leu Tyr Val Leu Phe Glu Val Phe Asp Val Val Arg
    930                 935                 940

gtg cac cag ccg cac cgc ggc gtc atc gaa acc gtg tac ctg cgc acg        2880
Val His Gln Pro His Arg Gly Val Ile Glu Thr Val Tyr Leu Arg Thr
945                 950                 955                 960

ccc ttc tcg gcc ggc aac gcc aca aca taa                                2910
Pro Phe Ser Ala Gly Asn Ala Thr Thr
                965

<210>  19
<211>  2934
<212>  DNA
<213>  Artificial Sequence

<220>
<223>  (NANP)18-HVR1 modified Hexon protein

<220>
<221>  CDS
<222>  (1)..(2931)

<400>  19
atg gct acc cct tcg atg atg ccg cag tgg tct tac atg cac atc tcg      48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat      432
Ala Pro Asn Pro Cys Glu Trp Asp Glu Asn Ala Asn Pro Asn Ala Asn
    130                 135                 140

cca aat gca aat cca aat gca aat cca aat gca aat cca aat gca aat      480
Pro Asn Ala Asn Pro Asn Ala Asn Pro Asn Ala Asn Pro Asn Ala Asn
145                 150                 155                 160

cca aat gca aat cca aat gca aat cca aat gca aat cca aat gca aat      528
Pro Asn Ala Asn Pro Asn Ala Asn Pro Asn Ala Asn Pro Asn Ala Asn
                165                 170                 175

cca aat gca aat cca aat gca aat cca aac gcg aac ccg aat gct aat      576
Pro Asn Ala Asn Pro Asn Ala Asn Pro Asn Ala Asn Pro Asn Ala Asn
            180                 185                 190

cct aat gca aat cca aat gca aat cca aat gca aat cca aat gca aat      624
Pro Asn Ala Asn Pro Asn Ala Asn Pro Asn Ala Asn Pro Asn Ala Asn
        195                 200                 205

cca aaa act cac gta ttt ggg cag gcg cct tat tct ggt ata aat att      672
Pro Lys Thr His Val Phe Gly Gln Ala Pro Tyr Ser Gly Ile Asn Ile
    210                 215                 220

aca aag gag ggt att caa ata ggt gtc gaa ggt caa aca cct aaa tat 720
Thr Lys Glu Gly Ile Gln Ile Gly Val Glu Gly Gln Thr Pro Lys Tyr
225                 230                 235                 240

gcc gat aaa aca ttt caa cct gaa cct caa ata gga gaa tct cag tgg 768
Ala Asp Lys Thr Phe Gln Pro Glu Pro Gln Ile Gly Glu Ser Gln Trp
                245                 250                 255

tac gaa aca gaa att aat cat gca gct ggg aga gtc cta aaa aag act 816
Tyr Glu Thr Glu Ile Asn His Ala Ala Gly Arg Val Leu Lys Lys Thr
            260                 265                 270

acc cca atg aaa cca tgt tac ggt tca tat gca aaa ccc aca aat gaa 864
Thr Pro Met Lys Pro Cys Tyr Gly Ser Tyr Ala Lys Pro Thr Asn Glu
        275                 280                 285

aat gga ggg caa ggc att ctt gta aag caa caa aat gga aag cta gaa 912
Asn Gly Gly Gln Gly Ile Leu Val Lys Gln Gln Asn Gly Lys Leu Glu
    290                 295                 300

agt caa gtg gaa atg caa ttt ttc tca act act gag gca gcc gca ggc 960
Ser Gln Val Glu Met Gln Phe Phe Ser Thr Thr Glu Ala Ala Ala Gly
305                 310                 315                 320

aat ggt gat aac ttg act cct aaa gtg gta ttg tac agt gaa gat gta 1008
Asn Gly Asp Asn Leu Thr Pro Lys Val Val Leu Tyr Ser Glu Asp Val
                325                 330                 335

gat ata gaa acc cca gac act cat att tct tac atg ccc act att aag 1056
Asp Ile Glu Thr Pro Asp Thr His Ile Ser Tyr Met Pro Thr Ile Lys
            340                 345                 350

gaa ggt aac tca cga gaa cta atg ggc caa caa tct atg ccc aac agg 1104
Glu Gly Asn Ser Arg Glu Leu Met Gly Gln Gln Ser Met Pro Asn Arg
        355                 360                 365

cct aat tac att gct ttt agg gac aat ttt att ggt cta atg tat tac 1152
Pro Asn Tyr Ile Ala Phe Arg Asp Asn Phe Ile Gly Leu Met Tyr Tyr
    370                 375                 380

aac agc acg ggt aat atg ggt gtt ctg gcg ggc caa gca tcg cag ttg 1200
Asn Ser Thr Gly Asn Met Gly Val Leu Ala Gly Gln Ala Ser Gln Leu
385                 390                 395                 400

aat gct gtt gta gat ttg caa gac aga aac aca gag ctt tca tac cag 1248
Asn Ala Val Val Asp Leu Gln Asp Arg Asn Thr Glu Leu Ser Tyr Gln
                405                 410                 415

ctt ttg ctt gat tcc att ggt gat aga acc agg tac ttt tct atg tgg 1296
Leu Leu Leu Asp Ser Ile Gly Asp Arg Thr Arg Tyr Phe Ser Met Trp
            420                 425                 430

aat cag ctt cca aat tac tgc ttt cca ctg gga ggt gtg att aat aca 1344
Asn Gln Leu Pro Asn Tyr Cys Phe Pro Leu Gly Gly Val Ile Asn Thr
        435                 440                 445

gag act ctt acc aag gta aaa cct aaa aca ggt cag gaa aat gga tgg 1392
Glu Thr Leu Thr Lys Val Lys Pro Lys Thr Gly Gln Glu Asn Gly Trp
    450                 455                 460

gaa aaa gat gct aca gaa ttt tca gat aaa aat gaa ata aga gtt gga 1440
Glu Lys Asp Ala Thr Glu Phe Ser Asp Lys Asn Glu Ile Arg Val Gly
465                 470                 475                 480

aat aat ttt gcc atg gaa atc aat cta aat gcc aac ctg tgg aga aat 1488
Asn Asn Phe Ala Met Glu Ile Asn Leu Asn Ala Asn Leu Trp Arg Asn
                485                 490                 495

ttc ctg tac tcc aac ata gcg ctg tat ttg ccc gac aag cta aag tac 1536
Phe Leu Tyr Ser Asn Ile Ala Leu Tyr Leu Pro Asp Lys Leu Lys Tyr
            500                 505                 510

agt cct tcc aac gta aaa att tct gat aac cca aac acc tac gac tac 1584
Ser Pro Ser Asn Val Lys Ile Ser Asp Asn Pro Asn Thr Tyr Asp Tyr
        515                 520                 525

atg aac aag cga gtg gtg gct ccc ggg cta gtg gac tgc tac att aac 1632
Met Asn Lys Arg Val Val Ala Pro Gly Leu Val Asp Cys Tyr Ile Asn
    530                 535                 540

ctt gga gca cgc tgg tcc ctt gac tat atg gac aac gtc aac cca ttt 1680
Leu Gly Ala Arg Trp Ser Leu Asp Tyr Met Asp Asn Val Asn Pro Phe
545                 550                 555                 560

aac cac cac cgc aat gct ggc ctg cgc tac cgc tca atg ttg ctg ggc 1728
Asn His His Arg Asn Ala Gly Leu Arg Tyr Arg Ser Met Leu Leu Gly
                565                 570                 575

aat ggt cgc tat gtg ccc ttc cac atc cag gtg cct cag aag ttc ttt 1776
Asn Gly Arg Tyr Val Pro Phe His Ile Gln Val Pro Gln Lys Phe Phe
            580                 585                 590

gcc att aaa aac ctc ctt ctc ctg ccg ggc tca tac acc tac gag tgg 1824
Ala Ile Lys Asn Leu Leu Leu Leu Pro Gly Ser Tyr Thr Tyr Glu Trp
        595                 600                 605

aac ttc agg aag gat gtt aac atg gtt ctg cag agc tcc cta gga aat 1872
Asn Phe Arg Lys Asp Val Asn Met Val Leu Gln Ser Ser Leu Gly Asn
    610                 615                 620

gac cta agg gtt gac gga gcc agc att aag ttt gat agc att tgc ctt 1920
Asp Leu Arg Val Asp Gly Ala Ser Ile Lys Phe Asp Ser Ile Cys Leu
625                 630                 635                 640

tac gcc acc ttc ttc ccc atg gcc cac aac acc gcc tcc acg ctt gag 1968
Tyr Ala Thr Phe Phe Pro Met Ala His Asn Thr Ala Ser Thr Leu Glu
                645                 650                 655

gcc atg ctt aga aac gac acc aac gac cag tcc ttt aac gac tat ctc 2016
Ala Met Leu Arg Asn Asp Thr Asn Asp Gln Ser Phe Asn Asp Tyr Leu
            660                 665                 670

tcc gcc gcc aac atg ctc tac cct ata ccc gcc aac gct acc aac gtg 2064
Ser Ala Ala Asn Met Leu Tyr Pro Ile Pro Ala Asn Ala Thr Asn Val
        675                 680                 685

ccc ata tcc atc ccc tcc cgc aac tgg gcg gct ttc cgc ggc tgg gcc 2112
Pro Ile Ser Ile Pro Ser Arg Asn Trp Ala Ala Phe Arg Gly Trp Ala
    690                 695                 700

ttc acg cgc ctt aag act aag gaa acc cca tca ctg ggc tcg ggc tac 2160
Phe Thr Arg Leu Lys Thr Lys Glu Thr Pro Ser Leu Gly Ser Gly Tyr
705                 710                 715                 720

gac cct tat tac acc tac tct ggc tct ata ccc tac cta gat gga acc 2208
Asp Pro Tyr Tyr Thr Tyr Ser Gly Ser Ile Pro Tyr Leu Asp Gly Thr

725                 730                 735

ttt tac ctc aac cac acc ttt aag aag gtg gcc att acc ttt gac tct      2256
Phe Tyr Leu Asn His Thr Phe Lys Lys Val Ala Ile Thr Phe Asp Ser
            740                 745                 750

tct gtc agc tgg cct ggc aat gac cgc ctg ctt acc ccc aac gag ttt      2304
Ser Val Ser Trp Pro Gly Asn Asp Arg Leu Leu Thr Pro Asn Glu Phe
        755                 760                 765

gaa att aag cgc tca gtt gac ggg gag ggt tac aac gtt gcc cag tgt      2352
Glu Ile Lys Arg Ser Val Asp Gly Glu Gly Tyr Asn Val Ala Gln Cys
    770                 775                 780

aac atg acc aaa gac tgg ttc ctg gta caa atg cta gct aac tat aac      2400
Asn Met Thr Lys Asp Trp Phe Leu Val Gln Met Leu Ala Asn Tyr Asn
785                 790                 795                 800

att ggc tac cag ggc ttc tat atc cca gag agc tac aag gac cgc atg      2448
Ile Gly Tyr Gln Gly Phe Tyr Ile Pro Glu Ser Tyr Lys Asp Arg Met
                805                 810                 815

tac tcc ttc ttt aga aac ttc cag ccc atg agc cgt cag gtg gtg gat      2496
Tyr Ser Phe Phe Arg Asn Phe Gln Pro Met Ser Arg Gln Val Val Asp
            820                 825                 830

gat act aaa tac aag gac tac caa cag gtg ggc atc cta cac caa cac      2544
Asp Thr Lys Tyr Lys Asp Tyr Gln Gln Val Gly Ile Leu His Gln His
        835                 840                 845

aac aac tct gga ttt gtt ggc tac ctt gcc ccc acc atg cgc gaa gga      2592
Asn Asn Ser Gly Phe Val Gly Tyr Leu Ala Pro Thr Met Arg Glu Gly
    850                 855                 860

cag gcc tac cct gct aac ttc ccc tat ccg ctt ata ggc aag acc gca      2640
Gln Ala Tyr Pro Ala Asn Phe Pro Tyr Pro Leu Ile Gly Lys Thr Ala
865                 870                 875                 880

gtt gac agc att acc cag aaa aag ttt ctt tgc gat cgc acc ctt tgg      2688
Val Asp Ser Ile Thr Gln Lys Lys Phe Leu Cys Asp Arg Thr Leu Trp
                885                 890                 895

cgc atc cca ttc tcc agt aac ttt atg tcc atg ggc gca ctc aca gac      2736
Arg Ile Pro Phe Ser Ser Asn Phe Met Ser Met Gly Ala Leu Thr Asp
            900                 905                 910

ctg ggc caa aac ctt ctc tac gcc aac tcc gcc cac gcg cta gac atg      2784
Leu Gly Gln Asn Leu Leu Tyr Ala Asn Ser Ala His Ala Leu Asp Met
        915                 920                 925

act ttt gag gtg gat ccc atg gac gag ccc acc ctt ctt tat gtt ttg      2832
Thr Phe Glu Val Asp Pro Met Asp Glu Pro Thr Leu Leu Tyr Val Leu
    930                 935                 940

ttt gaa gtc ttt gac gtg gtc cgt gtg cac cag ccg cac cgc ggc gtc      2880
Phe Glu Val Phe Asp Val Val Arg Val His Gln Pro His Arg Gly Val
945                 950                 955                 960

atc gaa acc gtg tac ctg cgc acg ccc ttc tcg gcc ggc aac gcc aca      2928
Ile Glu Thr Val Tyr Leu Arg Thr Pro Phe Ser Ala Gly Asn Ala Thr
                965                 970                 975

aca taa                                                              2934

Thr

<210>  20
<211>  2958
<212>  DNA
<213>  Artificial Sequence

<220>
<223>  (NANP)20-HVR1 modified Hexon protein

<220>
<221>  CDS
<222>  (1)..(2955)

<400>  20
atg gct acc cct tcg atg atg ccg cag tgg tct tac atg cac atc tcg      48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc      96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc      144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg      192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac      240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg      288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act      336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt      384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat      432
Ala Pro Asn Pro Cys Glu Trp Asp Glu Asn Ala Asn Pro Asn Ala Asn
    130                 135                 140

cca aat gca aat cca aat gca aat cca aat gca aat cca aat gca aat      480
Pro Asn Ala Asn Pro Asn Ala Asn Pro Asn Ala Asn Pro Asn Ala Asn
145                 150                 155                 160

cca aat gca aat cca aat gca aat cca aat gca aat cca aat gca aat      528
Pro Asn Ala Asn Pro Asn Ala Asn Pro Asn Ala Asn Pro Asn Ala Asn
                165                 170                 175

cca aat gca aat cca aat gca aat cca aac gcg aac ccg aat gct aat      576

Pro Asn Ala Asn Pro Asn Ala Asn Pro Asn Ala Asn Pro Asn Ala Asn
            180                 185                 190

cct aat gca aat cca aat gca aat cca aat gca aat cca aat gca aat      624
Pro Asn Ala Asn Pro Asn Ala Asn Pro Asn Ala Asn Pro Asn Ala Asn
        195                 200                 205

cca aat gca aat cca aat gca aat cca aaa act cac gta ttt ggg cag      672
Pro Asn Ala Asn Pro Asn Ala Asn Pro Lys Thr His Val Phe Gly Gln
    210                 215                 220

gcg cct tat tct ggt ata aat att aca aag gag ggt att caa ata ggt      720
Ala Pro Tyr Ser Gly Ile Asn Ile Thr Lys Glu Gly Ile Gln Ile Gly
225                 230                 235                 240

gtc gaa ggt caa aca cct aaa tat gcc gat aaa aca ttt caa cct gaa      768
Val Glu Gly Gln Thr Pro Lys Tyr Ala Asp Lys Thr Phe Gln Pro Glu
                245                 250                 255

cct caa ata gga gaa tct cag tgg tac gaa aca gaa att aat cat gca      816
Pro Gln Ile Gly Glu Ser Gln Trp Tyr Glu Thr Glu Ile Asn His Ala
            260                 265                 270

gct ggg aga gtc cta aaa aag act acc cca atg aaa cca tgt tac ggt      864
Ala Gly Arg Val Leu Lys Lys Thr Thr Pro Met Lys Pro Cys Tyr Gly
        275                 280                 285

tca tat gca aaa ccc aca aat gaa aat gga ggg caa ggc att ctt gta      912
Ser Tyr Ala Lys Pro Thr Asn Glu Asn Gly Gly Gln Gly Ile Leu Val
    290                 295                 300

aag caa caa aat gga aag cta gaa agt caa gtg gaa atg caa ttt ttc      960
Lys Gln Gln Asn Gly Lys Leu Glu Ser Gln Val Glu Met Gln Phe Phe
305                 310                 315                 320

tca act act gag gca gcc gca ggc aat ggt gat aac ttg act cct aaa     1008
Ser Thr Thr Glu Ala Ala Ala Gly Asn Gly Asp Asn Leu Thr Pro Lys
                325                 330                 335

gtg gta ttg tac agt gaa gat gta gat ata gaa acc cca gac act cat     1056
Val Val Leu Tyr Ser Glu Asp Val Asp Ile Glu Thr Pro Asp Thr His
            340                 345                 350

att tct tac atg ccc act att aag gaa ggt aac tca cga gaa cta atg     1104
Ile Ser Tyr Met Pro Thr Ile Lys Glu Gly Asn Ser Arg Glu Leu Met
        355                 360                 365

ggc caa caa tct atg ccc aac agg cct aat tac att gct ttt agg gac     1152
Gly Gln Gln Ser Met Pro Asn Arg Pro Asn Tyr Ile Ala Phe Arg Asp
    370                 375                 380

aat ttt att ggt cta atg tat tac aac agc acg ggt aat atg ggt gtt     1200
Asn Phe Ile Gly Leu Met Tyr Tyr Asn Ser Thr Gly Asn Met Gly Val
385                 390                 395                 400

ctg gcg ggc caa gca tcg cag ttg aat gct gtt gta gat ttg caa gac     1248
Leu Ala Gly Gln Ala Ser Gln Leu Asn Ala Val Val Asp Leu Gln Asp
                405                 410                 415

aga aac aca gag ctt tca tac cag ctt ttg ctt gat tcc att ggt gat     1296
Arg Asn Thr Glu Leu Ser Tyr Gln Leu Leu Leu Asp Ser Ile Gly Asp
            420                 425                 430

aga acc agg tac ttt tct atg tgg aat cag ctt cca aat tac tgc ttt 1344
Arg Thr Arg Tyr Phe Ser Met Trp Asn Gln Leu Pro Asn Tyr Cys Phe
        435                 440                 445

cca ctg gga ggt gtg att aat aca gag act ctt acc aag gta aaa cct 1392
Pro Leu Gly Gly Val Ile Asn Thr Glu Thr Leu Thr Lys Val Lys Pro
    450                 455                 460

aaa aca ggt cag gaa aat gga tgg gaa aaa gat gct aca gaa ttt tca 1440
Lys Thr Gly Gln Glu Asn Gly Trp Glu Lys Asp Ala Thr Glu Phe Ser
465                 470                 475                 480

gat aaa aat gaa ata aga gtt gga aat aat ttt gcc atg gaa atc aat 1488
Asp Lys Asn Glu Ile Arg Val Gly Asn Asn Phe Ala Met Glu Ile Asn
                485                 490                 495

cta aat gcc aac ctg tgg aga aat ttc ctg tac tcc aac ata gcg ctg 1536
Leu Asn Ala Asn Leu Trp Arg Asn Phe Leu Tyr Ser Asn Ile Ala Leu
            500                 505                 510

tat ttg ccc gac aag cta aag tac agt cct tcc aac gta aaa att tct 1584
Tyr Leu Pro Asp Lys Leu Lys Tyr Ser Pro Ser Asn Val Lys Ile Ser
        515                 520                 525

gat aac cca aac acc tac gac tac atg aac aag cga gtg gtg gct ccc 1632
Asp Asn Pro Asn Thr Tyr Asp Tyr Met Asn Lys Arg Val Val Ala Pro
    530                 535                 540

ggg cta gtg gac tgc tac att aac ctt gga gca cgc tgg tcc ctt gac 1680
Gly Leu Val Asp Cys Tyr Ile Asn Leu Gly Ala Arg Trp Ser Leu Asp
545                 550                 555                 560

tat atg gac aac gtc aac cca ttt aac cac cac cgc aat gct ggc ctg 1728
Tyr Met Asp Asn Val Asn Pro Phe Asn His His Arg Asn Ala Gly Leu
                565                 570                 575

cgc tac cgc tca atg ttg ctg ggc aat ggt cgc tat gtg ccc ttc cac 1776
Arg Tyr Arg Ser Met Leu Leu Gly Asn Gly Arg Tyr Val Pro Phe His
            580                 585                 590

atc cag gtg cct cag aag ttc ttt gcc att aaa aac ctc ctt ctc ctg 1824
Ile Gln Val Pro Gln Lys Phe Phe Ala Ile Lys Asn Leu Leu Leu Leu
        595                 600                 605

ccg ggc tca tac acc tac gag tgg aac ttc agg aag gat gtt aac atg 1872
Pro Gly Ser Tyr Thr Tyr Glu Trp Asn Phe Arg Lys Asp Val Asn Met
    610                 615                 620

gtt ctg cag agc tcc cta gga aat gac cta agg gtt gac gga gcc agc 1920
Val Leu Gln Ser Ser Leu Gly Asn Asp Leu Arg Val Asp Gly Ala Ser
625                 630                 635                 640

att aag ttt gat agc att tgc ctt tac gcc acc ttc ttc ccc atg gcc 1968
Ile Lys Phe Asp Ser Ile Cys Leu Tyr Ala Thr Phe Phe Pro Met Ala
                645                 650                 655

cac aac acc gcc tcc acg ctt gag gcc atg ctt aga aac gac acc aac 2016
His Asn Thr Ala Ser Thr Leu Glu Ala Met Leu Arg Asn Asp Thr Asn
            660                 665                 670

gac cag tcc ttt aac gac tat ctc tcc gcc gcc aac atg ctc tac cct 2064
Asp Gln Ser Phe Asn Asp Tyr Leu Ser Ala Ala Asn Met Leu Tyr Pro
        675                 680                 685

ata ccc gcc aac gct acc aac gtg ccc ata tcc atc ccc tcc cgc aac 2112
Ile Pro Ala Asn Ala Thr Asn Val Pro Ile Ser Ile Pro Ser Arg Asn
    690                 695                 700

tgg gcg gct ttc cgc ggc tgg gcc ttc acg cgc ctt aag act aag gaa 2160
Trp Ala Ala Phe Arg Gly Trp Ala Phe Thr Arg Leu Lys Thr Lys Glu
705                 710                 715                 720

acc cca tca ctg ggc tcg ggc tac gac cct tat tac acc tac tct ggc 2208
Thr Pro Ser Leu Gly Ser Gly Tyr Asp Pro Tyr Tyr Thr Tyr Ser Gly
                725                 730                 735

tct ata ccc tac cta gat gga acc ttt tac ctc aac cac acc ttt aag 2256
Ser Ile Pro Tyr Leu Asp Gly Thr Phe Tyr Leu Asn His Thr Phe Lys
            740                 745                 750

aag gtg gcc att acc ttt gac tct tct gtc agc tgg cct ggc aat gac 2304
Lys Val Ala Ile Thr Phe Asp Ser Ser Val Ser Trp Pro Gly Asn Asp
        755                 760                 765

cgc ctg ctt acc ccc aac gag ttt gaa att aag cgc tca gtt gac ggg 2352
Arg Leu Leu Thr Pro Asn Glu Phe Glu Ile Lys Arg Ser Val Asp Gly
    770                 775                 780

gag ggt tac aac gtt gcc cag tgt aac atg acc aaa gac tgg ttc ctg 2400
Glu Gly Tyr Asn Val Ala Gln Cys Asn Met Thr Lys Asp Trp Phe Leu
785                 790                 795                 800

gta caa atg cta gct aac tat aac att ggc tac cag ggc ttc tat atc 2448
Val Gln Met Leu Ala Asn Tyr Asn Ile Gly Tyr Gln Gly Phe Tyr Ile
                805                 810                 815

cca gag agc tac aag gac cgc atg tac tcc ttc ttt aga aac ttc cag 2496
Pro Glu Ser Tyr Lys Asp Arg Met Tyr Ser Phe Phe Arg Asn Phe Gln
            820                 825                 830

ccc atg agc cgt cag gtg gtg gat gat act aaa tac aag gac tac caa 2544
Pro Met Ser Arg Gln Val Val Asp Asp Thr Lys Tyr Lys Asp Tyr Gln
        835                 840                 845

cag gtg ggc atc cta cac caa cac aac aac tct gga ttt gtt ggc tac 2592
Gln Val Gly Ile Leu His Gln His Asn Asn Ser Gly Phe Val Gly Tyr
    850                 855                 860

ctt gcc ccc acc atg cgc gaa gga cag gcc tac cct gct aac ttc ccc 2640
Leu Ala Pro Thr Met Arg Glu Gly Gln Ala Tyr Pro Ala Asn Phe Pro
865                 870                 875                 880

tat ccg ctt ata ggc aag acc gca gtt gac agc att acc cag aaa aag 2688
Tyr Pro Leu Ile Gly Lys Thr Ala Val Asp Ser Ile Thr Gln Lys Lys
                885                 890                 895

ttt ctt tgc gat cgc acc ctt tgg cgc atc cca ttc tcc agt aac ttt 2736
Phe Leu Cys Asp Arg Thr Leu Trp Arg Ile Pro Phe Ser Ser Asn Phe
            900                 905                 910

atg tcc atg ggc gca ctc aca gac ctg ggc caa aac ctt ctc tac gcc 2784
Met Ser Met Gly Ala Leu Thr Asp Leu Gly Gln Asn Leu Leu Tyr Ala
        915                 920                 925

aac tcc gcc cac gcg cta gac atg act ttt gag gtg gat ccc atg gac 2832
Asn Ser Ala His Ala Leu Asp Met Thr Phe Glu Val Asp Pro Met Asp

930                 935                 940

gag ccc acc ctt ctt tat gtt ttg ttt gaa gtc ttt gac gtg gtc cgt         2880
Glu Pro Thr Leu Leu Tyr Val Leu Phe Glu Val Phe Asp Val Val Arg
945                 950                 955                 960

gtg cac cag ccg cac cgc ggc gtc atc gaa acc gtg tac ctg cgc acg         2928
Val His Gln Pro His Arg Gly Val Ile Glu Thr Val Tyr Leu Arg Thr
                965                 970                 975

ccc ttc tcg gcc ggc aac gcc aca aca taa                                 2958
Pro Phe Ser Ala Gly Asn Ala Thr Thr
            980                 985

<210>  21
<211>  2982
<212>  DNA
<213>  Artificial Sequence

<220>
<223>  (NANP)22-HVR1 modified Hexon protein

<220>
<221>  CDS
<222>  (1)..(2979)

<400>  21
atg gct acc cct tcg atg atg ccg cag tgg tct tac atg cac atc tcg           48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc           96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc          144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg          192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac          240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg          288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act          336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt          384
Phe Lys Pro Tyr Ser Gly Thr Ala Tyr Asn Ala Leu Ala Pro Lys Gly
        115                 120                 125

gcc cca aat cct tgc gaa tgg gat gaa aat gca aat cca aat gca aat          432

Ala Pro Asn Pro Cys Glu Trp Asp Glu Asn Ala Asn Pro Asn Ala Asn
    130                 135                 140

cca aat gca aat cca aat gca aat cca aat gca aat cca aat gca aat      480
Pro Asn Ala Asn Pro Asn Ala Asn Pro Asn Ala Asn Pro Asn Ala Asn
145                 150                 155                 160

cca aat gca aat cca aat gca aat cca aat gca aat cca aat gca aat      528
Pro Asn Ala Asn Pro Asn Ala Asn Pro Asn Ala Asn Pro Asn Ala Asn
                165                 170                 175

cca aat gca aat cca aat gca aat cca aac gcg aac ccg aat gct aat      576
Pro Asn Ala Asn Pro Asn Ala Asn Pro Asn Ala Asn Pro Asn Ala Asn
            180                 185                 190

cct aat gca aat cca aat gca aat cca aat gca aat cca aat gca aat      624
Pro Asn Ala Asn Pro Asn Ala Asn Pro Asn Ala Asn Pro Asn Ala Asn
        195                 200                 205

cca aat gca aat cca aat gca aat cca aat gca aat cca aat gca aat      672
Pro Asn Ala Asn Pro Asn Ala Asn Pro Asn Ala Asn Pro Asn Ala Asn
    210                 215                 220

cca aaa act cac gta ttt ggg cag gcg cct tat tct ggt ata aat att      720
Pro Lys Thr His Val Phe Gly Gln Ala Pro Tyr Ser Gly Ile Asn Ile
225                 230                 235                 240

aca aag gag ggt att caa ata ggt gtc gaa ggt caa aca cct aaa tat      768
Thr Lys Glu Gly Ile Gln Ile Gly Val Glu Gly Gln Thr Pro Lys Tyr
                245                 250                 255

gcc gat aaa aca ttt caa cct gaa cct caa ata gga gaa tct cag tgg      816
Ala Asp Lys Thr Phe Gln Pro Glu Pro Gln Ile Gly Glu Ser Gln Trp
            260                 265                 270

tac gaa aca gaa att aat cat gca gct ggg aga gtc cta aaa aag act      864
Tyr Glu Thr Glu Ile Asn His Ala Ala Gly Arg Val Leu Lys Lys Thr
        275                 280                 285

acc cca atg aaa cca tgt tac ggt tca tat gca aaa ccc aca aat gaa      912
Thr Pro Met Lys Pro Cys Tyr Gly Ser Tyr Ala Lys Pro Thr Asn Glu
    290                 295                 300

aat gga ggg caa ggc att ctt gta aag caa caa aat gga aag cta gaa      960
Asn Gly Gly Gln Gly Ile Leu Val Lys Gln Gln Asn Gly Lys Leu Glu
305                 310                 315                 320

agt caa gtg gaa atg caa ttt ttc tca act act gag gca gcc gca ggc     1008
Ser Gln Val Glu Met Gln Phe Phe Ser Thr Thr Glu Ala Ala Ala Gly
                325                 330                 335

aat ggt gat aac ttg act cct aaa gtg gta ttg tac agt gaa gat gta     1056
Asn Gly Asp Asn Leu Thr Pro Lys Val Val Leu Tyr Ser Glu Asp Val
            340                 345                 350

gat ata gaa acc cca gac act cat att tct tac atg ccc act att aag     1104
Asp Ile Glu Thr Pro Asp Thr His Ile Ser Tyr Met Pro Thr Ile Lys
        355                 360                 365

gaa ggt aac tca cga gaa cta atg ggc caa caa tct atg ccc aac agg     1152
Glu Gly Asn Ser Arg Glu Leu Met Gly Gln Gln Ser Met Pro Asn Arg
    370                 375                 380

cct aat tac att gct ttt agg gac aat ttt att ggt cta atg tat tac      1200
Pro Asn Tyr Ile Ala Phe Arg Asp Asn Phe Ile Gly Leu Met Tyr Tyr
385                 390                 395                 400

aac agc acg ggt aat atg ggt gtt ctg gcg ggc caa gca tcg cag ttg      1248
Asn Ser Thr Gly Asn Met Gly Val Leu Ala Gly Gln Ala Ser Gln Leu
                405                 410                 415

aat gct gtt gta gat ttg caa gac aga aac aca gag ctt tca tac cag      1296
Asn Ala Val Val Asp Leu Gln Asp Arg Asn Thr Glu Leu Ser Tyr Gln
            420                 425                 430

ctt ttg ctt gat tcc att ggt gat aga acc agg tac ttt tct atg tgg      1344
Leu Leu Leu Asp Ser Ile Gly Asp Arg Thr Arg Tyr Phe Ser Met Trp
        435                 440                 445

aat cag ctt cca aat tac tgc ttt cca ctg gga ggt gtg att aat aca      1392
Asn Gln Leu Pro Asn Tyr Cys Phe Pro Leu Gly Gly Val Ile Asn Thr
    450                 455                 460

gag act ctt acc aag gta aaa cct aaa aca ggt cag gaa aat gga tgg      1440
Glu Thr Leu Thr Lys Val Lys Pro Lys Thr Gly Gln Glu Asn Gly Trp
465                 470                 475                 480

gaa aaa gat gct aca gaa ttt tca gat aaa aat gaa ata aga gtt gga      1488
Glu Lys Asp Ala Thr Glu Phe Ser Asp Lys Asn Glu Ile Arg Val Gly
                485                 490                 495

aat aat ttt gcc atg gaa atc aat cta aat gcc aac ctg tgg aga aat      1536
Asn Asn Phe Ala Met Glu Ile Asn Leu Asn Ala Asn Leu Trp Arg Asn
            500                 505                 510

ttc ctg tac tcc aac ata gcg ctg tat ttg ccc gac aag cta aag tac      1584
Phe Leu Tyr Ser Asn Ile Ala Leu Tyr Leu Pro Asp Lys Leu Lys Tyr
        515                 520                 525

agt cct tcc aac gta aaa att tct gat aac cca aac acc tac gac tac      1632
Ser Pro Ser Asn Val Lys Ile Ser Asp Asn Pro Asn Thr Tyr Asp Tyr
    530                 535                 540

atg aac aag cga gtg gtg gct ccc ggg cta gtg gac tgc tac att aac      1680
Met Asn Lys Arg Val Val Ala Pro Gly Leu Val Asp Cys Tyr Ile Asn
545                 550                 555                 560

ctt gga gca cgc tgg tcc ctt gac tat atg gac aac gtc aac cca ttt      1728
Leu Gly Ala Arg Trp Ser Leu Asp Tyr Met Asp Asn Val Asn Pro Phe
                565                 570                 575

aac cac cac cgc aat gct ggc ctg cgc tac cgc tca atg ttg ctg ggc      1776
Asn His His Arg Asn Ala Gly Leu Arg Tyr Arg Ser Met Leu Leu Gly
            580                 585                 590

aat ggt cgc tat gtg ccc ttc cac atc cag gtg cct cag aag ttc ttt      1824
Asn Gly Arg Tyr Val Pro Phe His Ile Gln Val Pro Gln Lys Phe Phe
        595                 600                 605

gcc att aaa aac ctc ctt ctc ctg ccg ggc tca tac acc tac gag tgg      1872
Ala Ile Lys Asn Leu Leu Leu Leu Pro Gly Ser Tyr Thr Tyr Glu Trp
    610                 615                 620

aac ttc agg aag gat gtt aac atg gtt ctg cag agc tcc cta gga aat      1920
Asn Phe Arg Lys Asp Val Asn Met Val Leu Gln Ser Ser Leu Gly Asn
625                 630                 635                 640

gac cta agg gtt gac gga gcc agc att aag ttt gat agc att tgc ctt 1968
Asp Leu Arg Val Asp Gly Ala Ser Ile Lys Phe Asp Ser Ile Cys Leu
                645                 650                 655

tac gcc acc ttc ttc ccc atg gcc cac aac acc gcc tcc acg ctt gag 2016
Tyr Ala Thr Phe Phe Pro Met Ala His Asn Thr Ala Ser Thr Leu Glu
            660                 665                 670

gcc atg ctt aga aac gac acc aac gac cag tcc ttt aac gac tat ctc 2064
Ala Met Leu Arg Asn Asp Thr Asn Asp Gln Ser Phe Asn Asp Tyr Leu
        675                 680                 685

tcc gcc gcc aac atg ctc tac cct ata ccc gcc aac gct acc aac gtg 2112
Ser Ala Ala Asn Met Leu Tyr Pro Ile Pro Ala Asn Ala Thr Asn Val
    690                 695                 700

ccc ata tcc atc ccc tcc cgc aac tgg gcg gct ttc cgc ggc tgg gcc 2160
Pro Ile Ser Ile Pro Ser Arg Asn Trp Ala Ala Phe Arg Gly Trp Ala
705                 710                 715                 720

ttc acg cgc ctt aag act aag gaa acc cca tca ctg ggc tcg ggc tac 2208
Phe Thr Arg Leu Lys Thr Lys Glu Thr Pro Ser Leu Gly Ser Gly Tyr
                725                 730                 735

gac cct tat tac acc tac tct ggc tct ata ccc tac cta gat gga acc 2256
Asp Pro Tyr Tyr Thr Tyr Ser Gly Ser Ile Pro Tyr Leu Asp Gly Thr
            740                 745                 750

ttt tac ctc aac cac acc ttt aag aag gtg gcc att acc ttt gac tct 2304
Phe Tyr Leu Asn His Thr Phe Lys Lys Val Ala Ile Thr Phe Asp Ser
        755                 760                 765

tct gtc agc tgg cct ggc aat gac cgc ctg ctt acc ccc aac gag ttt 2352
Ser Val Ser Trp Pro Gly Asn Asp Arg Leu Leu Thr Pro Asn Glu Phe
    770                 775                 780

gaa att aag cgc tca gtt gac ggg gag ggt tac aac gtt gcc cag tgt 2400
Glu Ile Lys Arg Ser Val Asp Gly Glu Gly Tyr Asn Val Ala Gln Cys
785                 790                 795                 800

aac atg acc aaa gac tgg ttc ctg gta caa atg cta gct aac tat aac 2448
Asn Met Thr Lys Asp Trp Phe Leu Val Gln Met Leu Ala Asn Tyr Asn
                805                 810                 815

att ggc tac cag ggc ttc tat atc cca gag agc tac aag gac cgc atg 2496
Ile Gly Tyr Gln Gly Phe Tyr Ile Pro Glu Ser Tyr Lys Asp Arg Met
            820                 825                 830

tac tcc ttc ttt aga aac ttc cag ccc atg agc cgt cag gtg gtg gat 2544
Tyr Ser Phe Phe Arg Asn Phe Gln Pro Met Ser Arg Gln Val Val Asp
        835                 840                 845

gat act aaa tac aag gac tac caa cag gtg ggc atc cta cac caa cac 2592
Asp Thr Lys Tyr Lys Asp Tyr Gln Gln Val Gly Ile Leu His Gln His
    850                 855                 860

aac aac tct gga ttt gtt ggc tac ctt gcc ccc acc atg cgc gaa gga 2640
Asn Asn Ser Gly Phe Val Gly Tyr Leu Ala Pro Thr Met Arg Glu Gly
865                 870                 875                 880

cag gcc tac cct gct aac ttc ccc tat ccg ctt ata ggc aag acc gca 2688
Gln Ala Tyr Pro Ala Asn Phe Pro Tyr Pro Leu Ile Gly Lys Thr Ala

885                 890                 895

gtt gac agc att acc cag aaa aag ttt ctt tgc gat cgc acc ctt tgg        2736
Val Asp Ser Ile Thr Gln Lys Lys Phe Leu Cys Asp Arg Thr Leu Trp
            900                 905                 910

cgc atc cca ttc tcc agt aac ttt atg tcc atg ggc gca ctc aca gac        2784
Arg Ile Pro Phe Ser Ser Asn Phe Met Ser Met Gly Ala Leu Thr Asp
        915                 920                 925

ctg ggc caa aac ctt ctc tac gcc aac tcc gcc cac gcg cta gac atg        2832
Leu Gly Gln Asn Leu Leu Tyr Ala Asn Ser Ala His Ala Leu Asp Met
    930                 935                 940

act ttt gag gtg gat ccc atg gac gag ccc acc ctt ctt tat gtt ttg        2880
Thr Phe Glu Val Asp Pro Met Asp Glu Pro Thr Leu Leu Tyr Val Leu
945                 950                 955                 960

ttt gaa gtc ttt gac gtg gtc cgt gtg cac cag ccg cac cgc ggc gtc        2928
Phe Glu Val Phe Asp Val Val Arg Val His Gln Pro His Arg Gly Val
                965                 970                 975

atc gaa acc gtg tac ctg cgc acg ccc ttc tcg gcc ggc aac gcc aca        2976
Ile Glu Thr Val Tyr Leu Arg Thr Pro Phe Ser Ala Gly Asn Ala Thr
            980                 985                 990

aca taa                                                                2982
Thr


<210>  22
<211>  3054
<212>  DNA
<213>  Artificial Sequence

<220>
<223>  (NANP)28-HVR1 modified Hexon protein


<220>
<221>  CDS
<222>  (1)..(3051)

<400>  22
atg gct acc cct tcg atg atg ccg cag tgg tct tac atg cac atc tcg          48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc          96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc         144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg         192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac         240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg 288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act 336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt 384
Phe Lys Pro Tyr Ser Gly Thr Ala Tyr Asn Ala Leu Ala Pro Lys Gly
        115                 120                 125

gcc cca aat cct tgc gaa tgg gat gaa aac gcc aat cca aat gcg aat 432
Ala Pro Asn Pro Cys Glu Trp Asp Glu Asn Ala Asn Pro Asn Ala Asn
    130                 135                 140

ccc aac gct aat cct aac gca aac ccg aat gct aac cct aac gca aac 480
Pro Asn Ala Asn Pro Asn Ala Asn Pro Asn Ala Asn Pro Asn Ala Asn
145                 150                 155                 160

ccc aac gct aac ccc aac gcg aac ccc aat gcc aac ccc aac gcc aac 528
Pro Asn Ala Asn Pro Asn Ala Asn Pro Asn Ala Asn Pro Asn Ala Asn
                165                 170                 175

ccg aac gcc aat cca aac gct aac cct aat gcc aat cct aat gcc aat 576
Pro Asn Ala Asn Pro Asn Ala Asn Pro Asn Ala Asn Pro Asn Ala Asn
            180                 185                 190

ccg aac gcc aat cca aat gcc aat cca aat gct aat ccc aac aat gcc 624
Pro Asn Ala Asn Pro Asn Ala Asn Pro Asn Ala Asn Pro Asn Ala Asn
        195                 200                 205

ccc aac gcg aac cct aat gcc aac ccc aac gct aat cca aat gcg aac 672
Pro Asn Ala Asn Pro Asn Ala Asn Pro Asn Ala Asn Pro Asn Ala Asn
    210                 215                 220

cct aac gcc aac ccg aat gct aat ccc aat gcc aac ccc aat gct aat 720
Pro Asn Ala Asn Pro Asn Ala Asn Pro Asn Ala Asn Pro Asn Ala Asn
225                 230                 235                 240

ccc aat gcg aac cct aat gcc aat ccc aaa act cac gta ttt ggg cag 768
Pro Asn Ala Asn Pro Asn Ala Asn Pro Lys Thr His Val Phe Gly Gln
                245                 250                 255

gcg cct tat tct ggt ata aat att aca aag gag ggt att caa ata ggt 816
Ala Pro Tyr Ser Gly Ile Asn Ile Thr Lys Glu Gly Ile Gln Ile Gly
            260                 265                 270

gtc gaa ggt caa aca cct aaa tat gcc gat aaa aca ttt caa cct gaa 864
Val Glu Gly Gln Thr Pro Lys Tyr Ala Asp Lys Thr Phe Gln Pro Glu
        275                 280                 285

cct caa ata gga gaa tct cag tgg tac gaa aca gaa att aat cat gca 912
Pro Gln Ile Gly Glu Ser Gln Trp Tyr Glu Thr Glu Ile Asn His Ala
    290                 295                 300

gct ggg aga gtc cta aaa aag act acc cca atg aaa cca tgt tac ggt 960
Ala Gly Arg Val Leu Lys Lys Thr Thr Pro Met Lys Pro Cys Tyr Gly
305                 310                 315                 320

tca tat gca aaa ccc aca aat gaa aat gga ggg caa ggc att ctt gta 1008
Ser Tyr Ala Lys Pro Thr Asn Glu Asn Gly Gly Gln Gly Ile Leu Val

325                 330                 335

aag caa caa aat gga aag cta gaa agt caa gtg gaa atg caa ttt ttc      1056
Lys Gln Gln Asn Gly Lys Leu Glu Ser Gln Val Glu Met Gln Phe Phe
            340                 345                 350

tca act act gag gca gcc gca ggc aat ggt gat aac ttg act cct aaa      1104
Ser Thr Thr Glu Ala Ala Ala Gly Asn Gly Asp Asn Leu Thr Pro Lys
        355                 360                 365

gtg gta ttg tac agt gaa gat gta gat ata gaa acc cca gac act cat      1152
Val Val Leu Tyr Ser Glu Asp Val Asp Ile Glu Thr Pro Asp Thr His
    370                 375                 380

att tct tac atg ccc act att aag gaa ggt aac tca cga gaa cta atg      1200
Ile Ser Tyr Met Pro Thr Ile Lys Glu Gly Asn Ser Arg Glu Leu Met
385                 390                 395                 400

ggc caa caa tct atg ccc aac agg cct aat tac att gct ttt agg gac      1248
Gly Gln Gln Ser Met Pro Asn Arg Pro Asn Tyr Ile Ala Phe Arg Asp
                405                 410                 415

aat ttt att ggt cta atg tat tac aac agc acg ggt aat atg ggt gtt      1296
Asn Phe Ile Gly Leu Met Tyr Tyr Asn Ser Thr Gly Asn Met Gly Val
            420                 425                 430

ctg gcg ggc caa gca tcg cag ttg aat gct gtt gta gat ttg caa gac      1344
Leu Ala Gly Gln Ala Ser Gln Leu Asn Ala Val Val Asp Leu Gln Asp
        435                 440                 445

aga aac aca gag ctt tca tac cag ctt ttg ctt gat tcc att ggt gat      1392
Arg Asn Thr Glu Leu Ser Tyr Gln Leu Leu Leu Asp Ser Ile Gly Asp
    450                 455                 460

aga acc agg tac ttt tct atg tgg aat cag ctt cca aat tac tgc ttt      1440
Arg Thr Arg Tyr Phe Ser Met Trp Asn Gln Leu Pro Asn Tyr Cys Phe
465                 470                 475                 480

cca ctg gga ggt gtg att aat aca gag act ctt acc aag gta aaa cct      1488
Pro Leu Gly Gly Val Ile Asn Thr Glu Thr Leu Thr Lys Val Lys Pro
                485                 490                 495

aaa aca ggt cag gaa aat gga tgg gaa aaa gat gct aca gaa ttt tca      1536
Lys Thr Gly Gln Glu Asn Gly Trp Glu Lys Asp Ala Thr Glu Phe Ser
            500                 505                 510

gat aaa aat gaa ata aga gtt gga aat aat ttt gcc atg gaa atc aat      1584
Asp Lys Asn Glu Ile Arg Val Gly Asn Asn Phe Ala Met Glu Ile Asn
        515                 520                 525

cta aat gcc aac ctg tgg aga aat ttc ctg tac tcc aac ata gcg ctg      1632
Leu Asn Ala Asn Leu Trp Arg Asn Phe Leu Tyr Ser Asn Ile Ala Leu
    530                 535                 540

tat ttg ccc gac aag cta aag tac agt cct tcc aac gta aaa att tct      1680
Tyr Leu Pro Asp Lys Leu Lys Tyr Ser Pro Ser Asn Val Lys Ile Ser
545                 550                 555                 560

gat aac cca aac acc tac gac tac atg aac aag cga gtg gtg gct ccc      1728
Asp Asn Pro Asn Thr Tyr Asp Tyr Met Asn Lys Arg Val Val Ala Pro
                565                 570                 575

ggg cta gtg gac tgc tac att aac ctt gga gca cgc tgg tcc ctt gac      1776

Gly Leu Val Asp Cys Tyr Ile Asn Leu Gly Ala Arg Trp Ser Leu Asp
            580                 585                 590

tat atg gac aac gtc aac cca ttt aac cac cac cgc aat gct ggc ctg 1824
Tyr Met Asp Asn Val Asn Pro Phe Asn His His Arg Asn Ala Gly Leu
        595                 600                 605

cgc tac cgc tca atg ttg ctg ggc aat ggt cgc tat gtg ccc ttc cac 1872
Arg Tyr Arg Ser Met Leu Leu Gly Asn Gly Arg Tyr Val Pro Phe His
    610                 615                 620

atc cag gtg cct cag aag ttc ttt gcc att aaa aac ctc ctt ctc ctg 1920
Ile Gln Val Pro Gln Lys Phe Phe Ala Ile Lys Asn Leu Leu Leu Leu
625                 630                 635                 640

ccg ggc tca tac acc tac gag tgg aac ttc agg aag gat gtt aac atg 1968
Pro Gly Ser Tyr Thr Tyr Glu Trp Asn Phe Arg Lys Asp Val Asn Met
                645                 650                 655

gtt ctg cag agc tcc cta gga aat gac cta agg gtt gac gga gcc agc 2016
Val Leu Gln Ser Ser Leu Gly Asn Asp Leu Arg Val Asp Gly Ala Ser
            660                 665                 670

att aag ttt gat agc att tgc ctt tac gcc acc ttc ttc ccc atg gcc 2064
Ile Lys Phe Asp Ser Ile Cys Leu Tyr Ala Thr Phe Phe Pro Met Ala
        675                 680                 685

cac aac acc gcc tcc acg ctt gag gcc atg ctt aga aac gac acc aac 2112
His Asn Thr Ala Ser Thr Leu Glu Ala Met Leu Arg Asn Asp Thr Asn
    690                 695                 700

gac cag tcc ttt aac gac tat ctc tcc gcc gcc aac atg ctc tac cct 2160
Asp Gln Ser Phe Asn Asp Tyr Leu Ser Ala Ala Asn Met Leu Tyr Pro
705                 710                 715                 720

ata ccc gcc aac gct acc aac gtg ccc ata tcc atc ccc tcc cgc aac 2208
Ile Pro Ala Asn Ala Thr Asn Val Pro Ile Ser Ile Pro Ser Arg Asn
                725                 730                 735

tgg gcg gct ttc cgc ggc tgg gcc ttc acg cgc ctt aag act aag gaa 2256
Trp Ala Ala Phe Arg Gly Trp Ala Phe Thr Arg Leu Lys Thr Lys Glu
            740                 745                 750

acc cca tca ctg ggc tcg ggc tac gac cct tat tac acc tac tct ggc 2304
Thr Pro Ser Leu Gly Ser Gly Tyr Asp Pro Tyr Tyr Thr Tyr Ser Gly
        755                 760                 765

tct ata ccc tac cta gat gga acc ttt tac ctc aac cac acc ttt aag 2352
Ser Ile Pro Tyr Leu Asp Gly Thr Phe Tyr Leu Asn His Thr Phe Lys
    770                 775                 780

aag gtg gcc att acc ttt gac tct tct gtc agc tgg cct ggc aat gac 2400
Lys Val Ala Ile Thr Phe Asp Ser Ser Val Ser Trp Pro Gly Asn Asp
785                 790                 795                 800

cgc ctg ctt acc ccc aac gag ttt gaa att aag cgc tca gtt gac ggg 2448
Arg Leu Leu Thr Pro Asn Glu Phe Glu Ile Lys Arg Ser Val Asp Gly
                805                 810                 815

gag ggt tac aac gtt gcc cag tgt aac atg acc aaa gac tgg ttc ctg 2496
Glu Gly Tyr Asn Val Ala Gln Cys Asn Met Thr Lys Asp Trp Phe Leu
            820                 825                 830

gta caa atg cta gct aac tat aac att ggc tac cag ggc ttc tat atc          2544
Val Gln Met Leu Ala Asn Tyr Asn Ile Gly Tyr Gln Gly Phe Tyr Ile
        835                 840                 845

cca gag agc tac aag gac cgc atg tac tcc ttc ttt aga aac ttc cag          2592
Pro Glu Ser Tyr Lys Asp Arg Met Tyr Ser Phe Phe Arg Asn Phe Gln
    850                 855                 860

ccc atg agc cgt cag gtg gtg gat gat act aaa tac aag gac tac caa          2640
Pro Met Ser Arg Gln Val Val Asp Asp Thr Lys Tyr Lys Asp Tyr Gln
865                 870                 875                 880

cag gtg ggc atc cta cac caa cac aac aac tct gga ttt gtt ggc tac          2688
Gln Val Gly Ile Leu His Gln His Asn Asn Ser Gly Phe Val Gly Tyr
                885                 890                 895

ctt gcc ccc acc atg cgc gaa gga cag gcc tac cct gct aac ttc ccc          2736
Leu Ala Pro Thr Met Arg Glu Gly Gln Ala Tyr Pro Ala Asn Phe Pro
            900                 905                 910

tat ccg ctt ata ggc aag acc gca gtt gac agc att acc cag aaa aag          2784
Tyr Pro Leu Ile Gly Lys Thr Ala Val Asp Ser Ile Thr Gln Lys Lys
        915                 920                 925

ttt ctt tgc gat cgc acc ctt tgg cgc atc cca ttc tcc agt aac ttt          2832
Phe Leu Cys Asp Arg Thr Leu Trp Arg Ile Pro Phe Ser Ser Asn Phe
    930                 935                 940

atg tcc atg ggc gca ctc aca gac ctg ggc caa aac ctt ctc tac gcc          2880
Met Ser Met Gly Ala Leu Thr Asp Leu Gly Gln Asn Leu Leu Tyr Ala
945                 950                 955                 960

aac tcc gcc cac gcg cta gac atg act ttt gag gtg gat ccc atg gac          2928
Asn Ser Ala His Ala Leu Asp Met Thr Phe Glu Val Asp Pro Met Asp
                965                 970                 975

gag ccc acc ctt ctt tat gtt ttg ttt gaa gtc ttt gac gtg gtc cgt          2976
Glu Pro Thr Leu Leu Tyr Val Leu Phe Glu Val Phe Asp Val Val Arg
            980                 985                 990

gtg cac cag ccg cac cgc ggc gtc  atc gaa acc gtg tac  ctg cgc acg          3024
Val His Gln Pro His Arg Gly Val  Ile Glu Thr Val Tyr  Leu Arg Thr
        995                 1000                1005

ccc ttc  tcg gcc ggc aac gcc  aca aca taa                                 3054
Pro Phe  Ser Ala Gly Asn Ala  Thr Thr
    1010                1015


<210>  23
<211>  2928
<212>  DNA
<213>  Artificial Sequence

<220>
<223>  (QGPGAP)3-HVR5 modified Hexon protein


<220>
<221>  CDS
<222>  (1)..(2925)

<400>  23

atg gct acc cct tcg atg atg ccg cag tgg tct tac atg cac atc tcg 48
Met Ala Thr Pro Ser Met Met Pro Gln Trp Ser Tyr Met His Ile Ser
1               5                   10                  15

ggc cag gac gcc tcg gag tac ctg agc ccc ggg ctg gtg cag ttt gcc 96
Gly Gln Asp Ala Ser Glu Tyr Leu Ser Pro Gly Leu Val Gln Phe Ala
            20                  25                  30

cgc gcc acc gag acg tac ttc agc ctg aat aac aag ttt aga aac ccc 144
Arg Ala Thr Glu Thr Tyr Phe Ser Leu Asn Asn Lys Phe Arg Asn Pro
        35                  40                  45

acg gtg gcg cct acg cac gac gtg acc aca gac cgg tcc cag cgt ttg 192
Thr Val Ala Pro Thr His Asp Val Thr Thr Asp Arg Ser Gln Arg Leu
    50                  55                  60

acg ctg cgg ttc atc cct gtg gac cgt gag gat act gcg tac tcg tac 240
Thr Leu Arg Phe Ile Pro Val Asp Arg Glu Asp Thr Ala Tyr Ser Tyr
65                  70                  75                  80

aag gcg cgg ttc acc cta gct gtg ggt gat aac cgt gtg ctg gac atg 288
Lys Ala Arg Phe Thr Leu Ala Val Gly Asp Asn Arg Val Leu Asp Met
                85                  90                  95

gct tcc acg tac ttt gac atc cgc ggc gtg ctg gac agg ggc cct act 336
Ala Ser Thr Tyr Phe Asp Ile Arg Gly Val Leu Asp Arg Gly Pro Thr
            100                 105                 110

ttt aag ccc tac tct ggc act gcc tac aac gcc ctg gct ccc aag ggt 384
Phe Lys Pro Tyr Ser Gly Thr Ala Tyr Asn Ala Leu Ala Pro Lys Gly
        115                 120                 125

gcc cca aat cct tgc gaa tgg gat gaa gct gct act gct ctt gaa ata 432
Ala Pro Asn Pro Cys Glu Trp Asp Glu Ala Ala Thr Ala Leu Glu Ile
    130                 135                 140

aac cta gaa gaa gag gac gat gac aac gaa gac gaa gta gac gag caa 480
Asn Leu Glu Glu Glu Asp Asp Asp Asn Glu Asp Glu Val Asp Glu Gln
145                 150                 155                 160

gct gag cag caa aaa act cac gta ttt ggg cag gcg cct tat tct ggt 528
Ala Glu Gln Gln Lys Thr His Val Phe Gly Gln Ala Pro Tyr Ser Gly
                165                 170                 175

ata aat att aca aag gag ggt att caa ata ggt gtc gaa ggt caa aca 576
Ile Asn Ile Thr Lys Glu Gly Ile Gln Ile Gly Val Glu Gly Gln Thr
            180                 185                 190

cct aaa tat gcc gat aaa aca ttt caa cct gaa cct caa ata gga gaa 624
Pro Lys Tyr Ala Asp Lys Thr Phe Gln Pro Glu Pro Gln Ile Gly Glu
        195                 200                 205

tct cag tgg tac gaa aca gaa att aat cat gca gct ggg aga gtc cta 672
Ser Gln Trp Tyr Glu Thr Glu Ile Asn His Ala Ala Gly Arg Val Leu
    210                 215                 220

aaa aag act acc cca atg aaa cca tgt tac ggt tca tat gca aaa ccc 720
Lys Lys Thr Thr Pro Met Lys Pro Cys Tyr Gly Ser Tyr Ala Lys Pro
225                 230                 235                 240

aca aat gaa aat gga ggg caa ggc att ctt gta aag caa caa aat gga 768
Thr Asn Glu Asn Gly Gly Gln Gly Ile Leu Val Lys Gln Gln Asn Gly
                245                 250                 255

aag cta gaa agt caa gtg gaa atg caa ttt ttc tca gct agc cag ggc 816
Lys Leu Glu Ser Gln Val Glu Met Gln Phe Phe Ser Ala Ser Gln Gly
            260                 265                 270

cct gga gct cca cag gga cca ggt gca cct caa ggg cct gga gcc cct 864
Pro Gly Ala Pro Gln Gly Pro Gly Ala Pro Gln Gly Pro Gly Ala Pro
        275                 280                 285

ggc act agc act act gag gca gcc gca ggc aat ggt gat aac ttg act 912
Gly Thr Ser Thr Thr Glu Ala Ala Ala Gly Asn Gly Asp Asn Leu Thr
    290                 295                 300

cct aaa gtg gta ttg tac agt gaa gat gta gat ata gaa acc cca gac 960
Pro Lys Val Val Leu Tyr Ser Glu Asp Val Asp Ile Glu Thr Pro Asp
305                 310                 315                 320

act cat att tct tac atg ccc act att aag gaa ggt aac tca cga gaa 1008
Thr His Ile Ser Tyr Met Pro Thr Ile Lys Glu Gly Asn Ser Arg Glu
                325                 330                 335

cta atg ggc caa caa tct atg ccc aac agg cct aat tac att gct ttt 1056
Leu Met Gly Gln Gln Ser Met Pro Asn Arg Pro Asn Tyr Ile Ala Phe
            340                 345                 350

agg gac aat ttt att ggt cta atg tat tac aac agc acg ggt aat atg 1104
Arg Asp Asn Phe Ile Gly Leu Met Tyr Tyr Asn Ser Thr Gly Asn Met
        355                 360                 365

ggt gtt ctg gcg ggc caa gca tcg cag ttg aat gct gtt gta gat ttg 1152
Gly Val Leu Ala Gly Gln Ala Ser Gln Leu Asn Ala Val Val Asp Leu
    370                 375                 380

caa gac aga aac aca gag ctt tca tac cag ctt ttg ctt gat tcc att 1200
Gln Asp Arg Asn Thr Glu Leu Ser Tyr Gln Leu Leu Leu Asp Ser Ile
385                 390                 395                 400

ggt gat aga acc agg tac ttt tct atg tgg aat cag gct gtt gac agc 1248
Gly Asp Arg Thr Arg Tyr Phe Ser Met Trp Asn Gln Ala Val Asp Ser
                405                 410                 415

tat gat cca gat gtt aga att att gaa aat cat gga act gaa gat gaa 1296
Tyr Asp Pro Asp Val Arg Ile Ile Glu Asn His Gly Thr Glu Asp Glu
            420                 425                 430

ctt cca aat tac tgc ttt cca ctg gga ggt gtg att aat aca gag act 1344
Leu Pro Asn Tyr Cys Phe Pro Leu Gly Gly Val Ile Asn Thr Glu Thr
        435                 440                 445

ctt acc aag gta aaa cct aaa aca ggt cag gaa aat gga tgg gaa aaa 1392
Leu Thr Lys Val Lys Pro Lys Thr Gly Gln Glu Asn Gly Trp Glu Lys
    450                 455                 460

gat gct aca gaa ttt tca gat aaa aat gaa ata aga gtt gga aat aat 1440
Asp Ala Thr Glu Phe Ser Asp Lys Asn Glu Ile Arg Val Gly Asn Asn
465                 470                 475                 480

ttt gcc atg gaa atc aat cta aat gcc aac ctg tgg aga aat ttc ctg 1488
Phe Ala Met Glu Ile Asn Leu Asn Ala Asn Leu Trp Arg Asn Phe Leu
                485                 490                 495

tac tcc aac ata gcg ctg tat ttg ccc gac aag cta aag tac agt cct 1536
Tyr Ser Asn Ile Ala Leu Tyr Leu Pro Asp Lys Leu Lys Tyr Ser Pro

500                 505                 510

tcc aac gta aaa att tct gat aac cca aac acc tac gac tac atg aac       1584
Ser Asn Val Lys Ile Ser Asp Asn Pro Asn Thr Tyr Asp Tyr Met Asn
        515                 520                 525

aag cga gtg gtg gct ccc ggg cta gtg gac tgc tac att aac ctt gga       1632
Lys Arg Val Val Ala Pro Gly Leu Val Asp Cys Tyr Ile Asn Leu Gly
    530                 535                 540

gca cgc tgg tcc ctt gac tat atg gac aac gtc aac cca ttt aac cac       1680
Ala Arg Trp Ser Leu Asp Tyr Met Asp Asn Val Asn Pro Phe Asn His
545                 550                 555                 560

cac cgc aat gct ggc ctg cgc tac cgc tca atg ttg ctg ggc aat ggt       1728
His Arg Asn Ala Gly Leu Arg Tyr Arg Ser Met Leu Leu Gly Asn Gly
                565                 570                 575

cgc tat gtg ccc ttc cac atc cag gtg cct cag aag ttc ttt gcc att       1776
Arg Tyr Val Pro Phe His Ile Gln Val Pro Gln Lys Phe Phe Ala Ile
            580                 585                 590

aaa aac ctc ctt ctc ctg ccg ggc tca tac acc tac gag tgg aac ttc       1824
Lys Asn Leu Leu Leu Leu Pro Gly Ser Tyr Thr Tyr Glu Trp Asn Phe
        595                 600                 605

agg aag gat gtt aac atg gtt ctg cag agc tcc cta gga aat gac cta       1872
Arg Lys Asp Val Asn Met Val Leu Gln Ser Ser Leu Gly Asn Asp Leu
    610                 615                 620

agg gtt gac gga gcc agc att aag ttt gat agc att tgc ctt tac gcc       1920
Arg Val Asp Gly Ala Ser Ile Lys Phe Asp Ser Ile Cys Leu Tyr Ala
625                 630                 635                 640

acc ttc ttc ccc atg gcc cac aac acc gcc tcc acg ctt gag gcc atg       1968
Thr Phe Phe Pro Met Ala His Asn Thr Ala Ser Thr Leu Glu Ala Met
                645                 650                 655

ctt aga aac gac acc aac gac cag tcc ttt aac gac tat ctc tcc gcc       2016
Leu Arg Asn Asp Thr Asn Asp Gln Ser Phe Asn Asp Tyr Leu Ser Ala
            660                 665                 670

gcc aac atg ctc tac cct ata ccc gcc aac gct acc aac gtg ccc ata       2064
Ala Asn Met Leu Tyr Pro Ile Pro Ala Asn Ala Thr Asn Val Pro Ile
        675                 680                 685

tcc atc ccc tcc cgc aac tgg gcg gct ttc cgc ggc tgg gcc ttc acg       2112
Ser Ile Pro Ser Arg Asn Trp Ala Ala Phe Arg Gly Trp Ala Phe Thr
    690                 695                 700

cgc ctt aag act aag gaa acc cca tca ctg ggc tcg ggc tac gac cct       2160
Arg Leu Lys Thr Lys Glu Thr Pro Ser Leu Gly Ser Gly Tyr Asp Pro
705                 710                 715                 720

tat tac acc tac tct ggc tct ata ccc tac cta gat gga acc ttt tac       2208
Tyr Tyr Thr Tyr Ser Gly Ser Ile Pro Tyr Leu Asp Gly Thr Phe Tyr
                725                 730                 735

ctc aac cac acc ttt aag aag gtg gcc att acc ttt gac tct tct gtc       2256
Leu Asn His Thr Phe Lys Lys Val Ala Ile Thr Phe Asp Ser Ser Val
            740                 745                 750

agc tgg cct ggc aat gac cgc ctg ctt acc ccc aac gag ttt gaa att       2304

Ser Trp Pro Gly Asn Asp Arg Leu Leu Thr Pro Asn Glu Phe Glu Ile
        755                 760                 765

aag cgc tca gtt gac ggg gag ggt tac aac gtt gcc cag tgt aac atg      2352
Lys Arg Ser Val Asp Gly Glu Gly Tyr Asn Val Ala Gln Cys Asn Met
    770                 775                 780

acc aaa gac tgg ttc ctg gta caa atg cta gct aac tat aac att ggc      2400
Thr Lys Asp Trp Phe Leu Val Gln Met Leu Ala Asn Tyr Asn Ile Gly
785                 790                 795                 800

tac cag ggc ttc tat atc cca gag agc tac aag gac cgc atg tac tcc      2448
Tyr Gln Gly Phe Tyr Ile Pro Glu Ser Tyr Lys Asp Arg Met Tyr Ser
                805                 810                 815

ttc ttt aga aac ttc cag ccc atg agc cgt cag gtg gtg gat gat act      2496
Phe Phe Arg Asn Phe Gln Pro Met Ser Arg Gln Val Val Asp Asp Thr
            820                 825                 830

aaa tac aag gac tac caa cag gtg ggc atc cta cac caa cac aac aac      2544
Lys Tyr Lys Asp Tyr Gln Gln Val Gly Ile Leu His Gln His Asn Asn
        835                 840                 845

tct gga ttt gtt ggc tac ctt gcc ccc acc atg cgc gaa gga cag gcc      2592
Ser Gly Phe Val Gly Tyr Leu Ala Pro Thr Met Arg Glu Gly Gln Ala
    850                 855                 860

tac cct gct aac ttc ccc tat ccg ctt ata ggc aag acc gca gtt gac      2640
Tyr Pro Ala Asn Phe Pro Tyr Pro Leu Ile Gly Lys Thr Ala Val Asp
865                 870                 875                 880

agc att acc cag aaa aag ttt ctt tgc gat cgc acc ctt tgg cgc atc      2688
Ser Ile Thr Gln Lys Lys Phe Leu Cys Asp Arg Thr Leu Trp Arg Ile
                885                 890                 895

cca ttc tcc agt aac ttt atg tcc atg ggc gca ctc aca gac ctg ggc      2736
Pro Phe Ser Ser Asn Phe Met Ser Met Gly Ala Leu Thr Asp Leu Gly
            900                 905                 910

caa aac ctt ctc tac gcc aac tcc gcc cac gcg cta gac atg act ttt      2784
Gln Asn Leu Leu Tyr Ala Asn Ser Ala His Ala Leu Asp Met Thr Phe
        915                 920                 925

gag gtg gat ccc atg gac gag ccc acc ctt ctt tat gtt ttg ttt gaa      2832
Glu Val Asp Pro Met Asp Glu Pro Thr Leu Leu Tyr Val Leu Phe Glu
    930                 935                 940

gtc ttt gac gtg gtc cgt gtg cac cag ccg cac cgc ggc gtc atc gaa      2880
Val Phe Asp Val Val Arg Val His Gln Pro His Arg Gly Val Ile Glu
945                 950                 955                 960

acc gtg tac ctg cgc acg ccc ttc tcg gcc ggc aac gcc aca aca taa      2928
Thr Val Tyr Leu Arg Thr Pro Phe Ser Ala Gly Asn Ala Thr Thr
                965                 970                 975

<210>  24
<211>  1800
<212>  DNA
<213>  Artificial Sequence

<220>

<223>  (QGPGAP)3 modified fiber protein

<220>
<221>  CDS
<222>  (1)..(1797)

<400>  24
atg aag cgc gca aga ccg tct gaa gat acc ttc aac ccc gtg tat cca      48
Met Lys Arg Ala Arg Pro Ser Glu Asp Thr Phe Asn Pro Val Tyr Pro
1               5                   10                  15

tat gac acg gaa acc ggt cct cca act gtg cct ttt ctt act cct ccc      96
Tyr Asp Thr Glu Thr Gly Pro Pro Thr Val Pro Phe Leu Thr Pro Pro
            20                  25                  30

ttt gta tcc ccc aat ggg ttt caa gag agt ccc cct ggg gta ctc tct      144
Phe Val Ser Pro Asn Gly Phe Gln Glu Ser Pro Pro Gly Val Leu Ser
        35                  40                  45

ttg cgc cta tcc gaa cct cta gtt acc tcc aat ggc atg ctt gcg ctc      192
Leu Arg Leu Ser Glu Pro Leu Val Thr Ser Asn Gly Met Leu Ala Leu
    50                  55                  60

aaa atg ggc aac ggc ctc tct ctg gac gag gcc ggc aac ctt acc tcc      240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac      288
Gln Asn Val Thr Thr Val Ser Pro Pro Leu Lys Lys Thr Lys Ser Asn
                85                  90                  95

ata aac ctg gaa ata tct gca ccc ctc aca gtt acc tca gaa gcc cta      336
Ile Asn Leu Glu Ile Ser Ala Pro Leu Thr Val Thr Ser Glu Ala Leu
            100                 105                 110

act gtg gct gcc gcc gca cct cta atg gtc gcg ggc aac aca ctc acc      384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att      432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa      480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act      528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg      576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg      624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act      672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act 720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca 768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt 816
Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag 864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac 912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag 960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata 1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca 1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat 1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac 1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act 1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag 1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata 1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata 1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat 1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt 1440
Gly Val Leu Leu Asn Asn Ser Phe Leu Asp Pro Glu Tyr Trp Asn Phe

465                 470                 475                 480

aga aat gga gat ctt act gaa ggc aca gcc tat aca aac gct gtt gga      1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc      1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa      1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga cag      1632
Pro Val Thr Leu Thr Ile Thr Leu Asn Gly Thr Gln Glu Thr Gly Gln
    530                 535                 540

ggc cct gga gct cca cag gga cca ggt gca cct caa ggg cct gga gcc      1680
Gly Pro Gly Ala Pro Gln Gly Pro Gly Ala Pro Gln Gly Pro Gly Ala
545                 550                 555                 560

cct gac aca act cca agt gca tac tct atg tca ttt tca tgg gac tgg      1728
Pro Asp Thr Thr Pro Ser Ala Tyr Ser Met Ser Phe Ser Trp Asp Trp
                565                 570                 575

tct ggc cac aac tac att aat gaa ata ttt gcc aca tcc tct tac act      1776
Ser Gly His Asn Tyr Ile Asn Glu Ile Phe Ala Thr Ser Ser Tyr Thr
            580                 585                 590

ttt tca tac att gcc caa gaa taa                                      1800
Phe Ser Tyr Ile Ala Gln Glu
        595

<210>  25
<211>  1794
<212>  DNA
<213>  Artificial Sequence

<220>
<223>  (NANP)4 modified fiber protein

<220>
<221>  CDS
<222>  (1)..(1791)

<400>  25
atg aag cgc gca aga ccg tct gaa gat acc ttc aac ccc gtg tat cca        48
Met Lys Arg Ala Arg Pro Ser Glu Asp Thr Phe Asn Pro Val Tyr Pro
1               5                   10                  15

tat gac acg gaa acc ggt cct cca act gtg cct ttt ctt act cct ccc        96
Tyr Asp Thr Glu Thr Gly Pro Pro Thr Val Pro Phe Leu Thr Pro Pro
            20                  25                  30

ttt gta tcc ccc aat ggg ttt caa gag agt ccc cct ggg gta ctc tct       144
Phe Val Ser Pro Asn Gly Phe Gln Glu Ser Pro Pro Gly Val Leu Ser
        35                  40                  45

ttg cgc cta tcc gaa cct cta gtt acc tcc aat ggc atg ctt gcg ctc       192

Leu Arg Leu Ser Glu Pro Leu Val Thr Ser Asn Gly Met Leu Ala Leu
    50                  55                  60

aaa atg ggc aac ggc ctc tct ctg gac gag gcc ggc aac ctt acc tcc      240
Lys Met Gly Asn Gly Leu Ser Leu Asp Glu Ala Gly Asn Leu Thr Ser
65                  70                  75                  80

caa aat gta acc act gtg agc cca cct ctc aaa aaa acc aag tca aac      288
Gln Asn Val Thr Thr Val Ser Pro Pro Leu Lys Lys Thr Lys Ser Asn
                85                  90                  95

ata aac ctg gaa ata tct gca ccc ctc aca gtt acc tca gaa gcc cta      336
Ile Asn Leu Glu Ile Ser Ala Pro Leu Thr Val Thr Ser Glu Ala Leu
            100                 105                 110

act gtg gct gcc gcc gca cct cta atg gtc gcg ggc aac aca ctc acc      384
Thr Val Ala Ala Ala Ala Pro Leu Met Val Ala Gly Asn Thr Leu Thr
        115                 120                 125

atg caa tca cag gcc ccg cta acc gtg cac gac tcc aaa ctt agc att      432
Met Gln Ser Gln Ala Pro Leu Thr Val His Asp Ser Lys Leu Ser Ile
    130                 135                 140

gcc acc caa gga ccc ctc aca gtg tca gaa gga aag cta gcc ctg caa      480
Ala Thr Gln Gly Pro Leu Thr Val Ser Glu Gly Lys Leu Ala Leu Gln
145                 150                 155                 160

aca tca ggc ccc ctc acc acc acc gat agc agt acc ctt act atc act      528
Thr Ser Gly Pro Leu Thr Thr Thr Asp Ser Ser Thr Leu Thr Ile Thr
                165                 170                 175

gcc tca ccc cct cta act act gcc act ggt agc ttg ggc att gac ttg      576
Ala Ser Pro Pro Leu Thr Thr Ala Thr Gly Ser Leu Gly Ile Asp Leu
            180                 185                 190

aaa gag ccc att tat aca caa aat gga aaa cta gga cta aag tac ggg      624
Lys Glu Pro Ile Tyr Thr Gln Asn Gly Lys Leu Gly Leu Lys Tyr Gly
        195                 200                 205

gct cct ttg cat gta aca gac gac cta aac act ttg acc gta gca act      672
Ala Pro Leu His Val Thr Asp Asp Leu Asn Thr Leu Thr Val Ala Thr
    210                 215                 220

ggt cca ggt gtg act att aat aat act tcc ttg caa act aaa gtt act      720
Gly Pro Gly Val Thr Ile Asn Asn Thr Ser Leu Gln Thr Lys Val Thr
225                 230                 235                 240

gga gcc ttg ggt ttt gat tca caa ggc aat atg caa ctt aat gta gca      768
Gly Ala Leu Gly Phe Asp Ser Gln Gly Asn Met Gln Leu Asn Val Ala
                245                 250                 255

gga gga cta agg att gat tct caa aac aga cgc ctt ata ctt gat gtt      816
Gly Gly Leu Arg Ile Asp Ser Gln Asn Arg Arg Leu Ile Leu Asp Val
            260                 265                 270

agt tat ccg ttt gat gct caa aac caa cta aat cta aga cta gga cag      864
Ser Tyr Pro Phe Asp Ala Gln Asn Gln Leu Asn Leu Arg Leu Gly Gln
        275                 280                 285

ggc cct ctt ttt ata aac tca gcc cac aac ttg gat att aac tac aac      912
Gly Pro Leu Phe Ile Asn Ser Ala His Asn Leu Asp Ile Asn Tyr Asn
    290                 295                 300

aaa ggc ctt tac ttg ttt aca gct tca aac aat tcc aaa aag ctt gag     960
Lys Gly Leu Tyr Leu Phe Thr Ala Ser Asn Asn Ser Lys Lys Leu Glu
305                 310                 315                 320

gtt aac cta agc act gcc aag ggg ttg atg ttt gac gct aca gcc ata     1008
Val Asn Leu Ser Thr Ala Lys Gly Leu Met Phe Asp Ala Thr Ala Ile
                325                 330                 335

gcc att aat gca gga gat ggg ctt gaa ttt ggt tca cct aat gca cca     1056
Ala Ile Asn Ala Gly Asp Gly Leu Glu Phe Gly Ser Pro Asn Ala Pro
            340                 345                 350

aac aca aat ccc ctc aaa aca aaa att ggc cat ggc cta gaa ttt gat     1104
Asn Thr Asn Pro Leu Lys Thr Lys Ile Gly His Gly Leu Glu Phe Asp
        355                 360                 365

tca aac aag gct atg gtt cct aaa cta gga act ggc ctt agt ttt gac     1152
Ser Asn Lys Ala Met Val Pro Lys Leu Gly Thr Gly Leu Ser Phe Asp
    370                 375                 380

agc aca ggt gcc att aca gta gga aac aaa aat aat gat aag cta act     1200
Ser Thr Gly Ala Ile Thr Val Gly Asn Lys Asn Asn Asp Lys Leu Thr
385                 390                 395                 400

ttg tgg acc aca cca gct cca tct cct aac tgt aga cta aat gca gag     1248
Leu Trp Thr Thr Pro Ala Pro Ser Pro Asn Cys Arg Leu Asn Ala Glu
                405                 410                 415

aaa gat gct aaa ctc act ttg gtc tta aca aaa tgt ggc agt caa ata     1296
Lys Asp Ala Lys Leu Thr Leu Val Leu Thr Lys Cys Gly Ser Gln Ile
            420                 425                 430

ctt gct aca gtt tca gtt ttg gct gtt aaa ggc agt ttg gct cca ata     1344
Leu Ala Thr Val Ser Val Leu Ala Val Lys Gly Ser Leu Ala Pro Ile
        435                 440                 445

tct gga aca gtt caa agt gct cat ctt att ata aga ttt gac gaa aat     1392
Ser Gly Thr Val Gln Ser Ala His Leu Ile Ile Arg Phe Asp Glu Asn
    450                 455                 460

gga gtg cta cta aac aat tcc ttc ctg gac cca gaa tat tgg aac ttt     1440
Gly Val Leu Leu Asn Asn Ser Phe Leu Asp Pro Glu Tyr Trp Asn Phe
465                 470                 475                 480

aga aat gga gat ctt act gaa ggc aca gcc tat aca aac gct gtt gga     1488
Arg Asn Gly Asp Leu Thr Glu Gly Thr Ala Tyr Thr Asn Ala Val Gly
                485                 490                 495

ttt atg cct aac cta tca gct tat cca aaa tct cac ggt aaa act gcc     1536
Phe Met Pro Asn Leu Ser Ala Tyr Pro Lys Ser His Gly Lys Thr Ala
            500                 505                 510

aaa agt aac att gtc agt caa gtt tac tta aac gga gac aaa act aaa     1584
Lys Ser Asn Ile Val Ser Gln Val Tyr Leu Asn Gly Asp Lys Thr Lys
        515                 520                 525

cct gta aca cta acc att aca cta aac ggt aca cag gaa aca gga aac     1632
Pro Val Thr Leu Thr Ile Thr Leu Asn Gly Thr Gln Glu Thr Gly Asn
    530                 535                 540

gct aat ccc aac gct aac cca aat gca aat cct aat gcc aac ccc gac     1680
Ala Asn Pro Asn Ala Asn Pro Asn Ala Asn Pro Asn Ala Asn Pro Asp
545                 550                 555                 560

aca act cca agt gca tac tct atg tca ttt tca tgg gac tgg tct ggc 1728
Thr Thr Pro Ser Ala Tyr Ser Met Ser Phe Ser Trp Asp Trp Ser Gly
                565                 570                 575

cac aac tac att aat gaa ata ttt gcc aca tcc tct tac act ttt tca 1776
His Asn Tyr Ile Asn Glu Ile Phe Ala Thr Ser Ser Tyr Thr Phe Ser
            580                 585                 590

tac att gcc caa gaa taa 1794
Tyr Ile Ala Gln Glu
        595

<210> 26
<211> 684
<212> DNA
<213> Artificial Sequence

<220>
<223> P. yoelii CD4+ epitope modified pVII sequence

<220>
<221> CDS
<222> (1)..(681)

<400> 26
atg tcc atc ctt ata tcg ccc agc aat aac aca ggc tgg ggc ctg cgc 48
Met Ser Ile Leu Ile Ser Pro Ser Asn Asn Thr Gly Trp Gly Leu Arg
1               5                   10                  15

ttc cca agc aag atg ttt ggc ggg gcc aag aag cgc tcc gac caa cac 96
Phe Pro Ser Lys Met Phe Gly Gly Ala Lys Lys Arg Ser Asp Gln His
            20                  25                  30

cca gtg cgc gtg cgc ggg cac tac cgc gcg ccc tgg ggc gcg cac aaa 144
Pro Val Arg Val Arg Gly His Tyr Arg Ala Pro Trp Gly Ala His Lys
        35                  40                  45

cgc ggc cgc act ggg cgc acc acc gtc gat gac gcc atc gac gcg gtg 192
Arg Gly Arg Thr Gly Arg Thr Thr Val Asp Asp Ala Ile Asp Ala Val
    50                  55                  60

gtg gag gag gcg cgc aac tac acg ccc acg ccg cca cca gtg tcc aca 240
Val Glu Glu Ala Arg Asn Tyr Thr Pro Thr Pro Pro Pro Val Ser Thr
65                  70                  75                  80

gtg gac gcg gcc att cag acc gtg gtg cgc gga gcc cgg cgc tat gct 288
Val Asp Ala Ala Ile Gln Thr Val Val Arg Gly Ala Arg Arg Tyr Ala
                85                  90                  95

aaa atg aag aga cgg cgg agg cgc gta gca cgt cgc cac cgc cgc cga 336
Lys Met Lys Arg Arg Arg Arg Arg Val Ala Arg Arg His Arg Arg Arg
            100                 105                 110

ccc ggc act gcc gcc caa cgc gcg gcg gcg gcc ctg ctt aac cgc gca 384
Pro Gly Thr Ala Ala Gln Arg Ala Ala Ala Ala Leu Leu Asn Arg Ala
        115                 120                 125

cgt cgc acc ggc cga cgg gcg gcc atg cgg gcc gct cga agg ctg gcc 432
Arg Arg Thr Gly Arg Arg Ala Ala Met Arg Ala Ala Arg Arg Leu Ala

130                 135                 140

gcg ggt att gtc act gtg ccc ccc agg tcc agg cga cga gcg gcc gcc         480
Ala Gly Ile Val Thr Val Pro Pro Arg Ser Arg Arg Arg Ala Ala Ala
145                 150                 155                 160

gca gca gcc gcg gcc att agt gct atg act cag ggt cgc agg ggc aac         528
Ala Ala Ala Ala Ala Ile Ser Ala Met Thr Gln Gly Arg Arg Gly Asn
                165                 170                 175

gtg tat tgg gtg cgc gac tcg gtt agc ggc ctg cgc gtg ccc gtg cgc         576
Val Tyr Trp Val Arg Asp Ser Val Ser Gly Leu Arg Val Pro Val Arg
            180                 185                 190

acc cgc ccc ccg cgc aac gtg cgc acc cgc ccc ccg cgc aac tac aac         624
Thr Arg Pro Pro Arg Asn Val Arg Thr Arg Pro Pro Arg Asn Tyr Asn
        195                 200                 205

agg aac atc gtg aac agg ctg ctg ggc gac gcc ctg aac ggc aag ccc         672
Arg Asn Ile Val Asn Arg Leu Leu Gly Asp Ala Leu Asn Gly Lys Pro
    210                 215                 220

gag gag aag tag                                                         684
Glu Glu Lys
225

<210>  27
<211>  657
<212>  DNA
<213>  Artificial Sequence

<220>
<223>  P. falciparum CD4+ epitope modified pVII-1 sequence

<220>
<221>  CDS
<222>  (1)..(654)

<400>  27
atg tcc atc ctt ata tcg ccc agc aat aac aca ggc tgg ggc ctg cgc          48
Met Ser Ile Leu Ile Ser Pro Ser Asn Asn Thr Gly Trp Gly Leu Arg
1               5                   10                  15

ttc cca agc aag atg ttt ggc ggg gcc aag aag cgc tcc gac caa cac          96
Phe Pro Ser Lys Met Phe Gly Gly Ala Lys Lys Arg Ser Asp Gln His
            20                  25                  30

cca gtg cgc gtg cgc ggg cac tac cgc gcg ccc tgg ggc gcg cac aaa         144
Pro Val Arg Val Arg Gly His Tyr Arg Ala Pro Trp Gly Ala His Lys
        35                  40                  45

cgc ggc cgc act ggg cgc acc acc gtc gat gac gcc atc gac gcg gtg         192
Arg Gly Arg Thr Gly Arg Thr Thr Val Asp Asp Ala Ile Asp Ala Val
    50                  55                  60

gtg gag gag gcg cgc aac tac acg ccc acg ccg cca cca gtg tcc aca         240
Val Glu Glu Ala Arg Asn Tyr Thr Pro Thr Pro Pro Pro Val Ser Thr
65                  70                  75                  80

gtg gac gcg gcc att cag acc gtg gtg cgc gga gcc cgg cgc tat gct         288

Val Asp Ala Ala Ile Gln Thr Val Val Arg Gly Ala Arg Arg Tyr Ala
                85                  90                  95

aaa atg aag aga cgg cgg agg cgc gta gca cgt cgc cac cgc cgc cga      336
Lys Met Lys Arg Arg Arg Arg Arg Val Ala Arg Arg His Arg Arg Arg
            100                 105                 110

ccc ggc act gcc gcc caa cgc gcg gcg gcg gcc ctg ctt aac cgc gca      384
Pro Gly Thr Ala Ala Gln Arg Ala Ala Ala Ala Leu Leu Asn Arg Ala
        115                 120                 125

cgt cgc acc ggc cga cgg gcg gcc atg cgg gcc gct cga agg ctg gcc      432
Arg Arg Thr Gly Arg Arg Ala Ala Met Arg Ala Ala Arg Arg Leu Ala
    130                 135                 140

gcg ggt att gtc act gtg ccc ccc agg tcc agg cga cga gcg gcc gcc      480
Ala Gly Ile Val Thr Val Pro Pro Arg Ser Arg Arg Arg Ala Ala Ala
145                 150                 155                 160

gca gca gcc gcg gcc att agt gct atg act cag ggt cgc agg ggc aac      528
Ala Ala Ala Ala Ala Ile Ser Ala Met Thr Gln Gly Arg Arg Gly Asn
                165                 170                 175

gtg tat tgg gtg cgc gac tcg gtt agc ggc ctg cgc gtg ccc gtg cgc      576
Val Tyr Trp Val Arg Asp Ser Val Ser Gly Leu Arg Val Pro Val Arg
            180                 185                 190

acc cgc ccc ccg cgc aac gaa tat ttg aac aag att caa aac tcc ctc      624
Thr Arg Pro Pro Arg Asn Glu Tyr Leu Asn Lys Ile Gln Asn Ser Leu
        195                 200                 205

tca aca gaa tgg tct cct tgc agc gtg act tag                          657
Ser Thr Glu Trp Ser Pro Cys Ser Val Thr
    210                 215

<210>  28
<211>  741
<212>  DNA
<213>  Artificial Sequence

<220>
<223>  P. falciparum CD4+ epitope modified pVII-2 sequence

<220>
<221>  CDS
<222>  (1)..(738)

<400>  28
atg tcc atc ctt ata tcg ccc agc aat aac aca ggc tgg ggc ctg cgc       48
Met Ser Ile Leu Ile Ser Pro Ser Asn Asn Thr Gly Trp Gly Leu Arg
1               5                   10                  15

ttc cca agc aag atg ttt ggc ggg gcc aag aag cgc tcc gac caa cac       96
Phe Pro Ser Lys Met Phe Gly Gly Ala Lys Lys Arg Ser Asp Gln His
            20                  25                  30

cca gtg cgc gtg cgc ggg cac tac cgc gcg ccc tgg ggc gcg cac aaa      144
Pro Val Arg Val Arg Gly His Tyr Arg Ala Pro Trp Gly Ala His Lys
        35                  40                  45

cgc ggc cgc act ggg cgc acc acc gtc gat gac gcc atc gac gcg gtg     192
Arg Gly Arg Thr Gly Arg Thr Thr Val Asp Asp Ala Ile Asp Ala Val
    50                  55                  60

gtg gag gag gcg cgc aac tac acg ccc acg ccg cca cca gtg tcc aca     240
Val Glu Glu Ala Arg Asn Tyr Thr Pro Thr Pro Pro Pro Val Ser Thr
65                  70                  75                  80

gtg gac gcg gcc att cag acc gtg gtg cgc gga gcc gaa tat ttg aac     288
Val Asp Ala Ala Ile Gln Thr Val Val Arg Gly Ala Glu Tyr Leu Asn
                85                  90                  95

aag att caa aac tcc ctc tca aca gaa tgg tct cct tgc agc gtg act     336
Lys Ile Gln Asn Ser Leu Ser Thr Glu Trp Ser Pro Cys Ser Val Thr
            100                 105                 110

cgg cgc tat gct aaa atg aag aga cgg cgg agg cgc gta gca cgt cgc     384
Arg Arg Tyr Ala Lys Met Lys Arg Arg Arg Arg Arg Val Ala Arg Arg
        115                 120                 125

cac cgc cgc cga ccc ggc act gcc gcc caa cgc gcg gcg gcg gcc ctg     432
His Arg Arg Arg Pro Gly Thr Ala Ala Gln Arg Ala Ala Ala Ala Leu
    130                 135                 140

ctt aac cgc gca cgt cgc acc ggc cga cgg gcg gcc atg cgg gcc gct     480
Leu Asn Arg Ala Arg Arg Thr Gly Arg Arg Ala Ala Met Arg Ala Ala
145                 150                 155                 160

cga agg ctg gcc gcg ggt att gtc act gtg ccc ccc agg tcc agg cga     528
Arg Arg Leu Ala Ala Gly Ile Val Thr Val Pro Pro Arg Ser Arg Arg
                165                 170                 175

cga gcg gcc gcc gca gca gcc gcg gcc att agt gct atg act cag ggt     576
Arg Ala Ala Ala Ala Ala Ala Ala Ala Ile Ser Ala Met Thr Gln Gly
            180                 185                 190

cgc agg ggc aac gtg tat tgg gtg cgc gac tcg gtt agc ggc ctg cgc     624
Arg Arg Gly Asn Val Tyr Trp Val Arg Asp Ser Val Ser Gly Leu Arg
        195                 200                 205

gtg ccc gtg cgc acc cgc ccc ccg cgc aac gtg cgc acc cgc ccc ccg     672
Val Pro Val Arg Thr Arg Pro Pro Arg Asn Val Arg Thr Arg Pro Pro
    210                 215                 220

cgc aac tac aac agg aac atc gtg aac agg ctg ctg ggc gac gcc ctg     720
Arg Asn Tyr Asn Arg Asn Ile Val Asn Arg Leu Leu Gly Asp Ala Leu
225                 230                 235                 240

acc cgc ccc ccg cgc aac tag                                         741
Thr Arg Pro Pro Arg Asn
                245


<210>  29
<211>  681
<212>  DNA
<213>  Artificial Sequence

<220>
<223>  P. falciparum CD4+ epitope modified PVII-3 sequence


<220>

<221>  CDS
<222>  (1)..(678)

<400>  29
atg tcc atc ctt ata tcg ccc agc aat aac aca ggc tgg ggc ctg cgc      48
Met Ser Ile Leu Ile Ser Pro Ser Asn Asn Thr Gly Trp Gly Leu Arg
1               5                   10                  15

ttc cca agc aag atg ttt ggc ggg gcc aag aag cgc tcc gac caa cac      96
Phe Pro Ser Lys Met Phe Gly Gly Ala Lys Lys Arg Ser Asp Gln His
            20                  25                  30

cca gtg cgc gtg cgc ggg cac tac cgc gcg ccc tgg ggc gcg cac aaa      144
Pro Val Arg Val Arg Gly His Tyr Arg Ala Pro Trp Gly Ala His Lys
        35                  40                  45

cgc ggc cgc act ggg cgc acc acc gtc gat gac gcc atc gac gcg gtg      192
Arg Gly Arg Thr Gly Arg Thr Thr Val Asp Asp Ala Ile Asp Ala Val
    50                  55                  60

gtg gag gag gcg cgc aac tac acg ccc acg ccg cca cca gtg tcc aca      240
Val Glu Glu Ala Arg Asn Tyr Thr Pro Thr Pro Pro Pro Val Ser Thr
65                  70                  75                  80

gtg gac gcg gcc att cag acc gtg gtg cgc gga gcc cgg cgc tat gct      288
Val Asp Ala Ala Ile Gln Thr Val Val Arg Gly Ala Arg Arg Tyr Ala
                85                  90                  95

aaa atg aag aga cgg cgg agg cgc gta gca cgt cgc cac cgc cgc cga      336
Lys Met Lys Arg Arg Arg Arg Arg Val Ala Arg Arg His Arg Arg Arg
            100                 105                 110

ccc ggc act gcc gcc caa cgc gcg gcg gcg gcc ctg ctt aac cgc gca      384
Pro Gly Thr Ala Ala Gln Arg Ala Ala Ala Ala Leu Leu Asn Arg Ala
        115                 120                 125

cgt cgc acc ggc cga cgg gcg gcc atg cgg gcc gct gaa tat ttg aac      432
Arg Arg Thr Gly Arg Arg Ala Ala Met Arg Ala Ala Glu Tyr Leu Asn
    130                 135                 140

aag att caa aac tcc ctc tca aca gaa tgg tct cct tgc agc gtg act      480
Lys Ile Gln Asn Ser Leu Ser Thr Glu Trp Ser Pro Cys Ser Val Thr
145                 150                 155                 160

cga agg ctg gcc gcg ggt att gtc act gtg ccc ccc agg tcc agg cga      528
Arg Arg Leu Ala Ala Gly Ile Val Thr Val Pro Pro Arg Ser Arg Arg
                165                 170                 175

cga gcg gcc gcc gca gca gcc gcg gcc att agt gct atg act cag ggt      576
Arg Ala Ala Ala Ala Ala Ala Ala Ala Ile Ser Ala Met Thr Gln Gly
            180                 185                 190

cgc agg ggc aac gtg tat tgg gtg cgc gac tcg gtt agc ggc ctg cgc      624
Arg Arg Gly Asn Val Tyr Trp Val Arg Asp Ser Val Ser Gly Leu Arg
        195                 200                 205

gtg ccc gtg cgc acc cgc ccc ccg cgc aac gtg cgc acc cgc ccc ccg      672
Val Pro Val Arg Thr Arg Pro Pro Arg Asn Val Arg Thr Arg Pro Pro
    210                 215                 220

cgc aac tag                                                          681
Arg Asn
225

<210>  30
<211>  369
<212>  PRT
<213>  Artificial Sequence

<220>
<223>  Synthetic Construct

<400>  30

Met Lys Lys Cys Thr Ile Leu Val Val Ala Ser Leu Leu Leu Val Asp
1               5                   10                  15

Ser Leu Leu Pro Gly Tyr Gly Gln Asn Lys Ser Val Gln Ala Gln Arg
            20                  25                  30

Asn Leu Asn Glu Leu Cys Tyr Asn Glu Glu Asn Asp Asn Lys Leu Tyr
        35                  40                  45

His Val Leu Asn Ser Lys Asn Gly Lys Ile Tyr Asn Arg Asn Ile Val
    50                  55                  60

Asn Arg Leu Leu Gly Asp Ala Leu Asn Gly Lys Pro Glu Glu Lys Lys
65                  70                  75                  80

Asp Asp Pro Pro Lys Asp Asp Asn Lys Asp Asp Leu Pro Lys Glu Glu
                85                  90                  95

Lys Lys Asp Asp Leu Pro Lys Glu Glu Lys Lys Asp Asp Pro Pro Lys
            100                 105                 110

Asp Pro Lys Lys Asp Asp Pro Pro Lys Glu Ala Gln Asn Lys Leu Asn
        115                 120                 125

Gln Pro Val Val Ala Asp Glu Asn Val Asp Gln Gly Pro Gly Ala Pro
    130                 135                 140

Gln Gly Pro Gly Ala Pro Gln Gly Pro Gly Ala Pro Gln Gly Pro Gly
145                 150                 155                 160

Ala Pro Gln Gly Pro Gly Ala Pro Gln Gly Pro Gly Ala Pro Gln Gly
                165                 170                 175

Pro Gly Ala Pro Gln Gly Pro Gly Ala Pro Gln Gly Pro Gly Ala Pro
            180                 185                 190

Gln Gly Pro Gly Ala Pro Gln Gly Pro Gly Ala Pro Gln Gly Pro Gly
        195                 200                 205

Ala Pro Gln Gly Pro Gly Ala Pro Gln Gly Pro Gly Ala Pro Gln Gly
    210                 215                 220

Pro Gly Ala Pro Gln Gly Pro Gly Ala Pro Gln Gly Pro Gly Ala Pro
225                 230                 235                 240

Gln Gly Pro Gly Ala Pro Gln Gly Pro Gly Ala Pro Gln Glu Pro Pro
                245                 250                 255

Gln Gln Pro Pro Gln Gln Pro Pro Gln Gln Pro Pro Gln Gln Pro Pro
            260                 265                 270

Gln Gln Pro Pro Gln Gln Pro Asn Asn Asn Asn Asn Asn Asn Gly Asn
        275                 280                 285

Asn Asn Glu Asp Ser Tyr Val Pro Ser Ala Glu Gln Ile Leu Glu Phe
    290                 295                 300

Val Lys Gln Ile Ser Ser Gln Leu Thr Glu Glu Trp Ser Gln Cys Ser
305                 310                 315                 320

Val Thr Cys Gly Ser Gly Val Arg Val Arg Lys Arg Lys Asn Val Asn
                325                 330                 335

Lys Gln Pro Glu Asn Leu Thr Leu Glu Asp Ile Asp Thr Glu Ile Cys
            340                 345                 350

Lys Met Asp Lys Cys Ser Ser Ile Phe Asn Ile Val Ser Asn Ser Leu
        355                 360                 365

Gly

<210>  31
<211>  943
<212>  PRT
<213>  Artificial Sequence

<220>
<223>  Synthetic Construct

<400>  31

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Lys Thr His Val Phe
145                 150                 155                 160

Gly Gln Ala Pro Tyr Ser Gly Ile Asn Ile Thr Lys Glu Gly Ile Gln
                165                 170                 175

Ile Gly Val Glu Gly Gln Thr Pro Lys Tyr Ala Asp Lys Thr Phe Gln
            180                 185                 190

Pro Glu Pro Gln Ile Gly Glu Ser Gln Trp Tyr Glu Thr Glu Ile Asn
        195                 200                 205

His Ala Ala Gly Arg Val Leu Lys Lys Thr Thr Pro Met Lys Pro Cys
    210                 215                 220

Tyr Gly Ser Tyr Ala Lys Pro Thr Asn Glu Asn Gly Gly Gln Gly Ile
225                 230                 235                 240

Leu Val Lys Gln Gln Asn Gly Lys Leu Glu Ser Gln Val Glu Met Gln
                245                 250                 255

Phe Phe Ser Thr Thr Glu Ala Ala Ala Gly Asn Gly Asp Asn Leu Thr
            260                 265                 270

Pro Lys Val Val Leu Tyr Ser Glu Asp Val Asp Ile Glu Thr Pro Asp

275                 280                 285

Thr His Ile Ser Tyr Met Pro Thr Ile Lys Glu Gly Asn Ser Arg Glu
    290                 295                 300

Leu Met Gly Gln Gln Ser Met Pro Asn Arg Pro Asn Tyr Ile Ala Phe
305                 310                 315                 320

Arg Asp Asn Phe Ile Gly Leu Met Tyr Tyr Asn Ser Thr Gly Asn Met
                325                 330                 335

Gly Val Leu Ala Gly Gln Ala Ser Gln Leu Asn Ala Val Val Asp Leu
            340                 345                 350

Gln Asp Arg Asn Thr Glu Leu Ser Tyr Gln Leu Leu Leu Asp Ser Ile
        355                 360                 365

Gly Asp Arg Thr Arg Tyr Phe Ser Met Trp Asn Gln Ala Val Asp Ser
    370                 375                 380

Tyr Asp Pro Asp Val Arg Ile Ile Glu Asn His Gly Thr Glu Asp Glu
385                 390                 395                 400

Leu Pro Asn Tyr Cys Phe Pro Leu Gly Gly Val Ile Asn Thr Glu Thr
                405                 410                 415

Leu Thr Lys Val Lys Pro Lys Thr Gly Gln Glu Asn Gly Trp Glu Lys
            420                 425                 430

Asp Ala Thr Glu Phe Ser Asp Lys Asn Glu Ile Arg Val Gly Asn Asn
        435                 440                 445

Phe Ala Met Glu Ile Asn Leu Asn Ala Asn Leu Trp Arg Asn Phe Leu
    450                 455                 460

Tyr Ser Asn Ile Ala Leu Tyr Leu Pro Asp Lys Leu Lys Tyr Ser Pro
465                 470                 475                 480

Ser Asn Val Lys Ile Ser Asp Asn Pro Asn Thr Tyr Asp Tyr Met Asn
                485                 490                 495

Lys Arg Val Val Ala Pro Gly Leu Val Asp Cys Tyr Ile Asn Leu Gly
            500                 505                 510

Ala Arg Trp Ser Leu Asp Tyr Met Asp Asn Val Asn Pro Phe Asn His
        515                 520                 525

His Arg Asn Ala Gly Leu Arg Tyr Arg Ser Met Leu Leu Gly Asn Gly
    530                 535                 540

Arg Tyr Val Pro Phe His Ile Gln Val Pro Gln Lys Phe Phe Ala Ile
545                 550                 555                 560

Lys Asn Leu Leu Leu Leu Pro Gly Ser Tyr Thr Tyr Glu Trp Asn Phe
                565                 570                 575

Arg Lys Asp Val Asn Met Val Leu Gln Ser Ser Leu Gly Asn Asp Leu
            580                 585                 590

Arg Val Asp Gly Ala Ser Ile Lys Phe Asp Ser Ile Cys Leu Tyr Ala
        595                 600                 605

Thr Phe Phe Pro Met Ala His Asn Thr Ala Ser Thr Leu Glu Ala Met
    610                 615                 620

Leu Arg Asn Asp Thr Asn Asp Gln Ser Phe Asn Asp Tyr Leu Ser Ala
625                 630                 635                 640

Ala Asn Met Leu Tyr Pro Ile Pro Ala Asn Ala Thr Asn Val Pro Ile
                645                 650                 655

Ser Ile Pro Ser Arg Asn Trp Ala Ala Phe Arg Gly Trp Ala Phe Thr
            660                 665                 670

Arg Leu Lys Thr Lys Glu Thr Pro Ser Leu Gly Ser Gly Tyr Asp Pro
        675                 680                 685

Tyr Tyr Thr Tyr Ser Gly Ser Ile Pro Tyr Leu Asp Gly Thr Phe Tyr
    690                 695                 700

Leu Asn His Thr Phe Lys Lys Val Ala Ile Thr Phe Asp Ser Ser Val
705                 710                 715                 720

Ser Trp Pro Gly Asn Asp Arg Leu Leu Thr Pro Asn Glu Phe Glu Ile
                725                 730                 735

Lys Arg Ser Val Asp Gly Glu Gly Tyr Asn Val Ala Gln Cys Asn Met
            740                 745                 750

Thr Lys Asp Trp Phe Leu Val Gln Met Leu Ala Asn Tyr Asn Ile Gly
        755                 760                 765

Tyr Gln Gly Phe Tyr Ile Pro Glu Ser Tyr Lys Asp Arg Met Tyr Ser
    770                 775                 780

Phe Phe Arg Asn Phe Gln Pro Met Ser Arg Gln Val Val Asp Asp Thr
785                 790                 795                 800

Lys Tyr Lys Asp Tyr Gln Gln Val Gly Ile Leu His Gln His Asn Asn
                805                 810                 815

Ser Gly Phe Val Gly Tyr Leu Ala Pro Thr Met Arg Glu Gly Gln Ala
            820                 825                 830

Tyr Pro Ala Asn Phe Pro Tyr Pro Leu Ile Gly Lys Thr Ala Val Asp
        835                 840                 845

Ser Ile Thr Gln Lys Lys Phe Leu Cys Asp Arg Thr Leu Trp Arg Ile
    850                 855                 860

Pro Phe Ser Ser Asn Phe Met Ser Met Gly Ala Leu Thr Asp Leu Gly
865                 870                 875                 880

Gln Asn Leu Leu Tyr Ala Asn Ser Ala His Ala Leu Asp Met Thr Phe
                885                 890                 895

Glu Val Asp Pro Met Asp Glu Pro Thr Leu Leu Tyr Val Leu Phe Glu
            900                 905                 910

Val Phe Asp Val Val Arg Val His Gln Pro His Arg Gly Val Ile Glu
        915                 920                 925

Thr Val Tyr Leu Arg Thr Pro Phe Ser Ala Gly Asn Ala Thr Thr
    930                 935                 940

<210>  32
<211>  929
<212>  PRT
<213>  Artificial Sequence

<220>
<223>  Synthetic Construct

<400>  32

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Gln Gly Pro Gly Ala
145                 150                 155                 160

Pro Lys Thr His Val Phe Gly Gln Ala Pro Tyr Ser Gly Ile Asn Ile
                165                 170                 175

Thr Lys Glu Gly Ile Gln Ile Gly Val Glu Gly Gln Thr Pro Lys Tyr
            180                 185                 190

Ala Asp Lys Thr Phe Gln Pro Glu Pro Gln Ile Gly Glu Ser Gln Trp
        195                 200                 205

Tyr Glu Thr Glu Ile Asn His Ala Ala Gly Arg Val Leu Lys Lys Thr
    210                 215                 220

Thr Pro Met Lys Pro Cys Tyr Gly Ser Tyr Ala Lys Pro Thr Asn Glu
225                 230                 235                 240

Asn Gly Gly Gln Gly Ile Leu Val Lys Gln Gln Asn Gly Lys Leu Glu
                245                 250                 255

Ser Gln Val Glu Met Gln Phe Phe Ser Thr Thr Glu Ala Ala Ala Gly
            260                 265                 270

Asn Gly Asp Asn Leu Thr Pro Lys Val Val Leu Tyr Ser Glu Asp Val
        275                 280                 285

Asp Ile Glu Thr Pro Asp Thr His Ile Ser Tyr Met Pro Thr Ile Lys

290                 295                 300

Glu Gly Asn Ser Arg Glu Leu Met Gly Gln Gln Ser Met Pro Asn Arg
305                 310                 315                 320

Pro Asn Tyr Ile Ala Phe Arg Asp Asn Phe Ile Gly Leu Met Tyr Tyr
                325                 330                 335

Asn Ser Thr Gly Asn Met Gly Val Leu Ala Gly Gln Ala Ser Gln Leu
            340                 345                 350

Asn Ala Val Val Asp Leu Gln Asp Arg Asn Thr Glu Leu Ser Tyr Gln
        355                 360                 365

Leu Leu Leu Asp Ser Ile Gly Asp Arg Thr Arg Tyr Phe Ser Met Trp
    370                 375                 380

Asn Gln Leu Pro Asn Tyr Cys Phe Pro Leu Gly Gly Val Ile Asn Thr
385                 390                 395                 400

Glu Thr Leu Thr Lys Val Lys Pro Lys Thr Gly Gln Glu Asn Gly Trp
                405                 410                 415

Glu Lys Asp Ala Thr Glu Phe Ser Asp Lys Asn Glu Ile Arg Val Gly
            420                 425                 430

Asn Asn Phe Ala Met Glu Ile Asn Leu Asn Ala Asn Leu Trp Arg Asn
        435                 440                 445

Phe Leu Tyr Ser Asn Ile Ala Leu Tyr Leu Pro Asp Lys Leu Lys Tyr
    450                 455                 460

Ser Pro Ser Asn Val Lys Ile Ser Asp Asn Pro Asn Thr Tyr Asp Tyr
465                 470                 475                 480

Met Asn Lys Arg Val Val Ala Pro Gly Leu Val Asp Cys Tyr Ile Asn
                485                 490                 495

Leu Gly Ala Arg Trp Ser Leu Asp Tyr Met Asp Asn Val Asn Pro Phe
            500                 505                 510

Asn His His Arg Asn Ala Gly Leu Arg Tyr Arg Ser Met Leu Leu Gly
        515                 520                 525

Asn Gly Arg Tyr Val Pro Phe His Ile Gln Val Pro Gln Lys Phe Phe
    530                 535                 540

Ala Ile Lys Asn Leu Leu Leu Leu Pro Gly Ser Tyr Thr Tyr Glu Trp
545                 550                 555                 560

Asn Phe Arg Lys Asp Val Asn Met Val Leu Gln Ser Ser Leu Gly Asn
                565                 570                 575

Asp Leu Arg Val Asp Gly Ala Ser Ile Lys Phe Asp Ser Ile Cys Leu
            580                 585                 590

Tyr Ala Thr Phe Phe Pro Met Ala His Asn Thr Ala Ser Thr Leu Glu
        595                 600                 605

Ala Met Leu Arg Asn Asp Thr Asn Asp Gln Ser Phe Asn Asp Tyr Leu
    610                 615                 620

Ser Ala Ala Asn Met Leu Tyr Pro Ile Pro Ala Asn Ala Thr Asn Val
625                 630                 635                 640

Pro Ile Ser Ile Pro Ser Arg Asn Trp Ala Ala Phe Arg Gly Trp Ala
                645                 650                 655

Phe Thr Arg Leu Lys Thr Lys Glu Thr Pro Ser Leu Gly Ser Gly Tyr
            660                 665                 670

Asp Pro Tyr Tyr Thr Tyr Ser Gly Ser Ile Pro Tyr Leu Asp Gly Thr
        675                 680                 685

Phe Tyr Leu Asn His Thr Phe Lys Lys Val Ala Ile Thr Phe Asp Ser
    690                 695                 700

Ser Val Ser Trp Pro Gly Asn Asp Arg Leu Leu Thr Pro Asn Glu Phe
705                 710                 715                 720

Glu Ile Lys Arg Ser Val Asp Gly Glu Gly Tyr Asn Val Ala Gln Cys
                725                 730                 735

Asn Met Thr Lys Asp Trp Phe Leu Val Gln Met Leu Ala Asn Tyr Asn
            740                 745                 750

Ile Gly Tyr Gln Gly Phe Tyr Ile Pro Glu Ser Tyr Lys Asp Arg Met
        755                 760                 765

Tyr Ser Phe Phe Arg Asn Phe Gln Pro Met Ser Arg Gln Val Val Asp
    770                 775                 780

Asp Thr Lys Tyr Lys Asp Tyr Gln Gln Val Gly Ile Leu His Gln His
785                 790                 795                 800

Asn Asn Ser Gly Phe Val Gly Tyr Leu Ala Pro Thr Met Arg Glu Gly
                805                 810                 815

Gln Ala Tyr Pro Ala Asn Phe Pro Tyr Pro Leu Ile Gly Lys Thr Ala
            820                 825                 830

Val Asp Ser Ile Thr Gln Lys Lys Phe Leu Cys Asp Arg Thr Leu Trp
        835                 840                 845

Arg Ile Pro Phe Ser Ser Asn Phe Met Ser Met Gly Ala Leu Thr Asp
    850                 855                 860

Leu Gly Gln Asn Leu Leu Tyr Ala Asn Ser Ala His Ala Leu Asp Met
865                 870                 875                 880

Thr Phe Glu Val Asp Pro Met Asp Glu Pro Thr Leu Leu Tyr Val Leu
                885                 890                 895

Phe Glu Val Phe Asp Val Val Arg Val His Gln Pro His Arg Gly Val
            900                 905                 910

Ile Glu Thr Val Tyr Leu Arg Thr Pro Phe Ser Ala Gly Asn Ala Thr
        915                 920                 925

Thr

<210>  33
<211>  935
<212>  PRT
<213>  Artificial Sequence

<220>
<223>  Synthetic Construct

<400>  33

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Gln Gly Pro Gly Ala
145                 150                 155                 160

Pro Gln Gly Pro Gly Ala Pro Lys Thr His Val Phe Gly Gln Ala Pro
                165                 170                 175

Tyr Ser Gly Ile Asn Ile Thr Lys Glu Gly Ile Gln Ile Gly Val Glu
            180                 185                 190

Gly Gln Thr Pro Lys Tyr Ala Asp Lys Thr Phe Gln Pro Glu Pro Gln
        195                 200                 205

Ile Gly Glu Ser Gln Trp Tyr Glu Thr Glu Ile Asn His Ala Ala Gly
    210                 215                 220

Arg Val Leu Lys Lys Thr Thr Pro Met Lys Pro Cys Tyr Gly Ser Tyr
225                 230                 235                 240

Ala Lys Pro Thr Asn Glu Asn Gly Gly Gln Gly Ile Leu Val Lys Gln
                245                 250                 255

Gln Asn Gly Lys Leu Glu Ser Gln Val Glu Met Gln Phe Phe Ser Thr
            260                 265                 270

Thr Glu Ala Ala Ala Gly Asn Gly Asp Asn Leu Thr Pro Lys Val Val
        275                 280                 285

Leu Tyr Ser Glu Asp Val Asp Ile Glu Thr Pro Asp Thr His Ile Ser
    290                 295                 300

Tyr Met Pro Thr Ile Lys Glu Gly Asn Ser Arg Glu Leu Met Gly Gln

305                 310                 315                 320

Gln Ser Met Pro Asn Arg Pro Asn Tyr Ile Ala Phe Arg Asp Asn Phe
                325                 330                 335

Ile Gly Leu Met Tyr Tyr Asn Ser Thr Gly Asn Met Gly Val Leu Ala
            340                 345                 350

Gly Gln Ala Ser Gln Leu Asn Ala Val Val Asp Leu Gln Asp Arg Asn
        355                 360                 365

Thr Glu Leu Ser Tyr Gln Leu Leu Leu Asp Ser Ile Gly Asp Arg Thr
    370                 375                 380

Arg Tyr Phe Ser Met Trp Asn Gln Leu Pro Asn Tyr Cys Phe Pro Leu
385                 390                 395                 400

Gly Gly Val Ile Asn Thr Glu Thr Leu Thr Lys Val Lys Pro Lys Thr
                405                 410                 415

Gly Gln Glu Asn Gly Trp Glu Lys Asp Ala Thr Glu Phe Ser Asp Lys
            420                 425                 430

Asn Glu Ile Arg Val Gly Asn Asn Phe Ala Met Glu Ile Asn Leu Asn
        435                 440                 445

Ala Asn Leu Trp Arg Asn Phe Leu Tyr Ser Asn Ile Ala Leu Tyr Leu
    450                 455                 460

Pro Asp Lys Leu Lys Tyr Ser Pro Ser Asn Val Lys Ile Ser Asp Asn
465                 470                 475                 480

Pro Asn Thr Tyr Asp Tyr Met Asn Lys Arg Val Val Ala Pro Gly Leu
                485                 490                 495

Val Asp Cys Tyr Ile Asn Leu Gly Ala Arg Trp Ser Leu Asp Tyr Met
            500                 505                 510

Asp Asn Val Asn Pro Phe Asn His His Arg Asn Ala Gly Leu Arg Tyr
        515                 520                 525

Arg Ser Met Leu Leu Gly Asn Gly Arg Tyr Val Pro Phe His Ile Gln
    530                 535                 540

Val Pro Gln Lys Phe Phe Ala Ile Lys Asn Leu Leu Leu Leu Pro Gly
545                 550                 555                 560

Ser Tyr Thr Tyr Glu Trp Asn Phe Arg Lys Asp Val Asn Met Val Leu
                565                 570                 575

Gln Ser Ser Leu Gly Asn Asp Leu Arg Val Asp Gly Ala Ser Ile Lys
            580                 585                 590

Phe Asp Ser Ile Cys Leu Tyr Ala Thr Phe Phe Pro Met Ala His Asn
        595                 600                 605

Thr Ala Ser Thr Leu Glu Ala Met Leu Arg Asn Asp Thr Asn Asp Gln
    610                 615                 620

Ser Phe Asn Asp Tyr Leu Ser Ala Ala Asn Met Leu Tyr Pro Ile Pro
625                 630                 635                 640

Ala Asn Ala Thr Asn Val Pro Ile Ser Ile Pro Ser Arg Asn Trp Ala
                645                 650                 655

Ala Phe Arg Gly Trp Ala Phe Thr Arg Leu Lys Thr Lys Glu Thr Pro
            660                 665                 670

Ser Leu Gly Ser Gly Tyr Asp Pro Tyr Tyr Thr Tyr Ser Gly Ser Ile
        675                 680                 685

Pro Tyr Leu Asp Gly Thr Phe Tyr Leu Asn His Thr Phe Lys Lys Val
    690                 695                 700

Ala Ile Thr Phe Asp Ser Ser Val Ser Trp Pro Gly Asn Asp Arg Leu
705                 710                 715                 720

Leu Thr Pro Asn Glu Phe Glu Ile Lys Arg Ser Val Asp Gly Glu Gly
                725                 730                 735

Tyr Asn Val Ala Gln Cys Asn Met Thr Lys Asp Trp Phe Leu Val Gln
            740                 745                 750

Met Leu Ala Asn Tyr Asn Ile Gly Tyr Gln Gly Phe Tyr Ile Pro Glu
        755                 760                 765

Ser Tyr Lys Asp Arg Met Tyr Ser Phe Phe Arg Asn Phe Gln Pro Met
    770                 775                 780

Ser Arg Gln Val Val Asp Asp Thr Lys Tyr Lys Asp Tyr Gln Gln Val
785                 790                 795                 800

Gly Ile Leu His Gln His Asn Asn Ser Gly Phe Val Gly Tyr Leu Ala
                805                 810                 815

Pro Thr Met Arg Glu Gly Gln Ala Tyr Pro Ala Asn Phe Pro Tyr Pro
            820                 825                 830

Leu Ile Gly Lys Thr Ala Val Asp Ser Ile Thr Gln Lys Lys Phe Leu
        835                 840                 845

Cys Asp Arg Thr Leu Trp Arg Ile Pro Phe Ser Ser Asn Phe Met Ser
    850                 855                 860

Met Gly Ala Leu Thr Asp Leu Gly Gln Asn Leu Leu Tyr Ala Asn Ser
865                 870                 875                 880

Ala His Ala Leu Asp Met Thr Phe Glu Val Asp Pro Met Asp Glu Pro
                885                 890                 895

Thr Leu Leu Tyr Val Leu Phe Glu Val Phe Asp Val Val Arg Val His
            900                 905                 910

Gln Pro His Arg Gly Val Ile Glu Thr Val Tyr Leu Arg Thr Pro Phe
        915                 920                 925

Ser Ala Gly Asn Ala Thr Thr
    930                 935

<210>  34
<211>  941
<212>  PRT
<213>  Artificial Sequence

<220>
<223>  Synthetic Construct

<400>  34

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Gln Gly Pro Gly Ala
145                 150                 155                 160

Pro Gln Gly Pro Gly Ala Pro Gln Gly Pro Gly Ala Pro Lys Thr His
                165                 170                 175

Val Phe Gly Gln Ala Pro Tyr Ser Gly Ile Asn Ile Thr Lys Glu Gly
            180                 185                 190

Ile Gln Ile Gly Val Glu Gly Gln Thr Pro Lys Tyr Ala Asp Lys Thr
        195                 200                 205

Phe Gln Pro Glu Pro Gln Ile Gly Glu Ser Gln Trp Tyr Glu Thr Glu
    210                 215                 220

Ile Asn His Ala Ala Gly Arg Val Leu Lys Lys Thr Thr Pro Met Lys
225                 230                 235                 240

Pro Cys Tyr Gly Ser Tyr Ala Lys Pro Thr Asn Glu Asn Gly Gly Gln
                245                 250                 255

Gly Ile Leu Val Lys Gln Gln Asn Gly Lys Leu Glu Ser Gln Val Glu
            260                 265                 270

Met Gln Phe Phe Ser Thr Thr Glu Ala Ala Ala Gly Asn Gly Asp Asn
        275                 280                 285

Leu Thr Pro Lys Val Val Leu Tyr Ser Glu Asp Val Asp Ile Glu Thr
    290                 295                 300

Pro Asp Thr His Ile Ser Tyr Met Pro Thr Ile Lys Glu Gly Asn Ser
305                 310                 315                 320

Arg Glu Leu Met Gly Gln Gln Ser Met Pro Asn Arg Pro Asn Tyr Ile

                325                 330                 335

Ala Phe Arg Asp Asn Phe Ile Gly Leu Met Tyr Tyr Asn Ser Thr Gly
            340                 345                 350

Asn Met Gly Val Leu Ala Gly Gln Ala Ser Gln Leu Asn Ala Val Val
        355                 360                 365

Asp Leu Gln Asp Arg Asn Thr Glu Leu Ser Tyr Gln Leu Leu Leu Asp
    370                 375                 380

Ser Ile Gly Asp Arg Thr Arg Tyr Phe Ser Met Trp Asn Gln Leu Pro
385                 390                 395                 400

Asn Tyr Cys Phe Pro Leu Gly Gly Val Ile Asn Thr Glu Thr Leu Thr
                405                 410                 415

Lys Val Lys Pro Lys Thr Gly Gln Glu Asn Gly Trp Glu Lys Asp Ala
            420                 425                 430

Thr Glu Phe Ser Asp Lys Asn Glu Ile Arg Val Gly Asn Asn Phe Ala
        435                 440                 445

Met Glu Ile Asn Leu Asn Ala Asn Leu Trp Arg Asn Phe Leu Tyr Ser
    450                 455                 460

Asn Ile Ala Leu Tyr Leu Pro Asp Lys Leu Lys Tyr Ser Pro Ser Asn
465                 470                 475                 480

Val Lys Ile Ser Asp Asn Pro Asn Thr Tyr Asp Tyr Met Asn Lys Arg
                485                 490                 495

Val Val Ala Pro Gly Leu Val Asp Cys Tyr Ile Asn Leu Gly Ala Arg
            500                 505                 510

Trp Ser Leu Asp Tyr Met Asp Asn Val Asn Pro Phe Asn His His Arg
        515                 520                 525

Asn Ala Gly Leu Arg Tyr Arg Ser Met Leu Leu Gly Asn Gly Arg Tyr
    530                 535                 540

Val Pro Phe His Ile Gln Val Pro Gln Lys Phe Phe Ala Ile Lys Asn
545                 550                 555                 560

Leu Leu Leu Leu Pro Gly Ser Tyr Thr Tyr Glu Trp Asn Phe Arg Lys
                565                 570                 575

Asp Val Asn Met Val Leu Gln Ser Ser Leu Gly Asn Asp Leu Arg Val
            580                 585                 590

Asp Gly Ala Ser Ile Lys Phe Asp Ser Ile Cys Leu Tyr Ala Thr Phe
        595                 600                 605

Phe Pro Met Ala His Asn Thr Ala Ser Thr Leu Glu Ala Met Leu Arg
    610                 615                 620

Asn Asp Thr Asn Asp Gln Ser Phe Asn Asp Tyr Leu Ser Ala Ala Asn
625                 630                 635                 640

Met Leu Tyr Pro Ile Pro Ala Asn Ala Thr Asn Val Pro Ile Ser Ile
                645                 650                 655

Pro Ser Arg Asn Trp Ala Ala Phe Arg Gly Trp Ala Phe Thr Arg Leu
            660                 665                 670

Lys Thr Lys Glu Thr Pro Ser Leu Gly Ser Gly Tyr Asp Pro Tyr Tyr
        675                 680                 685

Thr Tyr Ser Gly Ser Ile Pro Tyr Leu Asp Gly Thr Phe Tyr Leu Asn
    690                 695                 700

His Thr Phe Lys Lys Val Ala Ile Thr Phe Asp Ser Ser Val Ser Trp
705                 710                 715                 720

Pro Gly Asn Asp Arg Leu Leu Thr Pro Asn Glu Phe Glu Ile Lys Arg
                725                 730                 735

Ser Val Asp Gly Glu Gly Tyr Asn Val Ala Gln Cys Asn Met Thr Lys
            740                 745                 750

Asp Trp Phe Leu Val Gln Met Leu Ala Asn Tyr Asn Ile Gly Tyr Gln
        755                 760                 765

Gly Phe Tyr Ile Pro Glu Ser Tyr Lys Asp Arg Met Tyr Ser Phe Phe
    770                 775                 780

Arg Asn Phe Gln Pro Met Ser Arg Gln Val Val Asp Asp Thr Lys Tyr
785                 790                 795                 800

Lys Asp Tyr Gln Gln Val Gly Ile Leu His Gln His Asn Asn Ser Gly
                805                 810                 815

Phe Val Gly Tyr Leu Ala Pro Thr Met Arg Glu Gly Gln Ala Tyr Pro
            820                 825                 830

Ala Asn Phe Pro Tyr Pro Leu Ile Gly Lys Thr Ala Val Asp Ser Ile
        835                 840                 845

Thr Gln Lys Lys Phe Leu Cys Asp Arg Thr Leu Trp Arg Ile Pro Phe
    850                 855                 860

Ser Ser Asn Phe Met Ser Met Gly Ala Leu Thr Asp Leu Gly Gln Asn
865                 870                 875                 880

Leu Leu Tyr Ala Asn Ser Ala His Ala Leu Asp Met Thr Phe Glu Val
                885                 890                 895

Asp Pro Met Asp Glu Pro Thr Leu Leu Tyr Val Leu Phe Glu Val Phe
            900                 905                 910

Asp Val Val Arg Val His Gln Pro His Arg Gly Val Ile Glu Thr Val
        915                 920                 925

Tyr Leu Arg Thr Pro Phe Ser Ala Gly Asn Ala Thr Thr
    930                 935                 940

<210>  35
<211>  947
<212>  PRT
<213>  Artificial Sequence

<220>
<223>  Synthetic Construct

<400>  35

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met

                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Gln Gly Pro Gly Ala
145                 150                 155                 160

Pro Gln Gly Pro Gly Ala Pro Gln Gly Pro Gly Ala Pro Gln Gly Pro
                165                 170                 175

Gly Ala Pro Lys Thr His Val Phe Gly Gln Ala Pro Tyr Ser Gly Ile
            180                 185                 190

Asn Ile Thr Lys Glu Gly Ile Gln Ile Gly Val Glu Gly Gln Thr Pro
        195                 200                 205

Lys Tyr Ala Asp Lys Thr Phe Gln Pro Glu Pro Gln Ile Gly Glu Ser
    210                 215                 220

Gln Trp Tyr Glu Thr Glu Ile Asn His Ala Ala Gly Arg Val Leu Lys
225                 230                 235                 240

Lys Thr Thr Pro Met Lys Pro Cys Tyr Gly Ser Tyr Ala Lys Pro Thr
                245                 250                 255

Asn Glu Asn Gly Gly Gln Gly Ile Leu Val Lys Gln Gln Asn Gly Lys
            260                 265                 270

Leu Glu Ser Gln Val Glu Met Gln Phe Phe Ser Thr Thr Glu Ala Ala
        275                 280                 285

Ala Gly Asn Gly Asp Asn Leu Thr Pro Lys Val Val Leu Tyr Ser Glu
    290                 295                 300

Asp Val Asp Ile Glu Thr Pro Asp Thr His Ile Ser Tyr Met Pro Thr
305                 310                 315                 320

Ile Lys Glu Gly Asn Ser Arg Glu Leu Met Gly Gln Gln Ser Met Pro
                325                 330                 335

Asn Arg Pro Asn Tyr Ile Ala Phe Arg Asp Asn Phe Ile Gly Leu Met
        340                 345                 350

Tyr Tyr Asn Ser Thr Gly Asn Met Gly Val Leu Ala Gly Gln Ala Ser
    355                 360                 365

Gln Leu Asn Ala Val Val Asp Leu Gln Asp Arg Asn Thr Glu Leu Ser
    370                 375                 380

Tyr Gln Leu Leu Leu Asp Ser Ile Gly Asp Arg Thr Arg Tyr Phe Ser
385                 390                 395                 400

Met Trp Asn Gln Leu Pro Asn Tyr Cys Phe Pro Leu Gly Gly Val Ile
                405                 410                 415

Asn Thr Glu Thr Leu Thr Lys Val Lys Pro Lys Thr Gly Gln Glu Asn
            420                 425                 430

Gly Trp Glu Lys Asp Ala Thr Glu Phe Ser Asp Lys Asn Glu Ile Arg
        435                 440                 445

Val Gly Asn Asn Phe Ala Met Glu Ile Asn Leu Asn Ala Asn Leu Trp
    450                 455                 460

Arg Asn Phe Leu Tyr Ser Asn Ile Ala Leu Tyr Leu Pro Asp Lys Leu
465                 470                 475                 480

Lys Tyr Ser Pro Ser Asn Val Lys Ile Ser Asp Asn Pro Asn Thr Tyr
                485                 490                 495

Asp Tyr Met Asn Lys Arg Val Val Ala Pro Gly Leu Val Asp Cys Tyr
            500                 505                 510

Ile Asn Leu Gly Ala Arg Trp Ser Leu Asp Tyr Met Asp Asn Val Asn
        515                 520                 525

Pro Phe Asn His His Arg Asn Ala Gly Leu Arg Tyr Arg Ser Met Leu
    530                 535                 540

Leu Gly Asn Gly Arg Tyr Val Pro Phe His Ile Gln Val Pro Gln Lys
545                 550                 555                 560

Phe Phe Ala Ile Lys Asn Leu Leu Leu Leu Pro Gly Ser Tyr Thr Tyr
                565                 570                 575

Glu Trp Asn Phe Arg Lys Asp Val Asn Met Val Leu Gln Ser Ser Leu
            580                 585                 590

Gly Asn Asp Leu Arg Val Asp Gly Ala Ser Ile Lys Phe Asp Ser Ile
        595                 600                 605

Cys Leu Tyr Ala Thr Phe Phe Pro Met Ala His Asn Thr Ala Ser Thr
    610                 615                 620

Leu Glu Ala Met Leu Arg Asn Asp Thr Asn Asp Gln Ser Phe Asn Asp
625                 630                 635                 640

Tyr Leu Ser Ala Ala Asn Met Leu Tyr Pro Ile Pro Ala Asn Ala Thr
                645                 650                 655

Asn Val Pro Ile Ser Ile Pro Ser Arg Asn Trp Ala Ala Phe Arg Gly
            660                 665                 670

Trp Ala Phe Thr Arg Leu Lys Thr Lys Glu Thr Pro Ser Leu Gly Ser
        675                 680                 685

Gly Tyr Asp Pro Tyr Tyr Thr Tyr Ser Gly Ser Ile Pro Tyr Leu Asp
    690                 695                 700

Gly Thr Phe Tyr Leu Asn His Thr Phe Lys Lys Val Ala Ile Thr Phe
705                 710                 715                 720

Asp Ser Ser Val Ser Trp Pro Gly Asn Asp Arg Leu Leu Thr Pro Asn
                725                 730                 735

Glu Phe Glu Ile Lys Arg Ser Val Asp Gly Glu Gly Tyr Asn Val Ala
            740                 745                 750

Gln Cys Asn Met Thr Lys Asp Trp Phe Leu Val Gln Met Leu Ala Asn
        755                 760                 765

Tyr Asn Ile Gly Tyr Gln Gly Phe Tyr Ile Pro Glu Ser Tyr Lys Asp
    770                 775                 780

Arg Met Tyr Ser Phe Phe Arg Asn Phe Gln Pro Met Ser Arg Gln Val
785                 790                 795                 800

Val Asp Asp Thr Lys Tyr Lys Asp Tyr Gln Gln Val Gly Ile Leu His
                805                 810                 815

Gln His Asn Asn Ser Gly Phe Val Gly Tyr Leu Ala Pro Thr Met Arg
            820                 825                 830

Glu Gly Gln Ala Tyr Pro Ala Asn Phe Pro Tyr Pro Leu Ile Gly Lys
        835                 840                 845

Thr Ala Val Asp Ser Ile Thr Gln Lys Lys Phe Leu Cys Asp Arg Thr
    850                 855                 860

Leu Trp Arg Ile Pro Phe Ser Ser Asn Phe Met Ser Met Gly Ala Leu
865                 870                 875                 880

Thr Asp Leu Gly Gln Asn Leu Leu Tyr Ala Asn Ser Ala His Ala Leu
                885                 890                 895

Asp Met Thr Phe Glu Val Asp Pro Met Asp Glu Pro Thr Leu Leu Tyr
            900                 905                 910

Val Leu Phe Glu Val Phe Asp Val Val Arg Val His Gln Pro His Arg
        915                 920                 925

Gly Val Ile Glu Thr Val Tyr Leu Arg Thr Pro Phe Ser Ala Gly Asn
    930                 935                 940

Ala Thr Thr
945

<210>  36
<211>  953
<212>  PRT
<213>  Artificial Sequence

<220>
<223>  Synthetic Construct

<400>  36

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Gln Gly Pro Gly Ala
145                 150                 155                 160

Pro Gln Gly Pro Gly Ala Pro Gln Gly Pro Gly Ala Pro Gln Gly Pro
                165                 170                 175

Gly Ala Pro Gln Gly Pro Gly Ala Pro Lys Thr His Val Phe Gly Gln
            180                 185                 190

Ala Pro Tyr Ser Gly Ile Asn Ile Thr Lys Glu Gly Ile Gln Ile Gly
        195                 200                 205

Val Glu Gly Gln Thr Pro Lys Tyr Ala Asp Lys Thr Phe Gln Pro Glu
    210                 215                 220

Pro Gln Ile Gly Glu Ser Gln Trp Tyr Glu Thr Glu Ile Asn His Ala
225                 230                 235                 240

Ala Gly Arg Val Leu Lys Lys Thr Thr Pro Met Lys Pro Cys Tyr Gly
                245                 250                 255

Ser Tyr Ala Lys Pro Thr Asn Glu Asn Gly Gly Gln Gly Ile Leu Val
            260                 265                 270

Lys Gln Gln Asn Gly Lys Leu Glu Ser Gln Val Glu Met Gln Phe Phe
        275                 280                 285

Ser Thr Thr Glu Ala Ala Ala Gly Asn Gly Asp Asn Leu Thr Pro Lys
    290                 295                 300

Val Val Leu Tyr Ser Glu Asp Val Asp Ile Glu Thr Pro Asp Thr His
305                 310                 315                 320

Ile Ser Tyr Met Pro Thr Ile Lys Glu Gly Asn Ser Arg Glu Leu Met
                325                 330                 335

Gly Gln Gln Ser Met Pro Asn Arg Pro Asn Tyr Ile Ala Phe Arg Asp

            340                 345                 350

Asn Phe Ile Gly Leu Met Tyr Tyr Asn Ser Thr Gly Asn Met Gly Val
        355                 360                 365

Leu Ala Gly Gln Ala Ser Gln Leu Asn Ala Val Val Asp Leu Gln Asp
    370                 375                 380

Arg Asn Thr Glu Leu Ser Tyr Gln Leu Leu Leu Asp Ser Ile Gly Asp
385                 390                 395                 400

Arg Thr Arg Tyr Phe Ser Met Trp Asn Gln Leu Pro Asn Tyr Cys Phe
                405                 410                 415

Pro Leu Gly Gly Val Ile Asn Thr Glu Thr Leu Thr Lys Val Lys Pro
            420                 425                 430

Lys Thr Gly Gln Glu Asn Gly Trp Glu Lys Asp Ala Thr Glu Phe Ser
        435                 440                 445

Asp Lys Asn Glu Ile Arg Val Gly Asn Asn Phe Ala Met Glu Ile Asn
    450                 455                 460

Leu Asn Ala Asn Leu Trp Arg Asn Phe Leu Tyr Ser Asn Ile Ala Leu
465                 470                 475                 480

Tyr Leu Pro Asp Lys Leu Lys Tyr Ser Pro Ser Asn Val Lys Ile Ser
                485                 490                 495

Asp Asn Pro Asn Thr Tyr Asp Tyr Met Asn Lys Arg Val Val Ala Pro
            500                 505                 510

Gly Leu Val Asp Cys Tyr Ile Asn Leu Gly Ala Arg Trp Ser Leu Asp
        515                 520                 525

Tyr Met Asp Asn Val Asn Pro Phe Asn His His Arg Asn Ala Gly Leu
    530                 535                 540

Arg Tyr Arg Ser Met Leu Leu Gly Asn Gly Arg Tyr Val Pro Phe His
545                 550                 555                 560

Ile Gln Val Pro Gln Lys Phe Phe Ala Ile Lys Asn Leu Leu Leu Leu
                565                 570                 575

Pro Gly Ser Tyr Thr Tyr Glu Trp Asn Phe Arg Lys Asp Val Asn Met
            580                 585                 590

Val Leu Gln Ser Ser Leu Gly Asn Asp Leu Arg Val Asp Gly Ala Ser
        595                 600                 605

Ile Lys Phe Asp Ser Ile Cys Leu Tyr Ala Thr Phe Phe Pro Met Ala
    610                 615                 620

His Asn Thr Ala Ser Thr Leu Glu Ala Met Leu Arg Asn Asp Thr Asn
625                 630                 635                 640

Asp Gln Ser Phe Asn Asp Tyr Leu Ser Ala Ala Asn Met Leu Tyr Pro
                645                 650                 655

Ile Pro Ala Asn Ala Thr Asn Val Pro Ile Ser Ile Pro Ser Arg Asn
            660                 665                 670

Trp Ala Ala Phe Arg Gly Trp Ala Phe Thr Arg Leu Lys Thr Lys Glu
        675                 680                 685

Thr Pro Ser Leu Gly Ser Gly Tyr Asp Pro Tyr Tyr Thr Tyr Ser Gly
    690                 695                 700

Ser Ile Pro Tyr Leu Asp Gly Thr Phe Tyr Leu Asn His Thr Phe Lys
705                 710                 715                 720

Lys Val Ala Ile Thr Phe Asp Ser Ser Val Ser Trp Pro Gly Asn Asp
                725                 730                 735

Arg Leu Leu Thr Pro Asn Glu Phe Glu Ile Lys Arg Ser Val Asp Gly
            740                 745                 750

Glu Gly Tyr Asn Val Ala Gln Cys Asn Met Thr Lys Asp Trp Phe Leu
        755                 760                 765

Val Gln Met Leu Ala Asn Tyr Asn Ile Gly Tyr Gln Gly Phe Tyr Ile
    770                 775                 780

Pro Glu Ser Tyr Lys Asp Arg Met Tyr Ser Phe Phe Arg Asn Phe Gln
785                 790                 795                 800

Pro Met Ser Arg Gln Val Val Asp Asp Thr Lys Tyr Lys Asp Tyr Gln
                805                 810                 815

Gln Val Gly Ile Leu His Gln His Asn Asn Ser Gly Phe Val Gly Tyr
            820                 825                 830

Leu Ala Pro Thr Met Arg Glu Gly Gln Ala Tyr Pro Ala Asn Phe Pro
        835                 840                 845

Tyr Pro Leu Ile Gly Lys Thr Ala Val Asp Ser Ile Thr Gln Lys Lys
    850                 855                 860

Phe Leu Cys Asp Arg Thr Leu Trp Arg Ile Pro Phe Ser Ser Asn Phe
865                 870                 875                 880

Met Ser Met Gly Ala Leu Thr Asp Leu Gly Gln Asn Leu Leu Tyr Ala
                885                 890                 895

Asn Ser Ala His Ala Leu Asp Met Thr Phe Glu Val Asp Pro Met Asp
            900                 905                 910

Glu Pro Thr Leu Leu Tyr Val Leu Phe Glu Val Phe Asp Val Val Arg
        915                 920                 925

Val His Gln Pro His Arg Gly Val Ile Glu Thr Val Tyr Leu Arg Thr
    930                 935                 940

Pro Phe Ser Ala Gly Asn Ala Thr Thr
945                 950

<210>  37
<211>  959
<212>  PRT
<213>  Artificial Sequence

<220>
<223>  Synthetic Construct

<400>  37

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
        100                 105                 110

Phe Lys Pro Tyr Ser Gly Thr Ala Tyr Asn Ala Leu Ala Pro Lys Gly
    115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Gln Gly Pro Gly Ala
145                 150                 155                 160

Pro Gln Gly Pro Gly Ala Pro Gln Gly Pro Gly Ala Pro Gln Gly Pro
                165                 170                 175

Gly Ala Pro Gln Gly Pro Gly Ala Pro Gln Gly Pro Gly Ala Pro Lys
            180                 185                 190

Thr His Val Phe Gly Gln Ala Pro Tyr Ser Gly Ile Asn Ile Thr Lys
        195                 200                 205

Glu Gly Ile Gln Ile Gly Val Glu Gly Gln Thr Pro Lys Tyr Ala Asp
    210                 215                 220

Lys Thr Phe Gln Pro Glu Pro Gln Ile Gly Glu Ser Gln Trp Tyr Glu
225                 230                 235                 240

Thr Glu Ile Asn His Ala Ala Gly Arg Val Leu Lys Lys Thr Thr Pro
                245                 250                 255

Met Lys Pro Cys Tyr Gly Ser Tyr Ala Lys Pro Thr Asn Glu Asn Gly
            260                 265                 270

Gly Gln Gly Ile Leu Val Lys Gln Gln Asn Gly Lys Leu Glu Ser Gln
        275                 280                 285

Val Glu Met Gln Phe Phe Ser Thr Thr Glu Ala Ala Ala Gly Asn Gly
    290                 295                 300

Asp Asn Leu Thr Pro Lys Val Val Leu Tyr Ser Glu Asp Val Asp Ile
305                 310                 315                 320

Glu Thr Pro Asp Thr His Ile Ser Tyr Met Pro Thr Ile Lys Glu Gly
                325                 330                 335

Asn Ser Arg Glu Leu Met Gly Gln Gln Ser Met Pro Asn Arg Pro Asn

            340                 345                 350

Tyr Ile Ala Phe Arg Asp Asn Phe Ile Gly Leu Met Tyr Tyr Asn Ser
        355                 360                 365

Thr Gly Asn Met Gly Val Leu Ala Gly Gln Ala Ser Gln Leu Asn Ala
    370                 375                 380

Val Val Asp Leu Gln Asp Arg Asn Thr Glu Leu Ser Tyr Gln Leu Leu
385                 390                 395                 400

Leu Asp Ser Ile Gly Asp Arg Thr Arg Tyr Phe Ser Met Trp Asn Gln
                405                 410                 415

Leu Pro Asn Tyr Cys Phe Pro Leu Gly Gly Val Ile Asn Thr Glu Thr
            420                 425                 430

Leu Thr Lys Val Lys Pro Lys Thr Gly Gln Glu Asn Gly Trp Glu Lys
        435                 440                 445

Asp Ala Thr Glu Phe Ser Asp Lys Asn Glu Ile Arg Val Gly Asn Asn
    450                 455                 460

Phe Ala Met Glu Ile Asn Leu Asn Ala Asn Leu Trp Arg Asn Phe Leu
465                 470                 475                 480

Tyr Ser Asn Ile Ala Leu Tyr Leu Pro Asp Lys Leu Lys Tyr Ser Pro
                485                 490                 495

Ser Asn Val Lys Ile Ser Asp Asn Pro Asn Thr Tyr Asp Tyr Met Asn
            500                 505                 510

Lys Arg Val Val Ala Pro Gly Leu Val Asp Cys Tyr Ile Asn Leu Gly
        515                 520                 525

Ala Arg Trp Ser Leu Asp Tyr Met Asp Asn Val Asn Pro Phe Asn His
    530                 535                 540

His Arg Asn Ala Gly Leu Arg Tyr Arg Ser Met Leu Leu Gly Asn Gly
545                 550                 555                 560

Arg Tyr Val Pro Phe His Ile Gln Val Pro Gln Lys Phe Phe Ala Ile
                565                 570                 575

Lys Asn Leu Leu Leu Leu Pro Gly Ser Tyr Thr Tyr Glu Trp Asn Phe
            580                 585                 590

Arg Lys Asp Val Asn Met Val Leu Gln Ser Ser Leu Gly Asn Asp Leu
        595                 600                 605

Arg Val Asp Gly Ala Ser Ile Lys Phe Asp Ser Ile Cys Leu Tyr Ala
    610                 615                 620

Thr Phe Phe Pro Met Ala His Asn Thr Ala Ser Thr Leu Glu Ala Met
625                 630                 635                 640

Leu Arg Asn Asp Thr Asn Asp Gln Ser Phe Asn Asp Tyr Leu Ser Ala
                645                 650                 655

Ala Asn Met Leu Tyr Pro Ile Pro Ala Asn Ala Thr Asn Val Pro Ile
            660                 665                 670

Ser Ile Pro Ser Arg Asn Trp Ala Ala Phe Arg Gly Trp Ala Phe Thr
        675                 680                 685

Arg Leu Lys Thr Lys Glu Thr Pro Ser Leu Gly Ser Gly Tyr Asp Pro
    690                 695                 700

Tyr Tyr Thr Tyr Ser Gly Ser Ile Pro Tyr Leu Asp Gly Thr Phe Tyr
705                 710                 715                 720

Leu Asn His Thr Phe Lys Lys Val Ala Ile Thr Phe Asp Ser Ser Val
                725                 730                 735

Ser Trp Pro Gly Asn Asp Arg Leu Leu Thr Pro Asn Glu Phe Glu Ile
            740                 745                 750

Lys Arg Ser Val Asp Gly Glu Gly Tyr Asn Val Ala Gln Cys Asn Met
        755                 760                 765

Thr Lys Asp Trp Phe Leu Val Gln Met Leu Ala Asn Tyr Asn Ile Gly
    770                 775                 780

Tyr Gln Gly Phe Tyr Ile Pro Glu Ser Tyr Lys Asp Arg Met Tyr Ser
785                 790                 795                 800

Phe Phe Arg Asn Phe Gln Pro Met Ser Arg Gln Val Val Asp Asp Thr
                805                 810                 815

Lys Tyr Lys Asp Tyr Gln Gln Val Gly Ile Leu His Gln His Asn Asn
            820                 825                 830

Ser Gly Phe Val Gly Tyr Leu Ala Pro Thr Met Arg Glu Gly Gln Ala
        835                 840                 845

Tyr Pro Ala Asn Phe Pro Tyr Pro Leu Ile Gly Lys Thr Ala Val Asp
    850                 855                 860

Ser Ile Thr Gln Lys Lys Phe Leu Cys Asp Arg Thr Leu Trp Arg Ile
865                 870                 875                 880

Pro Phe Ser Ser Asn Phe Met Ser Met Gly Ala Leu Thr Asp Leu Gly
                885                 890                 895

Gln Asn Leu Leu Tyr Ala Asn Ser Ala His Ala Leu Asp Met Thr Phe
            900                 905                 910

Glu Val Asp Pro Met Asp Glu Pro Thr Leu Leu Tyr Val Leu Phe Glu
        915                 920                 925

Val Phe Asp Val Val Arg Val His Gln Pro His Arg Gly Val Ile Glu
    930                 935                 940

Thr Val Tyr Leu Arg Thr Pro Phe Ser Ala Gly Asn Ala Thr Thr
945                 950                 955

<210>  38
<211>  971
<212>  PRT
<213>  Artificial Sequence

<220>
<223>  Synthetic Construct

<400>  38

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Gln Gly Pro Gly Ala
145                 150                 155                 160

Pro Gln Gly Pro Gly Ala Pro Gln Gly Pro Gly Ala Pro Gln Gly Pro
                165                 170                 175

Gly Ala Pro Gln Gly Pro Gly Ala Pro Gln Gly Pro Gly Ala Pro Gln
            180                 185                 190

Gly Pro Gly Ala Pro Gln Gly Pro Gly Ala Pro Lys Thr His Val Phe
        195                 200                 205

Gly Gln Ala Pro Tyr Ser Gly Ile Asn Ile Thr Lys Glu Gly Ile Gln
    210                 215                 220

Ile Gly Val Glu Gly Gln Thr Pro Lys Tyr Ala Asp Lys Thr Phe Gln
225                 230                 235                 240

Pro Glu Pro Gln Ile Gly Glu Ser Gln Trp Tyr Glu Thr Glu Ile Asn
                245                 250                 255

His Ala Ala Gly Arg Val Leu Lys Lys Thr Thr Pro Met Lys Pro Cys
            260                 265                 270

Tyr Gly Ser Tyr Ala Lys Pro Thr Asn Glu Asn Gly Gly Gln Gly Ile
        275                 280                 285

Leu Val Lys Gln Gln Asn Gly Lys Leu Glu Ser Gln Val Glu Met Gln
    290                 295                 300

Phe Phe Ser Thr Thr Glu Ala Ala Ala Gly Asn Gly Asp Asn Leu Thr
305                 310                 315                 320

Pro Lys Val Val Leu Tyr Ser Glu Asp Val Asp Ile Glu Thr Pro Asp
                325                 330                 335

Thr His Ile Ser Tyr Met Pro Thr Ile Lys Glu Gly Asn Ser Arg Glu
            340                 345                 350

Leu Met Gly Gln Gln Ser Met Pro Asn Arg Pro Asn Tyr Ile Ala Phe
        355                 360                 365

Arg Asp Asn Phe Ile Gly Leu Met Tyr Tyr Asn Ser Thr Gly Asn Met
    370                 375                 380

Gly Val Leu Ala Gly Gln Ala Ser Gln Leu Asn Ala Val Val Asp Leu
385                 390                 395                 400

Gln Asp Arg Asn Thr Glu Leu Ser Tyr Gln Leu Leu Leu Asp Ser Ile
                405                 410                 415

Gly Asp Arg Thr Arg Tyr Phe Ser Met Trp Asn Gln Leu Pro Asn Tyr
            420                 425                 430

Cys Phe Pro Leu Gly Gly Val Ile Asn Thr Glu Thr Leu Thr Lys Val
        435                 440                 445

Lys Pro Lys Thr Gly Gln Glu Asn Gly Trp Glu Lys Asp Ala Thr Glu
    450                 455                 460

Phe Ser Asp Lys Asn Glu Ile Arg Val Gly Asn Asn Phe Ala Met Glu
465                 470                 475                 480

Ile Asn Leu Asn Ala Asn Leu Trp Arg Asn Phe Leu Tyr Ser Asn Ile
                485                 490                 495

Ala Leu Tyr Leu Pro Asp Lys Leu Lys Tyr Ser Pro Ser Asn Val Lys
            500                 505                 510

Ile Ser Asp Asn Pro Asn Thr Tyr Asp Tyr Met Asn Lys Arg Val Val
        515                 520                 525

Ala Pro Gly Leu Val Asp Cys Tyr Ile Asn Leu Gly Ala Arg Trp Ser
    530                 535                 540

Leu Asp Tyr Met Asp Asn Val Asn Pro Phe Asn His His Arg Asn Ala
545                 550                 555                 560

Gly Leu Arg Tyr Arg Ser Met Leu Leu Gly Asn Gly Arg Tyr Val Pro
                565                 570                 575

Phe His Ile Gln Val Pro Gln Lys Phe Phe Ala Ile Lys Asn Leu Leu
            580                 585                 590

Leu Leu Pro Gly Ser Tyr Thr Tyr Glu Trp Asn Phe Arg Lys Asp Val

595                 600                 605

Asn Met Val Leu Gln Ser Ser Leu Gly Asn Asp Leu Arg Val Asp Gly
    610                 615                 620

Ala Ser Ile Lys Phe Asp Ser Ile Cys Leu Tyr Ala Thr Phe Phe Pro
625                 630                 635                 640

Met Ala His Asn Thr Ala Ser Thr Leu Glu Ala Met Leu Arg Asn Asp
                645                 650                 655

Thr Asn Asp Gln Ser Phe Asn Asp Tyr Leu Ser Ala Ala Asn Met Leu
            660                 665                 670

Tyr Pro Ile Pro Ala Asn Ala Thr Asn Val Pro Ile Ser Ile Pro Ser
        675                 680                 685

Arg Asn Trp Ala Ala Phe Arg Gly Trp Ala Phe Thr Arg Leu Lys Thr
    690                 695                 700

Lys Glu Thr Pro Ser Leu Gly Ser Gly Tyr Asp Pro Tyr Tyr Thr Tyr
705                 710                 715                 720

Ser Gly Ser Ile Pro Tyr Leu Asp Gly Thr Phe Tyr Leu Asn His Thr
                725                 730                 735

Phe Lys Lys Val Ala Ile Thr Phe Asp Ser Ser Val Ser Trp Pro Gly
            740                 745                 750

Asn Asp Arg Leu Leu Thr Pro Asn Glu Phe Glu Ile Lys Arg Ser Val
        755                 760                 765

Asp Gly Glu Gly Tyr Asn Val Ala Gln Cys Asn Met Thr Lys Asp Trp
    770                 775                 780

Phe Leu Val Gln Met Leu Ala Asn Tyr Asn Ile Gly Tyr Gln Gly Phe
785                 790                 795                 800

Tyr Ile Pro Glu Ser Tyr Lys Asp Arg Met Tyr Ser Phe Phe Arg Asn
                805                 810                 815

Phe Gln Pro Met Ser Arg Gln Val Val Asp Asp Thr Lys Tyr Lys Asp
            820                 825                 830

Tyr Gln Gln Val Gly Ile Leu His Gln His Asn Asn Ser Gly Phe Val
        835                 840                 845

Gly Tyr Leu Ala Pro Thr Met Arg Glu Gly Gln Ala Tyr Pro Ala Asn
    850                 855                 860

Phe Pro Tyr Pro Leu Ile Gly Lys Thr Ala Val Asp Ser Ile Thr Gln
865                 870                 875                 880

Lys Lys Phe Leu Cys Asp Arg Thr Leu Trp Arg Ile Pro Phe Ser Ser
                885                 890                 895

Asn Phe Met Ser Met Gly Ala Leu Thr Asp Leu Gly Gln Asn Leu Leu
            900                 905                 910

Tyr Ala Asn Ser Ala His Ala Leu Asp Met Thr Phe Glu Val Asp Pro
        915                 920                 925

Met Asp Glu Pro Thr Leu Leu Tyr Val Leu Phe Glu Val Phe Asp Val
    930                 935                 940

Val Arg Val His Gln Pro His Arg Gly Val Ile Glu Thr Val Tyr Leu
945                 950                 955                 960

Arg Thr Pro Phe Ser Ala Gly Asn Ala Thr Thr
                965                 970

<210>  39
<211>  977
<212>  PRT
<213>  Artificial Sequence

<220>
<223>  Synthetic Construct

<400>  39

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Gln Gly Pro Gly Ala Pro Gln
    130                 135                 140

Gly Pro Gly Ala Pro Gln Gly Pro Gly Ala Pro Gln Gly Pro Gly Ala
145                 150                 155                 160

Pro Gln Gly Pro Gly Ala Pro Gln Gly Pro Gly Ala Pro Gln Gly Pro
                165                 170                 175

Gly Ala Pro Gln Gly Pro Gly Ala Pro Gln Gly Pro Gly Ala Pro Gln
            180                 185                 190

Gly Pro Gly Ala Pro Gln Gly Pro Gly Ala Pro Gln Gly Pro Gly Ala
        195                 200                 205

Pro Lys Thr His Val Phe Gly Gln Ala Pro Tyr Ser Gly Ile Asn Ile
    210                 215                 220

Thr Lys Glu Gly Ile Gln Ile Gly Val Glu Gly Gln Thr Pro Lys Tyr
225                 230                 235                 240

Ala Asp Lys Thr Phe Gln Pro Glu Pro Gln Ile Gly Glu Ser Gln Trp
                245                 250                 255

Tyr Glu Thr Glu Ile Asn His Ala Ala Gly Arg Val Leu Lys Lys Thr
            260                 265                 270

Thr Pro Met Lys Pro Cys Tyr Gly Ser Tyr Ala Lys Pro Thr Asn Glu
        275                 280                 285

Asn Gly Gly Gln Gly Ile Leu Val Lys Gln Gln Asn Gly Lys Leu Glu
    290                 295                 300

Ser Gln Val Glu Met Gln Phe Phe Ser Thr Thr Glu Ala Ala Ala Gly
305                 310                 315                 320

Asn Gly Asp Asn Leu Thr Pro Lys Val Val Leu Tyr Ser Glu Asp Val
                325                 330                 335

Asp Ile Glu Thr Pro Asp Thr His Ile Ser Tyr Met Pro Thr Ile Lys
            340                 345                 350

Glu Gly Asn Ser Arg Glu Leu Met Gly Gln Gln Ser Met Pro Asn Arg
        355                 360                 365

Pro Asn Tyr Ile Ala Phe Arg Asp Asn Phe Ile Gly Leu Met Tyr Tyr
    370                 375                 380

Asn Ser Thr Gly Asn Met Gly Val Leu Ala Gly Gln Ala Ser Gln Leu
385                 390                 395                 400

Asn Ala Val Val Asp Leu Gln Asp Arg Asn Thr Glu Leu Ser Tyr Gln
                405                 410                 415

Leu Leu Leu Asp Ser Ile Gly Asp Arg Thr Arg Tyr Phe Ser Met Trp
            420                 425                 430

Asn Gln Leu Pro Asn Tyr Cys Phe Pro Leu Gly Gly Val Ile Asn Thr
        435                 440                 445

Glu Thr Leu Thr Lys Val Lys Pro Lys Thr Gly Gln Glu Asn Gly Trp
    450                 455                 460

Glu Lys Asp Ala Thr Glu Phe Ser Asp Lys Asn Glu Ile Arg Val Gly
465                 470                 475                 480

Asn Asn Phe Ala Met Glu Ile Asn Leu Asn Ala Asn Leu Trp Arg Asn
                485                 490                 495

Phe Leu Tyr Ser Asn Ile Ala Leu Tyr Leu Pro Asp Lys Leu Lys Tyr
            500                 505                 510

Ser Pro Ser Asn Val Lys Ile Ser Asp Asn Pro Asn Thr Tyr Asp Tyr
        515                 520                 525

Met Asn Lys Arg Val Val Ala Pro Gly Leu Val Asp Cys Tyr Ile Asn
    530                 535                 540

Leu Gly Ala Arg Trp Ser Leu Asp Tyr Met Asp Asn Val Asn Pro Phe
545                 550                 555                 560

Asn His His Arg Asn Ala Gly Leu Arg Tyr Arg Ser Met Leu Leu Gly
                565                 570                 575

Asn Gly Arg Tyr Val Pro Phe His Ile Gln Val Pro Gln Lys Phe Phe

580                 585                 590

Ala Ile Lys Asn Leu Leu Leu Leu Pro Gly Ser Tyr Thr Tyr Glu Trp
        595                 600                 605

Asn Phe Arg Lys Asp Val Asn Met Val Leu Gln Ser Ser Leu Gly Asn
    610                 615                 620

Asp Leu Arg Val Asp Gly Ala Ser Ile Lys Phe Asp Ser Ile Cys Leu
625                 630                 635                 640

Tyr Ala Thr Phe Phe Pro Met Ala His Asn Thr Ala Ser Thr Leu Glu
                645                 650                 655

Ala Met Leu Arg Asn Asp Thr Asn Asp Gln Ser Phe Asn Asp Tyr Leu
            660                 665                 670

Ser Ala Ala Asn Met Leu Tyr Pro Ile Pro Ala Asn Ala Thr Asn Val
        675                 680                 685

Pro Ile Ser Ile Pro Ser Arg Asn Trp Ala Ala Phe Arg Leu Leu Thr
    690                 695                 700

Pro Asn Glu Phe Glu Ile Lys Arg Ser Val Asp Gly Glu Gly Tyr Asn
705                 710                 715                 720

Asp Pro Tyr Tyr Thr Tyr Ser Gly Ser Ile Pro Tyr Leu Asp Gly Thr
                725                 730                 735

Phe Tyr Leu Asn His Thr Phe Lys Lys Val Ala Ile Thr Phe Asp Ser
            740                 745                 750

Ser Val Ser Trp Pro Gly Asn Asp Arg Leu Leu Thr Pro Asn Glu Phe
        755                 760                 765

Glu Ile Lys Arg Ser Val Asp Gly Glu Gly Tyr Asn Val Ala Gln Cys
    770                 775                 780

Asn Met Thr Lys Asp Trp Phe Leu Val Gln Met Leu Ala Asn Tyr Asn
785                 790                 795                 800

Ile Gly Tyr Gln Gly Phe Tyr Ile Pro Glu Ser Tyr Lys Asp Arg Met
                805                 810                 815

Tyr Ser Phe Phe Arg Asn Phe Gln Pro Met Ser Arg Gln Val Val Asp
            820                 825                 830

Asp Thr Lys Tyr Lys Asp Tyr Gln Gln Val Gly Ile Leu His Gln His
        835                 840                 845

Asn Asn Ser Gly Phe Val Gly Tyr Leu Ala Pro Thr Met Arg Glu Gly
    850                 855                 860

Gln Ala Tyr Pro Ala Asn Phe Pro Tyr Pro Leu Ile Gly Lys Thr Ala
865                 870                 875                 880

Val Asp Ser Ile Thr Gln Lys Lys Phe Leu Cys Asp Arg Thr Leu Trp
                885                 890                 895

Arg Ile Pro Phe Ser Ser Asn Phe Met Ser Met Gly Ala Leu Thr Asp
            900                 905                 910

Leu Gly Gln Asn Leu Leu Tyr Ala Asn Ser Ala His Ala Leu Asp Met
        915                 920                 925

Thr Phe Glu Val Asp Pro Met Asp Glu Pro Thr Leu Leu Tyr Val Leu
    930                 935                 940

Phe Glu Val Phe Asp Val Val Arg Val His Gln Pro His Arg Gly Val
945                 950                 955                 960

Ile Glu Thr Val Tyr Leu Arg Thr Pro Phe Ser Ala Gly Asn Ala Thr
                965                 970                 975

Thr

<210> 40
<211> 975
<212> PRT
<213> Artificial Sequence

<220>
<223> Synthetic Construct

<400> 40
   Met Ala Thr Pro Ser Met Met Pro Gln Trp Ser Tyr Met His Ile Ser
   1 5 10 15
   Gly Gln Asp Ala Ser Glu Tyr Leu Ser Pro Gly Leu Val Gln Phe Ala
   20 25 30
   Arg Ala Thr Glu Thr Tyr Phe Ser Leu Asn Asn Lys Phe Arg Asn Pro
   35 40 45
   Thr Val Ala Pro Thr His Asp Val Thr Thr Asp Arg Ser Gln Arg Leu
       50                  55                  60
   Thr Leu Arg Phe Ile Pro Val Asp Arg Glu Asp Thr Ala Tyr Ser Tyr
   65                  70                  75                  80
   Lys Ala Arg Phe Thr Leu Ala Val Gly Asp Asn Arg Val Leu Asp Met
                   85                  90                  95
   Ala Ser Thr Tyr Phe Asp Ile Arg Gly Val Leu Asp Arg Gly Pro Thr
               100                 105                 110
   Phe Lys Pro Tyr Ser Gly Thr Ala Tyr Asn Ala Leu Ala Pro Lys Gly
           115                 120                 125
   Ala Pro Asn Pro Cys Glu Trp Asp Glu Ala Ala Thr Ala Leu Glu Ile
       130                 135                 140
   Asn Leu Glu Glu Glu Asp Asp Asp Asn Glu Asp Glu Val Asp Glu Gln
   145                 150                 155                 160
   Ala Glu Gln Gln Lys Thr His Val Phe Gly Gln Ala Pro Tyr Ser Gly
                   165                 170                 175
   Ile Asn Ile Thr Lys Glu Gly Ile Gln Ile Gly Val Glu Gly Gln Thr
               180                 185                 190
   Pro Lys Tyr Ala Asp Lys Thr Phe Gln Pro Glu Pro Gln Ile Gly Glu
           195                 200                 205
   Ser Gln Trp Tyr Glu Thr Glu Ile Asn His Ala Ala Gly Arg Val Leu
       210                 215                 220
   Lys Lys Thr Thr Pro Met Lys Pro Cys Tyr Gly Ser Tyr Ala Lys Pro
   225                 230                 235                 240
   Thr Asn Glu Asn Gly Gly Gln Gly Ile Leu Val Lys Gln Gln Asn Gly
                   245                 250                 255
   Lys Leu Glu Ser Gln Val Glu Met Gln Phe Phe Ser Ala Ser Gln Gly
               260                 265                 270
   Pro Gly Ala Pro Gln Gly Pro Gly Ala Pro Gln Gly Pro Gly Ala Pro
           275                 280                 285
   Gly Thr Ser Thr Thr Glu Ala Ala Ala Gly Asn Gly Asp Asn Leu Thr
       290                 295                 300
   Pro Lys Val Val Leu Tyr Ser Glu Asp Val Asp Ile Glu Thr Pro Asp
   305                 310                 315                 320
   Thr His Ile Ser Tyr Met Pro Thr Ile Lys Glu Gly Asn Ser Arg Glu
                   325                 330                 335
   Leu Met Gly Gln Gln Ser Met Pro Asn Arg Pro Asn Tyr Ile Ala Phe
               340                 345                 350
   Arg Asp Asn Phe Ile Gly Leu Met Tyr Tyr Asn Ser Thr Gly Asn Met
           355                 360                 365
   Gly Val Leu Ala Gly Gln Ala Ser Gln Leu Asn Ala Val Val Asp Leu
       370                 375                 380
   Gln Asp Arg Asn Thr Glu Leu Ser Tyr Gln Leu Leu Leu Asp Ser Ile
   385                 390                 395                 400
   Gly Asp Arg Thr Arg Tyr Phe Ser Met Trp Asn Gln Ala Val Asp Ser
                   405                 410                 415
   Tyr Asp Pro Asp Val Arg Ile Ile Glu Asn His Gly Thr Glu Asp Glu
               420                 425                 430
   Leu Pro Asn Tyr Cys Phe Pro Leu Gly Gly Val Ile Asn Thr Glu Thr
           435                 440                 445
   Leu Thr Lys Val Lys Pro Lys Thr Gly Gln Glu Asn Gly Trp Glu Lys
       450                 455                 460
   Asp Ala Thr Glu Phe Ser Asp Lys Asn Glu Ile Arg Val Gly Asn Asn
   465                 470                 475                 480
   Phe Ala Met Glu Ile Asn Leu Asn Ala Asn Leu Trp Arg Asn Phe Leu
                   485                 490                 495
   Tyr Ser Asn Ile Ala Leu Tyr Leu Pro Asp Lys Leu Lys Tyr Ser Pro
               500                 505                 510
   Ser Asn Val Lys Ile Ser Asp Asn Pro Asn Thr Tyr Asp Tyr Met Asn
           515                 520                 525
   Lys Arg Val Val Ala Pro Gly Leu Val Asp Cys Tyr Ile Asn Leu Gly
       530                 535                 540
   Ala Arg Trp Ser Leu Asp Tyr Met Asp Asn Val Asn Pro Phe Asn His
   545                 550                 555                 560
   His Arg Asn Ala Gly Leu Arg Tyr Arg Ser Met Leu Leu Gly Asn Gly
   565 570 575
   Arg Tyr Val Pro Phe His Ile Gln Val Pro Gln Lys Phe Phe Ala Ile
   580 585 590
   Lys Asn Leu Leu Leu Leu Pro Gly Ser Tyr Thr Tyr Glu Trp Asn Phe
   595 600 605
   Arg Lys Asp Val Asn Met Val Leu Gln Ser Ser Leu Gly Asn Asp Leu
   610 615 620
   Arg Val Asp Gly Ala Ser Ile Lys Phe Asp Ser Ile Cys Leu Tyr Ala
   625 630 635 640
   Thr Phe Phe Pro Met Ala His Asn Thr Ala Ser Thr Leu Glu Ala Met
   645 650 655
   Leu Arg Asn Asp Thr Asn Asp Gln Ser Phe Asn Asp Tyr Leu Ser Ala
   660 665 670
   Ala Asn Met Leu Tyr Pro Ile Pro Ala Asn Ala Thr Asn Val Pro Ile
   675 680 685
   Ser Ile Pro Ser Arg Asn Trp Ala Ala Phe Arg Gly Trp Ala Phe Thr
   690 695 700
   Arg Leu Lys Thr Lys Glu Thr Pro Ser Leu Gly Ser Gly Tyr Asp Pro
   705 710 715 720
   Tyr Tyr Thr Tyr Ser Gly Ser Ile Pro Tyr Leu Asp Gly Thr Phe Tyr
   725 730 735
   Leu Asn His Thr Phe Lys Lys Val Ala Ile Thr Phe Asp Ser Ser Val
   740 745 750
   Ser Trp Pro Gly Asn Asp Arg Leu Leu Thr Pro Asn Glu Phe Glu Ile
   755 760 765
   Lys Arg Ser Val Asp Gly Glu Gly Tyr Asn Val Ala Gln Cys Asn Met
   770 775 780
   Thr Lys Asp Trp Phe Leu Val Gln Met Leu Ala Asn Tyr Asn Ile Gly
   785 790 795 800
   Tyr Gln Gly Phe Tyr Ile Pro Glu Ser Tyr Lys Asp Arg Met Tyr Ser
   805 810 815
   Phe Phe Arg Asn Phe Gln Pro Met Ser Arg Gln Val Val Asp Asp Thr
   820 825 830
   Lys Tyr Lys Asp Tyr Gln Gln Val Gly Ile Leu His Gln His Asn Asn
   835 840 845
   Ser Gly Phe Val Gly Tyr Leu Ala Pro Thr Met Arg Glu Gly Gln Ala
   850 855 860
   Tyr Pro Ala Asn Phe Pro Tyr Pro Leu Ile Gly Lys Thr Ala Val Asp
   865 870 875 880
   Ser Ile Thr Gln Lys Lys Phe Leu Cys Asp Arg Thr Leu Trp Arg Ile
   885 890 895
   Pro Phe Ser Ser Asn Phe Met Ser Met Gly Ala Leu Thr Asp Leu Gly
   900 905 910
   Gln Asn Leu Leu Tyr Ala Asn Ser Ala His Ala Leu Asp Met Thr Phe
   915 920 925
   Glu Val Asp Pro Met Asp Glu Pro Thr Leu Leu Tyr Val Leu Phe Glu
   930 935 940
   Val Phe Asp Val Val Arg Val His Gln Pro His Arg Gly Val Ile Glu
   945 950 955 960
   Thr Val Tyr Leu Arg Thr Pro Phe Ser Ala Gly Asn Ala Thr Thr
   965 970 975

<210> 41
<211> 599
<212> PRT
<213> Artificial Sequence

<220>
<223> Synthetic Construct

<400> 41

Met Lys Arg Ala Arg Pro Ser Glu Asp Thr Phe Asn Pro Val Tyr Pro
1               5                   10                  15

Tyr Asp Thr Glu Thr Gly Pro Pro Thr Val Pro Phe Leu Thr Pro Pro
            20                  25                  30

Phe Val Ser Pro Asn Gly Phe Gln Glu Ser Pro Pro Gly Val Leu Ser
        35                  40                  45

Leu Arg Leu Ser Glu Pro Leu Val Thr Ser Asn Gly Met Leu Ala Leu
    50                  55                  60

Lys Met Gly Asn Gly Leu Ser Leu Asp Glu Ala Gly Asn Leu Thr Ser
65                  70                  75                  80

Gln Asn Val Thr Thr Val Ser Pro Pro Leu Lys Lys Thr Lys Ser Asn
                85                  90                  95

Ile Asn Leu Glu Ile Ser Ala Pro Leu Thr Val Thr Ser Glu Ala Leu
            100                 105                 110

Thr Val Ala Ala Ala Ala Pro Leu Met Val Ala Gly Asn Thr Leu Thr
        115                 120                 125

Met Gln Ser Gln Ala Pro Leu Thr Val His Asp Ser Lys Leu Ser Ile
    130                 135                 140

Ala Thr Gln Gly Pro Leu Thr Val Ser Glu Gly Lys Leu Ala Leu Gln
145                 150                 155                 160

Thr Ser Gly Pro Leu Thr Thr Thr Asp Ser Ser Thr Leu Thr Ile Thr
                165                 170                 175

Ala Ser Pro Pro Leu Thr Thr Ala Thr Gly Ser Leu Gly Ile Asp Leu
            180                 185                 190

Lys Glu Pro Ile Tyr Thr Gln Asn Gly Lys Leu Gly Leu Lys Tyr Gly
        195                 200                 205

Ala Pro Leu His Val Thr Asp Asp Leu Asn Thr Leu Thr Val Ala Thr
    210                 215                 220

Gly Pro Gly Val Thr Ile Asn Asn Thr Ser Leu Gln Thr Lys Val Thr
225                 230                 235                 240

Gly Ala Leu Gly Phe Asp Ser Gln Gly Asn Met Gln Leu Asn Val Ala
                245                 250                 255

Gly Gly Leu Arg Ile Asp Ser Gln Asn Arg Arg Leu Ile Leu Asp Val
            260                 265                 270

Ser Tyr Pro Phe Asp Ala Gln Asn Gln Leu Asn Leu Arg Leu Gly Gln
        275                 280                 285

Gly Pro Leu Phe Ile Asn Ser Ala His Asn Leu Asp Ile Asn Tyr Asn
    290                 295                 300

Lys Gly Leu Tyr Leu Phe Thr Ala Ser Asn Asn Ser Lys Lys Leu Glu
305                 310                 315                 320

Val Asn Leu Ser Thr Ala Lys Gly Leu Met Phe Asp Ala Thr Ala Ile
                325                 330                 335

Ala Ile Asn Ala Gly Asp Gly Leu Glu Phe Gly Ser Pro Asn Ala Pro
            340                 345                 350

Asn Thr Asn Pro Leu Lys Thr Lys Ile Gly His Gly Leu Glu Phe Asp
        355                 360                 365

Ser Asn Lys Ala Met Val Pro Lys Leu Gly Thr Gly Leu Ser Phe Asp
    370                 375                 380

Ser Thr Gly Ala Ile Thr Val Gly Asn Lys Asn Asn Asp Lys Leu Thr
385                 390                 395                 400

Leu Trp Thr Thr Pro Ala Pro Ser Pro Asn Cys Arg Leu Asn Ala Glu
                405                 410                 415

Lys Asp Ala Lys Leu Thr Leu Val Leu Thr Lys Cys Gly Ser Gln Ile
            420                 425                 430

Leu Ala Thr Val Ser Val Leu Ala Val Lys Gly Ser Leu Ala Pro Ile
        435                 440                 445

Ser Gly Thr Val Gln Ser Ala His Leu Ile Ile Arg Phe Asp Glu Asn
    450                 455                 460

Gly Val Leu Leu Asn Asn Ser Phe Leu Asp Pro Glu Tyr Trp Asn Phe
465                 470                 475                 480

Arg Asn Gly Asp Leu Thr Glu Gly Thr Ala Tyr Thr Asn Ala Val Gly
                485                 490                 495

Phe Met Pro Asn Leu Ser Ala Tyr Pro Lys Ser His Gly Lys Thr Ala
            500                 505                 510

Lys Ser Asn Ile Val Ser Gln Val Tyr Leu Asn Gly Asp Lys Thr Lys
        515                 520                 525

Pro Val Thr Leu Thr Ile Thr Leu Asn Gly Thr Gln Glu Thr Gly Gln
    530                 535                 540

Gly Pro Gly Ala Pro Gln Gly Pro Gly Ala Pro Gln Gly Pro Gly Ala
545                 550                 555                 560

Pro Asp Thr Thr Pro Ser Ala Tyr Ser Met Ser Phe Ser Trp Asp Trp
                565                 570                 575

Ser Gly His Asn Tyr Ile Asn Glu Ile Phe Ala Thr Ser Ser Tyr Thr
            580                 585                 590

Phe Ser Tyr Ile Ala Gln Glu
        595

<210>  42
<211>  227
<212>  PRT
<213>  Artificial Sequence

<220>
<223>  Synthetic Construct

<400>  42

Met Ser Ile Leu Ile Ser Pro Ser Asn Asn Thr Gly Trp Gly Leu Arg
1               5                   10                  15

Phe Pro Ser Lys Met Phe Gly Gly Ala Lys Lys Arg Ser Asp Gln His
            20                  25                  30

Pro Val Arg Val Arg Gly His Tyr Arg Ala Pro Trp Gly Ala His Lys
        35                  40                  45

Arg Gly Arg Thr Gly Arg Thr Thr Val Asp Asp Ala Ile Asp Ala Val
    50                  55                  60

Val Glu Glu Ala Arg Asn Tyr Thr Pro Thr Pro Pro Pro Val Ser Thr
65                  70                  75                  80

Val Asp Ala Ala Ile Gln Thr Val Val Arg Gly Ala Arg Arg Tyr Ala
                85                  90                  95

Lys Met Lys Arg Arg Arg Arg Arg Val Ala Arg Arg His Arg Arg Arg
            100                 105                 110

Pro Gly Thr Ala Ala Gln Arg Ala Ala Ala Ala Leu Leu Asn Arg Ala
        115                 120                 125

Arg Arg Thr Gly Arg Arg Ala Ala Met Arg Ala Ala Arg Arg Leu Ala
    130                 135                 140

Ala Gly Ile Val Thr Val Pro Pro Arg Ser Arg Arg Arg Ala Ala Ala
145                 150                 155                 160

Ala Ala Ala Ala Ala Ile Ser Ala Met Thr Gln Gly Arg Arg Gly Asn
                165                 170                 175

Val Tyr Trp Val Arg Asp Ser Val Ser Gly Leu Arg Val Pro Val Arg
            180                 185                 190

Thr Arg Pro Pro Arg Asn Val Arg Thr Arg Pro Pro Arg Asn Tyr Asn
        195                 200                 205

Arg Asn Ile Val Asn Arg Leu Leu Gly Asp Ala Leu Asn Gly Lys Pro
    210                 215                 220

Glu Glu Lys
225

<210>  43
<211>  374
<212>  PRT
<213>  Artificial Sequence

<220>
<223>  Synthetic Construct

<400>  43

Met Met Arg Lys Leu Ala Ile Leu Ser Val Ser Ser Phe Leu Phe Val
1               5                   10                  15

Glu Ala Leu Phe Gln Glu Tyr Gln Cys Tyr Gly Ser Ser Ser Asn Thr
            20                  25                  30

Arg Val Leu Asn Glu Leu Asn Tyr Asp Asn Ala Gly Thr Asn Leu Tyr
        35                  40                  45

Asn Glu Leu Glu Met Asn Tyr Tyr Gly Lys Gln Glu Asn Trp Tyr Ser
    50                  55                  60

Leu Lys Lys Asn Ser Arg Ser Leu Gly Glu Asn Asp Asp Gly Asn Asn
65                  70                  75                  80

Glu Asp Asn Glu Lys Leu Arg Lys Pro Lys His Lys Lys Leu Lys Gln
                85                  90                  95

Pro Ala Asp Gly Asn Pro Asp Pro Asn Ala Asn Pro Asn Val Asp Pro
        100                 105                 110

Asn Ala Asn Pro Asn Val Asp Pro Asn Ala Asn Pro Asn Val Asp Pro
    115                 120                 125

Asn Ala Asn Pro Asn Ala Asn Pro Asn Ala Asn Pro Asn Ala Asn Pro
130                 135                 140

Asn Ala Asn Pro Asn Ala Asn Pro Asn Ala Asn Pro Asn Ala Asn Pro
145                 150                 155                 160

Asn Ala Asn Pro Asn Ala Asn Pro Asn Ala Asn Pro Asn Ala Asn Pro
                165                 170                 175

Asn Ala Asn Pro Asn Ala Asn Pro Asn Ala Asn Pro Asn Ala Asn Pro
            180                 185                 190

Asn Ala Asn Pro Asn Val Asp Pro Asn Ala Asn Pro Asn Ala Asn Pro
        195                 200                 205

Asn Ala Asn Pro Asn Ala Asn Pro Asn Ala Asn Pro Asn Ala Asn Pro
    210                 215                 220

Asn Ala Asn Pro Asn Ala Asn Pro Asn Ala Asn Pro Asn Ala Asn Pro
225                 230                 235                 240

Asn Ala Asn Pro Asn Ala Asn Pro Asn Ala Asn Pro Asn Ala Asn Pro
                245                 250                 255

Asn Ala Asn Pro Asn Ala Asn Pro Asn Ala Asn Pro Asn Ala Asn Pro
            260                 265                 270

Asn Lys Asn Asn Gln Gly Asn Gly Gln Gly His Asn Met Pro Asn Asp
        275                 280                 285

Pro Asn Arg Asn Val Asp Glu Asn Ala Asn Ala Asn Ser Ala Val Lys
    290                 295                 300

Asn Asn Asn Asn Glu Glu Pro Ser Asp Lys His Ile Lys Glu Tyr Leu
305                 310                 315                 320

Asn Lys Ile Gln Asn Ser Leu Ser Thr Glu Trp Ser Pro Cys Ser Val
                325                 330                 335

Thr Cys Gly Asn Gly Ile Gln Val Arg Ile Lys Pro Gly Ser Ala Asn
            340                 345                 350

Lys Pro Lys Asp Glu Leu Asp Tyr Ala Asn Asp Ile Glu Lys Lys Ile
        355                 360                 365

Cys Lys Met Glu Lys Cys
    370

<210>  44
<211>  941
<212>  PRT
<213>  Artificial Sequence

<220>
<223>  Synthetic Construct

<400>  44

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Lys Thr His Val Phe Gly Gln
145                 150                 155                 160

Ala Pro Tyr Ser Gly Ile Asn Ile Thr Lys Glu Gly Ile Gln Ile Gly
                165                 170                 175

Val Glu Gly Gln Thr Pro Lys Tyr Ala Asp Lys Thr Phe Gln Pro Glu
        180                 185                 190

Pro Gln Ile Gly Glu Ser Gln Trp Tyr Glu Thr Glu Ile Asn His Ala
    195                 200                 205

Ala Gly Arg Val Leu Lys Lys Thr Thr Pro Met Lys Pro Cys Tyr Gly
    210                 215                 220

Ser Tyr Ala Lys Pro Thr Asn Glu Asn Gly Gly Gln Gly Ile Leu Val
225                 230                 235                 240

Lys Gln Gln Asn Gly Lys Leu Glu Ser Gln Val Glu Met Gln Phe Phe
                245                 250                 255

Ser Thr Thr Glu Ala Ala Ala Gly Asn Gly Asp Asn Leu Thr Pro Lys
        260                 265                 270

Val Val Leu Tyr Ser Glu Asp Val Asp Ile Glu Thr Pro Asp Thr His
    275                 280                 285

Ile Ser Tyr Met Pro Thr Ile Lys Glu Gly Asn Ser Arg Glu Leu Met
    290                 295                 300

Gly Gln Gln Ser Met Pro Asn Arg Pro Asn Tyr Ile Ala Phe Arg Asp
305                 310                 315                 320

Asn Phe Ile Gly Leu Met Tyr Tyr Asn Ser Thr Gly Asn Met Gly Val
                325                 330                 335

Leu Ala Gly Gln Ala Ser Gln Leu Asn Ala Val Val Asp Leu Gln Asp
        340                 345                 350

Arg Asn Thr Glu Leu Ser Tyr Gln Leu Leu Leu Asp Ser Ile Gly Asp
    355                 360                 365

Arg Thr Arg Tyr Phe Ser Met Trp Asn Gln Ala Val Asp Ser Tyr Asp
    370                 375                 380

Pro Asp Val Arg Ile Ile Glu Asn His Gly Thr Glu Asp Glu Leu Pro
385                 390                 395                 400

Asn Tyr Cys Phe Pro Leu Gly Gly Val Ile Asn Thr Glu Thr Leu Thr
                405                 410                 415

Lys Val Lys Pro Lys Thr Gly Gln Glu Asn Gly Trp Glu Lys Asp Ala

            420                 425                 430

Thr Glu Phe Ser Asp Lys Asn Glu Ile Arg Val Gly Asn Asn Phe Ala
        435                 440                 445

Met Glu Ile Asn Leu Asn Ala Asn Leu Trp Arg Asn Phe Leu Tyr Ser
    450                 455                 460

Asn Ile Ala Leu Tyr Leu Pro Asp Lys Leu Lys Tyr Ser Pro Ser Asn
465                 470                 475                 480

Val Lys Ile Ser Asp Asn Pro Asn Thr Tyr Asp Tyr Met Asn Lys Arg
                485                 490                 495

Val Val Ala Pro Gly Leu Val Asp Cys Tyr Ile Asn Leu Gly Ala Arg
            500                 505                 510

Trp Ser Leu Asp Tyr Met Asp Asn Val Asn Pro Phe Asn His His Arg
        515                 520                 525

Asn Ala Gly Leu Arg Tyr Arg Ser Met Leu Leu Gly Asn Gly Arg Tyr
    530                 535                 540

Val Pro Phe His Ile Gln Val Pro Gln Lys Phe Phe Ala Ile Lys Asn
545                 550                 555                 560

Leu Leu Leu Leu Pro Gly Ser Tyr Thr Tyr Glu Trp Asn Phe Arg Lys
                565                 570                 575

Asp Val Asn Met Val Leu Gln Ser Ser Leu Gly Asn Asp Leu Arg Val
            580                 585                 590

Asp Gly Ala Ser Ile Lys Phe Asp Ser Ile Cys Leu Tyr Ala Thr Phe
        595                 600                 605

Phe Pro Met Ala His Asn Thr Ala Ser Thr Leu Glu Ala Met Leu Arg
    610                 615                 620

Asn Asp Thr Asn Asp Gln Ser Phe Asn Asp Tyr Leu Ser Ala Ala Asn
625                 630                 635                 640

Met Leu Tyr Pro Ile Pro Ala Asn Ala Thr Asn Val Pro Ile Ser Ile
                645                 650                 655

Pro Ser Arg Asn Trp Ala Ala Phe Arg Gly Trp Ala Phe Thr Arg Leu
            660                 665                 670

Lys Thr Lys Glu Thr Pro Ser Leu Gly Ser Gly Tyr Asp Pro Tyr Tyr
        675                 680                 685

Thr Tyr Ser Gly Ser Ile Pro Tyr Leu Asp Gly Thr Phe Tyr Leu Asn
    690                 695                 700

His Thr Phe Lys Lys Val Ala Ile Thr Phe Asp Ser Ser Val Ser Trp
705                 710                 715                 720

Pro Gly Asn Asp Arg Leu Leu Thr Pro Asn Glu Phe Glu Ile Lys Arg
                725                 730                 735

Ser Val Asp Gly Glu Gly Tyr Asn Val Ala Gln Cys Asn Met Thr Lys
            740                 745                 750

Asp Trp Phe Leu Val Gln Met Leu Ala Asn Tyr Asn Ile Gly Tyr Gln
        755                 760                 765

Gly Phe Tyr Ile Pro Glu Ser Tyr Lys Asp Arg Met Tyr Ser Phe Phe
    770                 775                 780

Arg Asn Phe Gln Pro Met Ser Arg Gln Val Val Asp Asp Thr Lys Tyr
785                 790                 795                 800

Lys Asp Tyr Gln Gln Val Gly Ile Leu His Gln His Asn Asn Ser Gly
                805                 810                 815

Phe Val Gly Tyr Leu Ala Pro Thr Met Arg Glu Gly Gln Ala Tyr Pro
            820                 825                 830

Ala Asn Phe Pro Tyr Pro Leu Ile Gly Lys Thr Ala Val Asp Ser Ile
        835                 840                 845

Thr Gln Lys Lys Phe Leu Cys Asp Arg Thr Leu Trp Arg Ile Pro Phe
    850                 855                 860

Ser Ser Asn Phe Met Ser Met Gly Ala Leu Thr Asp Leu Gly Gln Asn
865                 870                 875                 880

Leu Leu Tyr Ala Asn Ser Ala His Ala Leu Asp Met Thr Phe Glu Val
                885                 890                 895

Asp Pro Met Asp Glu Pro Thr Leu Leu Tyr Val Leu Phe Glu Val Phe
            900                 905                 910

Asp Val Val Arg Val His Gln Pro His Arg Gly Val Ile Glu Thr Val
        915                 920                 925

Tyr Leu Arg Thr Pro Phe Ser Ala Gly Asn Ala Thr Thr
    930                 935                 940

<210>  45
<211>  929
<212>  PRT
<213>  Artificial Sequence

<220>
<223>  Synthetic Construct

<400>  45

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Lys Thr His Val Phe Gly Gln Ala Pro Tyr Ser Gly Ile Asn Ile
                165                 170                 175

Thr Lys Glu Gly Ile Gln Ile Gly Val Glu Gly Gln Thr Pro Lys Tyr
            180                 185                 190

Ala Asp Lys Thr Phe Gln Pro Glu Pro Gln Ile Gly Glu Ser Gln Trp
        195                 200                 205

Tyr Glu Thr Glu Ile Asn His Ala Ala Gly Arg Val Leu Lys Lys Thr
    210                 215                 220

Thr Pro Met Lys Pro Cys Tyr Gly Ser Tyr Ala Lys Pro Thr Asn Glu
225                 230                 235                 240

Asn Gly Gly Gln Gly Ile Leu Val Lys Gln Gln Asn Gly Lys Leu Glu
                245                 250                 255

Ser Gln Val Glu Met Gln Phe Phe Ser Thr Thr Glu Ala Ala Ala Gly
            260                 265                 270

Asn Gly Asp Asn Leu Thr Pro Lys Val Val Leu Tyr Ser Glu Asp Val
        275                 280                 285

Asp Ile Glu Thr Pro Asp Thr His Ile Ser Tyr Met Pro Thr Ile Lys
    290                 295                 300

Glu Gly Asn Ser Arg Glu Leu Met Gly Gln Gln Ser Met Pro Asn Arg
305                 310                 315                 320

Pro Asn Tyr Ile Ala Phe Arg Asp Asn Phe Ile Gly Leu Met Tyr Tyr
                325                 330                 335

Asn Ser Thr Gly Asn Met Gly Val Leu Ala Gly Gln Ala Ser Gln Leu
            340                 345                 350

Asn Ala Val Val Asp Leu Gln Asp Arg Asn Thr Glu Leu Ser Tyr Gln
        355                 360                 365

Leu Leu Leu Asp Ser Ile Gly Asp Arg Thr Arg Tyr Phe Ser Met Trp
    370                 375                 380

Asn Gln Leu Pro Asn Tyr Cys Phe Pro Leu Gly Gly Val Ile Asn Thr
385                 390                 395                 400

Glu Thr Leu Thr Lys Val Lys Pro Lys Thr Gly Gln Glu Asn Gly Trp
                405                 410                 415

Glu Lys Asp Ala Thr Glu Phe Ser Asp Lys Asn Glu Ile Arg Val Gly
            420                 425                 430

Asn Asn Phe Ala Met Glu Ile Asn Leu Asn Ala Asn Leu Trp Arg Asn

435                 440                 445

Phe Leu Tyr Ser Asn Ile Ala Leu Tyr Leu Pro Asp Lys Leu Lys Tyr
    450                 455                 460

Ser Pro Ser Asn Val Lys Ile Ser Asp Asn Pro Asn Thr Tyr Asp Tyr
465                 470                 475                 480

Met Asn Lys Arg Val Val Ala Pro Gly Leu Val Asp Cys Tyr Ile Asn
                485                 490                 495

Leu Gly Ala Arg Trp Ser Leu Asp Tyr Met Asp Asn Val Asn Pro Phe
            500                 505                 510

Asn His His Arg Asn Ala Gly Leu Arg Tyr Arg Ser Met Leu Leu Gly
        515                 520                 525

Asn Gly Arg Tyr Val Pro Phe His Ile Gln Val Pro Gln Lys Phe Phe
    530                 535                 540

Ala Ile Lys Asn Leu Leu Leu Leu Pro Gly Ser Tyr Thr Tyr Glu Trp
545                 550                 555                 560

Asn Phe Arg Lys Asp Val Asn Met Val Leu Gln Ser Ser Leu Gly Asn
                565                 570                 575

Asp Leu Arg Val Asp Gly Ala Ser Ile Lys Phe Asp Ser Ile Cys Leu
            580                 585                 590

Tyr Ala Thr Phe Phe Pro Met Ala His Asn Thr Ala Ser Thr Leu Glu
        595                 600                 605

Ala Met Leu Arg Asn Asp Thr Asn Asp Gln Ser Phe Asn Asp Tyr Leu
    610                 615                 620

Ser Ala Ala Asn Met Leu Tyr Pro Ile Pro Ala Asn Ala Thr Asn Val
625                 630                 635                 640

Pro Ile Ser Ile Pro Ser Arg Asn Trp Ala Ala Phe Arg Gly Trp Ala
                645                 650                 655

Phe Thr Arg Leu Lys Thr Lys Glu Thr Pro Ser Leu Gly Ser Gly Tyr
            660                 665                 670

Asp Pro Tyr Tyr Thr Tyr Ser Gly Ser Ile Pro Tyr Leu Asp Gly Thr
        675                 680                 685

Phe Tyr Leu Asn His Thr Phe Lys Lys Val Ala Ile Thr Phe Asp Ser
    690                 695                 700

Ser Val Ser Trp Pro Gly Asn Asp Arg Leu Leu Thr Pro Asn Glu Phe
705                 710                 715                 720

Glu Ile Lys Arg Ser Val Asp Gly Glu Gly Tyr Asn Val Ala Gln Cys
                725                 730                 735

Asn Met Thr Lys Asp Trp Phe Leu Val Gln Met Leu Ala Asn Tyr Asn
            740                 745                 750

Ile Gly Tyr Gln Gly Phe Tyr Ile Pro Glu Ser Tyr Lys Asp Arg Met
        755                 760                 765

Tyr Ser Phe Phe Arg Asn Phe Gln Pro Met Ser Arg Gln Val Val Asp
    770                 775                 780

Asp Thr Lys Tyr Lys Asp Tyr Gln Gln Val Gly Ile Leu His Gln His
785                 790                 795                 800

Asn Asn Ser Gly Phe Val Gly Tyr Leu Ala Pro Thr Met Arg Glu Gly
                805                 810                 815

Gln Ala Tyr Pro Ala Asn Phe Pro Tyr Pro Leu Ile Gly Lys Thr Ala
            820                 825                 830

Val Asp Ser Ile Thr Gln Lys Lys Phe Leu Cys Asp Arg Thr Leu Trp
        835                 840                 845

Arg Ile Pro Phe Ser Ser Asn Phe Met Ser Met Gly Ala Leu Thr Asp
    850                 855                 860

Leu Gly Gln Asn Leu Leu Tyr Ala Asn Ser Ala His Ala Leu Asp Met
865                 870                 875                 880

Thr Phe Glu Val Asp Pro Met Asp Glu Pro Thr Leu Leu Tyr Val Leu
                885                 890                 895

Phe Glu Val Phe Asp Val Val Arg Val His Gln Pro His Arg Gly Val
            900                 905                 910

Ile Glu Thr Val Tyr Leu Arg Thr Pro Phe Ser Ala Gly Asn Ala Thr
        915                 920                 925

Thr

<210>  46
<211>  937
<212>  PRT
<213>  Artificial Sequence

<220>
<223>  Synthetic Construct

<400>  46

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Lys Thr His Val Phe Gly Gln
                165                 170                 175

Ala Pro Tyr Ser Gly Ile Asn Ile Thr Lys Glu Gly Ile Gln Ile Gly
            180                 185                 190

Val Glu Gly Gln Thr Pro Lys Tyr Ala Asp Lys Thr Phe Gln Pro Glu

195                 200                 205

Pro Gln Ile Gly Glu Ser Gln Trp Tyr Glu Thr Glu Ile Asn His Ala
    210                 215                 220

Ala Gly Arg Val Leu Lys Lys Thr Thr Pro Met Lys Pro Cys Tyr Gly
225                 230                 235                 240

Ser Tyr Ala Lys Pro Thr Asn Glu Asn Gly Gly Gln Gly Ile Leu Val
                245                 250                 255

Lys Gln Gln Asn Gly Lys Leu Glu Ser Gln Val Glu Met Gln Phe Phe
            260                 265                 270

Ser Thr Thr Glu Ala Ala Ala Gly Asn Gly Asp Asn Leu Thr Pro Lys
        275                 280                 285

Val Val Leu Tyr Ser Glu Asp Val Asp Ile Glu Thr Pro Asp Thr His
    290                 295                 300

Ile Ser Tyr Met Pro Thr Ile Lys Glu Gly Asn Ser Arg Glu Leu Met
305                 310                 315                 320

Gly Gln Gln Ser Met Pro Asn Arg Pro Asn Tyr Ile Ala Phe Arg Asp
                325                 330                 335

Asn Phe Ile Gly Leu Met Tyr Tyr Asn Ser Thr Gly Asn Met Gly Val
            340                 345                 350

Leu Ala Gly Gln Ala Ser Gln Leu Asn Ala Val Val Asp Leu Gln Asp
        355                 360                 365

Arg Asn Thr Glu Leu Ser Tyr Gln Leu Leu Leu Asp Ser Ile Gly Asp
    370                 375                 380

Arg Thr Arg Tyr Phe Ser Met Trp Asn Gln Leu Pro Asn Tyr Cys Phe
385                 390                 395                 400

Pro Leu Gly Gly Val Ile Asn Thr Glu Thr Leu Thr Lys Val Lys Pro
                405                 410                 415

Lys Thr Gly Gln Glu Asn Gly Trp Glu Lys Asp Ala Thr Glu Phe Ser
            420                 425                 430

Asp Lys Asn Glu Ile Arg Val Gly Asn Asn Phe Ala Met Glu Ile Asn
        435                 440                 445

Leu Asn Ala Asn Leu Trp Arg Asn Phe Leu Tyr Ser Asn Ile Ala Leu
    450                 455                 460

Tyr Leu Pro Asp Lys Leu Lys Tyr Ser Pro Ser Asn Val Lys Ile Ser
465                 470                 475                 480

Asp Asn Pro Asn Thr Tyr Asp Tyr Met Asn Lys Arg Val Val Ala Pro
                485                 490                 495

Gly Leu Val Asp Cys Tyr Ile Asn Leu Gly Ala Arg Trp Ser Leu Asp
            500                 505                 510

Tyr Met Asp Asn Val Asn Pro Phe Asn His His Arg Asn Ala Gly Leu
        515                 520                 525

Arg Tyr Arg Ser Met Leu Leu Gly Asn Gly Arg Tyr Val Pro Phe His
    530                 535                 540

Ile Gln Val Pro Gln Lys Phe Phe Ala Ile Lys Asn Leu Leu Leu Leu
545                 550                 555                 560

Pro Gly Ser Tyr Thr Tyr Glu Trp Asn Phe Arg Lys Asp Val Asn Met
                565                 570                 575

Val Leu Gln Ser Ser Leu Gly Asn Asp Leu Arg Val Asp Gly Ala Ser
            580                 585                 590

Ile Lys Phe Asp Ser Ile Cys Leu Tyr Ala Thr Phe Phe Pro Met Ala
        595                 600                 605

His Asn Thr Ala Ser Thr Leu Glu Ala Met Leu Arg Asn Asp Thr Asn
    610                 615                 620

Asp Gln Ser Phe Asn Asp Tyr Leu Ser Ala Ala Asn Met Leu Tyr Pro
625                 630                 635                 640

Ile Pro Ala Asn Ala Thr Asn Val Pro Ile Ser Ile Pro Ser Arg Asn
                645                 650                 655

Trp Ala Ala Phe Arg Gly Trp Ala Phe Thr Arg Leu Lys Thr Lys Glu
            660                 665                 670

Thr Pro Ser Leu Gly Ser Gly Tyr Asp Pro Tyr Tyr Thr Tyr Ser Gly
        675                 680                 685

Ser Ile Pro Tyr Leu Asp Gly Thr Phe Tyr Leu Asn His Thr Phe Lys
    690                 695                 700

Lys Val Ala Ile Thr Phe Asp Ser Ser Val Ser Trp Pro Gly Asn Asp
705                 710                 715                 720

Arg Leu Leu Thr Pro Asn Glu Phe Glu Ile Lys Arg Ser Val Asp Gly
                725                 730                 735

Glu Gly Tyr Asn Val Ala Gln Cys Asn Met Thr Lys Asp Trp Phe Leu
            740                 745                 750

Val Gln Met Leu Ala Asn Tyr Asn Ile Gly Tyr Gln Gly Phe Tyr Ile
        755                 760                 765

Pro Glu Ser Tyr Lys Asp Arg Met Tyr Ser Phe Phe Arg Asn Phe Gln
    770                 775                 780

Pro Met Ser Arg Gln Val Val Asp Asp Thr Lys Tyr Lys Asp Tyr Gln
785                 790                 795                 800

Gln Val Gly Ile Leu His Gln His Asn Asn Ser Gly Phe Val Gly Tyr
                805                 810                 815

Leu Ala Pro Thr Met Arg Glu Gly Gln Ala Tyr Pro Ala Asn Phe Pro
            820                 825                 830

Tyr Pro Leu Ile Gly Lys Thr Ala Val Asp Ser Ile Thr Gln Lys Lys
        835                 840                 845

Phe Leu Cys Asp Arg Thr Leu Trp Arg Ile Pro Phe Ser Ser Asn Phe
    850                 855                 860

Met Ser Met Gly Ala Leu Thr Asp Leu Gly Gln Asn Leu Leu Tyr Ala
865                 870                 875                 880

Asn Ser Ala His Ala Leu Asp Met Thr Phe Glu Val Asp Pro Met Asp
                885                 890                 895

Glu Pro Thr Leu Leu Tyr Val Leu Phe Glu Val Phe Asp Val Val Arg
            900                 905                 910

Val His Gln Pro His Arg Gly Val Ile Glu Thr Val Tyr Leu Arg Thr
        915                 920                 925

Pro Phe Ser Ala Gly Asn Ala Thr Thr
    930                 935

<210>  47
<211>  945
<212>  PRT
<213>  Artificial Sequence

<220>
<223>  Synthetic Construct

<400>  47
   Met Ala Thr Pro Ser Met Met Pro Gln Trp Ser Tyr Met His Ile Ser
   1               5                   10                  15
   Gly Gln Asp Ala Ser Glu Tyr Leu Ser Pro Gly Leu Val Gln Phe Ala
               20                  25                  30
   Arg Ala Thr Glu Thr Tyr Phe Ser Leu Asn Asn Lys Phe Arg Asn Pro
           35                  40                  45
   Thr Val Ala Pro Thr His Asp Val Thr Thr Asp Arg Ser Gln Arg Leu
       50                  55                  60
   Thr Leu Arg Phe Ile Pro Val Asp Arg Glu Asp Thr Ala Tyr Ser Tyr
   65                  70                  75                  80
   Lys Ala Arg Phe Thr Leu Ala Val Gly Asp Asn Arg Val Leu Asp Met
                   85                  90                  95
   Ala Ser Thr Tyr Phe Asp Ile Arg Gly Val Leu Asp Arg Gly Pro Thr
               100                 105                 110
   Phe Lys Pro Tyr Ser Gly Thr Ala Tyr Asn Ala Leu Ala Pro Lys Gly
           115                 120                 125
   Ala Pro Asn Pro Cys Glu Trp Asp Glu Asn Ala Asn Pro Asn Ala Asn
       130                 135                 140
   Pro Asn Ala Asn Pro Asn Ala Asn Pro Asn Ala Asn Pro Asn Ala Asn
   145                 150                 155                 160
   Pro Asn Ala Asn Pro Asn Ala Asn Pro Asn Ala Asn Pro Asn Ala Asn
                   165                 170                 175
   Pro Lys Thr His Val Phe Gly Gln Ala Pro Tyr Ser Gly Ile Asn Ile
               180                 185                 190
   Thr Lys Glu Gly Ile Gln Ile Gly Val Glu Gly Gln Thr Pro Lys Tyr
           195                 200                 205
   Ala Asp Lys Thr Phe Gln Pro Glu Pro Gln Ile Gly Glu Ser Gln Trp
   210                 215                 220
   Tyr Glu Thr Glu Ile Asn His Ala Ala Gly Arg Val Leu Lys Lys Thr
   225                 230                 235                 240
   Thr Pro Met Lys Pro Cys Tyr Gly Ser Tyr Ala Lys Pro Thr Asn Glu
                   245                 250                 255
   Asn Gly Gly Gln Gly Ile Leu Val Lys Gln Gln Asn Gly Lys Leu Glu
               260                 265                 270
   Ser Gln Val Glu Met Gln Phe Phe Ser Thr Thr Glu Ala Ala Ala Gly
           275                 280                 285
   Asn Gly Asp Asn Leu Thr Pro Lys Val Val Leu Tyr Ser Glu Asp Val
       290                 295                 300
   Asp Ile Glu Thr Pro Asp Thr His Ile Ser Tyr Met Pro Thr Ile Lys
   305                 310                 315                 320
   Glu Gly Asn Ser Arg Glu Leu Met Gly Gln Gln Ser Met Pro Asn Arg
                   325                 330                 335
   Pro Asn Tyr Ile Ala Phe Arg Asp Asn Phe Ile Gly Leu Met Tyr Tyr
               340                 345                 350
   Asn Ser Thr Gly Asn Met Gly Val Leu Ala Gly Gln Ala Ser Gln Leu
           355                 360                 365
   Asn Ala Val Val Asp Leu Gln Asp Arg Asn Thr Glu Leu Ser Tyr Gln
       370                 375                 380
   Leu Leu Leu Asp Ser Ile Gly Asp Arg Thr Arg Tyr Phe Ser Met Trp
   385                 390                 395                 400
   Asn Gln Leu Pro Asn Tyr Cys Phe Pro Leu Gly Gly Val Ile Asn Thr
                   405                 410                 415
   Glu Thr Leu Thr Lys Val Lys Pro Lys Thr Gly Gln Glu Asn Gly Trp
               420                 425                 430
   Glu Lys Asp Ala Thr Glu Phe Ser Asp Lys Asn Glu Ile Arg Val Gly
           435                 440                 445
   Asn Asn Phe Ala Met Glu Ile Asn Leu Asn Ala Asn Leu Trp Arg Asn
       450                 455                 460
   Phe Leu Tyr Ser Asn Ile Ala Leu Tyr Leu Pro Asp Lys Leu Lys Tyr
   465                 470                 475                 480
   Ser Pro Ser Asn Val Lys Ile Ser Asp Asn Pro Asn Thr Tyr Asp Tyr
                   485                 490                 495
   Met Asn Lys Arg Val Val Ala Pro Gly Leu Val Asp Cys Tyr Ile Asn
               500                 505                 510
   Leu Gly Ala Arg Trp Ser Leu Asp Tyr Met Asp Asn Val Asn Pro Phe
           515                 520                 525
   Asn His His Arg Asn Ala Gly Leu Arg Tyr Arg Ser Met Leu Leu Gly
       530                 535                 540
   Asn Gly Arg Tyr Val Pro Phe His Ile Gln Val Pro Gln Lys Phe Phe
   545                 550                 555                 560
   Ala Ile Lys Asn Leu Leu Leu Leu Pro Gly Ser Tyr Thr Tyr Glu Trp
                   565                 570                 575
   Asn Phe Arg Lys Asp Val Asn Met Val Leu Gln Ser Ser Leu Gly Asn
               580                 585                 590
   Asp Leu Arg Val Asp Gly Ala Ser Ile Lys Phe Asp Ser Ile Cys Leu
           595                 600                 605
   Tyr Ala Thr Phe Phe Pro Met Ala His Asn Thr Ala Ser Thr Leu Glu
       610                 615                 620
   Ala Met Leu Arg Asn Asp Thr Asn Asp Gln Ser Phe Asn Asp Tyr Leu
   625                 630                 635                 640
   Ser Ala Ala Asn Met Leu Tyr Pro Ile Pro Ala Asn Ala Thr Asn Val
                   645                 650                 655
   Pro Ile Ser Ile Pro Ser Arg Asn Trp Ala Ala Phe Arg Gly Trp Ala
               660                 665                 670
   Phe Thr Arg Leu Lys Thr Lys Glu Thr Pro Ser Leu Gly Ser Gly Tyr
           675                 680                 685
   Asp Pro Tyr Tyr Thr Tyr Ser Gly Ser Ile Pro Tyr Leu Asp Gly Thr
       690                 695                 700
   Phe Tyr Leu Asn His Thr Phe Lys Lys Val Ala Ile Thr Phe Asp Ser
   705                 710                 715                 720
   Ser Val Ser Trp Pro Gly Asn Asp Arg Leu Leu Thr Pro Asn Glu Phe
   725 730 735
   Glu Ile Lys Arg Ser Val Asp Gly Glu Gly Tyr Asn Val Ala Gln Cys
   740 745 750
   Asn Met Thr Lys Asp Trp Phe Leu Val Gln Met Leu Ala Asn Tyr Asn
   755 760 765
   Ile Gly Tyr Gln Gly Phe Tyr Ile Pro Glu Ser Tyr Lys Asp Arg Met
   770 775 780
   Tyr Ser Phe Phe Arg Asn Phe Gln Pro Met Ser Arg Gln Val Val Asp
   785 790 795 800
   Asp Thr Lys Tyr Lys Asp Tyr Gln Gln Val Gly Ile Leu His Gln His
   805 810 815
   Asn Asn Ser Gly Phe Val Gly Tyr Leu Ala Pro Thr Met Arg Glu Gly
   820 825 830
   Gln Ala Tyr Pro Ala Asn Phe Pro Tyr Pro Leu Ile Gly Lys Thr Ala
   835 840 845
   Val Asp Ser Ile Thr Gln Lys Lys Phe Leu Cys Asp Arg Thr Leu Trp
   850 855 860
   Arg Ile Pro Phe Ser Ser Asn Phe Met Ser Met Gly Ala Leu Thr Asp
   865 870 875 880
   Leu Gly Gln Asn Leu Leu Tyr Ala Asn Ser Ala His Ala Leu Asp Met
   885 890 895
   Thr Phe Glu Val Asp Pro Met Asp Glu Pro Thr Leu Leu Tyr Val Leu
   900 905 910
   Phe Glu Val Phe Asp Val Val Arg Val His Gln Pro His Arg Gly Val
   915 920 925
   Ile Glu Thr Val Tyr Leu Arg Thr Pro Phe Ser Ala Gly Asn Ala Thr
   930 935 940
   Thr
   945

<210>  48
<211>  953
<212>  PRT
<213>  Artificial Sequence

<220>
<223>  Synthetic Construct

<400>  48

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Asn Ala Asn Pro Asn Ala Asn
                165                 170                 175

Pro Asn Ala Asn Pro Asn Ala Asn Pro Lys Thr His Val Phe Gly Gln
            180                 185                 190

Ala Pro Tyr Ser Gly Ile Asn Ile Thr Lys Glu Gly Ile Gln Ile Gly
        195                 200                 205

Val Glu Gly Gln Thr Pro Lys Tyr Ala Asp Lys Thr Phe Gln Pro Glu

210                 215                 220

Pro Gln Ile Gly Glu Ser Gln Trp Tyr Glu Thr Glu Ile Asn His Ala
225                 230                 235                 240

Ala Gly Arg Val Leu Lys Lys Thr Thr Pro Met Lys Pro Cys Tyr Gly
                245                 250                 255

Ser Tyr Ala Lys Pro Thr Asn Glu Asn Gly Gly Gln Gly Ile Leu Val
            260                 265                 270

Lys Gln Gln Asn Gly Lys Leu Glu Ser Gln Val Glu Met Gln Phe Phe
        275                 280                 285

Ser Thr Thr Glu Ala Ala Ala Gly Asn Gly Asp Asn Leu Thr Pro Lys
    290                 295                 300

Val Val Leu Tyr Ser Glu Asp Val Asp Ile Glu Thr Pro Asp Thr His
305                 310                 315                 320

Ile Ser Tyr Met Pro Thr Ile Lys Glu Gly Asn Ser Arg Glu Leu Met
                325                 330                 335

Gly Gln Gln Ser Met Pro Asn Arg Pro Asn Tyr Ile Ala Phe Arg Asp
            340                 345                 350

Asn Phe Ile Gly Leu Met Tyr Tyr Asn Ser Thr Gly Asn Met Gly Val
        355                 360                 365

Leu Ala Gly Gln Ala Ser Gln Leu Asn Ala Val Val Asp Leu Gln Asp
    370                 375                 380

Arg Asn Thr Glu Leu Ser Tyr Gln Leu Leu Leu Asp Ser Ile Gly Asp
385                 390                 395                 400

Arg Thr Arg Tyr Phe Ser Met Trp Asn Gln Leu Pro Asn Tyr Cys Phe
                405                 410                 415

Pro Leu Gly Gly Val Ile Asn Thr Glu Thr Leu Thr Lys Val Lys Pro
            420                 425                 430

Lys Thr Gly Gln Glu Asn Gly Trp Glu Lys Asp Ala Thr Glu Phe Ser
        435                 440                 445

Asp Lys Asn Glu Ile Arg Val Gly Asn Asn Phe Ala Met Glu Ile Asn
    450                 455                 460

Leu Asn Ala Asn Leu Trp Arg Asn Phe Leu Tyr Ser Asn Ile Ala Leu
465                 470                 475                 480

Tyr Leu Pro Asp Lys Leu Lys Tyr Ser Pro Ser Asn Val Lys Ile Ser
                485                 490                 495

Asp Asn Pro Asn Thr Tyr Asp Tyr Met Asn Lys Arg Val Val Ala Pro
            500                 505                 510

Gly Leu Val Asp Cys Tyr Ile Asn Leu Gly Ala Arg Trp Ser Leu Asp
        515                 520                 525

Tyr Met Asp Asn Val Asn Pro Phe Asn His His Arg Asn Ala Gly Leu
    530                 535                 540

Arg Tyr Arg Ser Met Leu Leu Gly Asn Gly Arg Tyr Val Pro Phe His
545                 550                 555                 560

Ile Gln Val Pro Gln Lys Phe Phe Ala Ile Lys Asn Leu Leu Leu Leu
                565                 570                 575

Pro Gly Ser Tyr Thr Tyr Glu Trp Asn Phe Arg Lys Asp Val Asn Met
            580                 585                 590

Val Leu Gln Ser Ser Leu Gly Asn Asp Leu Arg Val Asp Gly Ala Ser
        595                 600                 605

Ile Lys Phe Asp Ser Ile Cys Leu Tyr Ala Thr Phe Phe Pro Met Ala
    610                 615                 620

His Asn Thr Ala Ser Thr Leu Glu Ala Met Leu Arg Asn Asp Thr Asn
625                 630                 635                 640

Asp Gln Ser Phe Asn Asp Tyr Leu Ser Ala Ala Asn Met Leu Tyr Pro
                645                 650                 655

Ile Pro Ala Asn Ala Thr Asn Val Pro Ile Ser Ile Pro Ser Arg Asn
            660                 665                 670

Trp Ala Ala Phe Arg Gly Trp Ala Phe Thr Arg Leu Lys Thr Lys Glu
        675                 680                 685

Thr Pro Ser Leu Gly Ser Gly Tyr Asp Pro Tyr Tyr Thr Tyr Ser Gly
    690                 695                 700

Ser Ile Pro Tyr Leu Asp Gly Thr Phe Tyr Leu Asn His Thr Phe Lys
705                 710                 715                 720

Lys Val Ala Ile Thr Phe Asp Ser Ser Val Ser Trp Pro Gly Asn Asp
                725                 730                 735

Arg Leu Leu Thr Pro Asn Glu Phe Glu Ile Lys Arg Ser Val Asp Gly
            740                 745                 750

Glu Gly Tyr Asn Val Ala Gln Cys Asn Met Thr Lys Asp Trp Phe Leu
        755                 760                 765

Val Gln Met Leu Ala Asn Tyr Asn Ile Gly Tyr Gln Gly Phe Tyr Ile
    770                 775                 780

Pro Glu Ser Tyr Lys Asp Arg Met Tyr Ser Phe Phe Arg Asn Phe Gln
785                 790                 795                 800

Pro Met Ser Arg Gln Val Val Asp Asp Thr Lys Tyr Lys Asp Tyr Gln
                805                 810                 815

Gln Val Gly Ile Leu His Gln His Asn Asn Ser Gly Phe Val Gly Tyr
            820                 825                 830

Leu Ala Pro Thr Met Arg Glu Gly Gln Ala Tyr Pro Ala Asn Phe Pro
        835                 840                 845

Tyr Pro Leu Ile Gly Lys Thr Ala Val Asp Ser Ile Thr Gln Lys Lys
    850                 855                 860

Phe Leu Cys Asp Arg Thr Leu Trp Arg Ile Pro Phe Ser Ser Asn Phe
865                 870                 875                 880

Met Ser Met Gly Ala Leu Thr Asp Leu Gly Gln Asn Leu Leu Tyr Ala
                885                 890                 895

Asn Ser Ala His Ala Leu Asp Met Thr Phe Glu Val Asp Pro Met Asp
            900                 905                 910

Glu Pro Thr Leu Leu Tyr Val Leu Phe Glu Val Phe Asp Val Val Arg
        915                 920                 925

Val His Gln Pro His Arg Gly Val Ile Glu Thr Val Tyr Leu Arg Thr
    930                 935                 940

Pro Phe Ser Ala Gly Asn Ala Thr Thr
945                 950

<210>  49
<211>  961
<212>  PRT
<213>  Artificial Sequence

<220>
<223>  Synthetic Construct

<400>  49

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Asn Ala Asn Pro Asn Ala Asn
                165                 170                 175

Pro Asn Ala Asn Pro Asn Ala Asn Pro Asn Ala Asn Pro Asn Ala Asn
            180                 185                 190

Pro Lys Thr His Val Phe Gly Gln Ala Pro Tyr Ser Gly Ile Asn Ile
        195                 200                 205

Thr Lys Glu Gly Ile Gln Ile Gly Val Glu Gly Gln Thr Pro Lys Tyr

210                 215                 220

Ala Asp Lys Thr Phe Gln Pro Glu Pro Gln Ile Gly Glu Ser Gln Trp
225                 230                 235                 240

Tyr Glu Thr Glu Ile Asn His Ala Ala Gly Arg Val Leu Lys Lys Thr
                245                 250                 255

Thr Pro Met Lys Pro Cys Tyr Gly Ser Tyr Ala Lys Pro Thr Asn Glu
            260                 265                 270

Asn Gly Gly Gln Gly Ile Leu Val Lys Gln Gln Asn Gly Lys Leu Glu
        275                 280                 285

Ser Gln Val Glu Met Gln Phe Phe Ser Thr Thr Glu Ala Ala Ala Gly
    290                 295                 300

Asn Gly Asp Asn Leu Thr Pro Lys Val Val Leu Tyr Ser Glu Asp Val
305                 310                 315                 320

Asp Ile Glu Thr Pro Asp Thr His Ile Ser Tyr Met Pro Thr Ile Lys
                325                 330                 335

Glu Gly Asn Ser Arg Glu Leu Met Gly Gln Gln Ser Met Pro Asn Arg
            340                 345                 350

Pro Asn Tyr Ile Ala Phe Arg Asp Asn Phe Ile Gly Leu Met Tyr Tyr
        355                 360                 365

Asn Ser Thr Gly Asn Met Gly Val Leu Ala Gly Gln Ala Ser Gln Leu
    370                 375                 380

Asn Ala Val Val Asp Leu Gln Asp Arg Asn Thr Glu Leu Ser Tyr Gln
385                 390                 395                 400

Leu Leu Leu Asp Ser Ile Gly Asp Arg Thr Arg Tyr Phe Ser Met Trp
                405                 410                 415

Asn Gln Leu Pro Asn Tyr Cys Phe Pro Leu Gly Gly Val Ile Asn Thr
            420                 425                 430

Glu Thr Leu Thr Lys Val Lys Pro Lys Thr Gly Gln Glu Asn Gly Trp
        435                 440                 445

Glu Lys Asp Ala Thr Glu Phe Ser Asp Lys Asn Glu Ile Arg Val Gly
    450                 455                 460

Asn Asn Phe Ala Met Glu Ile Asn Leu Asn Ala Asn Leu Trp Arg Asn
465                 470                 475                 480

Phe Leu Tyr Ser Asn Ile Ala Leu Tyr Leu Pro Asp Lys Leu Lys Tyr
                485                 490                 495

Ser Pro Ser Asn Val Lys Ile Ser Asp Asn Pro Asn Thr Tyr Asp Tyr
            500                 505                 510

Met Asn Lys Arg Val Val Ala Pro Gly Leu Val Asp Cys Tyr Ile Asn
        515                 520                 525

Leu Gly Ala Arg Trp Ser Leu Asp Tyr Met Asp Asn Val Asn Pro Phe
    530                 535                 540

Asn His His Arg Asn Ala Gly Leu Arg Tyr Arg Ser Met Leu Leu Gly
545                 550                 555                 560

Asn Gly Arg Tyr Val Pro Phe His Ile Gln Val Pro Gln Lys Phe Phe
                565                 570                 575

Ala Ile Lys Asn Leu Leu Leu Leu Pro Gly Ser Tyr Thr Tyr Glu Trp
            580                 585                 590

Asn Phe Arg Lys Asp Val Asn Met Val Leu Gln Ser Ser Leu Gly Asn
        595                 600                 605

Asp Leu Arg Val Asp Gly Ala Ser Ile Lys Phe Asp Ser Ile Cys Leu
    610                 615                 620

Tyr Ala Thr Phe Phe Pro Met Ala His Asn Thr Ala Ser Thr Leu Glu
625                 630                 635                 640

Ala Met Leu Arg Asn Asp Thr Asn Asp Gln Ser Phe Asn Asp Tyr Leu
                645                 650                 655

Ser Ala Ala Asn Met Leu Tyr Pro Ile Pro Ala Asn Ala Thr Asn Val
            660                 665                 670

Pro Ile Ser Ile Pro Ser Arg Asn Trp Ala Ala Phe Arg Gly Trp Ala
        675                 680                 685

Phe Thr Arg Leu Lys Thr Lys Glu Thr Pro Ser Leu Gly Ser Gly Tyr
    690                 695                 700

Asp Pro Tyr Tyr Thr Tyr Ser Gly Ser Ile Pro Tyr Leu Asp Gly Thr
705                 710                 715                 720

Phe Tyr Leu Asn His Thr Phe Lys Lys Val Ala Ile Thr Phe Asp Ser
                725                 730                 735

Ser Val Ser Trp Pro Gly Asn Asp Arg Leu Leu Thr Pro Asn Glu Phe
            740                 745                 750

Glu Ile Lys Arg Ser Val Asp Gly Glu Gly Tyr Asn Val Ala Gln Cys
        755                 760                 765

Asn Met Thr Lys Asp Trp Phe Leu Val Gln Met Leu Ala Asn Tyr Asn
    770                 775                 780

Ile Gly Tyr Gln Gly Phe Tyr Ile Pro Glu Ser Tyr Lys Asp Arg Met
785                 790                 795                 800

Tyr Ser Phe Phe Arg Asn Phe Gln Pro Met Ser Arg Gln Val Val Asp
                805                 810                 815

Asp Thr Lys Tyr Lys Asp Tyr Gln Gln Val Gly Ile Leu His Gln His
            820                 825                 830

Asn Asn Ser Gly Phe Val Gly Tyr Leu Ala Pro Thr Met Arg Glu Gly
        835                 840                 845

Gln Ala Tyr Pro Ala Asn Phe Pro Tyr Pro Leu Ile Gly Lys Thr Ala
    850                 855                 860

Val Asp Ser Ile Thr Gln Lys Lys Phe Leu Cys Asp Arg Thr Leu Trp
865                 870                 875                 880

Arg Ile Pro Phe Ser Ser Asn Phe Met Ser Met Gly Ala Leu Thr Asp
                885                 890                 895

Leu Gly Gln Asn Leu Leu Tyr Ala Asn Ser Ala His Ala Leu Asp Met
            900                 905                 910

Thr Phe Glu Val Asp Pro Met Asp Glu Pro Thr Leu Leu Tyr Val Leu
        915                 920                 925

Phe Glu Val Phe Asp Val Val Arg Val His Gln Pro His Arg Gly Val
    930                 935                 940

Ile Glu Thr Val Tyr Leu Arg Thr Pro Phe Ser Ala Gly Asn Ala Thr
945                 950                 955                 960

Thr

<210>  50
<211>  969
<212>  PRT
<213>  Artificial Sequence

<220>
<223>  Synthetic Construct

<400>  50

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Asn Ala Asn Pro Asn Ala Asn
                165                 170                 175

Pro Asn Ala Asn Pro Asn Ala Asn Pro Asn Ala Asn Pro Asn Ala Asn
            180                 185                 190

Pro Asn Ala Asn Pro Asn Ala Asn Pro Lys Thr His Val Phe Gly Gln
        195                 200                 205

Ala Pro Tyr Ser Gly Ile Asn Ile Thr Lys Glu Gly Ile Gln Ile Gly
    210                 215                 220

Val Glu Gly Gln Thr Pro Lys Tyr Ala Asp Lys Thr Phe Gln Pro Glu
225                 230                 235                 240

Pro Gln Ile Gly Glu Ser Gln Trp Tyr Glu Thr Glu Ile Asn His Ala
                245                 250                 255

Ala Gly Arg Val Leu Lys Lys Thr Thr Pro Met Lys Pro Cys Tyr Gly
            260                 265                 270

Ser Tyr Ala Lys Pro Thr Asn Glu Asn Gly Gly Gln Gly Ile Leu Val
        275                 280                 285

Lys Gln Gln Asn Gly Lys Leu Glu Ser Gln Val Glu Met Gln Phe Phe
    290                 295                 300

Ser Thr Thr Glu Ala Ala Ala Gly Asn Gly Asp Asn Leu Thr Pro Lys
305                 310                 315                 320

Val Val Leu Tyr Ser Glu Asp Val Asp Ile Glu Thr Pro Asp Thr His
                325                 330                 335

Ile Ser Tyr Met Pro Thr Ile Lys Glu Gly Asn Ser Arg Glu Leu Met
            340                 345                 350

Gly Gln Gln Ser Met Pro Asn Arg Pro Asn Tyr Ile Ala Phe Arg Asp
        355                 360                 365

Asn Phe Ile Gly Leu Met Tyr Tyr Asn Ser Thr Gly Asn Met Gly Val
    370                 375                 380

Leu Ala Gly Gln Ala Ser Gln Leu Asn Ala Val Val Asp Leu Gln Asp
385                 390                 395                 400

Arg Asn Thr Glu Leu Ser Tyr Gln Leu Leu Leu Asp Ser Ile Gly Asp
                405                 410                 415

Arg Thr Arg Tyr Phe Ser Met Trp Asn Gln Leu Pro Asn Tyr Cys Phe
            420                 425                 430

Pro Leu Gly Gly Val Ile Asn Thr Glu Thr Leu Thr Lys Val Lys Pro
        435                 440                 445

Lys Thr Gly Gln Glu Asn Gly Trp Glu Lys Asp Ala Thr Glu Phe Ser

450                 455                 460

Asp Lys Asn Glu Ile Arg Val Gly Asn Asn Phe Ala Met Glu Ile Asn
465                 470                 475                 480

Leu Asn Ala Asn Leu Trp Arg Asn Phe Leu Tyr Ser Asn Ile Ala Leu
                485                 490                 495

Tyr Leu Pro Asp Lys Leu Lys Tyr Ser Pro Ser Asn Val Lys Ile Ser
            500                 505                 510

Asp Asn Pro Asn Thr Tyr Asp Tyr Met Asn Lys Arg Val Val Ala Pro
        515                 520                 525

Gly Leu Val Asp Cys Tyr Ile Asn Leu Gly Ala Arg Trp Ser Leu Asp
    530                 535                 540

Tyr Met Asp Asn Val Asn Pro Phe Asn His His Arg Asn Ala Gly Leu
545                 550                 555                 560

Arg Tyr Arg Ser Met Leu Leu Gly Asn Gly Arg Tyr Val Pro Phe His
                565                 570                 575

Ile Gln Val Pro Gln Lys Phe Phe Ala Ile Lys Asn Leu Leu Leu Leu
            580                 585                 590

Pro Gly Ser Tyr Thr Tyr Glu Trp Asn Phe Arg Lys Asp Val Asn Met
        595                 600                 605

Val Leu Gln Ser Ser Leu Gly Asn Asp Leu Arg Val Asp Gly Ala Ser
    610                 615                 620

Ile Lys Phe Asp Ser Ile Cys Leu Tyr Ala Thr Phe Phe Pro Met Ala
625                 630                 635                 640

His Asn Thr Ala Ser Thr Leu Glu Ala Met Leu Arg Asn Asp Thr Asn
                645                 650                 655

Asp Gln Ser Phe Asn Asp Tyr Leu Ser Ala Ala Asn Met Leu Tyr Pro
            660                 665                 670

Ile Pro Ala Asn Ala Thr Asn Val Pro Ile Ser Ile Pro Ser Arg Asn
        675                 680                 685

Trp Ala Ala Phe Arg Gly Trp Ala Phe Thr Arg Leu Lys Thr Lys Glu
    690                 695                 700

Thr Pro Ser Leu Gly Ser Gly Tyr Asp Pro Tyr Tyr Thr Tyr Ser Gly
705                 710                 715                 720

Ser Ile Pro Tyr Leu Asp Gly Thr Phe Tyr Leu Asn His Thr Phe Lys
                725                 730                 735

Lys Val Ala Ile Thr Phe Asp Ser Ser Val Ser Trp Pro Gly Asn Asp
            740                 745                 750

Arg Leu Leu Thr Pro Asn Glu Phe Glu Ile Lys Arg Ser Val Asp Gly
        755                 760                 765

Glu Gly Tyr Asn Val Ala Gln Cys Asn Met Thr Lys Asp Trp Phe Leu
    770                 775                 780

Val Gln Met Leu Ala Asn Tyr Asn Ile Gly Tyr Gln Gly Phe Tyr Ile
785                 790                 795                 800

Pro Glu Ser Tyr Lys Asp Arg Met Tyr Ser Phe Phe Arg Asn Phe Gln
                805                 810                 815

Pro Met Ser Arg Gln Val Val Asp Asp Thr Lys Tyr Lys Asp Tyr Gln
            820                 825                 830

Gln Val Gly Ile Leu His Gln His Asn Asn Ser Gly Phe Val Gly Tyr
        835                 840                 845

Leu Ala Pro Thr Met Arg Glu Gly Gln Ala Tyr Pro Ala Asn Phe Pro
    850                 855                 860

Tyr Pro Leu Ile Gly Lys Thr Ala Val Asp Ser Ile Thr Gln Lys Lys
865                 870                 875                 880

Phe Leu Cys Asp Arg Thr Leu Trp Arg Ile Pro Phe Ser Ser Asn Phe
                885                 890                 895

Met Ser Met Gly Ala Leu Thr Asp Leu Gly Gln Asn Leu Leu Tyr Ala
            900                 905                 910

Asn Ser Ala His Ala Leu Asp Met Thr Phe Glu Val Asp Pro Met Asp
        915                 920                 925

Glu Pro Thr Leu Leu Tyr Val Leu Phe Glu Val Phe Asp Val Val Arg
    930                 935                 940

Val His Gln Pro His Arg Gly Val Ile Glu Thr Val Tyr Leu Arg Thr
945                 950                 955                 960

Pro Phe Ser Ala Gly Asn Ala Thr Thr
                965

<210>  51
<211>  977
<212>  PRT
<213>  Artificial Sequence

<220>
<223>  Synthetic Construct

<400>  51

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Asn Ala Asn Pro Asn Ala Asn
                165                 170                 175

Pro Asn Ala Asn Pro Asn Ala Asn Pro Asn Ala Asn Pro Asn Ala Asn
            180                 185                 190

Pro Asn Ala Asn Pro Asn Ala Asn Pro Asn Ala Asn Pro Asn Ala Asn
        195                 200                 205

Pro Lys Thr His Val Phe Gly Gln Ala Pro Tyr Ser Gly Ile Asn Ile
    210                 215                 220

Thr Lys Glu Gly Ile Gln Ile Gly Val Glu Gly Gln Thr Pro Lys Tyr
225                 230                 235                 240

Ala Asp Lys Thr Phe Gln Pro Glu Pro Gln Ile Gly Glu Ser Gln Trp
                245                 250                 255

Tyr Glu Thr Glu Ile Asn His Ala Ala Gly Arg Val Leu Lys Lys Thr
            260                 265                 270

Thr Pro Met Lys Pro Cys Tyr Gly Ser Tyr Ala Lys Pro Thr Asn Glu
        275                 280                 285

Asn Gly Gly Gln Gly Ile Leu Val Lys Gln Gln Asn Gly Lys Leu Glu
    290                 295                 300

Ser Gln Val Glu Met Gln Phe Phe Ser Thr Thr Glu Ala Ala Ala Gly
305                 310                 315                 320

Asn Gly Asp Asn Leu Thr Pro Lys Val Val Leu Tyr Ser Glu Asp Val
                325                 330                 335

Asp Ile Glu Thr Pro Asp Thr His Ile Ser Tyr Met Pro Thr Ile Lys
            340                 345                 350

Glu Gly Asn Ser Arg Glu Leu Met Gly Gln Gln Ser Met Pro Asn Arg
        355                 360                 365

Pro Asn Tyr Ile Ala Phe Arg Asp Asn Phe Ile Gly Leu Met Tyr Tyr
    370                 375                 380

Asn Ser Thr Gly Asn Met Gly Val Leu Ala Gly Gln Ala Ser Gln Leu
385                 390                 395                 400

Asn Ala Val Val Asp Leu Gln Asp Arg Asn Thr Glu Leu Ser Tyr Gln
                405                 410                 415

Leu Leu Leu Asp Ser Ile Gly Asp Arg Thr Arg Tyr Phe Ser Met Trp
            420                 425                 430

Asn Gln Leu Pro Asn Tyr Cys Phe Pro Leu Gly Gly Val Ile Asn Thr

435                 440                 445

Glu Thr Leu Thr Lys Val Lys Pro Lys Thr Gly Gln Glu Asn Gly Trp
    450                 455                 460

Glu Lys Asp Ala Thr Glu Phe Ser Asp Lys Asn Glu Ile Arg Val Gly
465                 470                 475                 480

Asn Asn Phe Ala Met Glu Ile Asn Leu Asn Ala Asn Leu Trp Arg Asn
                485                 490                 495

Phe Leu Tyr Ser Asn Ile Ala Leu Tyr Leu Pro Asp Lys Leu Lys Tyr
            500                 505                 510

Ser Pro Ser Asn Val Lys Ile Ser Asp Asn Pro Asn Thr Tyr Asp Tyr
        515                 520                 525

Met Asn Lys Arg Val Val Ala Pro Gly Leu Val Asp Cys Tyr Ile Asn
    530                 535                 540

Leu Gly Ala Arg Trp Ser Leu Asp Tyr Met Asp Asn Val Asn Pro Phe
545                 550                 555                 560

Asn His His Arg Asn Ala Gly Leu Arg Tyr Arg Ser Met Leu Leu Gly
                565                 570                 575

Asn Gly Arg Tyr Val Pro Phe His Ile Gln Val Pro Gln Lys Phe Phe
            580                 585                 590

Ala Ile Lys Asn Leu Leu Leu Leu Pro Gly Ser Tyr Thr Tyr Glu Trp
        595                 600                 605

Asn Phe Arg Lys Asp Val Asn Met Val Leu Gln Ser Ser Leu Gly Asn
    610                 615                 620

Asp Leu Arg Val Asp Gly Ala Ser Ile Lys Phe Asp Ser Ile Cys Leu
625                 630                 635                 640

Tyr Ala Thr Phe Phe Pro Met Ala His Asn Thr Ala Ser Thr Leu Glu
                645                 650                 655

Ala Met Leu Arg Asn Asp Thr Asn Asp Gln Ser Phe Asn Asp Tyr Leu
            660                 665                 670

Ser Ala Ala Asn Met Leu Tyr Pro Ile Pro Ala Asn Ala Thr Asn Val
        675                 680                 685

Pro Ile Ser Ile Pro Ser Arg Asn Trp Ala Ala Phe Arg Gly Trp Ala
    690                 695                 700

Phe Thr Arg Leu Lys Thr Lys Glu Thr Pro Ser Leu Gly Ser Gly Tyr
705                 710                 715                 720

Asp Pro Tyr Tyr Thr Tyr Ser Gly Ser Ile Pro Tyr Leu Asp Gly Thr
                725                 730                 735

Phe Tyr Leu Asn His Thr Phe Lys Lys Val Ala Ile Thr Phe Asp Ser
            740                 745                 750

Ser Val Ser Trp Pro Gly Asn Asp Arg Leu Leu Thr Pro Asn Glu Phe
        755                 760                 765

Glu Ile Lys Arg Ser Val Asp Gly Glu Gly Tyr Asn Val Ala Gln Cys
    770                 775                 780

Asn Met Thr Lys Asp Trp Phe Leu Val Gln Met Leu Ala Asn Tyr Asn
785                 790                 795                 800

Ile Gly Tyr Gln Gly Phe Tyr Ile Pro Glu Ser Tyr Lys Asp Arg Met
                805                 810                 815

Tyr Ser Phe Phe Arg Asn Phe Gln Pro Met Ser Arg Gln Val Val Asp
            820                 825                 830

Asp Thr Lys Tyr Lys Asp Tyr Gln Gln Val Gly Ile Leu His Gln His
        835                 840                 845

Asn Asn Ser Gly Phe Val Gly Tyr Leu Ala Pro Thr Met Arg Glu Gly
    850                 855                 860

Gln Ala Tyr Pro Ala Asn Phe Pro Tyr Pro Leu Ile Gly Lys Thr Ala
865                 870                 875                 880

Val Asp Ser Ile Thr Gln Lys Lys Phe Leu Cys Asp Arg Thr Leu Trp
                885                 890                 895

Arg Ile Pro Phe Ser Ser Asn Phe Met Ser Met Gly Ala Leu Thr Asp
            900                 905                 910

Leu Gly Gln Asn Leu Leu Tyr Ala Asn Ser Ala His Ala Leu Asp Met
        915                 920                 925

Thr Phe Glu Val Asp Pro Met Asp Glu Pro Thr Leu Leu Tyr Val Leu
    930                 935                 940

Phe Glu Val Phe Asp Val Val Arg Val His Gln Pro His Arg Gly Val
945                 950                 955                 960

Ile Glu Thr Val Tyr Leu Arg Thr Pro Phe Ser Ala Gly Asn Ala Thr
                965                 970                 975

Thr

<210>  52
<211>  985
<212>  PRT
<213>  Artificial Sequence

<220>
<223>  Synthetic Construct

<400>  52

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Asn Ala Asn Pro Asn Ala Asn
                165                 170                 175

Pro Asn Ala Asn Pro Asn Ala Asn Pro Asn Ala Asn Pro Asn Ala Asn
            180                 185                 190

Pro Asn Ala Asn Pro Asn Ala Asn Pro Asn Ala Asn Pro Asn Ala Asn
        195                 200                 205

Pro Asn Ala Asn Pro Asn Ala Asn Pro Lys Thr His Val Phe Gly Gln
    210                 215                 220

Ala Pro Tyr Ser Gly Ile Asn Ile Thr Lys Glu Gly Ile Gln Ile Gly
225                 230                 235                 240

Val Glu Gly Gln Thr Pro Lys Tyr Ala Asp Lys Thr Phe Gln Pro Glu
                245                 250                 255

Pro Gln Ile Gly Glu Ser Gln Trp Tyr Glu Thr Glu Ile Asn His Ala
            260                 265                 270

Ala Gly Arg Val Leu Lys Lys Thr Thr Pro Met Lys Pro Cys Tyr Gly
        275                 280                 285

Ser Tyr Ala Lys Pro Thr Asn Glu Asn Gly Gly Gln Gly Ile Leu Val
    290                 295                 300

Lys Gln Gln Asn Gly Lys Leu Glu Ser Gln Val Glu Met Gln Phe Phe
305                 310                 315                 320

Ser Thr Thr Glu Ala Ala Ala Gly Asn Gly Asp Asn Leu Thr Pro Lys
                325                 330                 335

Val Val Leu Tyr Ser Glu Asp Val Asp Ile Glu Thr Pro Asp Thr His
            340                 345                 350

Ile Ser Tyr Met Pro Thr Ile Lys Glu Gly Asn Ser Arg Glu Leu Met
        355                 360                 365

Gly Gln Gln Ser Met Pro Asn Arg Pro Asn Tyr Ile Ala Phe Arg Asp
    370                 375                 380

Asn Phe Ile Gly Leu Met Tyr Tyr Asn Ser Thr Gly Asn Met Gly Val
385                 390                 395                 400

Leu Ala Gly Gln Ala Ser Gln Leu Asn Ala Val Val Asp Leu Gln Asp
                405                 410                 415

Arg Asn Thr Glu Leu Ser Tyr Gln Leu Leu Leu Asp Ser Ile Gly Asp
        420                 425                 430

Arg Thr Arg Tyr Phe Ser Met Trp Asn Gln Leu Pro Asn Tyr Cys Phe
        435                 440                 445

Pro Leu Gly Gly Val Ile Asn Thr Glu Thr Leu Thr Lys Val Lys Pro
    450                 455                 460

Lys Thr Gly Gln Glu Asn Gly Trp Glu Lys Asp Ala Thr Glu Phe Ser
465                 470                 475                 480

Asp Lys Asn Glu Ile Arg Val Gly Asn Asn Phe Ala Met Glu Ile Asn
                485                 490                 495

Leu Asn Ala Asn Leu Trp Arg Asn Phe Leu Tyr Ser Asn Ile Ala Leu
            500                 505                 510

Tyr Leu Pro Asp Lys Leu Lys Tyr Ser Pro Ser Asn Val Lys Ile Ser
        515                 520                 525

Asp Asn Pro Asn Thr Tyr Asp Tyr Met Asn Lys Arg Val Val Ala Pro
    530                 535                 540

Gly Leu Val Asp Cys Tyr Ile Asn Leu Gly Ala Arg Trp Ser Leu Asp
545                 550                 555                 560

Tyr Met Asp Asn Val Asn Pro Phe Asn His His Arg Asn Ala Gly Leu
                565                 570                 575

Arg Tyr Arg Ser Met Leu Leu Gly Asn Gly Arg Tyr Val Pro Phe His
            580                 585                 590

Ile Gln Val Pro Gln Lys Phe Phe Ala Ile Lys Asn Leu Leu Leu Leu
        595                 600                 605

Pro Gly Ser Tyr Thr Tyr Glu Trp Asn Phe Arg Lys Asp Val Asn Met
    610                 615                 620

Val Leu Gln Ser Ser Leu Gly Asn Asp Leu Arg Val Asp Gly Ala Ser
625                 630                 635                 640

Ile Lys Phe Asp Ser Ile Cys Leu Tyr Ala Thr Phe Phe Pro Met Ala
                645                 650                 655

His Asn Thr Ala Ser Thr Leu Glu Ala Met Leu Arg Asn Asp Thr Asn
            660                 665                 670

Asp Gln Ser Phe Asn Asp Tyr Leu Ser Ala Ala Asn Met Leu Tyr Pro
        675                 680                 685

Ile Pro Ala Asn Ala Thr Asn Val Pro Ile Ser Ile Pro Ser Arg Asn
    690                 695                 700

Trp Ala Ala Phe Arg Gly Trp Ala Phe Thr Arg Leu Lys Thr Lys Glu
705                 710                 715                 720

Thr Pro Ser Leu Gly Ser Gly Tyr Asp Pro Tyr Tyr Thr Tyr Ser Gly
                725                 730                 735

Ser Ile Pro Tyr Leu Asp Gly Thr Phe Tyr Leu Asn His Thr Phe Lys
            740                 745                 750

Lys Val Ala Ile Thr Phe Asp Ser Ser Val Ser Trp Pro Gly Asn Asp
        755                 760                 765

Arg Leu Leu Thr Pro Asn Glu Phe Glu Ile Lys Arg Ser Val Asp Gly
    770                 775                 780

Glu Gly Tyr Asn Val Ala Gln Cys Asn Met Thr Lys Asp Trp Phe Leu
785                 790                 795                 800

Val Gln Met Leu Ala Asn Tyr Asn Ile Gly Tyr Gln Gly Phe Tyr Ile
                805                 810                 815

Pro Glu Ser Tyr Lys Asp Arg Met Tyr Ser Phe Phe Arg Asn Phe Gln
            820                 825                 830

Pro Met Ser Arg Gln Val Val Asp Asp Thr Lys Tyr Lys Asp Tyr Gln
        835                 840                 845

Gln Val Gly Ile Leu His Gln His Asn Asn Ser Gly Phe Val Gly Tyr
    850                 855                 860

Leu Ala Pro Thr Met Arg Glu Gly Gln Ala Tyr Pro Ala Asn Phe Pro
865                 870                 875                 880

Tyr Pro Leu Ile Gly Lys Thr Ala Val Asp Ser Ile Thr Gln Lys Lys
                885                 890                 895

Phe Leu Cys Asp Arg Thr Leu Trp Arg Ile Pro Phe Ser Ser Asn Phe
            900                 905                 910

Met Ser Met Gly Ala Leu Thr Asp Leu Gly Gln Asn Leu Leu Tyr Ala

915                 920                 925

Asn Ser Ala His Ala Leu Asp Met Thr Phe Glu Val Asp Pro Met Asp
    930                 935                 940

Glu Pro Thr Leu Leu Tyr Val Leu Phe Glu Val Phe Asp Val Val Arg
945                 950                 955                 960

Val His Gln Pro His Arg Gly Val Ile Glu Thr Val Tyr Leu Arg Thr
                965                 970                 975

Pro Phe Ser Ala Gly Asn Ala Thr Thr
            980                 985

<210>  53
<211>  993
<212>  PRT
<213>  Artificial Sequence

<220>
<223>  Synthetic Construct

<400>  53

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Asn Ala Asn Pro Asn Ala Asn
                165                 170                 175

Pro Asn Ala Asn Pro Asn Ala Asn Pro Asn Ala Asn Pro Asn Ala Asn
            180                 185                 190

Pro Asn Ala Asn Pro Asn Ala Asn Pro Asn Ala Asn Pro Asn Ala Asn
        195                 200                 205

Pro Asn Ala Asn Pro Asn Ala Asn Pro Asn Ala Asn Pro Asn Ala Asn
    210                 215                 220

Pro Lys Thr His Val Phe Gly Gln Ala Pro Tyr Ser Gly Ile Asn Ile
225                 230                 235                 240

Thr Lys Glu Gly Ile Gln Ile Gly Val Glu Gly Gln Thr Pro Lys Tyr
                245                 250                 255

Ala Asp Lys Thr Phe Gln Pro Glu Pro Gln Ile Gly Glu Ser Gln Trp
            260                 265                 270

Tyr Glu Thr Glu Ile Asn His Ala Ala Gly Arg Val Leu Lys Lys Thr
        275                 280                 285

Thr Pro Met Lys Pro Cys Tyr Gly Ser Tyr Ala Lys Pro Thr Asn Glu
    290                 295                 300

Asn Gly Gly Gln Gly Ile Leu Val Lys Gln Gln Asn Gly Lys Leu Glu
305                 310                 315                 320

Ser Gln Val Glu Met Gln Phe Phe Ser Thr Thr Glu Ala Ala Ala Gly
                325                 330                 335

Asn Gly Asp Asn Leu Thr Pro Lys Val Val Leu Tyr Ser Glu Asp Val
            340                 345                 350

Asp Ile Glu Thr Pro Asp Thr His Ile Ser Tyr Met Pro Thr Ile Lys
        355                 360                 365

Glu Gly Asn Ser Arg Glu Leu Met Gly Gln Gln Ser Met Pro Asn Arg
    370                 375                 380

Pro Asn Tyr Ile Ala Phe Arg Asp Asn Phe Ile Gly Leu Met Tyr Tyr
385                 390                 395                 400

Asn Ser Thr Gly Asn Met Gly Val Leu Ala Gly Gln Ala Ser Gln Leu
                405                 410                 415

Asn Ala Val Val Asp Leu Gln Asp Arg Asn Thr Glu Leu Ser Tyr Gln
            420                 425                 430

Leu Leu Leu Asp Ser Ile Gly Asp Arg Thr Arg Tyr Phe Ser Met Trp
        435                 440                 445

Asn Gln Leu Pro Asn Tyr Cys Phe Pro Leu Gly Gly Val Ile Asn Thr
    450                 455                 460

Glu Thr Leu Thr Lys Val Lys Pro Lys Thr Gly Gln Glu Asn Gly Trp
465                 470                 475                 480

Glu Lys Asp Ala Thr Glu Phe Ser Asp Lys Asn Glu Ile Arg Val Gly
                485                 490                 495

Asn Asn Phe Ala Met Glu Ile Asn Leu Asn Ala Asn Leu Trp Arg Asn
            500                 505                 510

Phe Leu Tyr Ser Asn Ile Ala Leu Tyr Leu Pro Asp Lys Leu Lys Tyr
        515                 520                 525

Ser Pro Ser Asn Val Lys Ile Ser Asp Asn Pro Asn Thr Tyr Asp Tyr
    530                 535                 540

Met Asn Lys Arg Val Val Ala Pro Gly Leu Val Asp Cys Tyr Ile Asn
545                 550                 555                 560

Leu Gly Ala Arg Trp Ser Leu Asp Tyr Met Asp Asn Val Asn Pro Phe
                565                 570                 575

Asn His His Arg Asn Ala Gly Leu Arg Tyr Arg Ser Met Leu Leu Gly
            580                 585                 590

Asn Gly Arg Tyr Val Pro Phe His Ile Gln Val Pro Gln Lys Phe Phe
        595                 600                 605

Ala Ile Lys Asn Leu Leu Leu Leu Pro Gly Ser Tyr Thr Tyr Glu Trp
    610                 615                 620

Asn Phe Arg Lys Asp Val Asn Met Val Leu Gln Ser Ser Leu Gly Asn
625                 630                 635                 640

Asp Leu Arg Val Asp Gly Ala Ser Ile Lys Phe Asp Ser Ile Cys Leu
                645                 650                 655

Tyr Ala Thr Phe Phe Pro Met Ala His Asn Thr Ala Ser Thr Leu Glu
            660                 665                 670

Ala Met Leu Arg Asn Asp Thr Asn Asp Gln Ser Phe Asn Asp Tyr Leu
        675                 680                 685

Ser Ala Ala Asn Met Leu Tyr Pro Ile Pro Ala Asn Ala Thr Asn Val
    690                 695                 700

Pro Ile Ser Ile Pro Ser Arg Asn Trp Ala Ala Phe Arg Gly Trp Ala
705                 710                 715                 720

Phe Thr Arg Leu Lys Thr Lys Glu Thr Pro Ser Leu Gly Ser Gly Tyr
                725                 730                 735

Asp Pro Tyr Tyr Thr Tyr Ser Gly Ser Ile Pro Tyr Leu Asp Gly Thr
            740                 745                 750

Phe Tyr Leu Asn His Thr Phe Lys Lys Val Ala Ile Thr Phe Asp Ser
        755                 760                 765

Ser Val Ser Trp Pro Gly Asn Asp Arg Leu Leu Thr Pro Asn Glu Phe
    770                 775                 780

Glu Ile Lys Arg Ser Val Asp Gly Glu Gly Tyr Asn Val Ala Gln Cys
785                 790                 795                 800

Asn Met Thr Lys Asp Trp Phe Leu Val Gln Met Leu Ala Asn Tyr Asn
                805                 810                 815

Ile Gly Tyr Gln Gly Phe Tyr Ile Pro Glu Ser Tyr Lys Asp Arg Met
            820                 825                 830

Tyr Ser Phe Phe Arg Asn Phe Gln Pro Met Ser Arg Gln Val Val Asp
        835                 840                 845

Asp Thr Lys Tyr Lys Asp Tyr Gln Gln Val Gly Ile Leu His Gln His
    850                 855                 860

Asn Asn Ser Gly Phe Val Gly Tyr Leu Ala Pro Thr Met Arg Glu Gly
865                 870                 875                 880

Gln Ala Tyr Pro Ala Asn Phe Pro Tyr Pro Leu Ile Gly Lys Thr Ala

885                 890                 895

Val Asp Ser Ile Thr Gln Lys Lys Phe Leu Cys Asp Arg Thr Leu Trp
            900                 905                 910

Arg Ile Pro Phe Ser Ser Asn Phe Met Ser Met Gly Ala Leu Thr Asp
        915                 920                 925

Leu Gly Gln Asn Leu Leu Tyr Ala Asn Ser Ala His Ala Leu Asp Met
    930                 935                 940

Thr Phe Glu Val Asp Pro Met Asp Glu Pro Thr Leu Leu Tyr Val Leu
945                 950                 955                 960

Phe Glu Val Phe Asp Val Val Arg Val His Gln Pro His Arg Gly Val
                965                 970                 975

Ile Glu Thr Val Tyr Leu Arg Thr Pro Phe Ser Ala Gly Asn Ala Thr
            980                 985                 990

Thr

<210>  54
<211>  1017
<212>  PRT
<213>  Artificial Sequence

<220>
<223>  Synthetic Construct

<400>  54

Met Ala Thr Pro Ser Met Met Pro Gln Trp Ser Tyr Met His Ile Ser
1               5                   10                  15

Gly Gln Asp Ala Ser Glu Tyr Leu Ser Pro Gly Leu Val Gln Phe Ala
            20                  25                  30

Arg Ala Thr Glu Thr Tyr Phe Ser Leu Asn Asn Lys Phe Arg Asn Pro
        35                  40                  45

Thr Val Ala Pro Thr His Asp Val Thr Thr Asp Arg Ser Gln Arg Leu
    50                  55                  60

Thr Leu Arg Phe Ile Pro Val Asp Arg Glu Asp Thr Ala Tyr Ser Tyr
65                  70                  75                  80

Lys Ala Arg Phe Thr Leu Ala Val Gly Asp Asn Arg Val Leu Asp Met
                85                  90                  95

Ala Ser Thr Tyr Phe Asp Ile Arg Gly Val Leu Asp Arg Gly Pro Thr
            100                 105                 110

Phe Lys Pro Tyr Ser Gly Thr Ala Tyr Asn Ala Leu Ala Pro Lys Gly
        115                 120                 125

Ala Pro Asn Pro Cys Glu Trp Asp Glu Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Asn Ala Asn Pro Asn Ala Asn
                165                 170                 175

Pro Asn Ala Asn Pro Asn Ala Asn Pro Asn Ala Asn Pro Asn Ala Asn
            180                 185                 190

Pro Asn Ala Asn Pro Asn Ala Asn Pro Asn Ala Asn Pro Asn Ala Asn
        195                 200                 205

Pro Asn Ala Asn Pro Asn Ala Asn Pro Asn Ala Asn Pro Asn Ala Asn
    210                 215                 220

Pro Asn Ala Asn Pro Asn Ala Asn Pro Asn Ala Asn Pro Asn Ala Asn
225                 230                 235                 240

Pro Asn Ala Asn Pro Asn Ala Asn Pro Lys Thr His Val Phe Gly Gln
                245                 250                 255

Ala Pro Tyr Ser Gly Ile Asn Ile Thr Lys Glu Gly Ile Gln Ile Gly
            260                 265                 270

Val Glu Gly Gln Thr Pro Lys Tyr Ala Asp Lys Thr Phe Gln Pro Glu
        275                 280                 285

Pro Gln Ile Gly Glu Ser Gln Trp Tyr Glu Thr Glu Ile Asn His Ala
    290                 295                 300

Ala Gly Arg Val Leu Lys Lys Thr Thr Pro Met Lys Pro Cys Tyr Gly
305                 310                 315                 320

Ser Tyr Ala Lys Pro Thr Asn Glu Asn Gly Gly Gln Gly Ile Leu Val
                325                 330                 335

Lys Gln Gln Asn Gly Lys Leu Glu Ser Gln Val Glu Met Gln Phe Phe
            340                 345                 350

Ser Thr Thr Glu Ala Ala Ala Gly Asn Gly Asp Asn Leu Thr Pro Lys
        355                 360                 365

Val Val Leu Tyr Ser Glu Asp Val Asp Ile Glu Thr Pro Asp Thr His
    370                 375                 380

Ile Ser Tyr Met Pro Thr Ile Lys Glu Gly Asn Ser Arg Glu Leu Met
385                 390                 395                 400

Gly Gln Gln Ser Met Pro Asn Arg Pro Asn Tyr Ile Ala Phe Arg Asp
                405                 410                 415

Asn Phe Ile Gly Leu Met Tyr Tyr Asn Ser Thr Gly Asn Met Gly Val
            420                 425                 430

Leu Ala Gly Gln Ala Ser Gln Leu Asn Ala Val Val Asp Leu Gln Asp
        435                 440                 445

Arg Asn Thr Glu Leu Ser Tyr Gln Leu Leu Leu Asp Ser Ile Gly Asp
    450                 455                 460

Arg Thr Arg Tyr Phe Ser Met Trp Asn Gln Leu Pro Asn Tyr Cys Phe
465                 470                 475                 480

Pro Leu Gly Gly Val Ile Asn Thr Glu Thr Leu Thr Lys Val Lys Pro
                485                 490                 495

Lys Thr Gly Gln Glu Asn Gly Trp Glu Lys Asp Ala Thr Glu Phe Ser
            500                 505                 510

Asp Lys Asn Glu Ile Arg Val Gly Asn Asn Phe Ala Met Glu Ile Asn
        515                 520                 525

Leu Asn Ala Asn Leu Trp Arg Asn Phe Leu Tyr Ser Asn Ile Ala Leu
    530                 535                 540

Tyr Leu Pro Asp Lys Leu Lys Tyr Ser Pro Ser Asn Val Lys Ile Ser
545                 550                 555                 560

Asp Asn Pro Asn Thr Tyr Asp Tyr Met Asn Lys Arg Val Val Ala Pro
                565                 570                 575

Gly Leu Val Asp Cys Tyr Ile Asn Leu Gly Ala Arg Trp Ser Leu Asp
            580                 585                 590

Tyr Met Asp Asn Val Asn Pro Phe Asn His His Arg Asn Ala Gly Leu

595                 600                 605

Arg Tyr Arg Ser Met Leu Leu Gly Asn Gly Arg Tyr Val Pro Phe His
    610                 615                 620

Ile Gln Val Pro Gln Lys Phe Phe Ala Ile Lys Asn Leu Leu Leu Leu
625                 630                 635                 640

Pro Gly Ser Tyr Thr Tyr Glu Trp Asn Phe Arg Lys Asp Val Asn Met
                645                 650                 655

Val Leu Gln Ser Ser Leu Gly Asn Asp Leu Arg Val Asp Gly Ala Ser
            660                 665                 670

Ile Lys Phe Asp Ser Ile Cys Leu Tyr Ala Thr Phe Phe Pro Met Ala
        675                 680                 685

His Asn Thr Ala Ser Thr Leu Glu Ala Met Leu Arg Asn Asp Thr Asn
    690                 695                 700

Asp Gln Ser Phe Asn Asp Tyr Leu Ser Ala Ala Asn Met Leu Tyr Pro
705                 710                 715                 720

Ile Pro Ala Asn Ala Thr Asn Val Pro Ile Ser Ile Pro Ser Arg Asn
                725                 730                 735

Trp Ala Ala Phe Arg Gly Trp Ala Phe Thr Arg Leu Lys Thr Lys Glu
            740                 745                 750

Thr Pro Ser Leu Gly Ser Gly Tyr Asp Pro Tyr Tyr Thr Tyr Ser Gly
        755                 760                 765

Ser Ile Pro Tyr Leu Asp Gly Thr Phe Tyr Leu Asn His Thr Phe Lys
    770                 775                 780

Lys Val Ala Ile Thr Phe Asp Ser Ser Val Ser Trp Pro Gly Asn Asp
785                 790                 795                 800

Arg Leu Leu Thr Pro Asn Glu Phe Glu Ile Lys Arg Ser Val Asp Gly
                805                 810                 815

Glu Gly Tyr Asn Val Ala Gln Cys Asn Met Thr Lys Asp Trp Phe Leu
            820                 825                 830

Val Gln Met Leu Ala Asn Tyr Asn Ile Gly Tyr Gln Gly Phe Tyr Ile
        835                 840                 845

Pro Glu Ser Tyr Lys Asp Arg Met Tyr Ser Phe Phe Arg Asn Phe Gln
    850                 855                 860

Pro Met Ser Arg Gln Val Val Asp Asp Thr Lys Tyr Lys Asp Tyr Gln
865                 870                 875                 880

Gln Val Gly Ile Leu His Gln His Asn Asn Ser Gly Phe Val Gly Tyr
                885                 890                 895

Leu Ala Pro Thr Met Arg Glu Gly Gln Ala Tyr Pro Ala Asn Phe Pro
            900                 905                 910

Tyr Pro Leu Ile Gly Lys Thr Ala Val Asp Ser Ile Thr Gln Lys Lys
        915                 920                 925

Phe Leu Cys Asp Arg Thr Leu Trp Arg Ile Pro Phe Ser Ser Asn Phe
    930                 935                 940

Met Ser Met Gly Ala Leu Thr Asp Leu Gly Gln Asn Leu Leu Tyr Ala
945                 950                 955                 960

Asn Ser Ala His Ala Leu Asp Met Thr Phe Glu Val Asp Pro Met Asp
                965                 970                 975

Glu Pro Thr Leu Leu Tyr Val Leu Phe Glu Val Phe Asp Val Val Arg
            980                 985                 990

Val His Gln Pro His Arg Gly Val  Ile Glu Thr Val Tyr  Leu Arg Thr
        995                 1000                1005

Pro Phe  Ser Ala Gly Asn Ala  Thr Thr
    1010                1015

<210>  55
<211>  597
<212>  PRT
<213>  Artificial Sequence

<220>
<223>  Synthetic Construct

<400>  55

Met Lys Arg Ala Arg Pro Ser Glu Asp Thr Phe Asn Pro Val Tyr Pro
1               5                   10                  15

Tyr Asp Thr Glu Thr Gly Pro Pro Thr Val Pro Phe Leu Thr Pro Pro
            20                  25                  30

Phe Val Ser Pro Asn Gly Phe Gln Glu Ser Pro Pro Gly Val Leu Ser
        35                  40                  45

Leu Arg Leu Ser Glu Pro Leu Val Thr Ser Asn Gly Met Leu Ala Leu
    50                  55                  60

Lys Met Gly Asn Gly Leu Ser Leu Asp Glu Ala Gly Asn Leu Thr Ser
65                  70                  75                  80

Gln Asn Val Thr Thr Val Ser Pro Pro Leu Lys Lys Thr Lys Ser Asn
                85                  90                  95

Ile Asn Leu Glu Ile Ser Ala Pro Leu Thr Val Thr Ser Glu Ala Leu
            100                 105                 110

Thr Val Ala Ala Ala Ala Pro Leu Met Val Ala Gly Asn Thr Leu Thr
        115                 120                 125

Met Gln Ser Gln Ala Pro Leu Thr Val His Asp Ser Lys Leu Ser Ile
    130                 135                 140

Ala Thr Gln Gly Pro Leu Thr Val Ser Glu Gly Lys Leu Ala Leu Gln
145                 150                 155                 160

Thr Ser Gly Pro Leu Thr Thr Thr Asp Ser Ser Thr Leu Thr Ile Thr
                165                 170                 175

Ala Ser Pro Pro Leu Thr Thr Ala Thr Gly Ser Leu Gly Ile Asp Leu
            180                 185                 190

Lys Glu Pro Ile Tyr Thr Gln Asn Gly Lys Leu Gly Leu Lys Tyr Gly
        195                 200                 205

Ala Pro Leu His Val Thr Asp Asp Leu Asn Thr Leu Thr Val Ala Thr
    210                 215                 220

Gly Pro Gly Val Thr Ile Asn Asn Thr Ser Leu Gln Thr Lys Val Thr
225                 230                 235                 240

Gly Ala Leu Gly Phe Asp Ser Gln Gly Asn Met Gln Leu Asn Val Ala
                245                 250                 255

Gly Gly Leu Arg Ile Asp Ser Gln Asn Arg Arg Leu Ile Leu Asp Val
            260                 265                 270

Ser Tyr Pro Phe Asp Ala Gln Asn Gln Leu Asn Leu Arg Leu Gly Gln
        275                 280                 285

Gly Pro Leu Phe Ile Asn Ser Ala His Asn Leu Asp Ile Asn Tyr Asn
    290                 295                 300

Lys Gly Leu Tyr Leu Phe Thr Ala Ser Asn Asn Ser Lys Lys Leu Glu
305                 310                 315                 320

Val Asn Leu Ser Thr Ala Lys Gly Leu Met Phe Asp Ala Thr Ala Ile
                325                 330                 335

Ala Ile Asn Ala Gly Asp Gly Leu Glu Phe Gly Ser Pro Asn Ala Pro
            340                 345                 350

Asn Thr Asn Pro Leu Lys Thr Lys Ile Gly His Gly Leu Glu Phe Asp
        355                 360                 365

Ser Asn Lys Ala Met Val Pro Lys Leu Gly Thr Gly Leu Ser Phe Asp
    370                 375                 380

Ser Thr Gly Ala Ile Thr Val Gly Asn Lys Asn Asn Asp Lys Leu Thr
385                 390                 395                 400

Leu Trp Thr Thr Pro Ala Pro Ser Pro Asn Cys Arg Leu Asn Ala Glu
                405                 410                 415

Lys Asp Ala Lys Leu Thr Leu Val Leu Thr Lys Cys Gly Ser Gln Ile
            420                 425                 430

Leu Ala Thr Val Ser Val Leu Ala Val Lys Gly Ser Leu Ala Pro Ile
        435                 440                 445

Ser Gly Thr Val Gln Ser Ala His Leu Ile Ile Arg Phe Asp Glu Asn
    450                 455                 460

Gly Val Leu Leu Asn Asn Ser Phe Leu Asp Pro Glu Tyr Trp Asn Phe
465                 470                 475                 480

Arg Asn Gly Asp Leu Thr Glu Gly Thr Ala Tyr Thr Asn Ala Val Gly
                485                 490                 495

Phe Met Pro Asn Leu Ser Ala Tyr Pro Lys Ser His Gly Lys Thr Ala
            500                 505                 510

Lys Ser Asn Ile Val Ser Gln Val Tyr Leu Asn Gly Asp Lys Thr Lys
        515                 520                 525

Pro Val Thr Leu Thr Ile Thr Leu Asn Gly Thr Gln Glu Thr Gly Asn

530                 535                 540

Ala Asn Pro Asn Ala Asn Pro Asn Ala Asn Pro Asn Ala Asn Pro Asp
545                 550                 555                 560

Thr Thr Pro Ser Ala Tyr Ser Met Ser Phe Ser Trp Asp Trp Ser Gly
                565                 570                 575

His Asn Tyr Ile Asn Glu Ile Phe Ala Thr Ser Ser Tyr Thr Phe Ser
            580                 585                 590

Tyr Ile Ala Gln Glu
        595

<210>  56
<211>  218
<212>  PRT
<213>  Artificial Sequence

<220>
<223>  Synthetic Construct

<400>  56

Met Ser Ile Leu Ile Ser Pro Ser Asn Asn Thr Gly Trp Gly Leu Arg
1               5                   10                  15

Phe Pro Ser Lys Met Phe Gly Gly Ala Lys Lys Arg Ser Asp Gln His
            20                  25                  30

Pro Val Arg Val Arg Gly His Tyr Arg Ala Pro Trp Gly Ala His Lys
        35                  40                  45

Arg Gly Arg Thr Gly Arg Thr Thr Val Asp Asp Ala Ile Asp Ala Val
    50                  55                  60

Val Glu Glu Ala Arg Asn Tyr Thr Pro Thr Pro Pro Pro Val Ser Thr
65                  70                  75                  80

Val Asp Ala Ala Ile Gln Thr Val Val Arg Gly Ala Arg Arg Tyr Ala
                85                  90                  95

Lys Met Lys Arg Arg Arg Arg Arg Val Ala Arg Arg His Arg Arg Arg
            100                 105                 110

Pro Gly Thr Ala Ala Gln Arg Ala Ala Ala Ala Leu Leu Asn Arg Ala
        115                 120                 125

Arg Arg Thr Gly Arg Arg Ala Ala Met Arg Ala Ala Arg Arg Leu Ala

130                 135                 140

Ala Gly Ile Val Thr Val Pro Pro Arg Ser Arg Arg Arg Ala Ala Ala
145                 150                 155                 160

Ala Ala Ala Ala Ala Ile Ser Ala Met Thr Gln Gly Arg Arg Gly Asn
                165                 170                 175

Val Tyr Trp Val Arg Asp Ser Val Ser Gly Leu Arg Val Pro Val Arg
            180                 185                 190

Thr Arg Pro Pro Arg Asn Glu Tyr Leu Asn Lys Ile Gln Asn Ser Leu
        195                 200                 205

Ser Thr Glu Trp Ser Pro Cys Ser Val Thr
    210                 215

<210>  57
<211>  246
<212>  PRT
<213>  Artificial Sequence

<220>
<223>  Synthetic Construct

<400>  57

Met Ser Ile Leu Ile Ser Pro Ser Asn Asn Thr Gly Trp Gly Leu Arg
1               5                   10                  15

Phe Pro Ser Lys Met Phe Gly Gly Ala Lys Lys Arg Ser Asp Gln His
            20                  25                  30

Pro Val Arg Val Arg Gly His Tyr Arg Ala Pro Trp Gly Ala His Lys
        35                  40                  45

Arg Gly Arg Thr Gly Arg Thr Thr Val Asp Asp Ala Ile Asp Ala Val
    50                  55                  60

Val Glu Glu Ala Arg Asn Tyr Thr Pro Thr Pro Pro Pro Val Ser Thr
65                  70                  75                  80

Val Asp Ala Ala Ile Gln Thr Val Val Arg Gly Ala Glu Tyr Leu Asn
                85                  90                  95

Lys Ile Gln Asn Ser Leu Ser Thr Glu Trp Ser Pro Cys Ser Val Thr
            100                 105                 110

Arg Arg Tyr Ala Lys Met Lys Arg Arg Arg Arg Arg Val Ala Arg Arg

115                 120                 125

His Arg Arg Arg Pro Gly Thr Ala Ala Gln Arg Ala Ala Ala Ala Leu
    130                 135                 140

Leu Asn Arg Ala Arg Arg Thr Gly Arg Arg Ala Ala Met Arg Ala Ala
145                 150                 155                 160

Arg Arg Leu Ala Ala Gly Ile Val Thr Val Pro Pro Arg Ser Arg Arg
                165                 170                 175

Arg Ala Ala Ala Ala Ala Ala Ala Ala Ile Ser Ala Met Thr Gln Gly
            180                 185                 190

Arg Arg Gly Asn Val Tyr Trp Val Arg Asp Ser Val Ser Gly Leu Arg
        195                 200                 205

Val Pro Val Arg Thr Arg Pro Pro Arg Asn Val Arg Thr Arg Pro Pro
    210                 215                 220

Arg Asn Tyr Asn Arg Asn Ile Val Asn Arg Leu Leu Gly Asp Ala Leu
225                 230                 235                 240

Thr Arg Pro Pro Arg Asn
                245

<210>  58
<211>  226
<212>  PRT
<213>  Artificial Sequence

<220>
<223>  Synthetic Construct

<400>  58

Met Ser Ile Leu Ile Ser Pro Ser Asn Asn Thr Gly Trp Gly Leu Arg
1               5                   10                  15

Phe Pro Ser Lys Met Phe Gly Gly Ala Lys Lys Arg Ser Asp Gln His
            20                  25                  30

Pro Val Arg Val Arg Gly His Tyr Arg Ala Pro Trp Gly Ala His Lys
        35                  40                  45

Arg Gly Arg Thr Gly Arg Thr Thr Val Asp Asp Ala Ile Asp Ala Val
    50                  55                  60

Val Glu Glu Ala Arg Asn Tyr Thr Pro Thr Pro Pro Pro Val Ser Thr
65                  70                  75                  80

Val Asp Ala Ala Ile Gln Thr Val Val Arg Gly Ala Arg Arg Tyr Ala
                85                  90                  95

Lys Met Lys Arg Arg Arg Arg Arg Val Ala Arg Arg His Arg Arg Arg
            100                 105                 110

Pro Gly Thr Ala Ala Gln Arg Ala Ala Ala Ala Leu Leu Asn Arg Ala
        115                 120                 125

Arg Arg Thr Gly Arg Arg Ala Ala Met Arg Ala Ala Glu Tyr Leu Asn
    130                 135                 140

Lys Ile Gln Asn Ser Leu Ser Thr Glu Trp Ser Pro Cys Ser Val Thr
145                 150                 155                 160

Arg Arg Leu Ala Ala Gly Ile Val Thr Val Pro Pro Arg Ser Arg Arg
                165                 170                 175

Arg Ala Ala Ala Ala Ala Ala Ala Ala Ile Ser Ala Met Thr Gln Gly
            180                 185                 190

Arg Arg Gly Asn Val Tyr Trp Val Arg Asp Ser Val Ser Gly Leu Arg
        195                 200                 205

Val Pro Val Arg Thr Arg Pro Pro Arg Asn Val Arg Thr Arg Pro Pro
    210                 215                 220

Arg Asn
225

<210>  59
<211>  6
<212>  PRT
<213>  Plasmodium yoelii

<400>  59

Gln Gly Pro Gly Ala Pro
1               5

<210>  60
<211>  4
<212>  PRT
<213>  Plasmodium falciparum

<400>  60

Asn Ala Asn Pro

1



<210>  61
<211>  21
<212>  PRT
<213>  Plasmodium yoelii

<400>  61

Tyr Asn Arg Asn Ile Val Asn Arg Leu Leu Gly Asp Ala Leu Asn Gly
1               5                   10                  15


Lys Pro Glu Glu Lys
            20


<210>  62
<211>  20
<212>  PRT
<213>  Plasmodium falciparum

<400>  62

Glu Tyr Leu Asn Lys Ile Gln Asn Ser Leu Ser Thr Glu Trp Ser Pro
1               5                   10                  15


Cys Ser Val Thr
            20


<210>  63
<211>  9
<212>  PRT
<213>  Plasmodium vivax

<400>  63

Ala Asn Gly Ala Gly Asn Gln Pro Gly
1               5


<210>  64
<211>  4
<212>  PRT
<213>  Plasmodium malariae

<400>  64

Asn Ala Ala Gly
1


<210>  65
<211>  4
<212>  PRT
<213>  Plasmodium yoelii

<400>  65

Gln Gln Pro Pro

1

<210> 66
<211> 9
<212> PRT
<213> Plasmodium yoelii

<400> 66

Ser Tyr Val Pro Ser Ala Glu Gln Ile
1               5

## Description

### PRIORITY CLAIM

This application claims priority to and is a continuation-in-part of International Application No. PCT/US09/054212, filed on August 18, 2009, which is incorporated by reference in its entirety, as if fully set forth herein.

### STATEMENT REGARDING FEDERALLY SPONSORED RESEARCH

This invention was made with government support under Grant No. 1R01AI081510-01A1 awarded by the National Institute of Allergy and Infectious Diseases (NIAID), an institute that is part of the National Institutes of Health. The government has certain rights in the invention.

### FIELD OF THE INVENTION

The invention relates to the field of medicine and biotechnology. More particularly, the invention relates to the use of capsid-modified adenoviral vectors to induce a potent immune response to a malaria parasite antigen such as *Plasmodium* circumsporozoite protein, which are suitable for vaccines against malaria.

### BACKGROUND

Malaria is a severe disease that ranks among the most prevalent infections in tropical areas throughout the world. Approximately 300-500 million people become infected yearly, with relatively high rates of morbidity and mortality. Severe morbidity and mortality occur particularly in young children and in adults migrating to a malaria endemic area without having undergone prior malaria exposure. The World Health Organization (WHO) estimates that 2 - 3 million children die of malaria in Africa alone, every year. The widespread occurrence and the increasing incidence of malaria in many countries, caused by drug-resistant parasites (*Plasmodium falciparum,* recently also *Plasmodium vivax*) and insecticide-resistant vectors (Anopheles mosquitoes), underscore the need for developing new methods for the control of this disease (Nussenzweig and Long 1994).

Malaria parasites have a complicated life cycle consisting of pre-erythrocytic, erythrocytic and sexual parasitic forms, representing a potential target for the development of a malaria vaccine. The pre-erythrocytic and erythrocytic forms are found in the host, while the sexual forms occur in the vector. Immunization with live-attenuated irradiated sporozoites (IrSp) has been shown to induce sterile protection (i.e., complete resistance against parasite challenge) in mice (Nussenzweig et al. 1967), non-human primates (Gwadz et al. 1979) and human (Clyde et al. 1973, Edelman et al. 1993). Protection conferred by IrSp is mediated by sporozoite neutralization by both humoral (B cell) and cellular (T cell) immune responses (Tsuji et al. 2001). Although an IrSp vaccination is an attractive solution, the only way to obtain sporozoites is by dissecting mosquito salivary glands, and there is currently no known technology to grow large numbers of sporozoites *in vitro.* Therefore, an alternate vaccine vector that can elicit an equally strong protective immunity against malaria is needed.

One promising target for such a vaccine vector is the circumsporozoite (CS) protein, which is expressed on the surface of the sporozoite. Effective neutralizing antibodies are directed against the immunodominant, species specific, repeat domains of the circumsporozoite (CS) protein. In *Plasmodium falciparum* (human malaria parasite), the repeats (NANP)ₙ are conserved among isolates from all areas of the world. This central repeat contains multiple repeat of B cell epitopes, and, therefore, the CS protein can induce a strong humoral immune response by triggering B cells (Tsuji et al. 2001). At the C-terminal region of the CS protein, there are several T cell epitopes, which can induce a significant cellular immune response (Tsuji et al. 2001). The humoral (antibody) response can eliminate parasites by interacting and neutralizing the infectivity of sporozoites (extra-cellular parasite) prior to entering hepatocyte, whereas the cellular (T cell) response can attack EEF (an intra-cellular parasite) by secreting interferon-gamma. These immune responses prevent the EEFs from maturing and dividing rapidly to form thousands of merozoites that reenter the blood and infect erythrocytes causing the disease we recognize as malaria.

One CS-based malarial vaccine that is currently undergoing human trials is GlaxoSmithKline's RTS, S, fusion protein of the Hepatitis B surface antigen and a portion of *Plasmodium falciparum* circumsporozoite protein (PfCSP) in a form of virus-like particle (International Patent Application No. PCT/EP1992/002591 to SmithKline Beecham Biologicals S.A., filed November 11, 1992), has been shown to decrease malaria infection in clinical trials (Alonso et al. 2004, Alonso et al. 2005, Bejon et al. 2008). RTS, S induces an anti-PfCSP humoral immune response, but a relatively weak PfCSP-specific cellular (CD8+) response (Kester et al. 2008), which might be the reason for the relatively weak protection by RTS, S. In contrast, adenovirus-based malaria vaccines can induce a protective cellular immune response (International Patent Application No. PCT/EP2003/051019, filed December 16, 2003, Rodrigues et al. 1997). However, there are currently two obstacles that limit the use of an adenovirus-based platform as a malaria vaccine: (1) lack of a capability of inducing a potent humoral response against a transgene product, and (2) pre-existing immunity to adenovirus, especially adenovirus serotype 5, which hampers the immunogenicity of adenovirus-based vaccine.

One approach that has recently been taken in an attempt to augment adenovirus-induced humoral response is to insert a B cell antigenic epitope (e.g., a bacterial or viral epitope) in adenovirus capsid proteins such as Hexon, Fiber, Penton and pIX (Worgall et al. 2005, McConnell et al. 2006, Krause et al. 2006, Worgall et al. 2007).

In addition, to circumvent pre-existing immunity to adenovirus serotype 5 (Ad5), other adenovirus serotypes with lower seroprevalence, such as adenovirus serotype 11, 35, 26, 48, 49 and 50, have been evaluated as a vaccine platform and shown to induce immune response to a transgene in spite of the presence of anti-Ad5 immunity (International Patent Application No. PCT/EP2005/055183 to Crucell Holland B.V., filed October 12, 2005, Abbink et al. 2007). Substitution of Ad5 Hexon, which is the target capsid protein of neutralizing antibody, with that of other serotypes has also been constructed in order to escape pre-existing anti-Ad5 immunity (Wu et al. 2002, Roberts et al. 2006).

There is, however, no improved adenoviral vector reported to have overcome the two obstacles at the same time in applying an adenoviral vector to a malaria vaccine mentioned above. Given that seroprevalence to Ad5 is high in malaria endemic areas (Ophorst et al. 2006.), there is a need for an adenovirus-based malaria vaccine that induces both protective humoral and cellular immune responses even in the presence of pre-exiting immunity to adenovirus.

### SUMMARY

The present disclosure relates to various adenovirus protein modifications to augment immune response to a transgene of a recombinant adenoviral vaccine and to circumvent pre-existing anti-adenovirus immunity.

More specifically, one embodiment is directed to a recombinant adenovirus derived from a recombinant adenovirus plasmid vector, wherein the recombinant adenovirus plasmid vector comprises a nucleotide sequence encoding (i) a Plasmodium circumsporozoite protein, or antigenic portion thereof, operably linked to a heterologous promoter: and (ii) a modified capsid or core protein, wherein an immunogenic epitope of Plasmodium circumsporozoite has been inserted into or replaces at least part of a capsid or core protein.

In some embodiments, the Plasmodium circumsporozoite protein further comprises a *Plasmodium falciparum* or *Plasmodium yoelii* circumsporozoite protein. The circumsporozoite protein may further comprise a codon-optimized *Plasmodium falciparum* or *Plasmodium yoelii* circumsporozoite protein, and in some aspects, may be encoded by the nucleotide sequence of SEQ ID NO:2 or SEQ ID NO:1, respectively.

In other embodiments, the immunogenic epitope further comprises a B cell epitope of Plasmodium circumsporozoite protein. The B cell epitope may be incorporated in a modified capsid protein, and in some aspects, the capsid protein may comprise a Hexon hypervariable region (HVR). The HVR may further comprise HVR1 or HVR5, wherein a portion of HVR1 or HVR5 is replaced with the B cell epitope. In other aspects, the capsid protein may further comprise a capsid Fiber protein wherein the B cell epitope is inserted into the Fiber protein. In some aspects, the B cell epitope is a *Plasmodium falciparum* circumsporozoite protein B cell epitope, wherein the B cell epitope is a repeat sequence, for example, (NANP)ₙ (SEQ ID NO:60), wherein the repeat sequence may be (NANP)₄, (NANP)₆, (NANP)₈, (NANP)₁₀, (NANP)₁₂, (NANP)₁₄, (NANP)₁₆, (NANP)₁₈, (NANP)₂₀, (NANP)₂₂ or (NANP)₂₈. In other aspects, the B cell epitope is a *Plasmodium yoelii* circumsporozoite protein B cell epitope, wherein the B cell epitope is a repeat sequence, for example, (QGPGAP)ₙ (SEQ ID NO:59), wherein the repeat sequence may be (QGPGAP)₃, (QGPGAP)₄, (QGPGAP)₅, (QGPGAP)₆, (QGPGAP)₇, (QGPGAP)₈, (QGPGAP)₉, (QGPGAP)₁₁, or (QGPGAP)₁₂.

In yet other embodiments, the immunogenic epitope further comprises a CD4+ or CD8+ T cell epitope of Plasmodium circumsporozoite protein. The CD4+ or CD8+ T cell epitope may be incorporated in a modified capsid or core protein. In some aspects, the capsid protein may comprise a Hexon hypervariable region (HVR). The HVR may further comprise HVR1 wherein a portion of HVR1 is replaced with the CD4+ or CD8+ T cell epitope. In other aspects, the core protein further comprises a pVII protein and a CD4+ T cell epitope is inserted into the pVII protein. In some aspects the CD4+ T cell epitope is a *Plasmodium falciparum* circumsporozoite CD4+ T cell epitope, wherein the CD4+ T cell epitope is EYLNKIQNSLSTEWSPCSVT (SEQ ID NO:62). In other aspects the CD4+ T cell epitope is a *Plasmodium yoelii* circumsporozoite CD4+ T cell epitope, wherein the CD4+ T cell epitope is YNRNIVNRLLGDALNGKPEEK (SEQ ID NO:61)

Other embodiments are directed to a pharmaceutical composition or malaria vaccine composition comprising a recombinant adenovirus according to the above embodiments. Further embodiments include a method of treating, preventing, or diagnosing malaria, comprising administering a therapeutic amount of the pharmaceutical composition or malaria vaccine composition in accordance with the above embodiments.

In another embodiment, a method for treatment comprising administering a prime-boost vaccination, wherein a subject is given a series of increasing dosages or same dosages at a given time interval. The time interval may be any length sufficient to generate a humoral and/or cellular immune response. For example, as described below, the interval may be, but is not limited to, once every 3 weeks.

### BRIEF DESCRIPTION OF THE DRAWINGS

Fig. 1 is a schematic diagram of a capsid-modified recombinant adenovirus in accordance with embodiments of the disclosure.
Fig. 2 is a schematic diagram illustrating the construction of the HVR1-Modified Adenovirus DNA of an HVR1-modified recombinant adenovirus plasmid vector.
Fig. 3 is a schematic diagram illustrating the construction of the HVR5-Modified Adenovirus DNA of an HVR5-modified recombinant adenovirus plasmid vector.
Fig. 4 is a schematic diagram illustrating the construction of the Fiber-Modified Adenovirus DNA of a Fiber-modified recombinant adenovirus plasmid vector.
Fig. 5 is a schematic diagram illustrating the construction of the HVR1 and Fiber-Modified Adenovirus DNA of an HVR1 and Fiber-modified recombinant adenovirus plasmid vector.
Fig. 6 is a schematic diagram illustrating the construction of the Fiber and pVII-Modified Adenovirus DNA of a Fiber and pVII-modified recombinant adenovirus plasmid vector.
Fig. 7 is a schematic diagram illustrating the construction of the HVR1 and pVII-Modified Adenovirus DNA of an HVR1 and pVII-modified recombinant adenovirus plasmid vector.
Fig. 8 is a schematic diagram illustrating the construction of the HVR1, Fiber and pVII-Modified Adenovirus DNA of an HVR1, Fiber and pVII-modified recombinant adenovirus plasmid vector.
Fig. 9 is the nucleic acid sequence of codon-optimized *Plasmodium yoelii* circumsporozoite protein (PyCS, SEQ ID NO:1) and the corresponding amino acid sequence (SEQ ID NO:30)
Fig. 10 is the nucleic acid sequence of codon-optimized *Plasmodium falciparum* circumsporozoite protein (PfCSP, SEQ ID NO:2) and the corresponding amino acid sequence (SEQ ID NO:43)
Fig. 11 is the nucleic acid and amino acid sequences of a modified Hexon having three repeats of the PyCS B cell epitope sequence (QGPGAP)ₙ, (SEQ ID NO:59; n=3) in HVR1(SEQ ID NO:3, nucleic acid; SEQ ID NO:31, amino acid). The inserted (QGPGAP)₃ sequence is underlined.
Fig. 12 is the nucleic acid and amino acid sequences of a modified Hexon having four repeats of the PyCS B cell epitope sequence (QGPGAP)ₙ, (SEQ ID NO:59; n=4) in HVR1 (SEQ ID NO:4, nucleic acid; SEQ ID NO: 32, amino acid). The inserted (QGPGAP)₄ sequence is underlined.
Fig. 13 is the nucleic acid and amino acid sequences of modified Hexon having five repeats of the PyCS B cell epitope sequence (QGPGAP)ₙ, (SEQ ID NO:59; n=5) in HVR1 (SEQ ID NO:5, nucleic acid; SEQ ID NO: 33, amino acid). The inserted (QGPGAP)₅ sequence is underlined.
Fig. 14 is the nucleic acid and amino acid sequences of modified Hexon having six repeats of the PyCS B cell epitope sequence (QGPGAP)ₙ, (SEQ ID NO:59; n=6) in HVR1 (SEQ ID NO:6, nucleic acid; SEQ ID NO: 34, amino acid). The inserted (QGPGAP)₆ sequence is underlined.
Fig. 15 is the nucleic acid and amino acid sequences of modified Hexon having seven repeats of the PyCS B cell epitope sequence (QGPGAP)ₙ, (SEQ ID NO:59; n=7) in HVR1 (SEQ ID NO:7, nucleic acid; SEQ ID NO: 35, amino acid). The inserted (QGPGAP)₇ sequence is underlined.
Fig. 16 is the nucleic acid and amino acid sequences of modified Hexon having eight repeats of the PyCS B cell epitope sequence (QGPGAP)ₙ, (SEQ ID NO:59; n=8) in HVR1 (SEQ ID NO:8, nucleic acid; SEQ ID NO: 36, amino acid). The inserted (QGPGAP)₈ sequence is underlined.
Fig. 17 is the nucleic acid and amino acid sequences of modified Hexon having nine repeats of the PyCS B cell epitope sequence (QGPGAP)ₙ, (SEQ ID NO:59; n=9) in HVR1 (SEQ ID NO:9, nucleic acid; SEQ ID NO: 37, amino acid). The inserted (QGPGAP)₉ sequence is underlined.
Fig. 18 is the nucleic acid and amino acid sequences of modified Hexon having eleven repeats of the PyCS B cell epitope sequence (QGPGAP)ₙ, (SEQ ID NO:59; n=11) in HVR1 (SEQ ID NO:10, nucleic acid; SEQ ID NO: 38, amino acid). The inserted (QGPGAP)₁₁ sequence is underlined.
Fig. 19 is the nucleic acid and amino acid sequences of modified Hexon having twelve repeats of the PyCS B cell epitope sequence (QGPGAP)ₙ, (SEQ ID NO:59; n=12) in HVR1 (SEQ ID NO:11, nucleic acid; SEQ ID NO: 39, amino acid). The inserted (QGPGAP)₁₂ sequence is underlined.
Fig. 20 is the nucleic acid and amino acid sequences of modified Hexon having four repeats of the PfCSP B cell epitope sequence (NANP)ₙ, (SEQ ID NO:60; n=4) in HVR1 (SEQ ID NO:12, nucleic acid; SEQ ID NO: 44, amino acid). The inserted (NANP)₄ sequence is underlined.
Fig. 21 is the nucleic acid and amino acid sequences of modified Hexon having six repeats of the PfCSP B cell epitope sequence (NANP)ₙ, (SEQ ID NO:60; n=6) in HVR1 (SEQ ID NO:13, nucleic acid; SEQ ID NO: 45, amino acid). The inserted (NANP)₆ sequence is underlined.
Fig. 22 is the nucleic acid and amino acid sequences of modified Hexon having eight repeats of the PfCSP B cell epitope sequence (NANP)ₙ, (SEQ ID NO:60; n=8) in HVR1 (SEQ ID NO:14, nucleic acid; SEQ ID NO: 46, amino acid). The inserted (NANP)₈ sequence is underlined.
Fig. 23 is the nucleic acid and amino acid sequences of modified Hexon having ten repeats of the PfCSP B cell epitope sequence (NANP)ₙ, (SEQ ID NO:60; n=10) in HVR1 (SEQ ID NO:15, nucleic acid; SEQ ID NO: 47, amino acid). The inserted (NANP)₁₀ sequence is underlined.
Fig. 24 is the nucleic acid and amino acid sequences of modified Hexon having twelve repeats of the PfCSP B cell epitope sequence (NANP)ₙ, (SEQ ID NO:60; n=12) in HVR1 (SEQ ID NO:16, nucleic acid; SEQ ID NO: 48, amino acid). The inserted (NANP)₁₂ sequence is underlined.
Fig. 25 is the nucleic acid and amino acid sequences of modified Hexon having fourteen repeats of the PfCSP B cell epitope sequence (NANP)ₙ, (SEQ ID NO:60; n=14) in HVR1 (SEQ ID NO:17, nucleic acid; SEQ ID NO: 49, amino acid). The inserted (NANP)₁₄ sequence is underlined.
Fig. 26 is the nucleic acid and amino acid sequences of modified Hexon having sixteen repeats of the PfCSP B cell epitope sequence (NANP)ₙ, (SEQ ID NO:60; n=16) in HVR1 (SEQ ID NO:18, nucleic acid; SEQ ID NO: 50, amino acid). The inserted (NANP)₁₆ sequence is underlined.
Fig. 27 is the nucleic acid and amino acid sequences of modified Hexon having eighteen repeats of the PfCSP B cell epitope sequence (NANP)ₙ, (SEQ ID NO:60; n=18) in HVR1 (SEQ ID NO:19, nucleic acid; SEQ ID NO: 51, amino acid). The inserted (NANP)₁₈ sequence is underlined.
Fig. 28 is the nucleic acid and amino acid sequences of modified Hexon having twenty repeats of the PfCSP B cell epitope sequence (NANP)ₙ, (SEQ ID NO:60; n=20) in HVR1 (SEQ ID NO:20, nucleic acid; SEQ ID NO: 52, amino acid). The inserted (NANP)₂₀ sequence is underlined.
Fig. 29 is the nucleic acid and amino acid sequences of modified Hexon having twenty-two repeats of the PfCSP B cell epitope sequence (NANP)ₙ, (SEQ ID NO:60; n=22) in HVR1 (SEQ ID NO:21, nucleic acid; SEQ ID NO: 53, amino acid). The inserted (NANP)₂₂ sequence is underlined.
Fig. 30 is the nucleic acid and amino acid sequences of modified Hexon having twenty-eight repeats of the PfCSP B cell epitope sequence (NANP)ₙ, (SEQ ID NO:60; n=28) in HVR1 (SEQ ID NO:22, nucleic acid; SEQ ID NO: 54, amino acid). The inserted (NANP)₂₈ sequence is underlined.
Fig. 31 is the nucleic acid and amino acid sequences of modified Hexon having three repeats of the PyCS B cell epitope sequence (QGPGAP)ₙ, (SEQ ID NO:59; n=3) in HVR5 (SEQ ID NO:23, nucleic acid; SEQ ID NO:40, amino acid). The inserted (QGPGAP)₃ sequence is underlined.
Fig. 32 is the nucleic acid and amino acid sequences of modified Fiber having three repeats of the PyCS B cell epitope sequence (QGPGAP)ₙ, (SEQ ID NO:59; n=3) in Fiber (SEQ ID NO:24, nucleic acid; SEQ ID NO:41, amino acid). The inserted (QGPGAP)₃ sequence is underlined.
Fig. 33 is the nucleic acid and amino acid sequences of modified Fiber having four repeats of the PfCSP B cell epitope sequence (NANP)ₙ, (SEQ ID NO:60; n=4) in Fiber (SEQ ID NO:25, nucleic acid; SEQ ID NO:55, amino acid). The inserted (NANP)₄ sequence is underlined.
Fig. 34 is the nucleic acid and amino acid sequences of the modified pVII having the PyCS CD4+ epitope sequence YNRNIVNRLLGDALNGKPEEK, (SEQ ID NO:61) at the N-terminus of pVII (SEQ ID NO:26, nucleic acid; SEQ ID NO:42, amino acid). The inserted YNRNIVNRLLGDALNGKPEEK sequence is underlined.
Fig. 35 is the nucleic acid and amino acid sequences of the modified pVII having the PfCSP CD4+ epitope sequence EYLNKIQNSLSTEWSPCSVT, (SEQ ID NO:62) at the C-terminus of pVII (pVII-1; SEQ ID NO:27, nucleic acid; SEQ ID NO:56, amino acid). The inserted EYLNKIQNSLSTEWSPCSVTsequence is underlined.
Fig. 36 is the nucleic acid and amino acid sequences of the modified pVII having the PfCSP CD4+ epitope sequence EYLNKIQNSLSTEWSPCSVT, (SEQ ID NO:62) before the first Nuclear Localization Signal (NLS) of pVII (pVII-2; SEQ ID NO:28, nucleic acid; SEQ ID NO:57, amino acid). The inserted EYLNKIQNSLSTEWSPCSVT sequence is underlined.
Fig. 37 is the nucleic acid and amino acid sequences of the modified pVII having the PfCSP CD4+ epitope sequence EYLNKIQNSLSTEWSPCSVT, (SEQ ID NO:62) between the two NLSs of pVII (pVII-3; SEQ ID NO:29, nucleic acid; SEQ ID NO:58, amino acid). The inserted EYLNKIQNSLSTEWSPCSVTsequence is underlined.
Fig. 38 shows PyCS protein expression in AD293 cells after transient transfection with PyCS-GFP/pShuttle-CMV. PyCS protein was detected by western blotting using mouse monoclonal anti-PyCS antibody (9D3).
Fig. 39 shows the results of silver staining and western blotting (A) and ELISA assay (B) of the purified capsid-modified recombinant PyCS-GFP adenoviruses to confirm the (QGPGAP)₃ epitope (SEQ ID NO:59; n=3) insertion into adenovirus capsid proteins. In the ELISA assay, ELISA plates were coated directly with purified adenoviruses and the inserted epitope in adenovirus particles was detected with anti-PyCS antibody.
Fig. 40 shows the results of silver staining and western blotting (A) and ELISA assay (B) of the purified capsid-modified recombinant PyCS adenoviruses to confirm the (QGPGAP)ₙ epitope (SEQ ID NO:59) insertion into adenovirus capsid proteins. In the ELISA assay, ELISA plates were coated directly with purified adenoviruses and the inserted epitope in adenovirus particles was detected with anti-PyCS antibody.
Fig. 41 illustrates a single immunization regimen with capsid-modified PyCS adenoviruses having (QGPGAP)ₙ repeats (SEQ ID NO:59, n=3, 4, 5, 6, 9, 12) (A) and PyCS-specific CD8+ response two weeks after immunization (B).
Fig. 42 illustrates a prime and boost immunization regimen with capsid-modified PyCS adenoviruses having (QGPGAP)ₙ repeats (SEQ ID NO:59, n=3) (A), PyCS-specific humoral responses at week 10 (B), and malaria parasite burden in liver 42 hours after sporozoite challenge (C).
Fig. 43 illustrates anti-sporozoite antibody titer determined by indirect immunofluorescene assay (IFA) (A) and *in vitro* sporozoite neutralizing activity (B) of pooled serum samples prepared from mice given the regimen in Fig. 42.
Fig. 44 illustrates a prime and boost immunization regimen with capsid-modified PyCS adenoviruses having (QGPGAP)ₙ repeats (SEQ ID NO:59, n=4, 6) in HVR1(A), PyCS-specific humoral responses at week 9 (B), malaria parasite burden in liver 42 hours after sporozoite challenge (C), and *in vitro* sporozoite neutralizing activity of pooled serum samples (D). Mice were immunized with or without adjuvant.
Fig. 45 illustrates a prime and boost immunization regimen with capsid-modified PyCS adenoviruses having (QGPGAP)ₙ repeats (SEQ ID NO:59, n=6, 9, 12) in HVR1(A), PyCS-specific humoral responses at week 9 (B), PyCS-specific CD8+ T cell responses at week 9 (C), and malaria parasite burden in liver 42 hours after sporozoite challenge (D). Mice were immunized with or without adjuvant.
Fig. 46 shows PfCSP protein expression in AD293 cells after transient transfection with PfCSP/pShuttle-CMV. PfCSP was detected by western blotting using mouse monoclonal anti-NANP antibody (2A10).
Fig. 47 illustrates the results of silver staining and western blotting (A), and ELISA assay (B) of the purified capsid-modified recombinant PfCSP adenoviruses to confirm the (NANP)₄ epitope (SEQ ID NO:60; n=4) insertion into adenovirus capsid proteins. The inserted (NANP)₄ epitope (SEQ ID NO:60; n=4) was detected with mouse monoclonal anti-NANP antibody (2A10). In the ELISA assay, ELISA plates were coated directly with purified adenoviruses.
Fig. 48 shows the results of silver staining and western blotting (A) and ELISA assay (B) of the purified capsid-modified recombinant PfCSP adenoviruses to confirm the (NANP)ₙ epitope (SEQ ID NO:60; n=4, 6, 8, 10, 12, 14, 16, 18, 20, 22) insertion into adenovirus capsid proteins. In the ELISA assay, ELISA plates were coated directly with purified adenoviruses and the inserted epitope in adenovirus particles was detected with anti-PfCSP antibody (2A10).
Fig. 49 illustrates the prime and boast immunization regimen with capsid-modified recombinant PfCSP adenoviruses having (NANP)₄ (SEQ ID NO:60; n=4) (A) and PfCSP-specific humoral responses at week 9 (B).
Fig. 50 illustrates the prime and boast immunization regimen with capsid-modified recombinant PfCSP adenoviruses having (NANP)ₙ (SEQ ID NO:60; n=4, 6, 8, 10) in HVR1 (A) and PfCSP-specific humoral responses at week 9 (B).
Fig. 51 illustrates the prime and boast immunization regimen with capsid-modified recombinant PfCSP adenoviruses having (NANP)ₙ (SEQ ID NO:60; n=10, 16, 22) in HVR1 (A) and PfCSP-specific humoral responses at week 9 (B). Mice were immunized with or without adjuvant.
Fig. 52 illustrates the result of sliver staining analysis of purified (QGPGAP)₃-modified Fiber and pVII-1 ((QGPGAP)₆-Fib/CD4-pVII-1/PyCS-GFP) adenovirus (A) and anti-QGPGAP antibody titer at week 10 in mice immunized with (QGPGAP)₃-Fib/PyCS-GFP or (QGPGAP)₃-Fib/CD4-pVII-1/PyCS-GFP as described in Fig. 49 (B). The results of two independent experiments were plotted in the figure after normalization with the median antibody titers in B-Fib/PyCS-GFP-immunized group.
Fig. 53 shows schematic diagrams of the structure of the adenovirus pVII proteins with the PfCSP CD4+ epitope sequence EYLNKIQNSLSTEWSPCSVT(SEQ ID NO:62) inserted at the before the first Nuclear Localization Signal (NLS) of pVII (PfCD4-pVII-2; SEQ ID NO:28, nucleic acid; SEQ ID NO:57, amino acid) and between the two NLSs of pVII (PfCD4-pVII-3; SEQ ID NO:29, nucleic acid; SEQ ID NO:58, amino acid) (A) and the results of silver staining to confirm the epitope insertion into pVII (B).
Fig. 54 illustrates the prime and boast immunization regimen with HVR1 and pVII-modified recombinant PfCSP adenoviruses (A), PfCSP-specific humoral responses at week 6 (B), and PfCSP-specific CD4+ (EYLNKIQNSLSTEWSPCSVT ; SEQ ID NO:62) response at week 9 (C).
Fig. 55 illustrates *in vitro* neutralization of recombinant adenovirus by human serum samples. AD293 cells were infected with recombinant adenoviruses in the presence of diluted human serum for overnight and GFP expression was measured as a marker of infection.
Fig. 56 illustrates the effect of anti-adenovirus immunity on the induction of PyCS-specific T cell response by capsid-modified PyCS-GFP adenoviruses *in vivo.* (A) is the brief description of the study design. (B) shows PyCS-specific CD8+ T cell response in mice immunized with wild-type (wt)/empty adenovirus twice followed by priming with capsid-modified PyCS-GFP adenoviruses.
Fig. 57 illustrates the effect of anti-adenovirus immunity on the induction of PyCS-specific humoral immune response by capsid-modified PyCS-GFP adenoviruses *in vivo.* (A) is the brief description of the study design. (B) shows PyCS-specific humoral immune response in mice immunized with wild-type (wt)/empty adenovirus twice followed by two doses of capsid-modified PyCS-GFP adenoviruses.

### MEANS FOR SOLVING THE PROBLEMS

The present inventors have found a novel recombinant adenovirus having a novel, capsid-modified structure that is derived from a recombinant adenovirus plasmid vector. The recombinant adenovirus is capable of infecting mammalian cells, causing the cells to express a *Plasmodium* circumsporozoite protein. The recombinant adenovirus also has one or more capsid proteins that have been modified by having a desired immunogenic antigen, such as B cell epitope, T cell epitope of *Plasmodium* circumsporozoite protein. The recombinant adenovirus is obtained by the method of transfecting cells with the linearized recombinant adenovirus plasmid vector. Using the obtained recombinant adenovirus, the present inventors carried out extensive research on pharmaceuticals containing as an active ingredient a recombinant adenovirus having malaria infection preventive and therapeutic effects. As a result, the inventors found that the obtained recombinant adenovirus has the desired pharmaceutical effects.

### DETAILED DESCRIPTION

The following description provides specific details for a thorough understanding of, and enabling description for, embodiments of the disclosure. However, one skilled in the art will understand that the disclosure may be practiced without these details. In other instances, well-known structures and functions have not been shown or described in detail to avoid unnecessarily obscuring the description of the embodiments of the disclosure.

The abbreviations used for the amino acids, peptides, base sequences, and nucleic acids in the present disclosure are based on the abbreviations specified in the IUPAC-IUB Communication on Biochemical Nomenclature, Eur. J. Biochem., 138: 9 (1984), "Guideline for Preparing Specifications Including Base Sequences and Amino Acid Sequences" (United States Patent and Trademark Office), and those commonly used in this technical field.

A "nucleotide sequence," "polynucleotide" or "DNA molecule" as contemplated by the current disclosure, may include double strand DNA or single strand DNA (i.e., a sense chain and an antisense chain constituting the double strand DNA), and is not limited to a full length thereof. Nucleotide sequences encoding an immunogenic foreign gene, such as those disclosed herein below, encompass double strand DNA containing genomic DNA, single strand DNA (sense chain) containing cDNA, single strand DNA (antisense chain) having a sequence complementary to the sense chain, synthetic DNA, and fragments thereof, unless otherwise mentioned.

Nucleotide sequences, polynucleotides or DNA molecules as used herein are not limited to the functional region, and may include at least one of an expression suppression region, a coding region, a leader sequence, an exon, and an intron. Further, examples of nucleotide sequences or polynucleotides may include RNA or DNA. A polypeptide containing a specific amino acid sequence and a polynucleotide containing a specific DNA sequence may include fragments, homologs, derivatives, and mutants of the polynucleotide. Examples of mutants of a nucleotide sequence or polynucleotide (such as mutant DNA), include naturally occurring allelic mutants; artificial mutants; and mutants having deletion, substitution, addition, and/or insertion. It should be understood that such mutants encode polypeptides having substantially the same function as the polypeptide encoded by the original non-mutated polynucleotide.

The present disclosure relates to a recombinant adenovirus that can express an antigenic determinant of a *Plasmodium* parasite, and comprises one or more modified capsid and/or core proteins. The recombinant adenovirus is derived from a recombinant adenovirus plasmid vector, the generation of which is described in the Examples below. The use of adenovirus as a vector is discussed further below. The recombinant adenovirus plasmid vectors described herein may be used as a malaria vaccine or pharmaceutical composition, wherein both humoral and cellular immune responses against the *Plasmodium* parasite are induced.

The *Plasmodium* parasite may be selected from any of the known *Plasmodium* (P.) species, for example, *P*. *falciparum, P. malariae, P. ovale, P*. *vivax, P. knowlesi, P. berghei, P. chabaudi* and *P*. *yoelii.* In some embodiments, the antigenic determinant is derived from the rodent-specific *Plasmodium yoelii* or the human-specific *Plasmodium falciparum*

In one embodiment, a recombinant adenovirus capsid-modified plasmid vector (also described as a recombinant adenovirus plasmid vector herein) is a plasmid that encodes and produces a capsid and/or core-modified recombinant adenovirus (also described as a recombinant adenovirus herein) that has a structure comprising one or more modified capsid and/or core proteins. In accordance with the embodiments of the disclosure, the modification of the capsid and/or core proteins may be accomplished by insertion of at least one immunogenic epitope of a *Plasmodium* circumsporozoite protein. Alternatively, at least part of the capsid and/or core protein may be deleted and replaced by at least one immunogenic epitope of a *Plasmodium* circumsporozoite protein. In some embodiments, the immunogenic epitope is a B-cell and/or T-cell epitope of a *Plasmodium* circumsporozoite protein. The addition of a B cell or T cell epitope may serve to enhance the efficacy of an adenoviral vector used as a malaria vaccine by establishing or enhancing the humoral immune response to the CS protein. The modified capsid and core proteins and their significance with respect to their use in the recombinant adenovirus described herein are discussed further below.

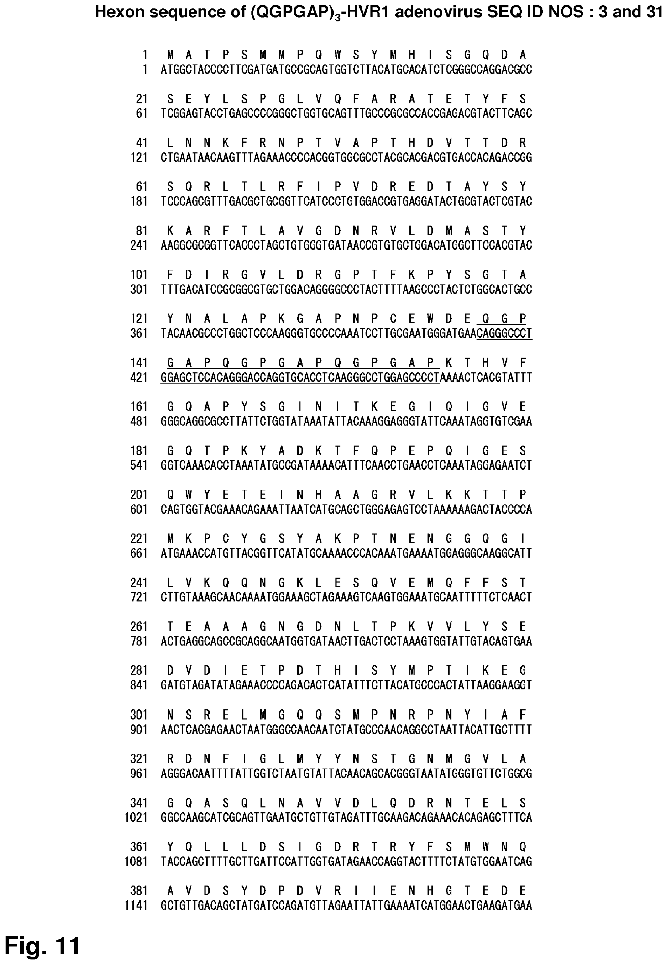

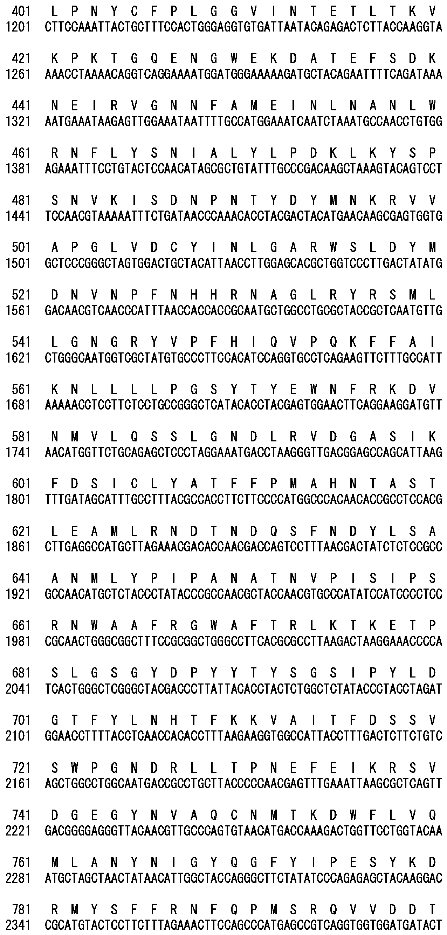

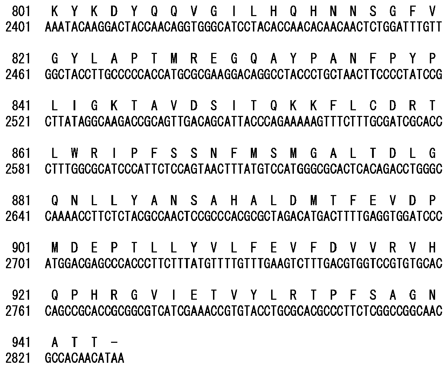

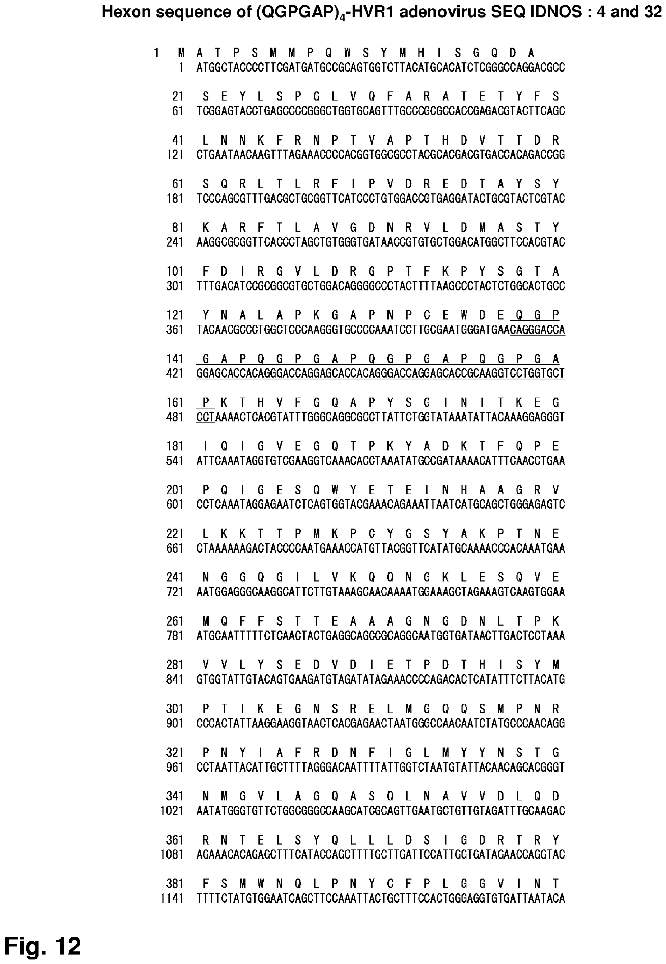

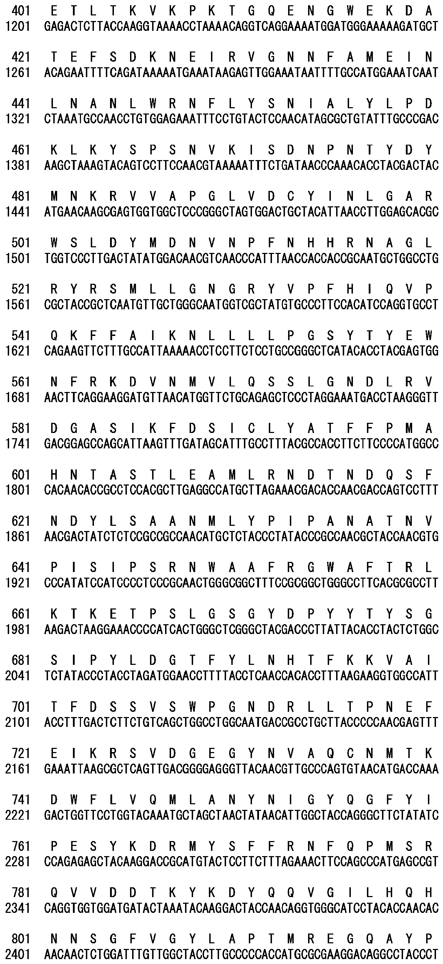

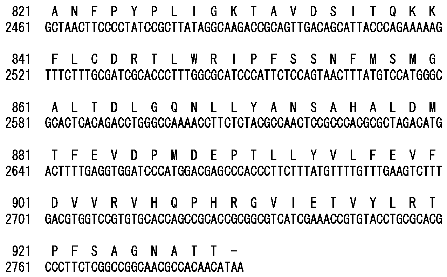

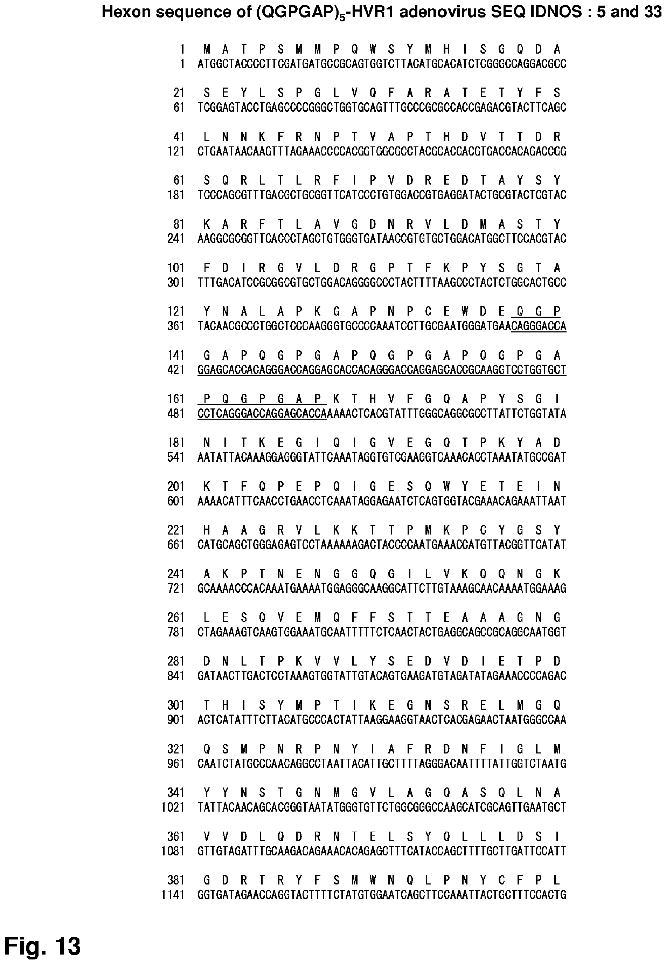

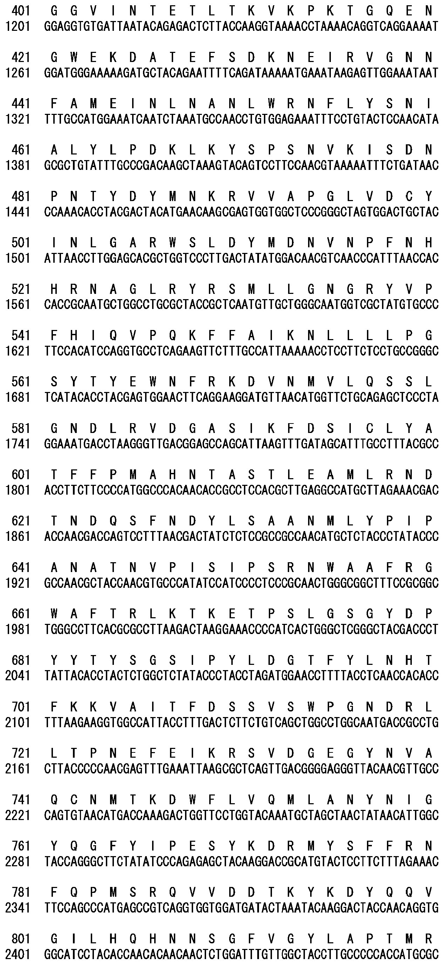

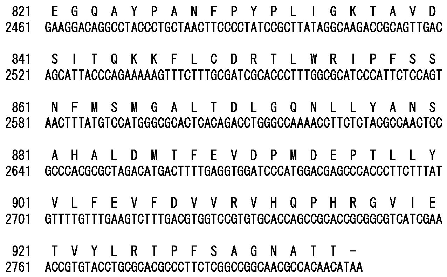

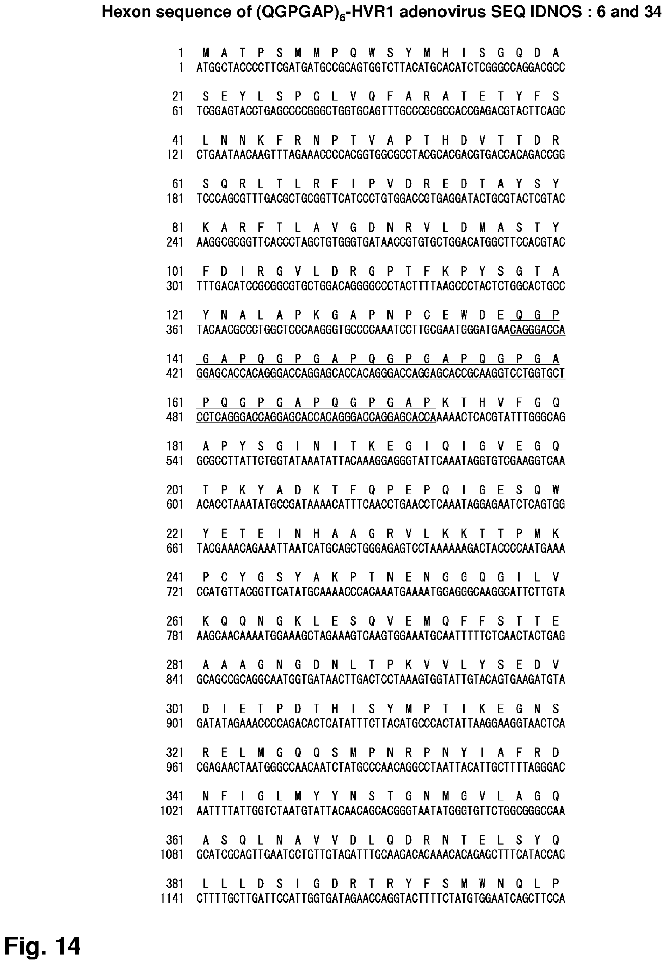

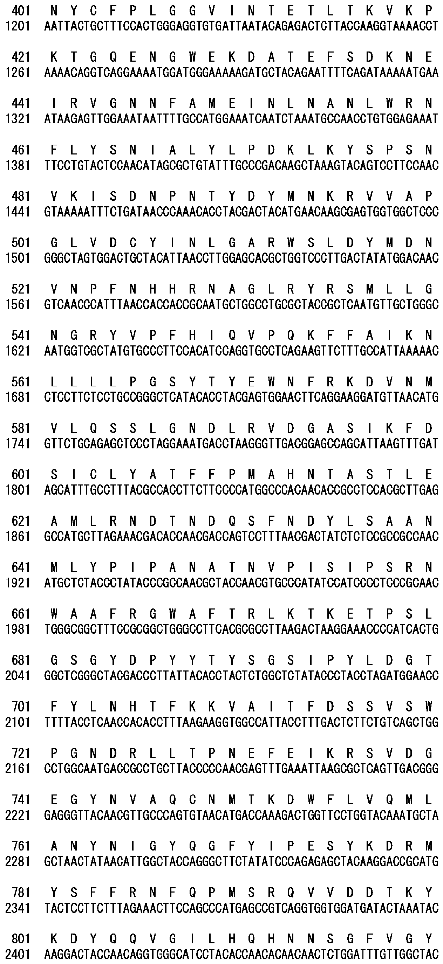

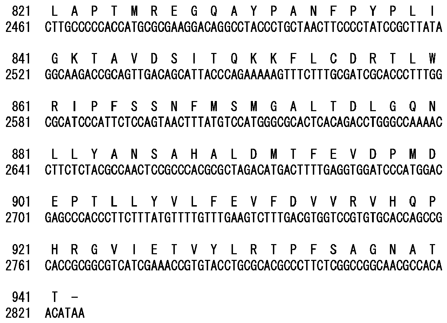

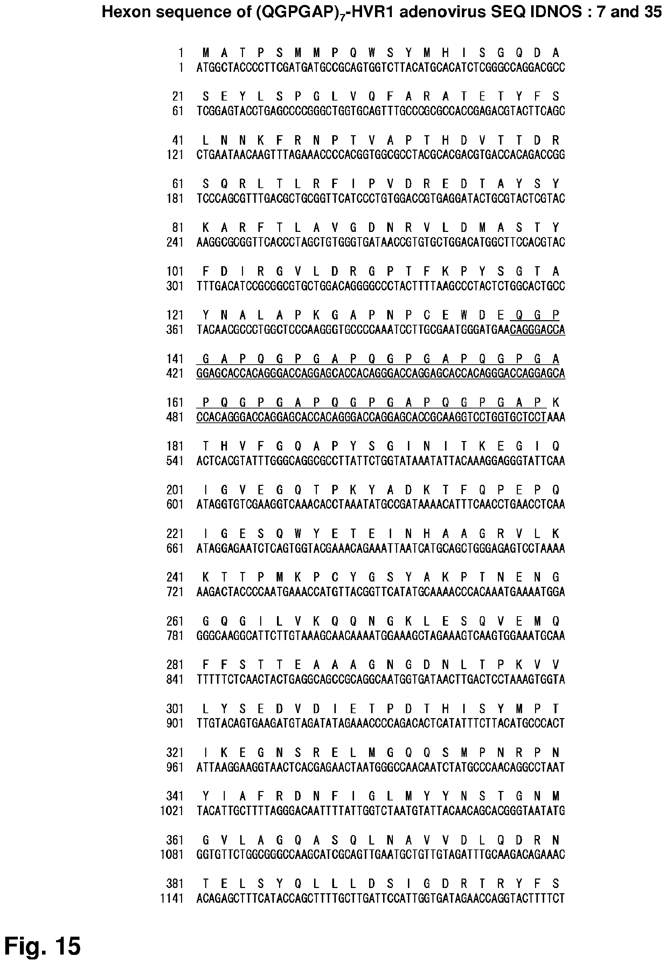

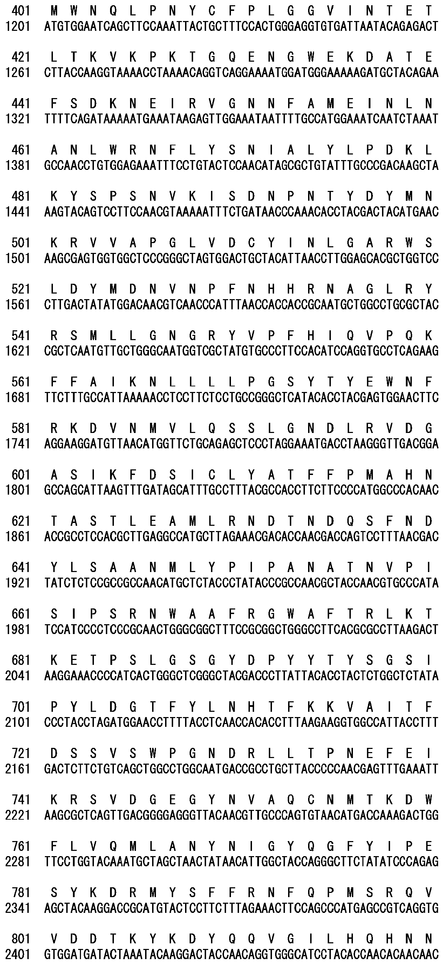

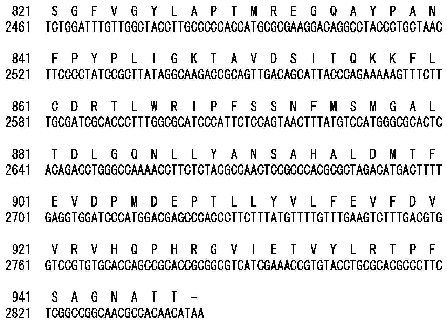

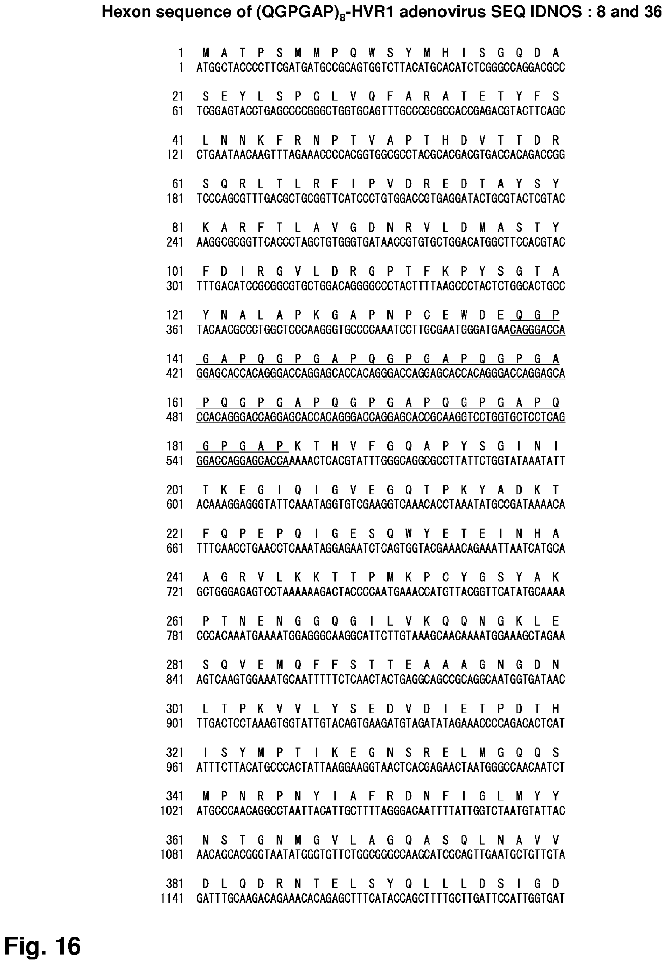

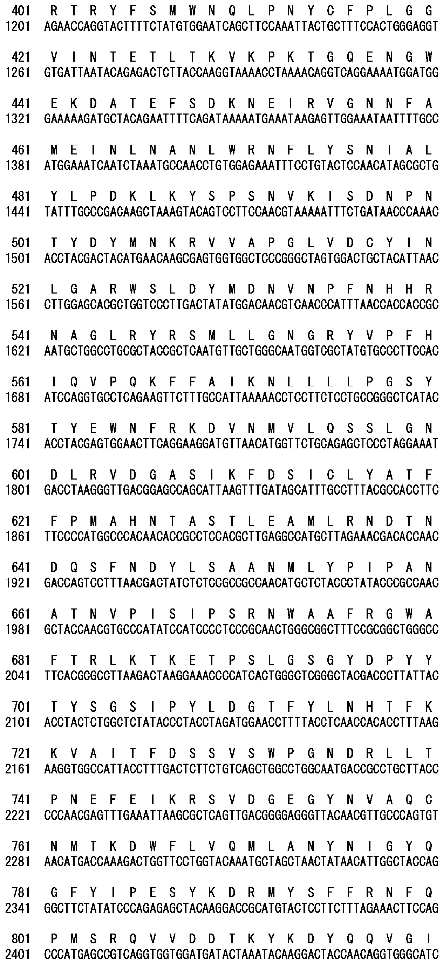

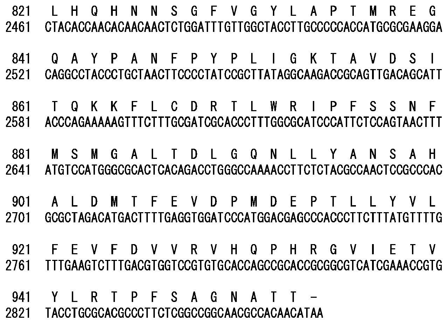

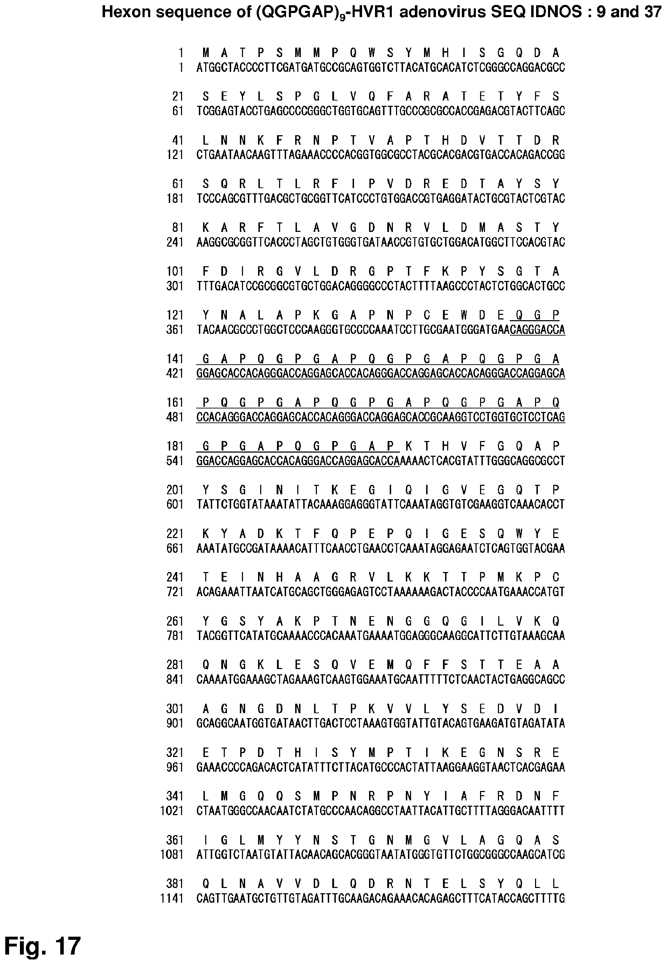

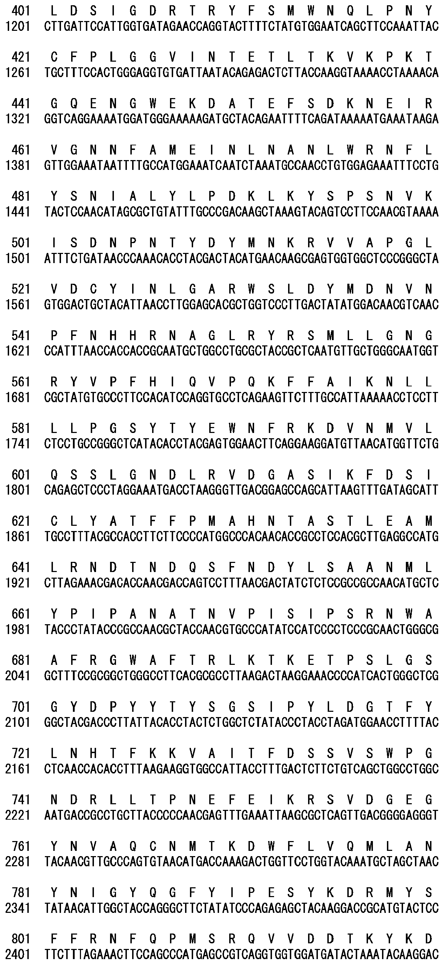

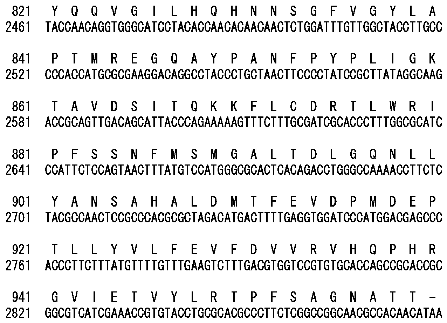

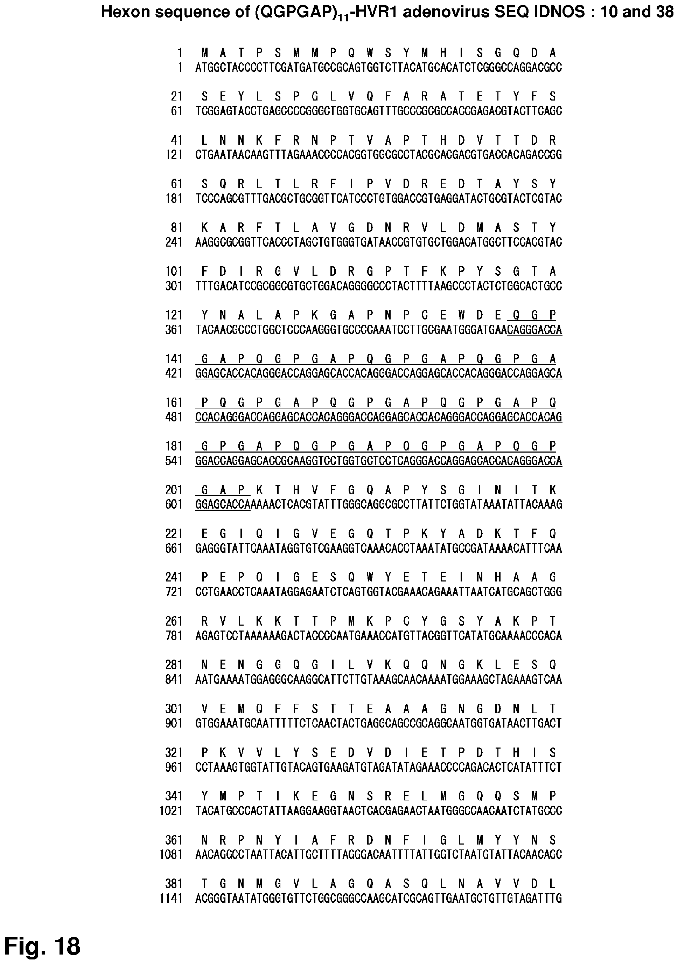

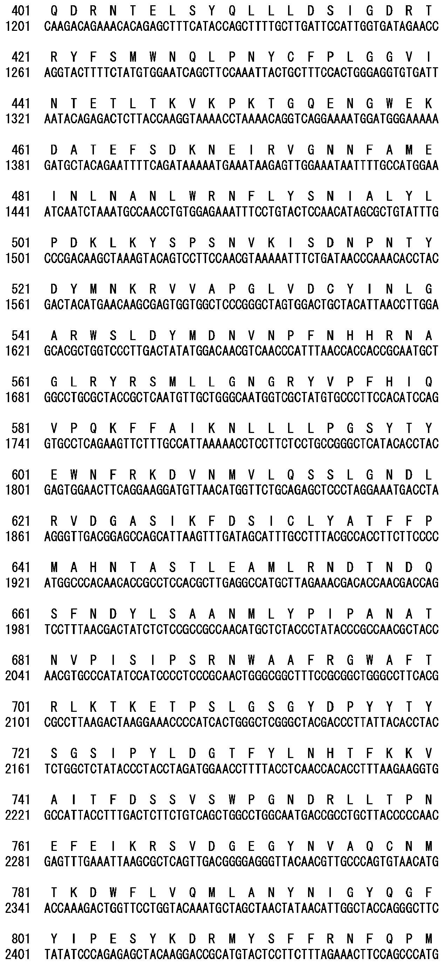

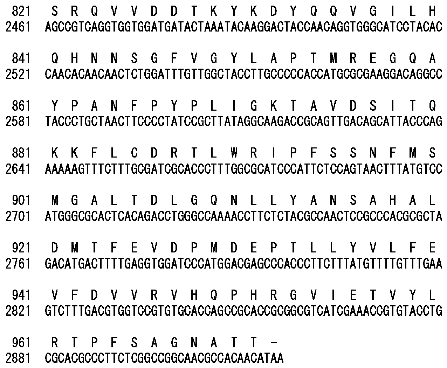

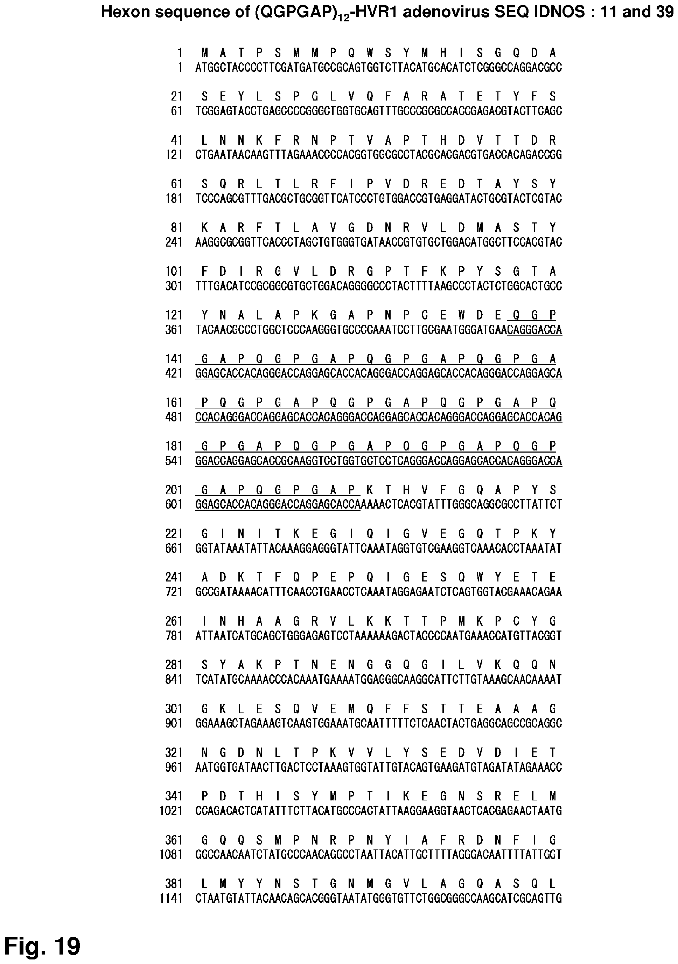

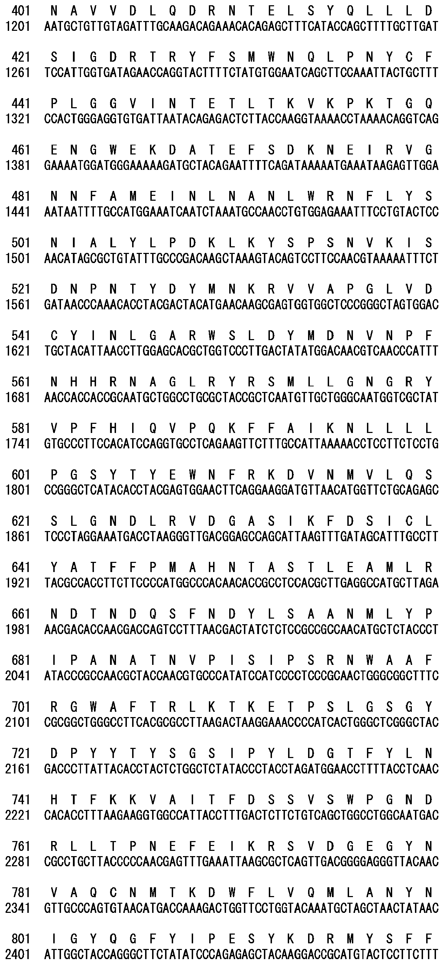

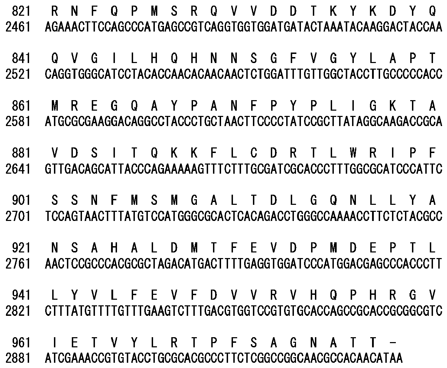

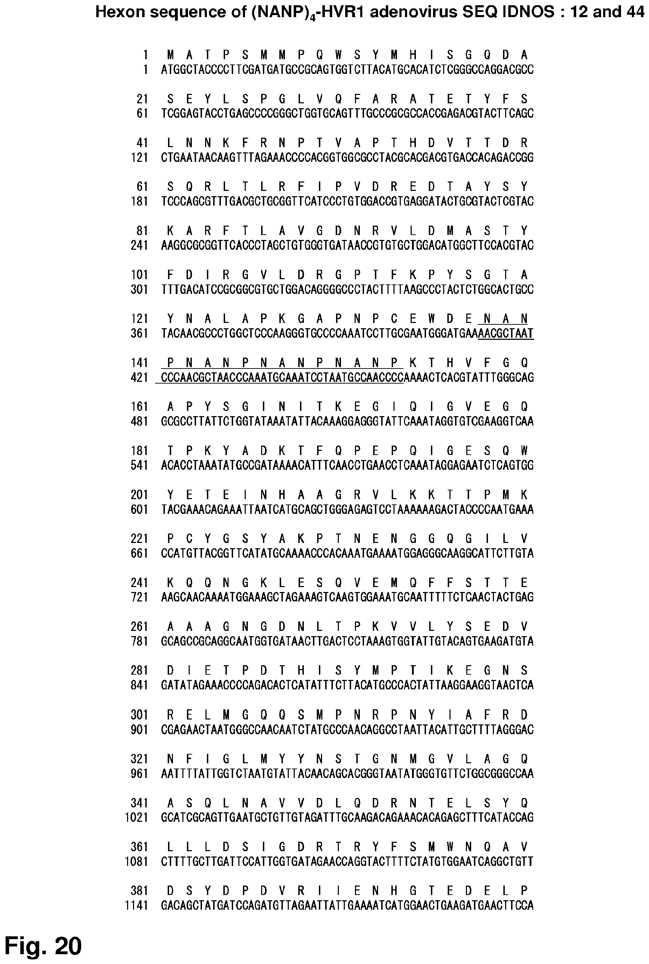

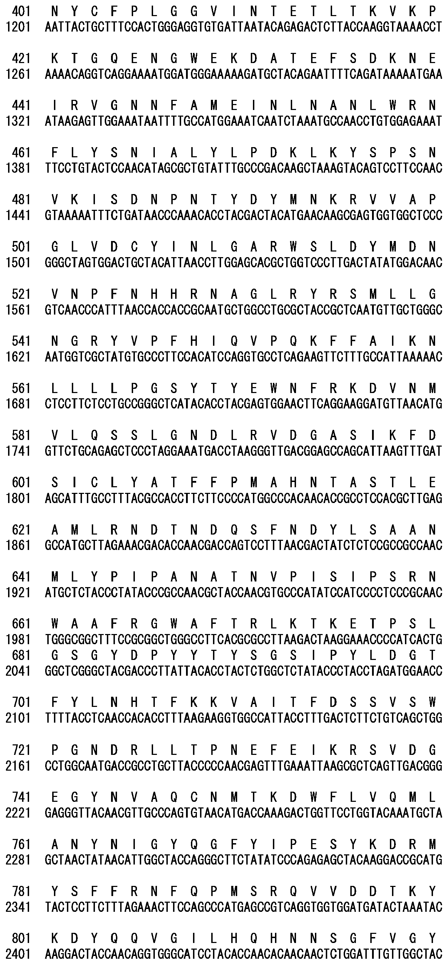

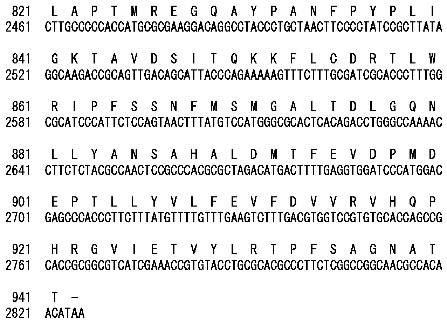

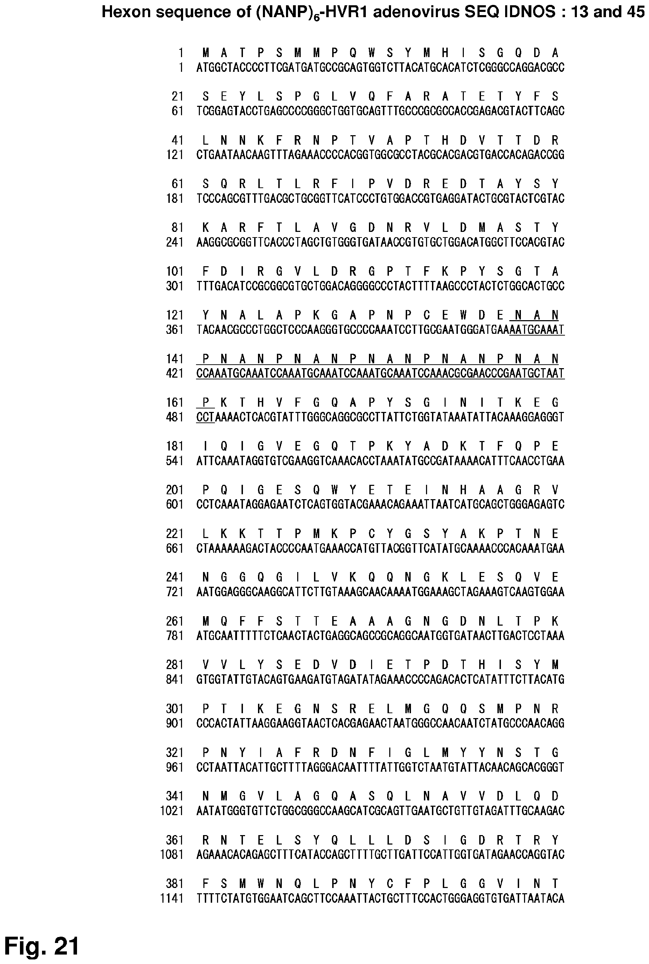

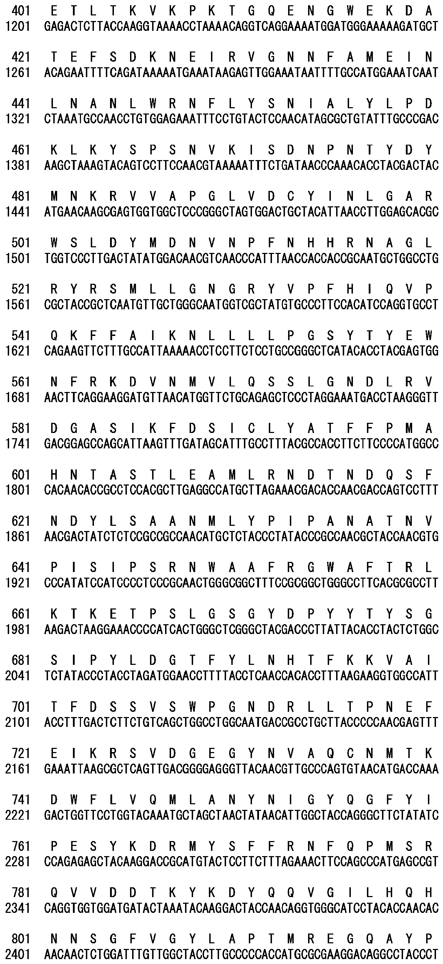

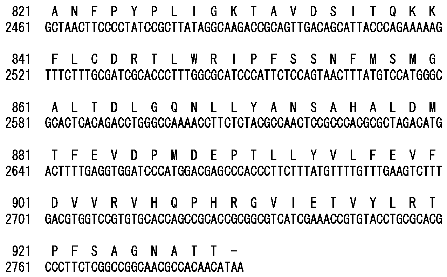

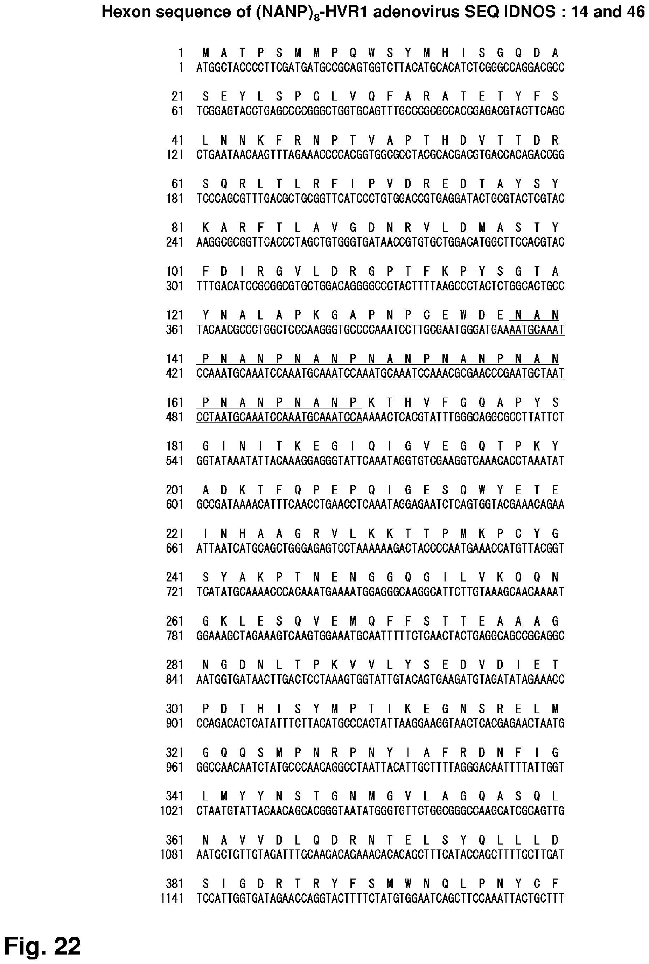

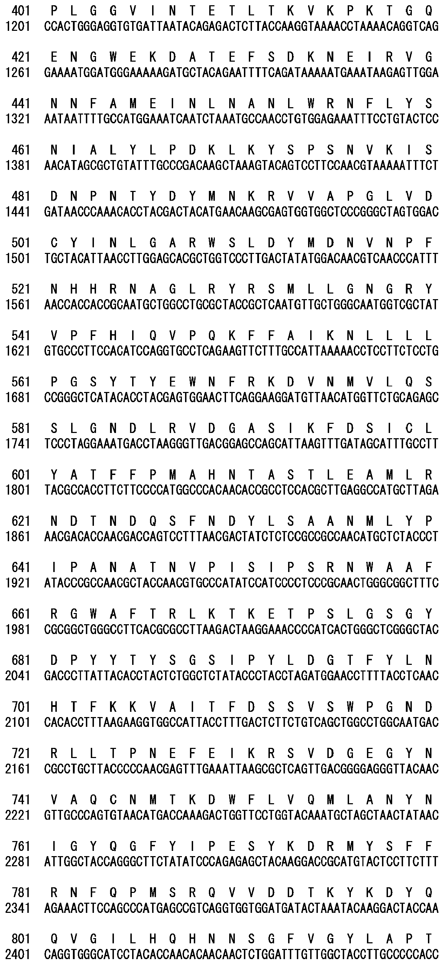

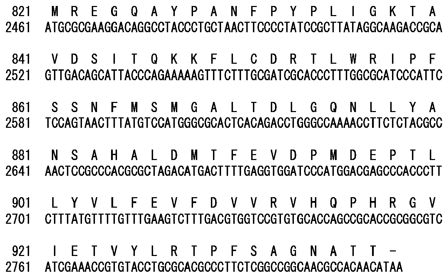

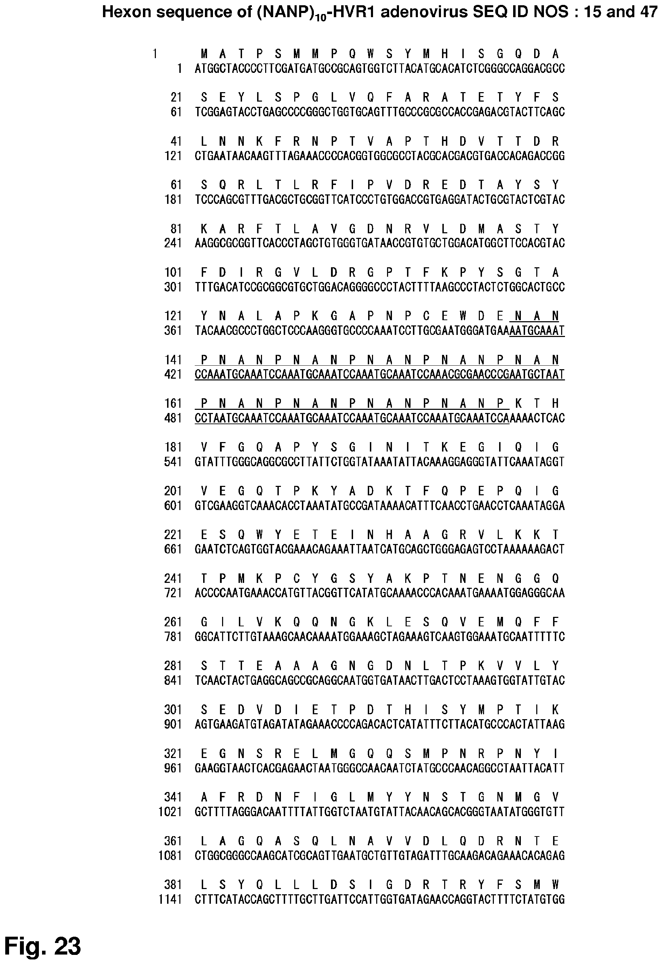

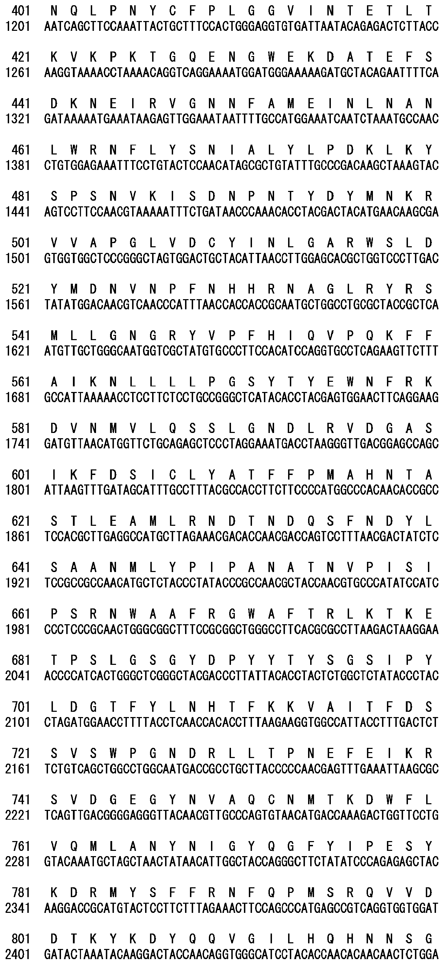

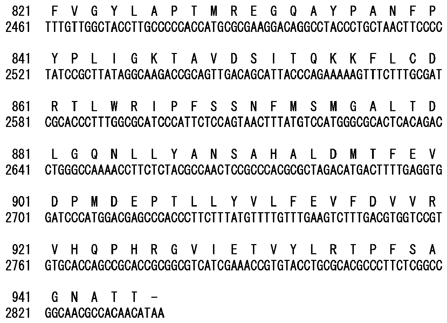

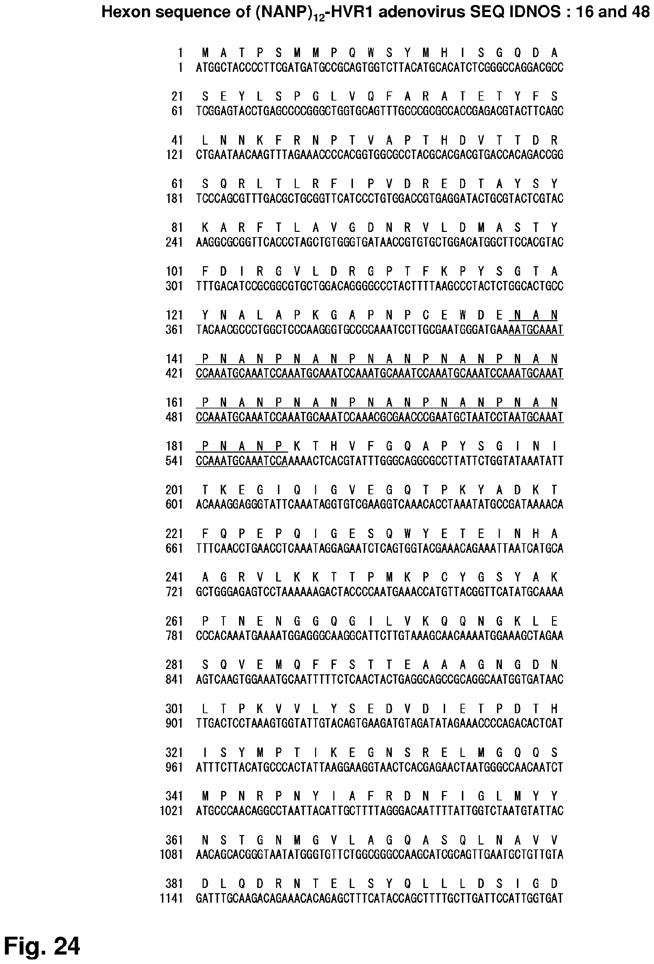

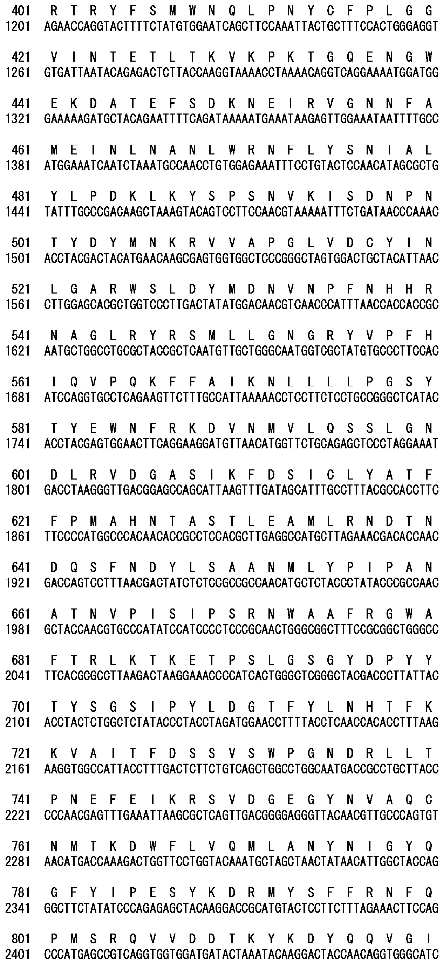

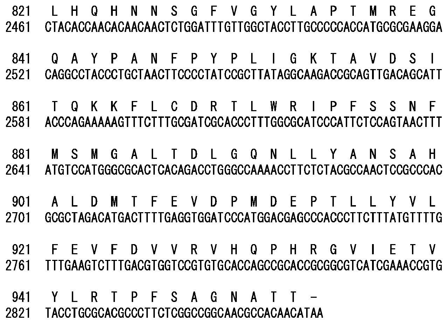

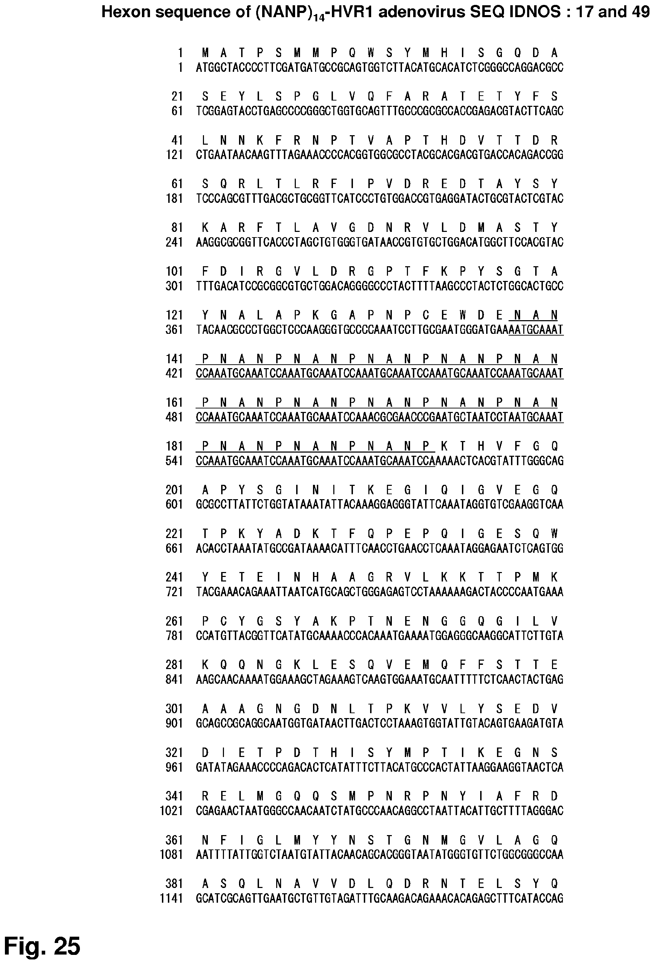

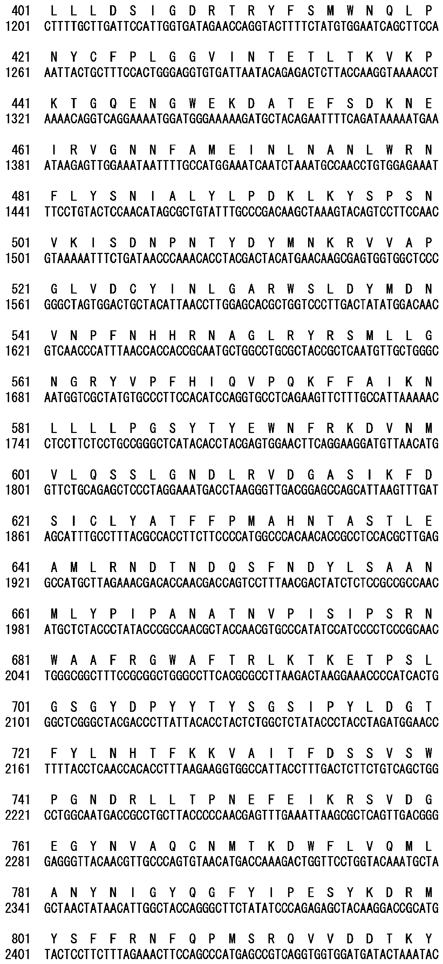

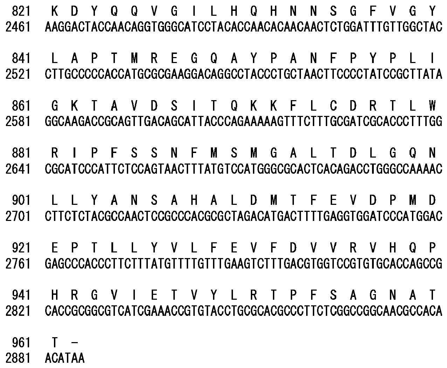

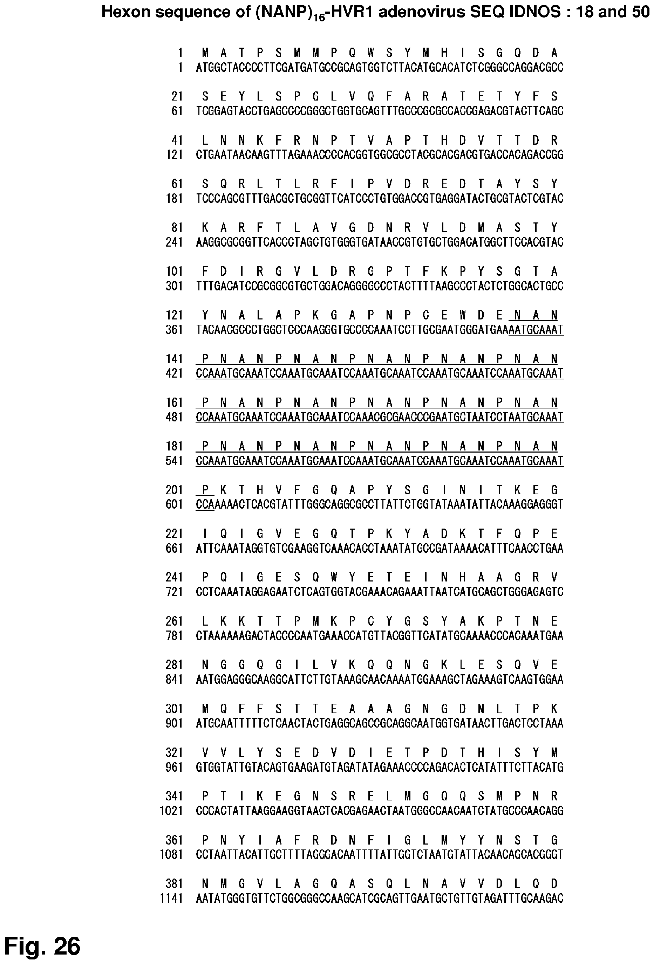

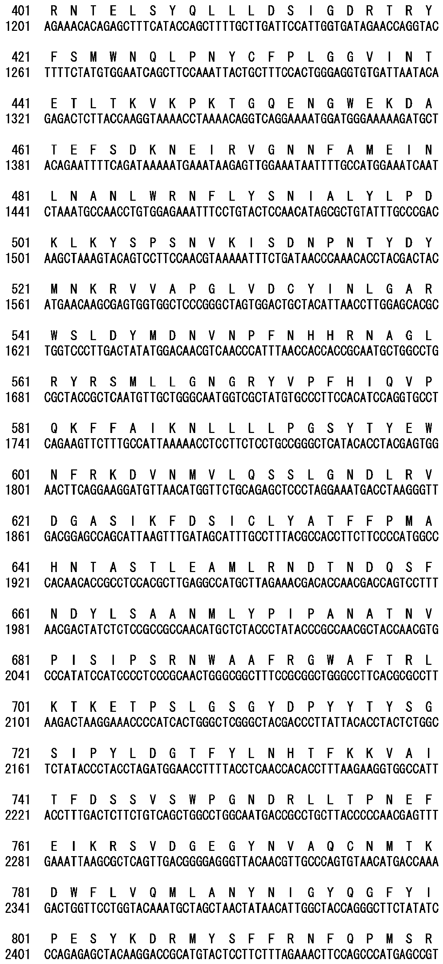

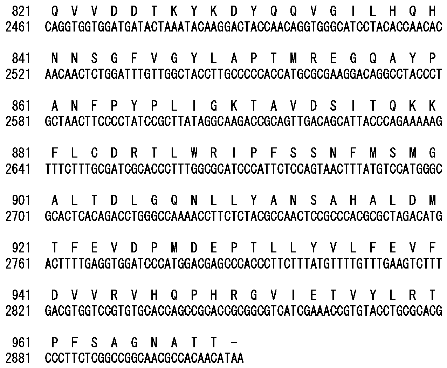

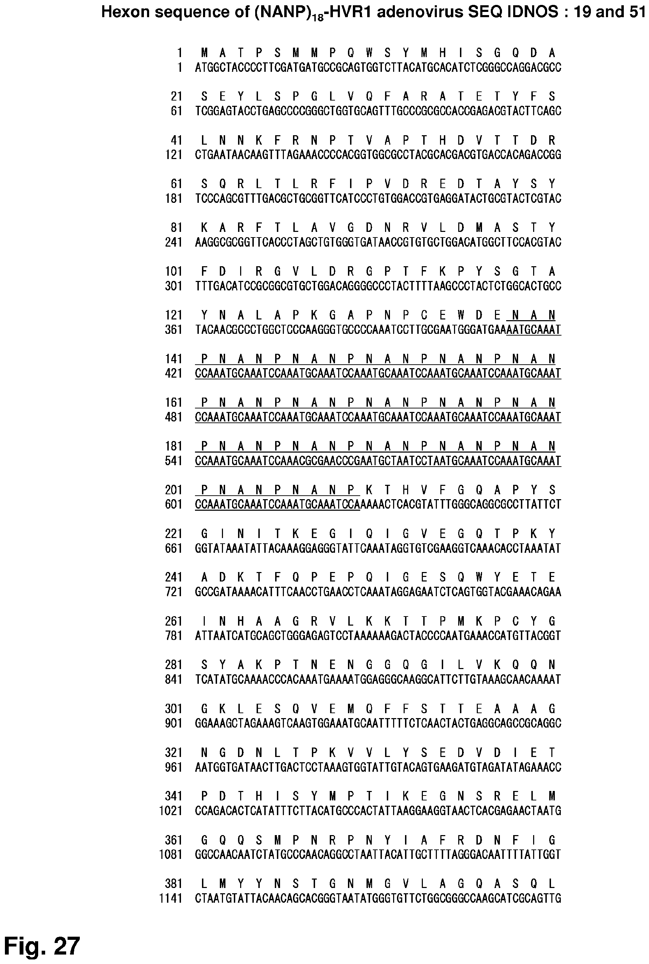

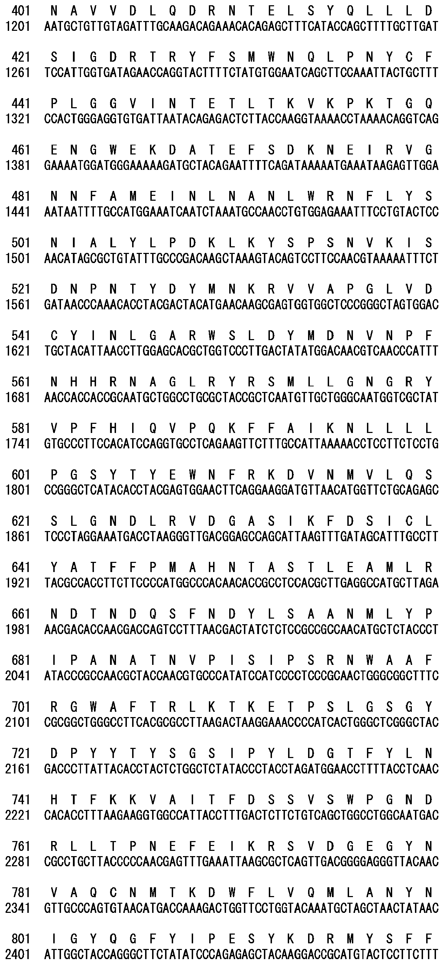

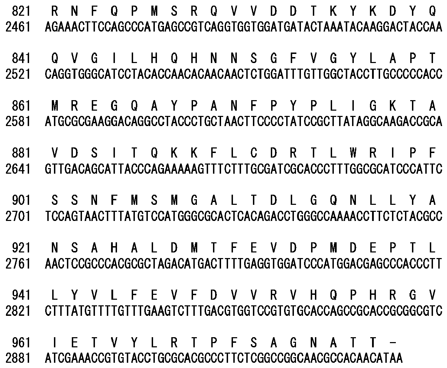

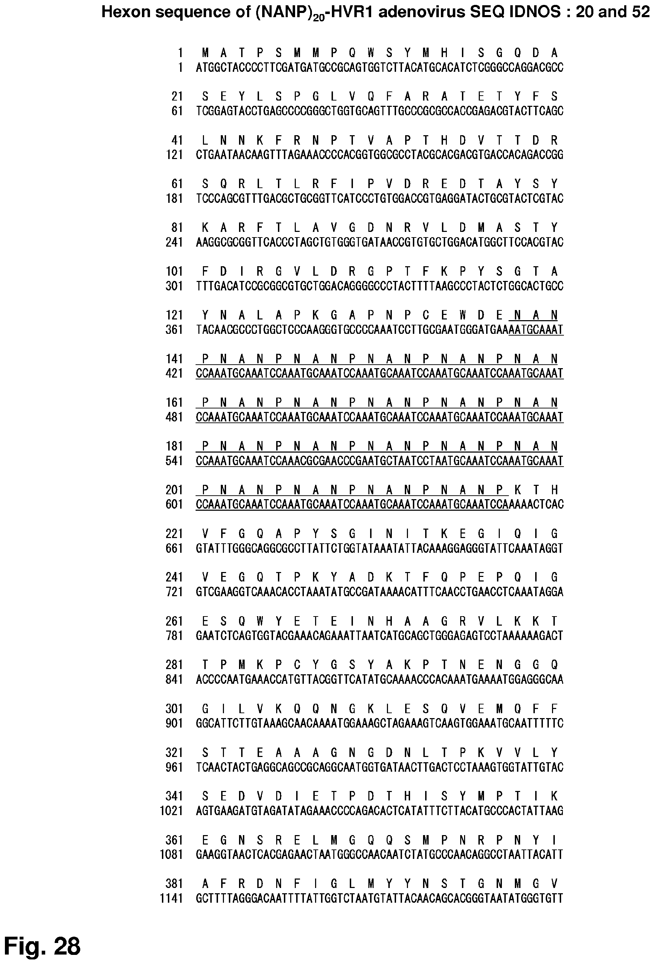

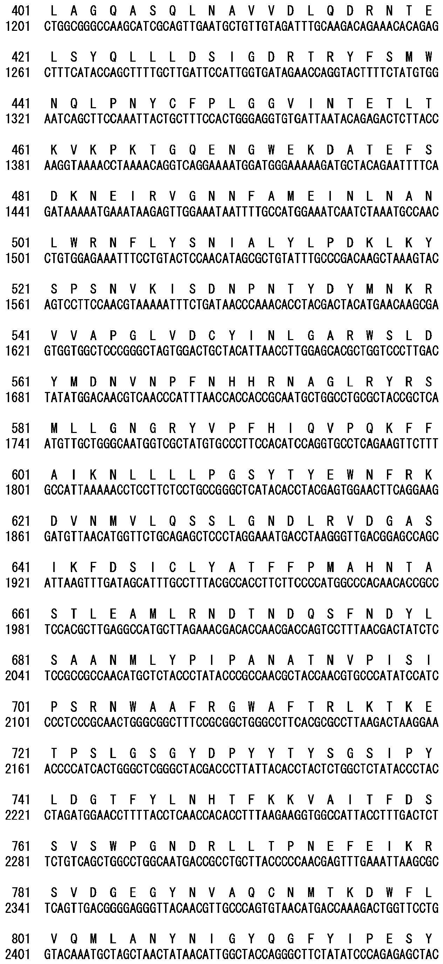

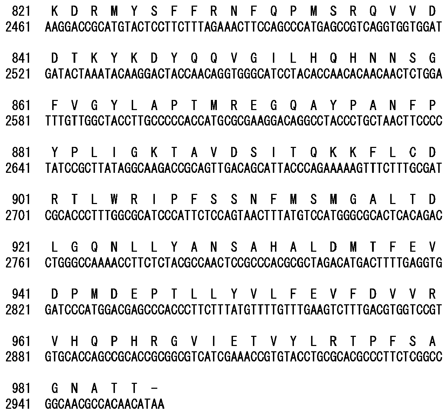

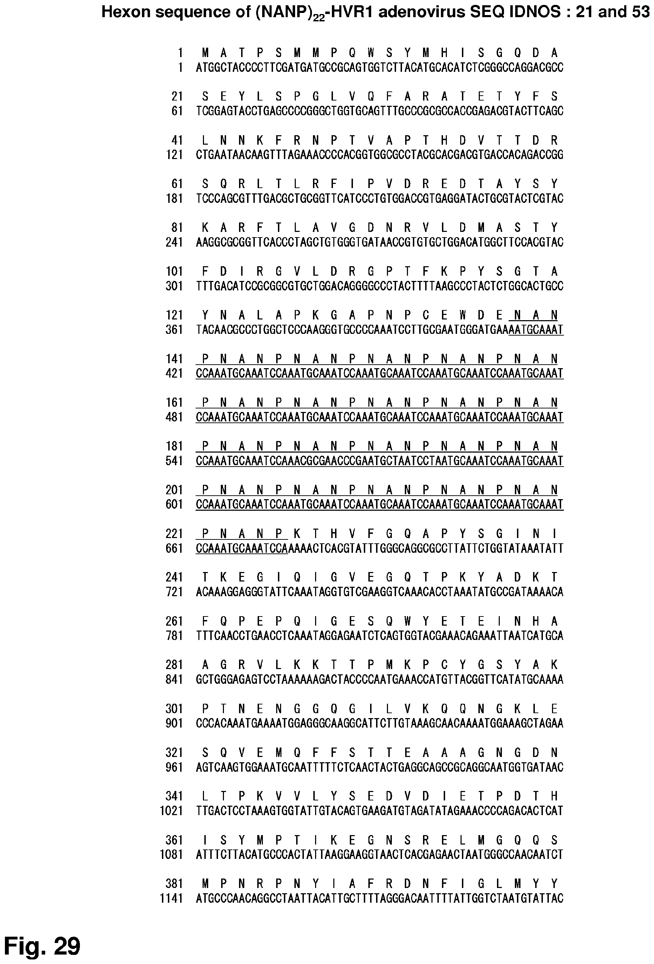

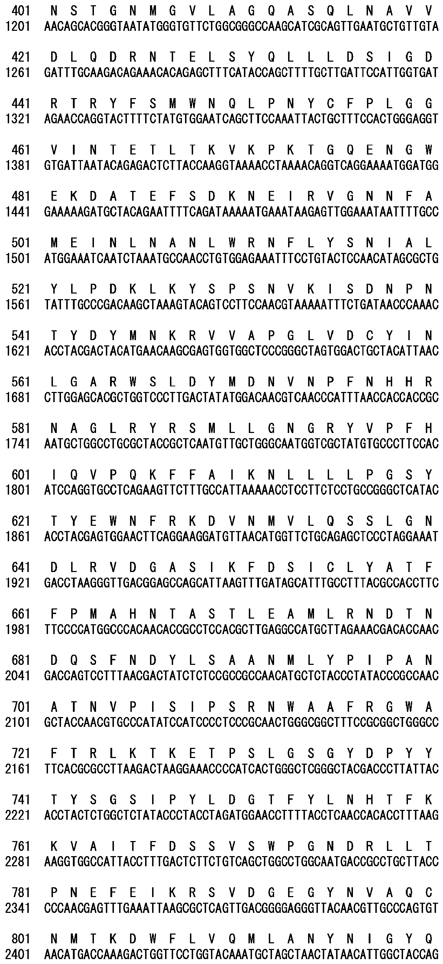

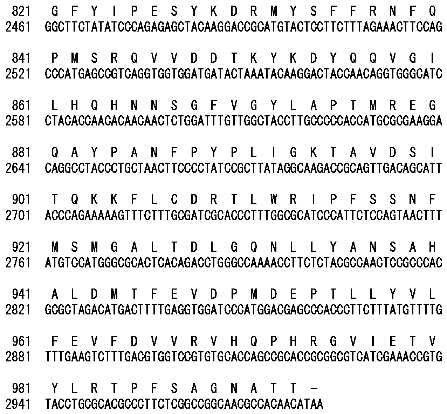

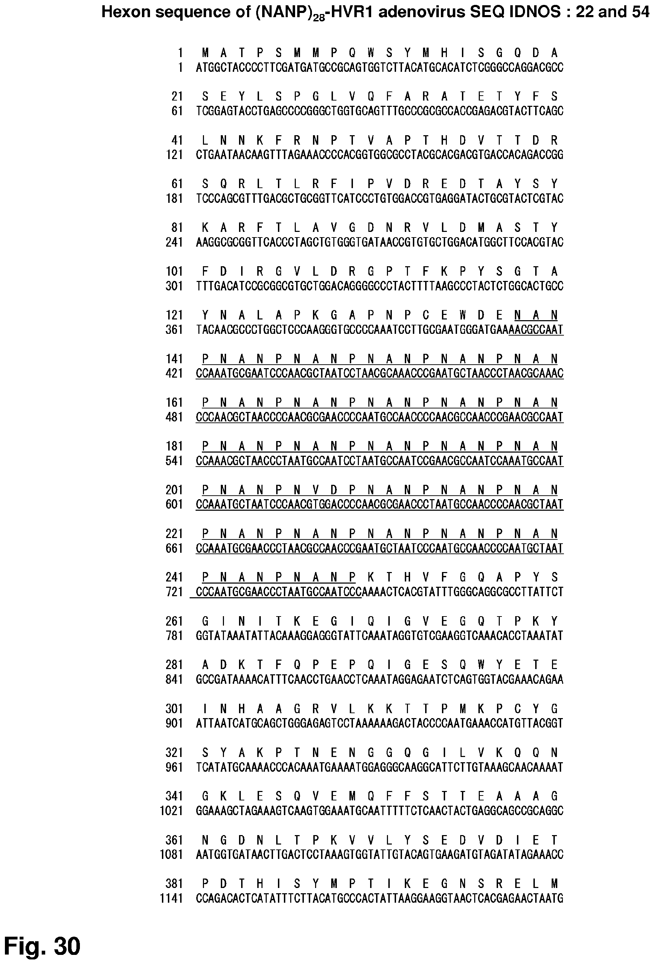

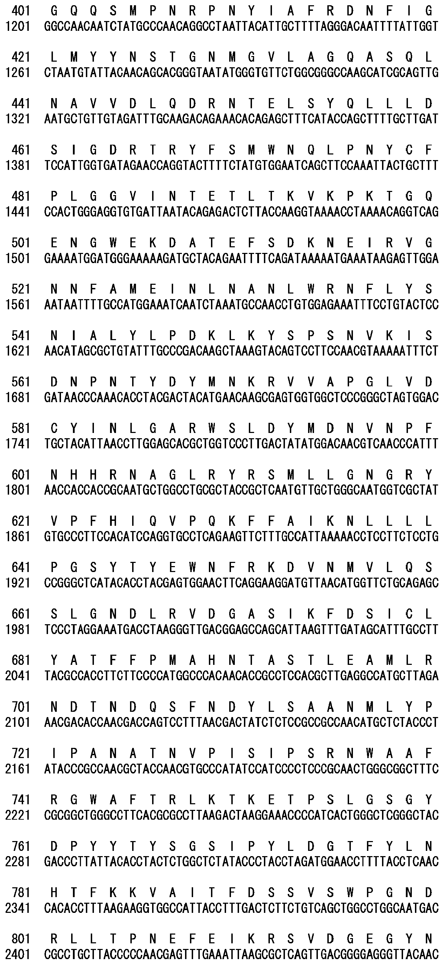

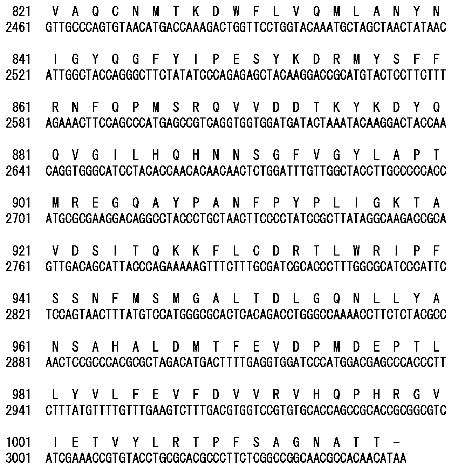

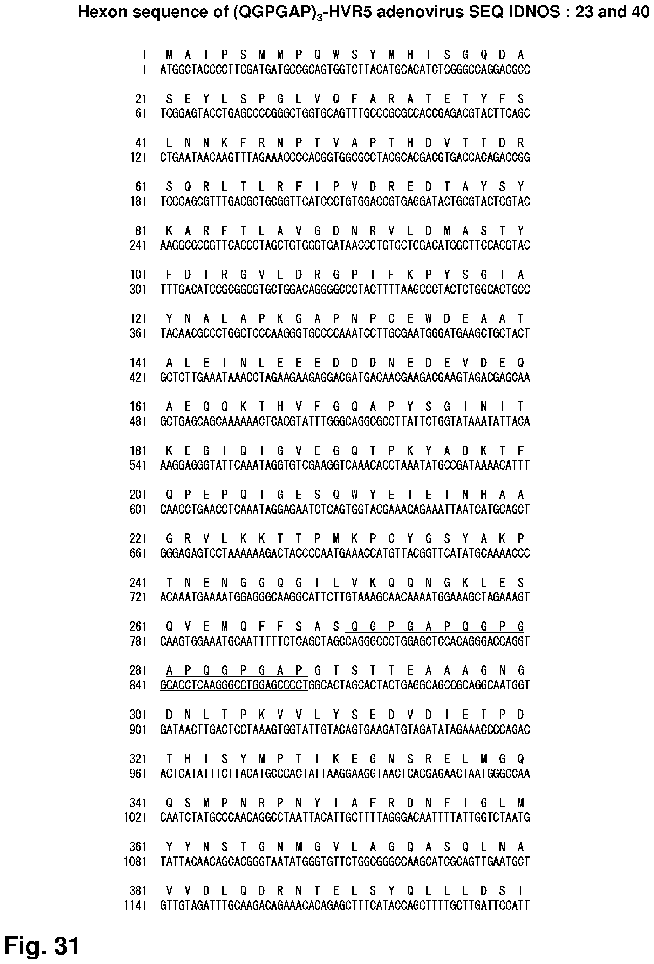

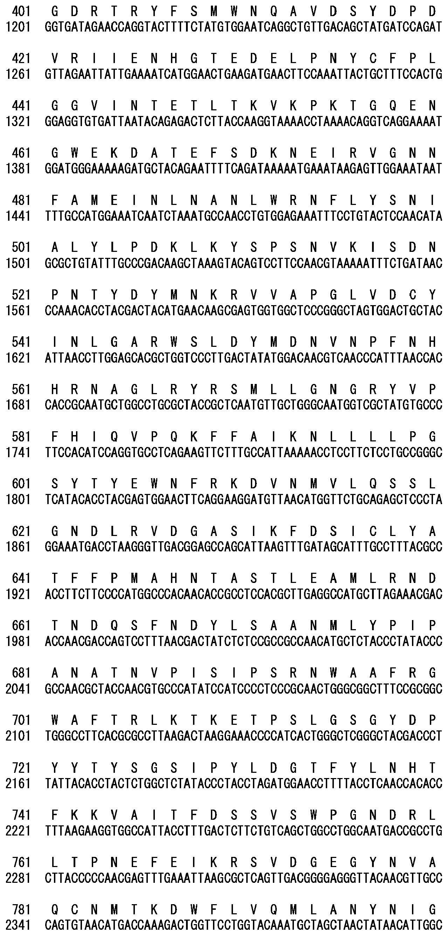

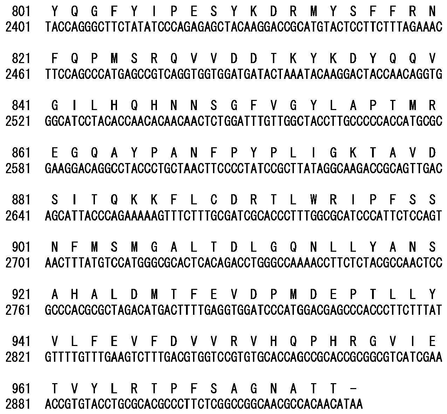

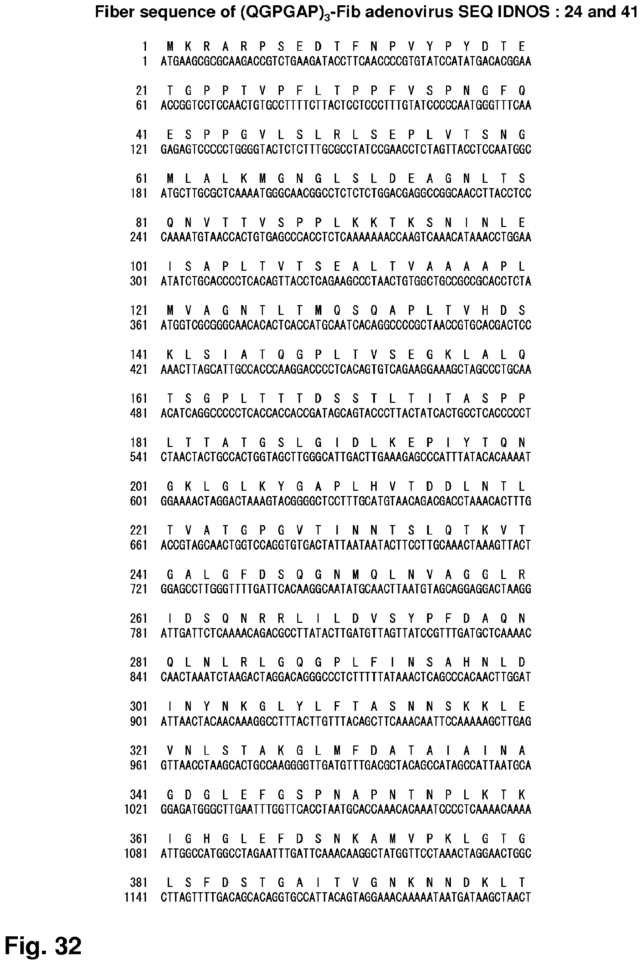

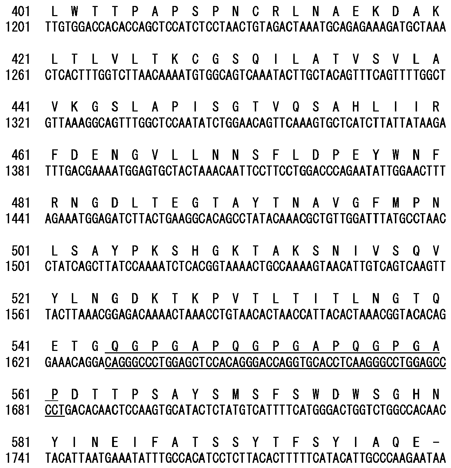

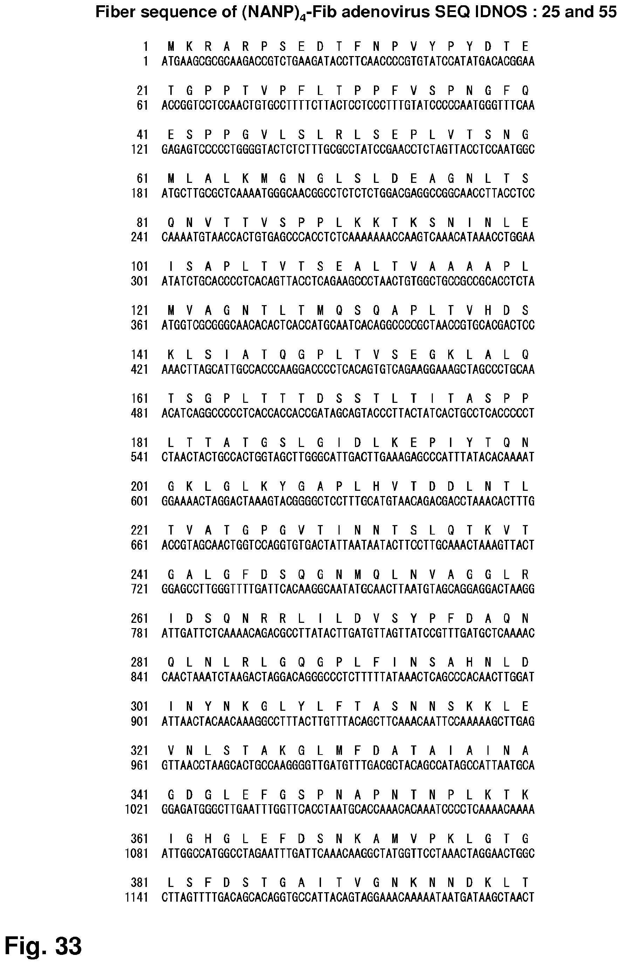

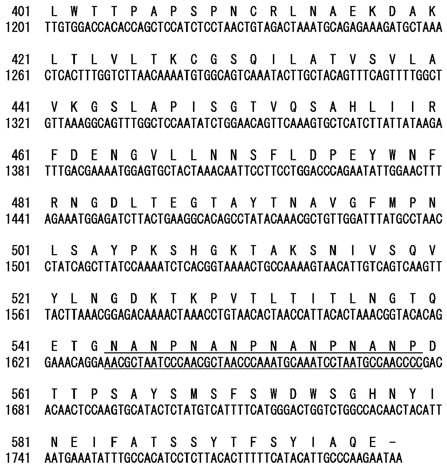

The one or more modified capsid and/or core proteins may be a modified Hexon protein, a modified Fiber protein, a modified pVII protein or a combination thereof. In one embodiment, a portion of a Hexon hypervariable region (HVR) and/or a portion of Fiber protein is replaced by at least one B-cell and/or T-cell epitope of a *Plasmodium* circumsporozoite protein. Alternatively, one or more B-cell and/or T cell epitope of a *Plasmodium* circumsporozoite protein may be inserted in the Fiber protein or Hexon HVR. In some aspects, the modified HVR may be HVR1, HVR2, HVR3, HVR4, HVR5, HVR6 or HVR7. In other aspects, the modified HVR may be HVR1 or HVR5. In some embodiments, the HVR-modified Hexon may have a nucleic acid sequence of SEQ ID NO:3 (Fig. 11), SEQ ID NO:4 (Fig. 12), SEQ ID NO:5 (Fig. 13), SEQ ID NO:6 (Fig. 14), SEQ ID NO:7 (Fig. 15), SEQ ID NO:8 (Fig. 16), SEQ ID NO:9 (Fig. 17), SEQ ID NO:10 (Fig. 18), SEQ ID NO:11 (Fig. 19), SEQ ID NO:12 (Fig. 20), SEQ ID NO:13 (Fig. 21), SEQ ID NO:14 (Fig. 22), SEQ ID NO:15 (Fig. 23), SEQ ID NO:16 (Fig 24), SEQ ID NO:17 (Fig. 25), SEQ ID NO:18 (Fig. 26), SEQ ID NO:19 (Fig. 27), SEQ ID NO:20 (Fig. 28), SEQ ID NO:21 (Fig. 29), SEQ ID NO:22 (Fig. 30), or SEQ ID NO:23 (Fig. 31). In other embodiments, the modified Fiber protein may have a nucleic acid sequence of SEQ ID NO:24 (Fig. 32) or SEQ ID NO:25 (Fig. 33).

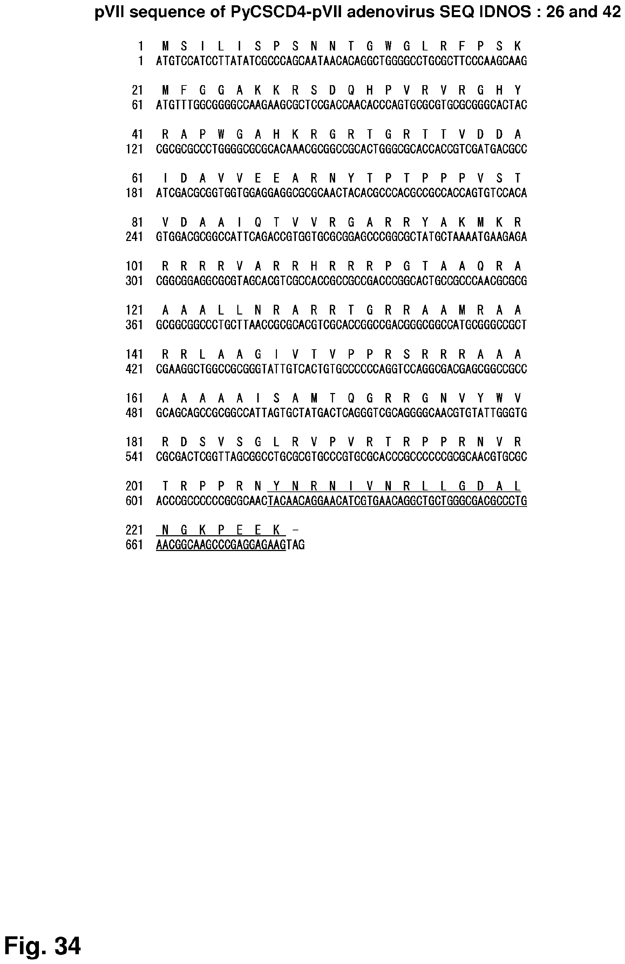

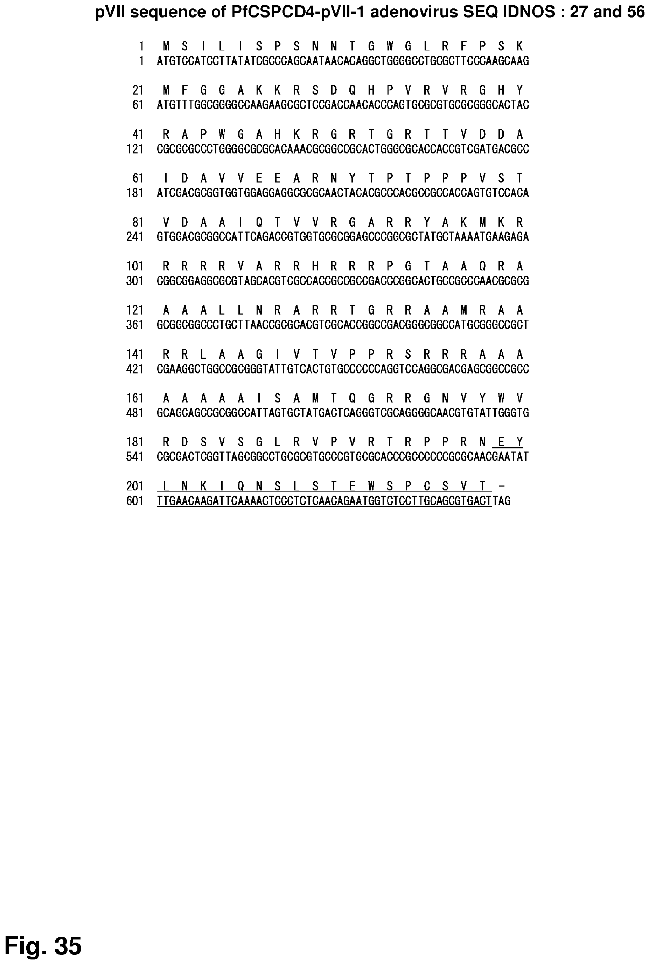

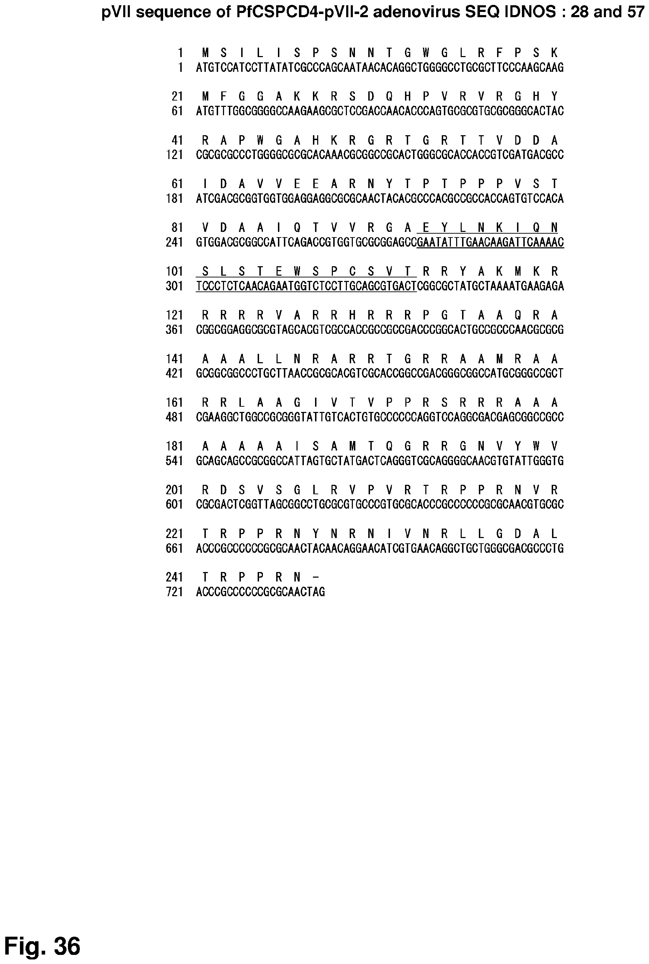

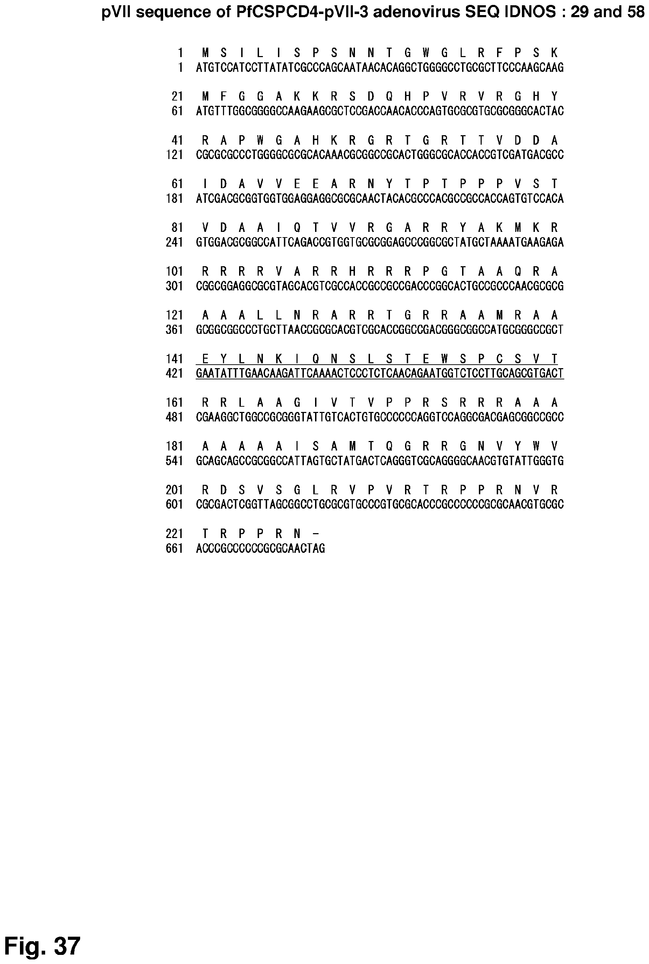

In another embodiment, a T-cell epitope of a *Plasmodium* circumsporozoite protein may be inserted into an adenovirus core pVII protein at any of the following sites: the C-terminus, before the first Nuclear Localization Signal (NLS) or between the two NLS. Alternatively, a T-cell epitope of a *Plasmodium* circumsporozoite protein may replace a portion of the pVII protein. In some embodiments, the modified pVII protein may have a nucleic acid sequence of SEQ ID NO:26 (Fig.34), SEQ ID NO:27 (Fig.35), SEQ ID NO:28 (Fig.36) or SEQ ID NO:29 (Fig.37).

In the recombinant adenovirus may express a transgenic protein or recombinant transgenic protein. In some embodiments, the transgenic protein or recombinant transgenic protein is a *Plasmodium* circumsporozoite protein or an antigenic determinant that is encoded by a recombinant adenovirus plasmid vector as described herein, and is expressed by a recombinant adenovirus produced by said recombinant adenovirus plasmid vector after infection of one or more host cells,

Thus, in some embodiments, the recombinant adenovirus plasmid vectors comprise a nucleotide sequence encoding a recombinant transgenic protein. In one embodiment, the recombinant transgenic protein may comprise an antigenic determinant of *P*. *yoelii,* a rodent-specific parasite, wherein the antigenic determinant comprises a *P*. *yoelii* circumsporozoite (CS) protein gene or an antigenic portion thereof. In another embodiment, the recombinant transgenic protein may comprise an antigenic determinant of *P*. *falciparum,* a human-specific parasite, wherein the antigenic determinant comprises a *P*. *falciparum* circumsporozoite gene (CS) protein or an antigenic portion thereof. The *P*. *falciparum* CS protein has demonstrated prevention of malaria when used as the basis of active immunization in humans against mosquito-borne infection. The antigenic determinant may further comprise an immunogenic epitope, such as a B cell and/or T cell epitope.

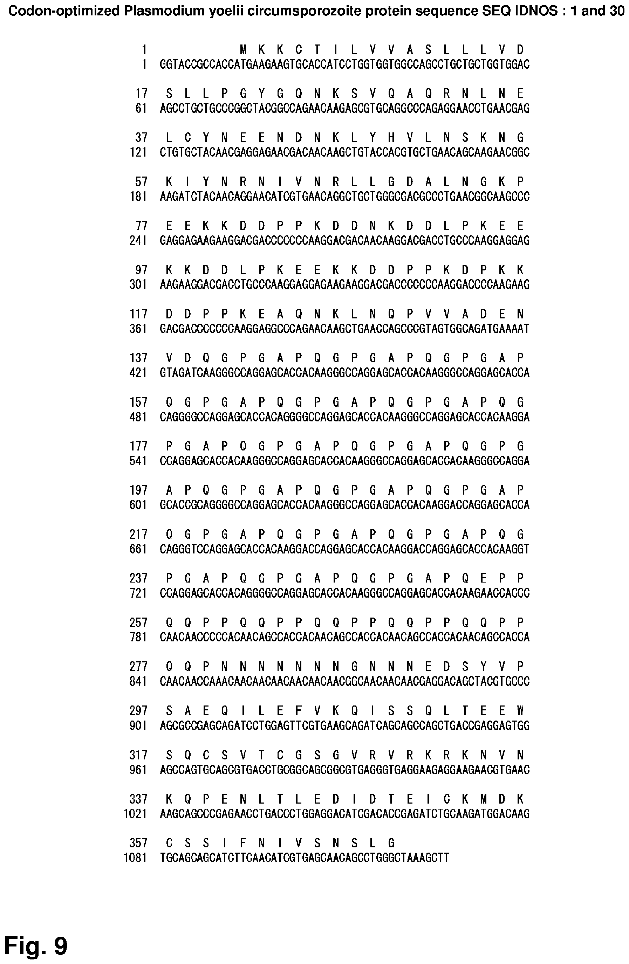

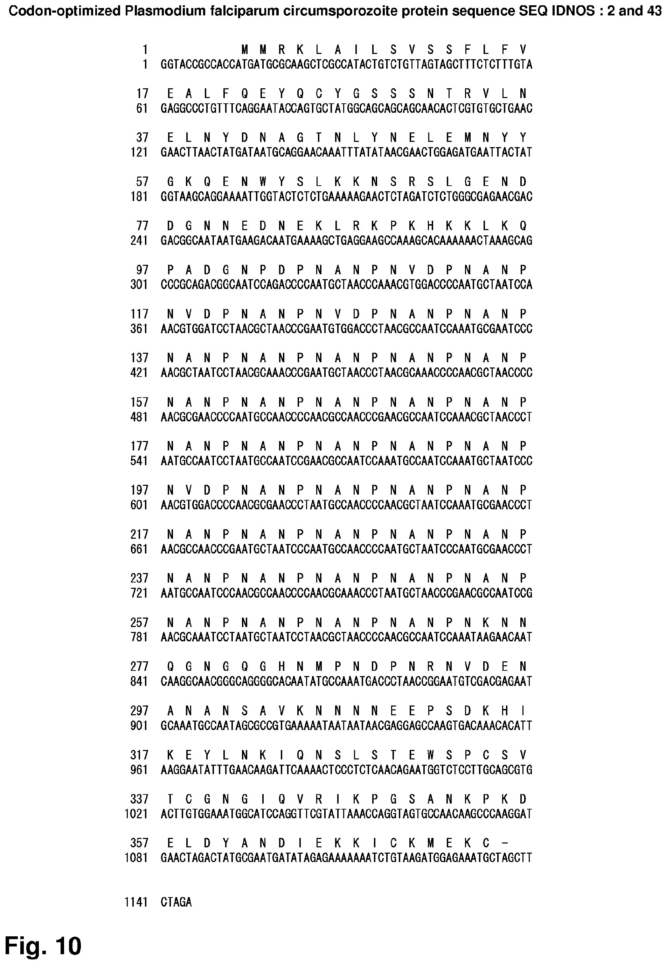

In some embodiments, the CS protein is codon-optimized for enhanced expression in a subject. Codon-optimization is based on the required amino acid content, the general optimal codon usage in the subject of interest as well as any aspects that should be avoided to ensure proper expression. Such aspects may be splice donor or acceptor sites, stop codons, polyadenylation (pA) signals, GC- and AT-rich sequences, internal TATA boxes, or any other aspects known in the art. In some embodiments, the DNA sequence of the codon-optimized CS transgene is shown in Fig. 9 (SEQ ID NO:1, *P. yoelii*) and Fig. 10 (SEQ ID NO: 2, *P. falciparum*).

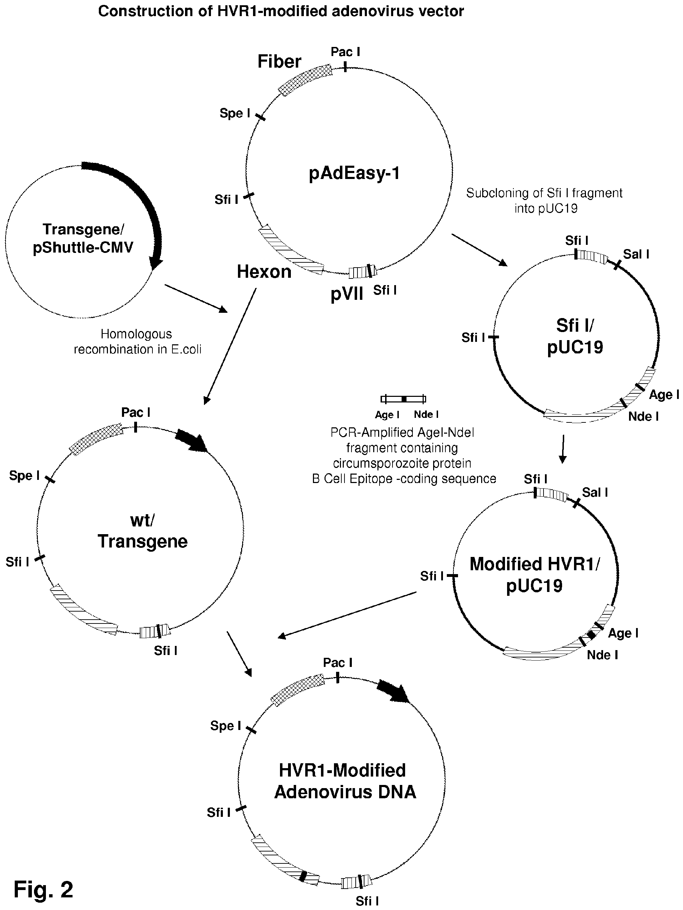

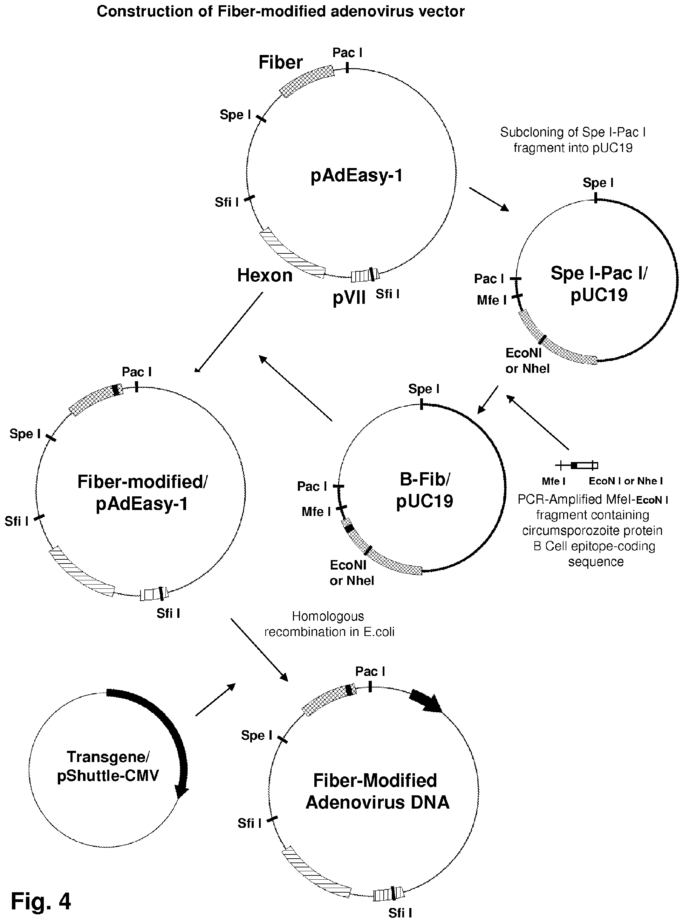

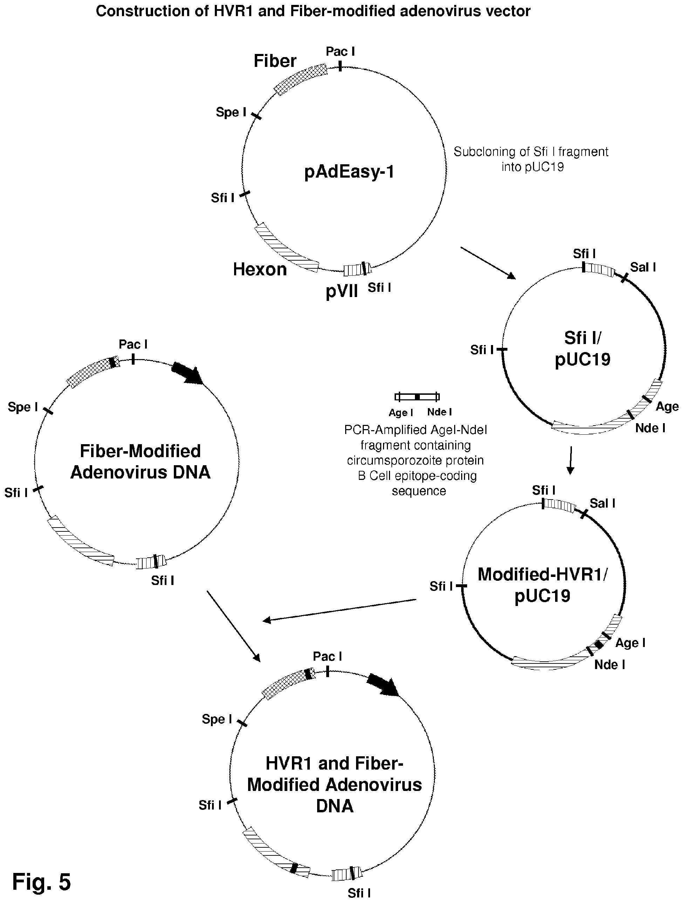

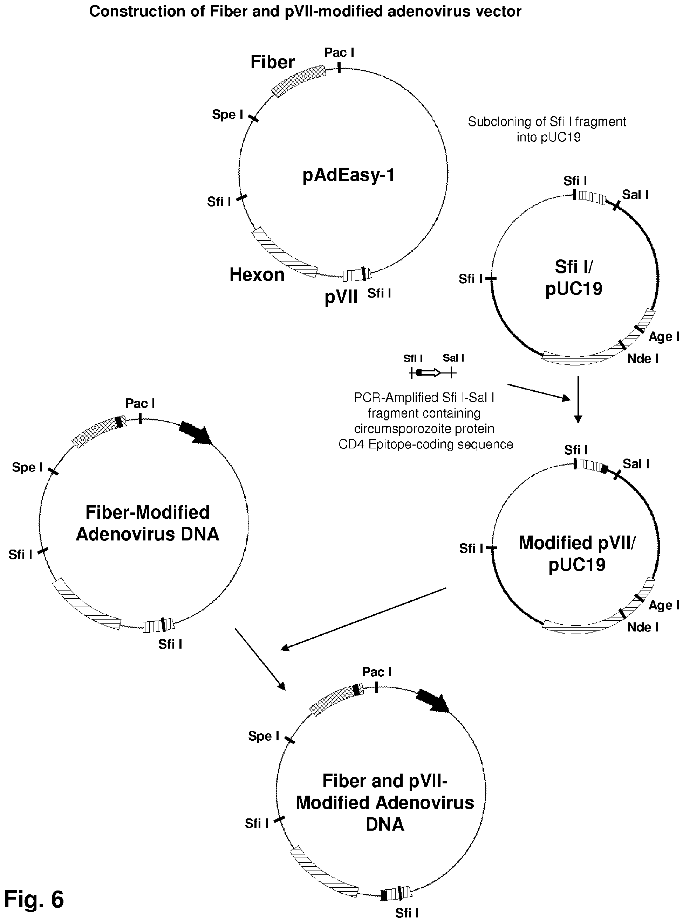

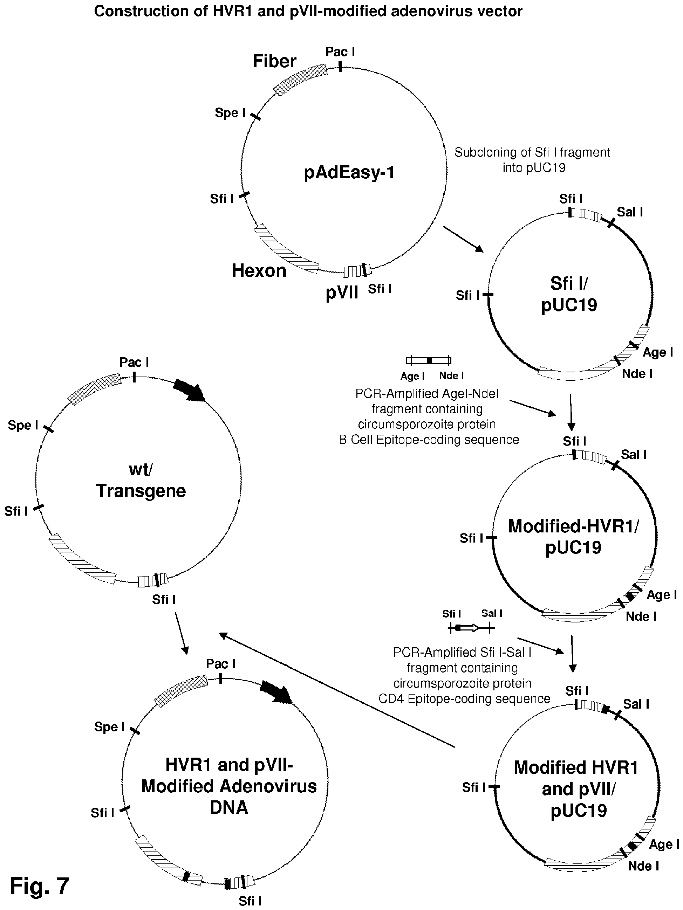

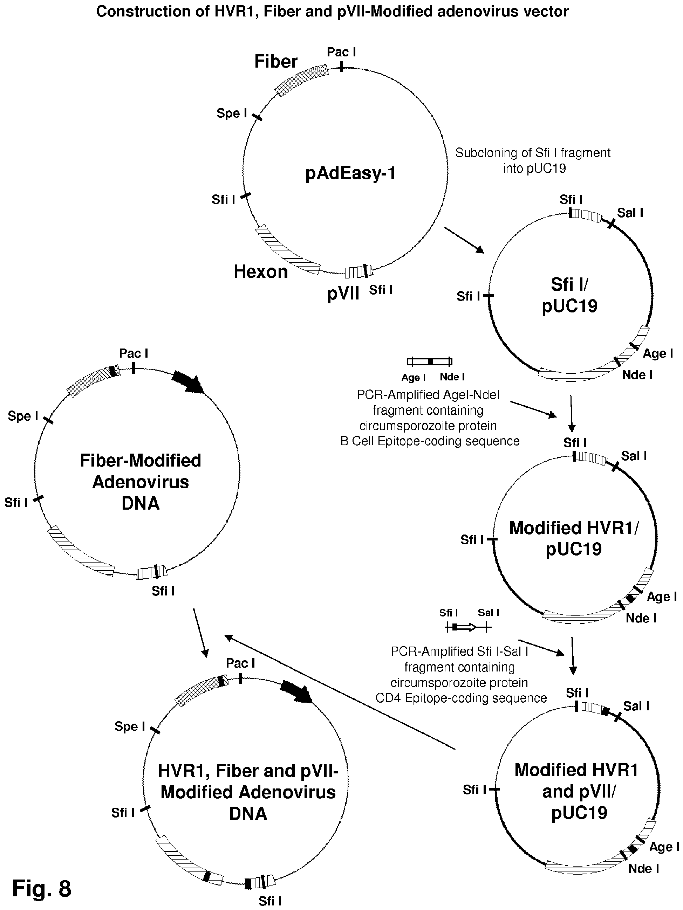

In some embodiments, the recombinant adenovirus plasmid vector may be one of the following modified *P. falciparum* recombinant adenovirus plasmid vectors: HVR1-modified adenovirus vector (NANP-HVR1/PfCSP) constructed as shown in Fig. 2, using a B cell epitope coding sequence of (NANP)ₙ (SEQ ID NO:60); Fiber-modified adenovirus vector (NANP-Fib/PfCSP) constructed as shown in Fig. 4, using a B cell epitope coding sequence of (NANP)ₙ (SEQ ID NO:60); HVR1 and Fiber-modified adenovirus vector (NANP-HVR1/B-Fib/PfCSP) constructed as shown in Fig. 5, using a B cell epitope coding sequence of (NANP)ₙ (SEQ ID NO:60); HVR1 and pVII-modified adenovirus vector (NANP-HVR1/CD4-pVII/PfCSP) constructed as shown in Fig.7, using a B cell epitope of (NANP)ₙ (SEQ ID NO:60) and a CD4 epitope coding sequence of EYLNKIQNSLSTEWSPCSVT(SEQ ID NO:62); Fiber and pVII-modified adenovirus vector (NANP-Fib/CD4-pVII/PfCSP) constructed as shown in Fig. 6, using a B cell epitope of (NANP)ₙ (SEQ ID NO:60) and a CD4 epitope coding sequence of EYLNKIQNSLSTEWSPCSVT(SEQ ID NO:62); and HVR1, Fiber and pVII-modified adenovirus vector (NANP-HVR1/Fib/CD4-pVII/PfCSP) constructed as shown in Fig. 8, using a B cell epitope of (NANP)ₙ (SEQ ID NO:60) and a CD4 epitope coding sequence of EYLNKIQNSLSTEWSPCSVT(SEQ ID NO:62).

In other embodiments, the recombinant adenovirus plasmid vector may be one of the following modified *P. yoelii* recombinant adenovirus plasmid vectors: HVR1-modified adenovirus vector (QGPGAP-HVR1/PyCS) constructed as shown in Fig. 2, using a B cell epitope coding sequence of (QGPGAP)ₙ (SEQ ID NO:59); Fiber-modified adenovirus vector (QGPGAP-Fib/PyCS) constructed as shown in Fig. 4, using a B cell epitope coding sequence of (QGPGAP)ₙ (SEQ ID NO:59); HVR1 and Fiber-modified adenovirus vector (QGPGAP-HVR1/B-Fib/PyCS) constructed as shown in Fig. 5, using a B cell epitope coding sequence of (QGPGAP)ₙ (SEQ ID NO:59); HVR1 and pVII-modified adenovirus vector (QGPGAP-HVR1/CD4-pVII/PyCS) constructed as shown in Fig.7, using a B cell epitope of (QGPGAP)ₙ (SEQ ID NO:59) and a CD4 epitope coding sequence of YNRNIVNRLLGDALNGKPEEK, (SEQ ID NO:61); Fiber and pVII-modified adenovirus vector (QGPGAP-Fib/CD4-pVII/PyCS) constructed as shown in Fig. 6, using a B cell epitope of (QGPGAP)ₙ (SEQ ID NO:59) and a CD4 epitope coding sequence of YNRNIVNRLLGDALNGKPEEK, (SEQ ID NO:61); and HVR1, Fiber and pVII-modified adenovirus vector (QGPGAP-HVR1/Fib/CD4-pVII/PyCS) constructed as shown in Fig. 8, using a B cell epitope of (QGPGAP)ₙ (SEQ ID NO:59) and a CD4 epitope coding sequence of YNRNIVNRLLGDALNGKPEEK, (SEQ ID NO:61).

In other embodiments, a recombinant adenovirus may be produced by one of the following modified *P. falciparum* or *P*. *yoelii* recombinant adenovirus plasmid vectors: NANP-HVR1/PfCSP or QGPGAP-HVR1/PyCS (Fig. 2), NANP-Fib/PfCSP or QGPGAP-Fib/PyCS (Fig. 4), NANP-HVR1/B-Fib/PfCSP or QGPGAP-HVR1/B-Fib/PyCS (Fig. 5), NANP-HVR1/CD4-pVII/PfCSP or QGPGAP-HVR1/CD4-pVII/PyCS (Fig.7), NANP-Fib/CD4-pVII/PfCSP or QGPGAP-Fib/CD4-pVII/PyCS (Fig. 6), NANP-HVR1/Fib/CD4-pVII/PfCSP or QGPGAP-HVR1/Fib/CD4-pVII/PyCS (Fig. 8). The recombinant adenovirus may be produced in accordance with the methods described herein for producing a recombinant adenovirus plasmid vector with the ability to express a recombinant transgenic protein (e.g., *Plasmodium* CS protein) in mammalian host cells.

Purification of a recombinant adenovirus may be performed by using known virus purification methods. For example, purification of 0.5 to 1.0 mL of a stock virus obtained by the method of producing a recombinant adenovirus protein by inoculating insect cells (1 x 10⁷ cells/10 cm dish), such as AD293 cells. The culture supernatant is then collected several days after the infection, and a virus pellet obtained by centrifugation is suspended in a buffer, such as PBS (Phosphate Buffered Saline). The resulting suspension is subjected to a sucrose gradient of 10 to 60% and then centrifuged (25,000 rpm for 60 minutes at 4°C) to collect a virus band. The collected virus is further suspended in PBS, subsequently centrifuged under the same conditions as above, and the resulting purified recombinant virus pellet is stored at 4°C in a buffer, such as PBS.

Another embodiment is directed to a pharmaceutical composition essentially comprising at least one active ingredient. In one embodiment, an active ingredient of the pharmaceutical composition may comprise a recombinant adenovirus, which may be obtained by the genetic engineering techniques described herein. More specifically, the active ingredient may be a recombinant adenovirus comprising modified capsid and/or core proteins, wherein a portion of a Hexon hypervariable region (HVR), a portion of Fiber protein, a portion of pVII protein or a combination thereof is replaced by at least one immunogenic epitope of *Plasmodium* circumsporozoite protein. Alternatively, one or more B-cell and/or T cell epitope of a *Plasmodium* circumsporozoite protein may be inserted in the Fiber protein, Hexon HVR or pVII protein. The recombinant adenovirus plasmid vector further comprises a transgenic protein or recombinant transgenic protein that is expressed by the recombinant adenovirus after infecting one or more host cells. The transgenic protein or recombinant transgenic protein may be a *Plasmodium* circumsporozoite protein or a malaria antigen of a *Plasmodium* circumsporozoite protein, wherein the malaria antigen comprises at least one immunogenic epitope (e.g., a B cell or T cell epitope) of *Plasmodium* circumsporozoite protein.

In some embodiments, the active ingredient of the pharmaceutical composition is a recombinant adenovirus derived from a recombinant adenovirus plasmid vector, wherein the recombinant adenovirus plasmid vector is one of the following modified *P. falciparum* or *P*. *yoelii* recombinant adenovirus plasmid vectors: NANP-HVR1/PfCSP or QGPGAP-HVR1/PyCS (Fig. 2), NANP-Fib/PfCSP or QGPGAP-Fib/PyCS (Fig. 4), NANP-HVR1/B-Fib/PfCSP or QGPGAP-HVR1/B-Fib/PyCS (Fig. 5), NANP-HVR1/CD4-pVII/PfCSP or QGPGAP-HVR1/CD4-pVII/PyCS (Fig.7), NANP-Fib/CD4-pVII/PfCSP or QGPGAP-Fib/CD4-pVII/PyCS (Fig. 6), NANP-HVR1/Fib/CD4-pVII/PfCSP or QGPGAP-HVR1/Fib/CD4-pVII/PyCS (Fig. 8). These recombinant adenovirus plasmid vectors are capable of producing recombinant adenoviruses when transfected into cells (e.g., AD293 cells) and wherein the recombinant transgenic protein may be expressed in mammalian cells, including human cells.

. When given to a subject, a pharmaceutical composition having an active ingredient is a recombinant adenovirus as described herein enhances malaria infection-preventing effects against a malaria infectious antigen and reduces the infectivity titer, as described further in the Examples below. Thus, the recombinant adenovirus may be used for the treatment of malaria infections associated with infection of target cells and tissues. Examples of target cells affected by such malaria infection include blood cells, hepatic cells, renal cells, brain cells, lung cells, epithelial cells, and muscular cells. Examples of tissues comprising such cells include the lung, liver, kidney, brain, arteries and veins, the stomach, intestines, urethra, skin, and muscle.

In some aspects, the pharmaceutical composition may enhance malaria infection- preventing effects against infectious antigens, for example, malaria antigens such as sporozoite surface antigens (Circumsporozoite Protein (CSP) and Thrombospondin Related Adhesive Protein (TRAP)) of malaria parasites, merozoite surface membrane protein (MSPI), malaria S antigen secreted from erythrocytes infected with malaria, and *P. falciparum* Erythrocyte Membrane Protein-1 (PfEMPI) protein present in the knobs of erythrocytes infected with malaria. The pharmaceutical composition may enhance malaria infection-preventing effects against a *Plasmodium* parasite, selected from any known *Plasmodium* (*P*) species, for example, *P. falciparum, P. malariae, P. ovale, P. vivax, P. knowlesi, P. berghei, P. chabaudi* and *P. yoelii,* by reducing the infectivity titer. When administered to a subject, a reduction of the infectivity titer by the pharmaceutical composition may result in an increased survival, disease-free survival, or infection-free survival period and survival, disease-free survival, or infection-free survival rate when compared to subjects not administered the pharmaceutical composition. Thus, in some aspects, the pharmaceutical composition is useful as a preventive or therapeutic agent for malaria infections caused by pathogens such as *Plasmodium.* In further aspects, the pharmaceutical composition is useful as a preventive or therapeutic agent for complications resulting from a malaria infection caused by pathogens such as *Plasmodium.*

The infection-preventing effect of the recombinant adenovirus of the present invention in a subject can be provided, for example, by administering the pharmaceutical composition containing the capsid-modified recombinant adenovirus of the present invention and additives for pharmaceutical administration to vertebrates, particularly mammals, including humans, by intramuscular (i.m.), subcutaneous (s.c.), intracutaneous (i.c.), intradermal (i.d.), intraperitoneal (i.p.), nasal, or respiratory route, and then immunizing the vertebrates with the pharmaceutical composition containing the recombinant adenovirus described herein as an active ingredient several times. To evaluate the infection-preventing effect, the survival rate, disease-free survival, or infection-free survival of subjects immunized with the pharmaceutical composition several times followed by infection by a target pathogen (such as a selected *Plasmodium* species) may be compared with the survival rate, disease-free survival, or infection-free survival of subjects not given the pharmaceutical composition.

In some embodiments, the pharmaceutical composition may additionally comprise a pharmaceutically effective amount of capsid and/or core-modified recombinant adenovirus as described herein and a pharmaceutically acceptable carrier, which is described further below.

Another embodiment is directed to a vaccine composition essentially comprising at least one active ingredient. In one embodiment, an active ingredient of the vaccine composition may comprise a recombinant adenovirus, derived from a recombinant adenovirus plasmid vector as described herein. More specifically, the active ingredient may be a recombinant adenovirus comprising modified capsid or core proteins, wherein a portion of a Hexon hypervariable region (HVR), a portion of Fiber protein, a portion of pVII protein or a combination thereof are replaced by at least one immunogenic epitope of *Plasmodium* circumsporozoite protein. Alternatively, at least one immunogenic epitope of a *Plasmodium* circumsporozoite protein may be inserted in the pVII protein, Fiber protein or Hexon HVR, or a combination thereof. In some embodiments, the active ingredient of the vaccine composition may be derived from a recombinant adenovirus plasmid vector illustrated in Figs. 2-8, for example, NANP-HVR1/PfCSP or QGPGAP-HVR1/PyCS (Fig. 2), NANP-Fib/PfCSP or QGPGAP-Fib/PyCS (Fig. 4), NANP-HVR1/B-Fib/PfCSP or QGPGAP-HVR1/B-Fib/PyCS (Fig. 5), NANP-HVR1/CD4-pVII/PfCSP or QGPGAP-HVR1/CD4-pVII/PyCS (Fig.7), NANP-Fib/CD4-pVII/PfCSP or QGPGAP-Fib/CD4-pVII/PyCS (Fig. 6), NANP-HVR1/Fib/CD4-pVII/PfCSP or QGPGAP-HVR1/Fib/CD4-pVII/PyCS (Fig. 8).

In some aspects, the vaccine composition, when administered to a subject, first comprises a recombinant adenovirus having one or more antigenic portions of a *Plasmodium* CS protein (i.e., a B cell epitope, T cell epitope or both) inserted into or replacing at least a part of a capsid or core protein. The vaccine composition may then express a recombinant transgenic protein, wherein the recombinant transgenic protein is a *Plasmodium* CS protein comprising a B cell epitope, T cell epitope or both. The antigenic portions of the *Plasmodium* CS protein are found in the recombinant transgenic protein and the modified capsid or core proteins promote or enhance acquired humoral immunity, cellular immunity, or both as described in the Examples below. Thus, in some aspects, the recombinant adenovirus as described herein is useful as a vaccine to promote or enhance humoral immunity, cellular immunity, or both.

In further embodiments, the vaccine composition may enhance infection-preventing effects against infectious antigens, for example, malaria antigens such as sporozoite surface antigens (CSP and TRAP) of malaria parasites, merozoite surface membrane protein MSPI, malaria S antigen secreted from erythrocytes infected with malaria, PfEMPI protein present in the knobs of erythrocytes infected with malaria, Serine-Rich Antigen (SERA) protein, Tyrosine-Rich Acidic Matrix Protein (TRAMP), and Apical Membrane Antigen-1 (AMAI) protein. Further, a reduced infectivity titer (e.g., the viral infectivity titer) resulting from administration of a vaccine composition described herein may result in an increased survival, disease-free survival or infection-free survival period and survival, disease-free survival or infection-free survival rate when compared to subjects not administered the vaccine composition. Thus, in some aspects, the vaccine composition is also useful as a preventive or therapeutic agent for malaria infections caused by pathogens such as *Plasmodium.* In further aspects, the vaccine composition is also useful as a preventive or therapeutic agent for complications resulting from a malaria infection by pathogens such as *Plasmodium.*

A vaccine composition as described herein may comprise a therapeutically effective amount of a recombinant adenovirus as described herein, and further comprising a pharmaceutically acceptable carrier according to a standard method. Examples of acceptable carriers include physiologically acceptable solutions, such as sterile saline and sterile buffered saline.

In some embodiments, the vaccine or pharmaceutical composition may be used in combination with a pharmaceutically effective amount of an adjuvant to enhance the anti-malaria effects. Any immunologic adjuvant that may stimulate the immune system and increase the response to a vaccine, without having any specific antigenic effect itself may be used as the adjuvant. Many immunologic adjuvants mimic evolutionarily conserved molecules known as pathogen-associated molecular patterns (PAMPs) and are recognized by a set of immune receptors known as Toll-like Receptors (TLRs). Examples of adjuvants that may be used in accordance with the embodiments described herein include Freund's complete adjuvant, Freund's incomplete adjuvant, double stranded RNA (a TLR3 ligand), LPS, LPS analogs such as monophosphoryl lipid A (MPL) (a TLR4 ligand), flagellin (a TLR5 ligand), lipoproteins, lipopeptides, single stranded RNA, single stranded DNA, imidazoquinolin analogs (TLR7 and TLR8 ligands), CpG DNA (a TLR9 ligand), Ribi's adjuvant (monophosphoryl-lipid A/trehalose dicorynoycolate), glycolipids (α-GalCer analogs), unmethylated CpG islands, oil emulsion, liposomes, virosomes, saponins (active fractions of saponin such as QS21), muramyl dipeptide, alum, aluminum hydroxide, squalene, BCG, cytokines such as GM-CSF and IL-12, chemokines such as MIP 1-a and RANTES, N-acetylmuramine-L-alanyl-D-isoglutamine (MDP), thymosin al and MF59. The amount of adjuvant used can be suitably selected according to the degree of symptoms, such as softening of the skin, pain, erythema, fever, headache, and muscular pain, which might be expressed as part of the immune response in humans or animals after the administration of this type of vaccine.

In some embodiments, the vaccine or pharmaceutical composition described herein may be used in combination with other known pharmaceutical products, such as immune response-promoting peptides and antibacterial agents (synthetic antibacterial agents). The vaccine or pharmaceutical composition may further comprise other drugs and additives. Examples of drugs or additives that may be used in conjunction with a vaccine or pharmaceutical composition described herein include drugs that aid intracellular uptake of the recombinant adenovirus or recombinant transgenic protein of the present invention, liposome and other drugs and/or additives that facilitate transfection, (e.g., fluorocarbon emulsifiers, cochleates, tubules, golden particles, biodegradable microspheres, and cationic polymers).

In some embodiments, the amount of the active ingredient contained in the vaccine or pharmaceutical composition described herein may be selected from a wide range of concentrations, Virus Particle Unit (VPU), Plaque Forming Unit (PFU), weight to volume percent (w/v %) or other quantitative measure of active ingredient amount, as long as it is a therapeutically or pharmaceutically effective amount. The dosage of the vaccine or pharmaceutical composition may be appropriately selected from a wide range according to the desired therapeutic effect, the administration method (administration route), the therapeutic period, the patient's age, gender, and other conditions, etc.

In some aspects, when a recombinant adenovirus is administered to a human subject as an active ingredient of the vaccine or pharmaceutical composition, the dosage of the recombinant adenovirus may be administered in an amount approximately corresponding to 10² to 10¹⁴ PFU, preferably 10⁵ to 10¹² PFU, and more preferably 10⁶ to 10¹⁰ PFU per patient, calculated as the PFU of the recombinant virus.

In further aspects, when a recombinant adenovirus is administered to a subject as an active ingredient of the vaccine or pharmaceutical composition, the dosage may be selected from a wide range in terms of the amount of expressible DNA introduced into the vaccine host or the amount of transcribed RNA. The dosage also depends on the strength of the transcription and translation promoters used in any transfer vectors used.

In some embodiments, the vaccine composition or pharmaceutical composition described herein may be administered by directly injecting a recombinant adenovirus suspension prepared by suspending the recombinant adenovirus in PBS (phosphate buffered saline) or saline into a local site (e.g., into the lung tissue, liver, muscle or brain), by nasal or respiratory inhalation, or by intravascular (i.v.) (e.g., intraarterial, intravenous, and portal venous), subcutaneous (s.c.), intracutaneous (i.c.), intradermal (i.d.), or intraperitoneal (i.p.) administration. The vaccine or pharmaceutical composition of the present invention may be administered more than once. More specifically, after the initial administration, one or more additional vaccinations may be given as a booster. One or more booster administrations can enhance the desired effect. After the administration of the vaccine or pharmaceutical composition, booster immunization with a pharmaceutical composition containing the recombinant adenovirus as described herein may be performed.

In further embodiments, use of various other adjuvants, drugs or additives with the vaccine of the invention, as discussed above, may enhance the therapeutic effect achieved by the administration of the vaccine or pharmaceutical composition. The pharmaceutically acceptable carrier may contain a trace amount of additives, such as substances that enhance the isotonicity and chemical stability. Such additives should be non-toxic to a human or other mammalian subject in the dosage and concentration used, and examples thereof include buffers such as phosphoric acid, citric acid, succinic acid, acetic acid, and other organic acids, and salts thereof; antioxidants such as ascorbic acid; low molecular weight (e.g., less than about 10 residues) polypeptides (e.g., polyarginine and tripeptide) proteins (e.g., serum albumin, gelatin, and immunoglobulin); amino acids (e.g., glycine, glutamic acid, aspartic acid, and arginine) ; monosaccharides, disaccharides, and other carbohydrates (e.g., cellulose and derivatives thereof, glucose, mannose, and dextrin), chelating agents (e.g., EDTA); sugar alcohols (e.g., mannitol and sorbitol); counterions (e.g., sodium); nonionic surfactants (e.g., polysorbate and poloxamer) ; and PEG.

The vaccine or pharmaceutical composition containing a recombinant adenovirus described herein may be stored as an aqueous solution or a lyophilized product in a unit or multiple dose container such as a sealed ampoule or a vial.

Another embodiment further provides a method of preventing malaria infection, or a method of treating malaria comprising administering an effective amount of the recombinant adenoviral vaccine, formulation, or pharmaceutical composition. The present invention further provides a method of immunostimulation comprising administering an effective amount of a recombinant adenoviral vaccine composition, formulation, pharmaceutical composition or a combination thereof to a subject. Subjects may include humans, animals (such as mammals, birds, reptiles, fish, and amphibians), or any other subjects that may become infected with a malaria parasite. Malaria parasites may include a *Plasmodium* parasite, selected from any of known *Plasmodium* (*P*) species, for example, *P*. *falciparum, P. malariae, P. ovale, P. vivax, P. knowlesi, P. berghei, P. chabaudi* and *P. yoelii.*

In some embodiments, a recombinant adenovirus as described herein may be formed alone or may be together with a pharmaceutically acceptable carrier into a vaccine composition, formulation, or pharmaceutical composition, and administered to the subject. The administration route may be, for example, any administration route mentioned above. The pharmaceutically acceptable carrier for use in the present invention can be suitably selected from carriers commonly used in this technical field, according to the form of the pharmaceutical composition to be produced. For example, when the pharmacological composition is formed into an aqueous solution, purified water (sterile water) or a physiological buffer solution can be used as the carrier. When the pharmaceutical composition is formed into other appropriate solutions, organic esters capable of being injected, such as glycol, glycerol and olive oil may be used as the carrier. The composition may contain stabilizers, excipients and other commonly used substances in this technical field, and particularly in the field of vaccine formulations.

In further embodiments, the amount of recombinant adenovirus used in a vaccine composition, formulation, or pharmaceutical composition may be suitably selected from a wide range of concentrations, VPU, PFU, weight to volume percent (w/v %) or other quantitative measure of active ingredient amount. In some aspects, a suitable range of recombinant adenovirus in the composition is preferably about 0.0002 to about 0.2 (w/v %), and more preferably 0.001 to 0.1 (w/v %). The method of administration of a recombinant adenovirus vaccine composition, formulation, or pharmaceutical composition according to some embodiments may be suitably selected according to the dosage form, the patient's age, gender and other conditions such as the severity of the disease. A suitable dosage form is a form for parenteral administration, such as injections, drops, nasal drops, and inhalants. When the composition is formed into an injection or drops, the injection can be intravenously administered and mixed with a replacement fluid such as a glucose solution or an amino acid solution as appropriate, or can be administered intramuscularly (i.m.), intracutaneously (i.c.), subcutaneously (s.c.) intradermally (i.d.), or intraperitoneally (i.p.).

In other embodiments, the daily dosage of a recombinant adenovirus vaccine composition, formulation, or pharmaceutical composition may vary depending on the subject's condition, body weight, age, gender, etc. In some aspects, the dosage of a recombinant adenovirus is administered in an amount of approximately 0.001 to 100 mg per kg of body weight per day. The vaccine, formulation, or composition of the invention may be administered in one or more administrations per day.

In further embodiments, when a recombinant adenovirus is administered to a human subject as an active ingredient of the vaccine composition, formulation or pharmaceutical composition, the dosage of the recombinant adenovirus is administered in an amount approximately corresponding to 10² to 10¹⁴ PFU, preferably 10⁵ to 10¹² PFU, and more preferably 10⁶ to 10¹⁰ PFU per patient, calculated as the PFU of the recombinant adenovirus particle. The vaccine composition of the present invention should be administered according to Good Medical Practice, considering the clinical condition (for example, the condition to be prevented or treated) of each patient, the delivery site of the vaccine composition containing the recombinant adenovirus, the target tissue, the administration method, the dosage regimen, and other factors known to those skilled in the art. Therefore, the proper dosage of the vaccine composition herein is determined in consideration of the above.

Yet another embodiment of the disclosure relates to a method of treating or preventing a malaria infection in a subject, the method comprising administering an immunologic or therapeutic amount of a malaria vaccine composition comprising a recombinant adenovirus. The recombinant adenovirus of the malaria vaccine may comprise an antigenic determinant of a *Plasmodium* parasite, and may further comprise one or more modified capsid or core proteins. An immunologic, pharmacologic or therapeutic amount may be any suitable amount wherein a potent immune response is generated against one or more antigenic portions of the (CS) protein (i.e., the transgene, B cell epitope, or CD4+ T cell epitope) such that malarial infection is prevented or reduced in severity.

When a subject is first exposed or "primed" to an adenovirus vector, the immune system produces neutralizing antibodies against that specific vector. The immune response to the adenovirus is generally directed against the capsid proteins. Therefore, subsequent exposure to the same adenovirus vector, or "boosts," can reduce the efficacy of transgene expression. Therefore, in some embodiments, the method of treating or preventing a malaria infection described above may comprise a priming step using a first recombinant adenovirus vector followed by one or more boosting steps using one or more different recombinant adenovirus vectors. This method may be used in subjects that have not yet been exposed to a wild-type adenovirus, or in a subject that has been previously exposed to a wild-type adenovirus vector, wherein the priming step recombinant adenovirus vector is used to circumvent existing adenovirus immunity. Further embodiments and examples are described below.

### Adenovirus as a vector

Adenoviruses are non-enveloped DNA viruses comprising a set of viral capsid proteins (described below) and a viral genome, that have been widely used to deliver one or more therapeutic or antigenic transgene to a variety of cells *in vitro* and *in vivo.* Many adenovirus serotypes exist. Of the known adenovirus serotypes, serotype 5 (Ad5) is preferably used as a vector for foreign gene transduction because of its strong infectivity *in vivo* (Abbink et al. 2007). Expression of the antigenic transgene may be controlled by any promoter or enhancer element known in the art. Promoters which may be used to control gene expression include, but are not limited to, cytomegalovirus immediate early promoter (CMV), simian virus 40 (SV40) early promoter, cellular polypeptide chain elongation factor 1 alpha (EF1) promoter, Rous sarcoma virus (RSV) promoter, and tetracycline-regulated (TR) promoter. A polyadenylation (pA) signal after the coding sequence may also be used for efficient transcription and translation. The recombinant adenovirus vector described herein may be replication-defective, having a deletion at least in the E1 region of the adenoviral genome, since the E1 region is required for replication, transcription, translation and packaging processes. In some aspects, the E2, E3 and/or E4 regions may also be deleted. In further aspects, a Kozak consensus sequence may be used for a more efficient translation (Kozak 1987).

The adenovirus (Ad) system is an attractive vector for the development of recombinant vaccines for a number of reasons. One reason is that recombinant adenoviral vectors infect most mammalian cell types (both replicative and non-replicative), including, but not limited to, mouse and human cell types. Thus, the same vector may be used successfully in mouse models and human clinical trials alike. Another reason is that any transferred genetic information remains epichromosomal, avoiding insertional mutagenesis and alteration of the cellular genotype (Crystal 1995). Yet another reason is that the transgene remains unaltered after successive rounds of viral replication. Other advantages of using adenovirus include that recombinant adenovirus: 1) has a high virion stability, 2) is well tolerated, 3) may be grown at high titer, 4) can accommodate large transgenes, 5) has a genome that has been extensively studied for many years such that the complete DNA sequence of several serotypes is known, facilitating the manipulation of the Ad genome by recombinant DNA techniques (Graham and Prevec 1992).

In one embodiment, the adenovirus vaccine platform is used as a viral vector for development of a vaccine that targets a pre-erythrocytic malaria parasite, and provides protection from malaria infection. Among known recombinant viral vectors (Rodrigues et al. 1997, Bruña-Romero et al. 2001, Anderson et al. 2004, Tao et al. 2005), adenovirus has been shown to be a suitable viral vector for a malaria vaccine because it can induce a strong protective cellular immune response to pre-erythrocytic malaria parasites (Rodrigues et al. 1997). The malaria parasite may be any one of the *Plasmodium* family. In some embodiments, the targeted parasite may be *P. yoelii* or *P*. *falciparum.*

### Adenovirus vectors expressing PyCS as a transgene elicits a malaria-specific CD8+ T cell response

Adenovirus is an attractive vector for inducing a significant CD8+ T cell-mediated protective immunity against malaria (Rodrigues et al. 1997, Rodrigues et al. 1998). The immunogenicity of a recombinant adenovirus expressing the *P. yoelii* (a rodent malaria parasite) CS protein, AdPyCS, was determined using a rodent malaria model. The inoculation of mice with AdPyCS induces complete immunity in a significant proportion of mice, preventing the occurrence of parasitemia (Rodrigues et al. 1997). This protective effect is primarily mediated by CD8+ T cells, as evidenced by depletion of the T cell population and is corroborated by the fact that AdPyCS was unable to induce high titers of antibody response against malaria parasites.

To quantitatively measure the infectivity of capsid-modified adenovirus, the shuttle vector may contain a GFP expression cassette and cloning sites for a transgene. The resulting shuttle vector (GFP/pShuttle-CMV) has dual pCMV promoters and SV40pAs for a transgene and GFP from pmaxGFP (Amaxa, Germany). The optimized PyCS fragment was inserted into Kpnl and Hindlll sites of GFP/pShuttle-CMV.

The immunogenicity of Ad(PyCS+GFP) was determined by measuring the magnitude of the CS-specific CD8+ T cell response and the level of protective immunity against the plasmodial liver stages. Administration of Ad(PyCS+GFP) via different routes, at an optimal dose, 10¹⁰ viral particle (v.p.) elicited the same pattern of anti-malarial protective responses that AdPyCS was shown to elicit, with the s.c. and i.m. routes inducing the strongest response resulted in the highest degree of liver stage inhibition in mice challenged with live *P. yoelii* sporozoites. This illustrates that as a vaccine, Ad(PyCS+GFP) behaves equivalently to AdPyCS (Rodrigues et al 1997), and is a potentially useful tool in determining the *in vivo* tropism of AdPyCS.

### Adenovirus capsid and core proteins

The studies above confirm that recombinant adenoviral vectors expressing a CS protein elicit a strong cellular immune response by CD8+ T cells, but no appreciable humoral response. Therefore, because the humoral response to wild-type adenovirus can often be attributed to capsid proteins, recombinant adenoviral vectors with modified capsid and core proteins were constructed to 1) enhance humoral immunity via B cell activation, 2) enhance humoral immunity via T helper cell activation, and 3) circumvent existing adenoviral immunity.

Adenovirus is a non-enveloped naked double stranded DNA virus with an icosahedral shape, having 20 faces of equilateral triangles. The adenovirus capsid consists of 252 capsomers, of which 240 are Hexon trimers and 12 are penton pentamers. A Fiber protein, which projects from each penton base, mediates attachment to host cells by interaction with the cellular receptor. A secondary interaction occurs between the RGD (Asp-Arg-Gly) motif in the penton base with αvβ3, αvβ5 and similar integrins, facilitating subsequent internalization of adenovirus into the cell (Mathias et al. 1994, Wickham et al. 1993). Most of the adenovirus use the coxsackie-adenovirus receptor, CAR, as a cellular receptor (Bergelson et al. 1997). In addition, MHC class I molecules, VCAM, and heparan sulfate, are shown to mediate attachment and entry of Ad5 (Chu et al. 2001, Hong et al. 1997). Following entry via endocytosis, the Ad5 rapidly escapes from endocytic compartments into the cytosol (Meier and Greber 2003, Leopold and Crystal 2007). The virion then translocates to the nucleus using microtubules. The Fiber protein is shed as the earliest capsid protein in the process (Nakano et al. 2000, Hong et al 2003). Adenoviruses of different serotypes demonstrate different trafficking patterns (Miyazawa et al. 1999, Miyazawa et al. 2001). Changing or modifying the Fiber protein can impact trafficking, which may be particularly important with regard to antigen processing and presentation, following infection of antigen presenting cells (APC).

The adenovirus Fiber is a trimer divided into Fiber tail, shaft and knob domains (Henry et al. 1994, Rux and Burnett 2004, Chroboczek et al. 1995). The three dimensional structure of the knob domain is known, and together with mutagenesis studies, these studies allow the areas involved in CAR interaction and trimerization to be visualized (Kirby et al. 1999, Xia et al. 1995). The Fiber shaft projects from the virion and the Fiber knob contains the Coxsackie and Adenovirus Receptor (CAR) interaction domain (Roelvink et al. 1999, Bewley et al. 1999). The CAR-binding site of the Fiber knob consists primarily of residues from the AB loop and CD loop and extends secondarily to the FG and HI loop and the B, E and F β sheets (Roelvink et al. 1999, Bewley et al. 1999). The HI loop has been the best studied insertion site on the Fiber knob (Worgall et al. 2004, Mizuguchi and Hayakawa 2004, Koizumi et al. 2003, Belousova et al. 2002, Noureddini and Curiel 2005, Nicklin et al. 2001), and incorporation of an epitope into the HI loop (residue 543 and 544) resulted in potent anti-epitope immunity (Krause et al. 2006). Therefore, an immunodominant CS-derived B cell epitope was initially inserted into the HI loop of the Fiber protein.

Hexon is the most abundant protein of the adenovirus capsid with 720 copies per virion. In the mature virus, Hexon exists as homotrimeric capsomeres which make up the facets of the icosahedral virion (Rux and Burnett 2004). The crystal structures of adenovirus serotypes 2 and 5 (Ad2 and Ad5) Hexons have been solved, revealing a complex molecular architecture (Athapilly et al. 1994, Roberts et al. 1986, Rux and Burnett 2000). The base of each monomeric subunit consists of two beta-barrel motifs that are present in the capsid proteins of many icosahedral viruses. Three long loops (DE1, FG1, and FG2) extend out from the base structure to form the tower region of each molecule (Rux and Burnett 2004). Sequences within these loop domains protrude to the surface of the capsid to form the exterior of the virion. Alignments from different adenovirus serotypes show that the sequences located on the capsid exterior are poorly conserved in both length and amino acid sequence (Crawford-Miksza and Schnurr 1996). Furthermore, it has been shown that the sequences located in these poorly conserved domains, termed hypervariable regions (HVRs), contain the determinants against which serotype-specific antibodies are produced (Top 1975, Rux and Burnett 2000, Top et al. 1971).

Based on early sequence alignments, seven HVRs were identified throughout the Hexon molecule (Crawford-Miksza and Schnurr 1996, Roberts et al 2006). Because the HVRs are poorly conserved between serotypes and do not appear to be involved in maintaining the structural integrity of Hexon, small changes could be made to these domains without affecting the viability of the virus (Rux and Burnett 2000). For example a hexahistidine tag can be inserted into HVR2, HVR3, HVR5, HVR6, and HVR7 without compromising virus viability (Wu et al. 2005). Thus, Hexon HVRs are often used as targets to efficiently induce an antibody response against peptides located in Hexon HVRs (Worgall et al. 2005, Crompton et al. 1994). Due to its poor conservation in length between serotypes and its position on the outermost surface of the adenovirus capsid (Rux and Burnett 2000, Crawford-Miksza and Schnurr 1996), Hexon HVR5 was initially chosen as a site for epitope insertion. Further, the crystal structure of Hexon indicates that HVR5 is a flexible loop on the capsid surface, suggesting that HVR5 can accommodate relatively large peptides without compromising the structural integrity of the capsid (Roberts et al. 1986). Hexon-specific CD4+ and CD8+ epitopes have recently been identified (Leen et al. 2008), and the CD4+ T cell response to adenovirus is focused against conserved residues within the Hexon protein in humans (Onion et al. 2007, Heemskerk et al. 2006).

The adenovirus core is composed of the viral genome and four core proteins. The terminal protein (TP) is covalently linked to the 5' end of each linear viral DNA strand at two copies per virion. Noncovalently and nonspecifically bound to the viral DNA through arginine-rich portions are three other core proteins mu (µ), V (pV) and VII (pVII). pVII is the major core protein contributing roughly 700-800 copies per virion, and serves as a histone-like center around which viral DNA is wrapped to form nucleosome structures.

### Modification of adenovirus capsid proteins to enhance humoral immunity

In some embodiments, circumsporozoite (CS) adenoviral vectors that have an immunodominant CS protein B epitope in an adenovirus capsid protein (inserted in the Hexon or Fiber) are described. The transgene may be under a promoter such as CMV to augment cell-mediated and humoral immune responses to CS protein.

A central repeat region is the conserved structure of CS protein among *Plasmodium* species, and antibody against this repeat sequence has been shown to have sporozoite neutralizing activity. Examples of a repeat sequence in *Plasmodium* CS protein are (NANP)ₙ repeat (*P. falciparum;* SEQ ID NO:60), ANGAGNQPG repeat (*P. vivax;* SEQ ID NO:63) and NAAG repeat (*P. malariae;* SEQ ID NO:64), which can be inserted into adenovirus capsid proteins. In some embodiments, four or more (NANP)ₙ repeats (SEQ ID NO:60) of PfCSP may be inserted into HVR1 of adenovirus serotype 5 Hexon. In some embodiments, two, four, six, eight, ten, fourteen, sixteen, eighteen, twenty, twenty-two, twenty-four, twenty-six, or twenty-eight (NANP)ₙ repeats (SEQ ID NO:60; n=2, 4, 6, 8, 10, 12, 14, 16, 18, 20, 22, 24, 26, 28) of PfCSP are inserted into HVR1 of adenovirus serotype 5 Hexon. The (NANP)ₙ repeat sequence may be additionally inserted in the in the HI loop of Fiber.

In some embodiments, immunodominant neutralizing B cell epitopes to CS were mapped to develop improved CS protein adenovirus vaccines. Mice immunized with recombinant *P*. *yoelii* CS protein (PyCS) generated high titers against the two major immunodominant B epitopes, QGPGAP (SEQ ID NO:59) and QQPP (SEQ ID NO:65), but the *in vitro* neutralization assay indicated that the humoral immune response to QGPGAP epitope (SEQ ID NO:59) itself may account for neutralizing activity because the neutralization could be reversed by adding (QGPGAP)₃ peptide (SEQ ID NO:59; n=3) to the medium. In some embodiments, three or more (QGPGAP)ₙ repeats (SEQ ID NO:59) of PyCS are inserted into HVR1 of adenovirus serotype 5 Hexon. In some embodiments, three, four, five, six, seven, eight, nine, ten, eleven, or twelve (QGPGAP)ₙ repeats (SEQ ID NO:59; n=3, 4, 5, 6, 7, 8, 9, 10, 11, 12) of PyCS may be inserted into HVR1 of adenovirus serotype 5 Hexon. The (QGPGAP)ₙ repeat sequence may be additionally inserted in the in the HI loop of Fiber.

The B cell epitope peptide should be presented on the surface of adenovirus virions so that immune system can recognize the epitope efficiently. Such insertion sites could be HVRs of Hexon and Loop structures in Fiber, and different insertion sites can be combined.

### Modification of adenovirus capsid and core proteins to enhance T helper cell activation

In another embodiment, a CD4+ epitope specific to the transgene used in an adenoviral vector may be incorporated into adenovirus proteins such as pVII, pV and Hexon to augment immunogenicity of the adenoviral-based vaccine. Professional antigen presenting cells (APC) such as dendritic cells (DC) and B cells can uptake particulated pathogens like virus particles via endocytosis and present CD4+ epitopes in the pathogen to CD4+ T cells which acts as helper cells for humoral and/or cellular immune responses. pVII and Hexon may easily be used as adenovirus target proteins to insert antigenic CD4+ peptides because of high copy number of pVII (700-800 copies) and Hexon (720 copies) in one virion.

### Modification of adenovirus capsid proteins to circumvent existing adenovirus immunity

In some embodiments, adenovirus Fiber and Hexon capsid proteins may be modified to insert a B cell or T helper cell epitope to overcome existing immunity to adenovirus and/or enhance the humoral response to an adenovirus vaccine. An estimated 80% of young adults in human population have circulating neutralizing antibodies to adenovirus (Douglas 2007), especially to serotype 5 (Ad5). In studies utilizing adenovirus as a gene therapy vector, it was found that the presence of neutralizing antibodies in animals limits the expression of transgenes delivered by adenovirus. In addition to neutralizing antibodies, CD8+ T cell responses also contributed to the limitation of recombinant gene expression (Yang et al. 1995, Yang et al 1996). Such pre-existing immunity to adenovirus has previously been reported to inhibit the efficacy of a recombinant adenovirus vaccine (Papp et al. 1999) and also reduces immunogenicity of adenovirus-based vaccines in a clinical trial (Priddy et al. 2008).

Hexon is a major target for anti-Ad capsid immune responses (Roy et al. 2005, Wohlfart 1988), and is likely responsible for the potent adjuvant effect of adenovirus, including the induction of CD4+ and CD8+ T cell responses. Therefore, one strategy that has been employed to circumvent pre-existing anti-adenovirus immunity is to replace all or part of the Hexon with a different protein, for example, rare serotypes such as adenovirus 11, 24, 26 and 35. Because Hexon is a major target of anti-adenovirus neutralizing antibody (Youil et al. 2002, Sumida et al. 2005), the entire Hexon or HVRs of Hexon may be swapped with the rare serotypes (Wu et al. 2002, Roberts et al. 2006).

In another strategy as described in one embodiment herein, an adenoviral Hexon may be modified by replacement of HVR1 or HVR5 with an antigenic peptide to circumvent pre-existing anti-adenovirus immunity or anti-adenovirus neutralizing antibody induced by previous vaccination with adenoviral vector. In some embodiments, an antigenic peptide may be an immunogenic epitope of *Plasmodium* CS protein, and in certain aspects, the epitope may comprise a central repeat sequence, CD4+ epitope sequence or CD8+ epitope sequence.

Repeat administration with an Ad vector of the same serotype is prevented due to anti-Ad immunity following immunization. Therefore, many Ad vaccines impede boosting of the vaccine by preventing expression and presentation of the antigen encoded by the transgene (Yang 1995, Hackett et al. 2000, Harvey et al. 1999, Mastrangeli et al. 1996). The addition of a specific epitope to the Ad capsid, such as those described in the examples below, may reduce or eliminate this impediment according to some embodiments.

The following examples are provided to better illustrate the embodiments and are not to be interpreted as limiting the scope of any claimed embodiment. The extent that specific materials are mentioned, it is merely for purposes of illustration and is not intended to limit the invention. One skilled in the art may develop equivalent means or reactants without the exercise of inventive capacity and without departing from the scope of the invention. It will be understood that many variations can be made in the procedures herein described while still remaining within the bounds of the present invention. It is the intention of the inventors that such variations are included within the scope of the invention.

### Example 1: Construction of capsid-modified Plasmodium circumsporozoite protein adenovirus plasmid vectors and recombinant adenovirus particles

### Epitope Mapping

First, an immunodominant neutralizing B cell epitope in PyCS was chosen. Naïve Balb/c mice were immunized with recombinant PyCS-protein with incomplete freund adjuvant three times and the pooled serum was used to determine the critical epitope of neutralizing antibody.

Briefly, in the neutralizing assay, human CD81 expressing HepG2 cells were used as target cells. CD81 is a molecule necessary for malaria parasites to form parasitophorous vacuoles in hepatocytes where they multiply and develop into schizonts (Silvie et al 2006), thus greatly increasing the *in vitro* infectivity of sporozoites.

In this assay, PyCS synthetic peptides were added to the wells to block peptide specific antibody. The results of epitope mapping indicated that the PyCS central repeat sequence, QGPGAP (SEQ ID NO:59), is a more potent neutralizing epitope in PyCS than QQPP (SEQ ID NO:65). Therefore, insertion of the (QGPGAP)ₙ epitope (SEQ ID NO:59) was used to modify adenovirus capsid proteins, Hexon and/or Fiber.

### Construction of capsid-modified plasmid vectors

Adenovirus shuttle vector pShuttle-CMV (STRATAGENE) was modified by inserting a GFP-expression cassette under the cloning site. First, BsmBI-SacI fragment (pCMV+GFP) and SacI-BsmBI fragment (SV40 poly A signal) of pmaxGFP (Lonza, Cologne, Germany) were blunted and inserted into the blunted SalI and Kpnl sites of pUC19 respectively. The BamHI-EcoRI fragment of the resulting pCMV-GFP/pUC19 was inserted into the same sites of SV40pA/ pUC19 to create SV40pA-pCMV-GFP fragment. The fragment was blunted and inserted into the EcoRV site of pShuttle-CMV. The resulting shuttle vector (GFP/pShuttle-CMV) has dual pCMV promoters and SV40pAs for a transgene and GFP.

Another modification of the Adenovirus shuttle vector pShuttle-CMV was done to replace the CMV promoter region with CMV5 promoter from pQBI-AdCMV5 (QBIOgene). The SgrAI-KpnI fragment of pShuttle-CMV was replaced with the fragment containing the CMV5 promoter sequence and the upstream sequence from CMV promoter in pShuttle-CMV to construct pShuttle-CMV5 vector.

The *P*. *yoelii* CS (PyCS) gene was codon-optimized except for the (QGPGAP)ₙ repeats (SEQ ID NO:59) by overlapping PCR reaction based on JCat codon-optimization algorithm (http://www jcat de/).

The PfCSP amino acid sequence of *P*. *falciparum* 3D7 strain was used as a template sequence for codon-optimization. Codon-optimization for protein expression in humans was done by Integrated DNA Technologies' (Coralville, IA USA) optimization software. DNA fragments that encode whole PfCSP except for the GPI-anchored motif at the C-terminus (Fig. 10; SEQ ID NO:2) were synthesized by Integrated DNA Technologies

Codon-optimized PyCS gene (Fig. 9; SEQ ID NO:1) or PfCSP gene (Fig. 10; SEQ ID NO:2) was inserted into Kpnl and Hindlll sites of pShuttle-CMV, pShuttle-CMV5, or GFP/pShuttle-CMV. The resulting Plasmodium circumsporozoite protein coding adenovirus shuttle vectors were used for homologous recombination with AdEasy-1 to construct adenovirus genome which has Plasmodium circumsporozoite antigenic gene and intact adenovirus protein coding sequences. Briefly, Plasmodium circumsporozoite protein coding adenovirus shuttle vectors were linearized by Pmel digestion, and E. coli BJ5183 cells were co-transformed with the linearized shuttle vector and pAdEasy-1 vector (Bruna-Romero et al 2003) for homologous recombination.

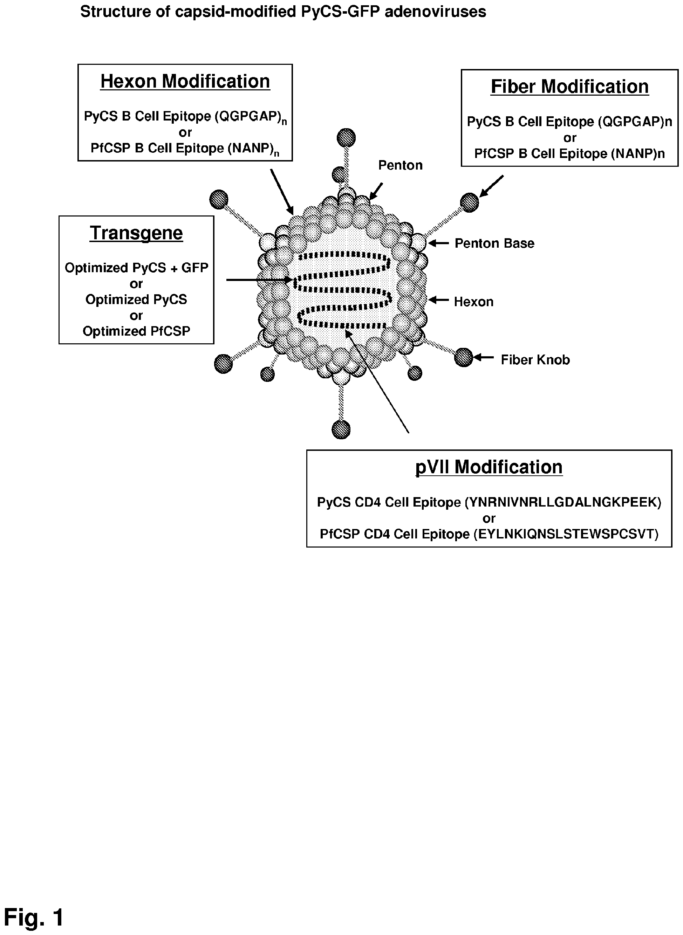

Modification of adenovirus capsid proteins is summarized and illustrated in Fig. 1. Modification of HVR1 sequence in the adenovirus genome DNA is illustrated in Fig. 2. Briefly, AdEasy-1 was digested with Sfil and the 6.4kbp fragment was subcloned into EcoRI and Pstl sites of pUC19 using EcoRI-SfiI and Pstl-Sfil linker oligomers. To replace HVR1 with a Plasmodium circumsporozoite protein B cell epitope, the region containing Agel and Ndel sites was amplified by two-step PCR using primers which have the epitope sequence instead of HVR1 sequence. The PCR product was digested with Agel and Ndel, and then used to replace the native Agel-Ndel region of Sfil fragment in Sfil/pUC19 vector. After confirming the sequence, the Sfil fragment of adenovirus genome DNA was replaced with the Sfil fragment containing the circumsporozoite epitope sequence to produce an HVR1- modified Hexon. In some embodiments, an HVR-modified Hexon may have a nucleic acid sequence of SEQ ID NO:3 (Fig. 11), SEQ ID NO:4 (Fig. 12), SEQ ID NO:5 (Fig. 13), SEQ ID NO:6 (Fig. 14), SEQ ID NO:7 (Fig. 15), SEQ ID NO:8 (Fig. 16), SEQ ID NO:9 (Fig. 17), SEQ ID NO:10 (Fig. 18), SEQ ID NO:11 (Fig. 19), SEQ ID NO:12 (Fig. 20), SEQ ID NO:13 (Fig. 21), SEQ ID NO:14 (Fig. 22), SEQ ID NO:15 (Fig. 23), SEQ ID NO:16 (Fig 24), SEQ ID NO:17 (Fig. 25), SEQ ID NO:18 (Fig. 26), SEQ ID NO:19 (Fig. 27), SEQ ID NO:20 (Fig. 28), SEQ ID NO:21 (Fig. 29), SEQ ID NO:22 (Fig. 30), or SEQ ID NO:23 (Fig. 31).

To insert (NANP)₂₈ (SEQ ID NO:60; n=28) in HVR1, a part of the central repeat region of codon-optimized PfCSP was amplified by PCR using primers having hexon-specific sequence at 5' and NANP-specific sequence at 3', and the resulting DNA fragment was inserted into the Agel-Ndel region by second PCR.

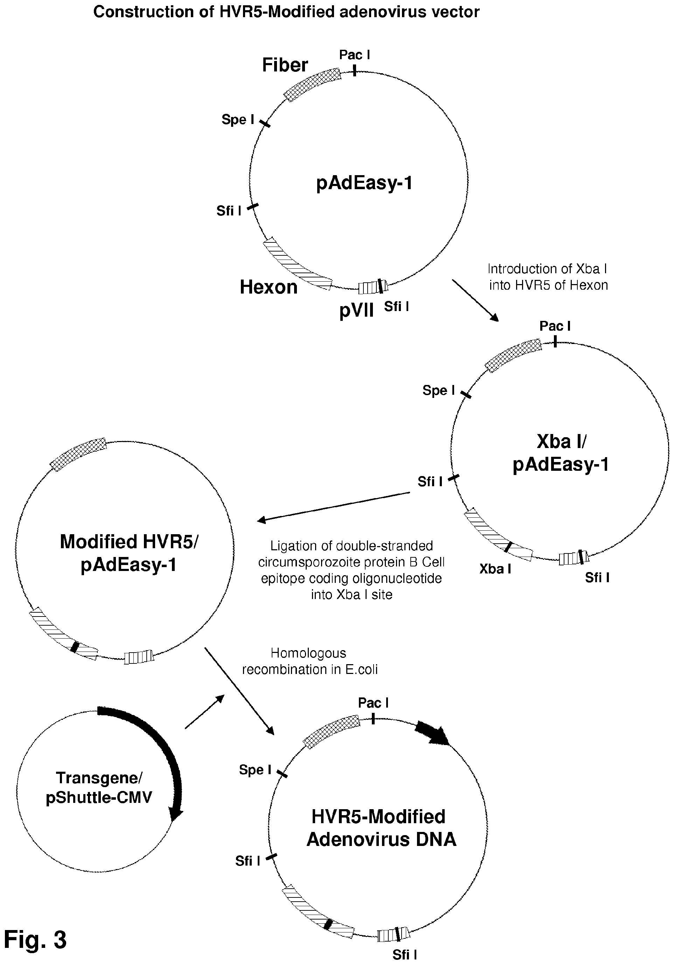

For HVR5-modification, as illustrated in Fig. 3, Xbal site was introduced into HVR5 in the L1 Loop of Hexon in AdEasy-1 and then synthesized, phosphorylated double strand oligomer coding the Plasmodium circumsporozoite protein epitope was inserted into the Xbal site. The insertion was confirmed by sequencing (Fig 31; SEQ ID NO:23).

For Fiber-modification, as illustrated in Fig. 4, the Spel-Pacl fragment of AdEasy-1 was subcloned into EcoRI and Pstl sites of pUC19 using EcoRI-PacI and PstI-SpeI linker oligomers. To insert a Plasmodium circumsporozoite protein B-cell epitope sequence into HI loop of Fiber knob, the region containing EcoNI (or Nhel) and Mfel sites was amplified by two-step PCR using primers which have the epitope sequence. The PCR product was digested with EcoNI (or Nhel) and Mfel, and then used to replace the native EcoNI (or Nhel) -Mfel region of Fiber in Spel-Pacl/pUC19 vector. After confirming the sequence (Fig 32, SEQ ID NO:24; Fig 33, SEQ ID NO:25), the Spel-Pacl fragment of AdEasy-1 was replaced with the Spel-Pacl fragment containing the epitope sequence. The resulting Fiber-modified adenovirus DNA was used for homologous recombination with Plasmodium circumsporozoite protein coding adenovirus shuttle vector to produce Fiber-modified Plasmodium circumsporozoite protein adenovirus DNA.

To construct HVR1 and Fiber-modified adenovirus DNA which has two epitope insertions, Sfil-Sfil fragment of Fiber-modified adenovirus DNA was replaced with Sfil-Sfil fragment having the circumsporozoite protein epitope in HVR1 as illustrated in Fig. 5.

To modify the C-terminus of pVII, the region containing Sfi I and Sal I sites was amplified by two-step PCR using primers which have the circumsporozoite protein epitope sequence. The PCR product was digested with Sfil and SalI, and then used to replace the native Sfil-Sall region of Sfil/pUC19 vector (Fig. 6, 7 and 8). After confirming the sequence (Fig. 34, SEQ ID NO:26; Fig. 35, SEQ ID NO:27), the Sfil-Sfil fragment of HVR1 and/or Fiber-modified circumsporozoite protein adenovirus DNA was replaced with Sfil-Sfil fragment having the circumsporozoite protein epitope in pVII.

To insert the circumsporozoite protein CD4+ epitope sequence EYLNKIQNSLSTEWSPCSVT(SEQ ID NO:62) in the middle of pVII, about 7.7kb fragment of pAdEasy-1 was prepared by Rsrll digestion and cloned between the EcoRI and Hindlll sites of pUC19 plasmid using RsrlI linker (Rsrll/pUC19). The region containing AscI and BglII sites in Rsrll/pUC19 was amplified by two-step PCR using primers which have the epitope sequence. The PCR product was digested with AscI and BglII, and then used to replace the native AscI and Bglll region in Rsrll/pUC19 plasmid. After confirming the sequence of the replaced region (Fig. 36, SEQ ID NO:28; Fig. 37, SEQ ID NO:29), the Rsrll fragment of HVR1-modified adenovirus DNA was replaced with the Rsrll fragment containing the epitope sequence.

The recombinant adenoviruses listed in Table 1 (*P. yoelii*) and Table 2 (*P*. *falciparum*) below were produced to evaluate the effect of epitope insertion on infectivity, immunogenicity and sensitivity to pre-existing, anti-adenovirus immunity. Recombinant adenovirus vectors used were replication defective, E1 and E3-deleted adenovirus serotype 5 (STRATAGENE). Fig. 1 shows the schematic structure of capsid-modified Plasmodium circumsporozoite protein recombinant adenovirus.

**Table 1. Recombinant adenoviruses (Plasmodium yoelii circumsporozoite protein)**

| Antigen | Recombinant Adenovirus | Promoter | Transgene | Adenovirus Protein | Insertion Site | Position (a.a.) | Inserted Sequence | Length |
|---|---|---|---|---|---|---|---|---|
| | wt/Empty | CMV | None | Hexon | - | - | - | - |
| | | | | Fiber | - | - | - | - |
| | | | | pVII | - | - | - | - |
| | wt/GFP | CMV | GFP | Hexon | - | - | - | - |
| | | | | Fiber | - | - | - | - |
| | | | | pVII | - | - | - | - |
| | wt/PyCS-GFP | CMV | PyCS+GFP | Hexon | - | - | - | - |
| | | | | Fiber | - | - | - | - |
| | | | | pVII | - | - | - | - |
| | (QGPGAP) ₃-HVR1/PyCS-GFP | CMV | PyCS+GFP | Hexon | HVR1 | from138 to164 | (QGPGAP)₃ | 18 |
| | | | | Fiber | - | - | - | - |
| | | | | pVII | - | - | - | - |
| | (QGPGAP) ₃-HVR5/PyCS-GFP | CMV | PyCS+GFP | Hexon | HVR5 | between 268 and 269 | (QGPGAP)₃ | 18 |
| | | | | Fiber | - | - | - | - |
| | | | | pVII | - | - | - | - |
| | (QGPGAP) ₃-Fib/PyCS-GFP | CMV | PyCS+GFP | Hexon | - | - | - | - |
| | | | | Fiber | HI Loop | between 543 and 544 | (QGPGAP)₃ | 18 |
| | | | | pVII | - | - | - | - |
| | (QGPGAP) ₃-HVR1/Fib/PyCS-GFP | CMV | PyCS+GFP | Hexon | HVR1 | from138 to 164 | (QGPGAP)₃ | 18 |
| | | | | Fiber | HI Loop | between 543 and 544 | (QGPGAP)₃ | 18 |
| | | | | pVII | - | - | - | - |
| | (QGPGAP) ₃-Fib/PyCD4-pVII-1/PyCS-GFP | CMV | PyCS+GFP | Hexon | - | - | - | - |
| | | | | Fiber | HI Loop | between 543 and 544 | (QGPGAP)₃ | 18 |
| | | | | pVII | C-terminus | between 198 and STOP Codon | YNRNIVNRLLGDALNGKPEEK | 21 |
| P. | wt/cmv5-PyCS | CMV5 | PyCS | Hexon | - | - | - | - |
| yoelii circumsporozoite | | | | Fiber | - | - | - | - |
| protein | | | | pVII | - | - | - | - |
| (PyCS) | (QGPGAP) ₃-HVR₁/cmv5-PyCS | CMV5 | PyCS | Hexon | HVR1 | from 138 to 164 | (QGPGAP) ₃ | 18 |
| | | | | Fiber | - | - | - | - |
| | | | | pVII | - | - | - | - |
| | (QGPGAP) ₄-HVR1/cmv5-PyCS | CMV5 | PyCS | Hexon | HVR1 | from138 to164 | (QGPGAP) ₄ | 24 |
| | | | | Fiber | - | - | - | - |
| | | | | pVII | - | - | - | - |
| | (OGPGAP) ₅-HVR11cmv5-PyCS | CMV5 | PyCS | Hexon | HVR1 | from 138 to 164 | (QGPGAP) ₅ | 30 |
| | | | | Fiber | - | - | - | - |
| | | | | pVII | - | - | - | - |
| | (QGPGAP) ₆-HVR1/cmv5-PyCS | CMV5 | PyCS | Hexon | HVR1 | from 138 to 164 | (QGPGAP) ₆ | 36 |
| | | | | Fiber | - | - | - | - |
| | | | | pVII | - | - | - | - |
| | (QGPGAP) ₇-HVR1/cmv5-PyCS | CMV5 | PyCS | Hexon | HVR1 | from138 to164 | (QGPGAP) ₇ | 42 |
| | | | | Fiber | - | - | - | - |
| | | | | pVII | - | - | - | - |
| | (QGPGAP) ₈-HVR1/cmv5-PyCS | CMV5 | PyCS | Hexon | HVR1 | from 138 to 164 | (QGPGAP) ₈ | 48 |
| | | | | Fiber | - | - | - | - |
| | | | | pVII | - | - | - | - |
| | (QGPGAP) ₉-HVR1/cmv5-PyCS | CMV5 | PyCS | Hexon | HVR1 | from 138 to 164 | (QGPGAP) ₉ | 54 |
| | | | | Fiber | - | - | - | - |
| | | | | pVII | - | - | - | - |
| | (QGPGAP) ₁₁-HVR1/cmv5-PyCS | CMV5 | PyCS | Hexon | HVR1 | from 138 to 164 | (QGPGAP) ₁₁ | 66 |
| | | | | Fiber | - | - | - | - |
| | | | | pVII | - | - | - | - |
| | (QGPGAP) ₁₂-HVR1/cmv5-PyCS | CMV5 | PyCS | Hexon | HVR1 | from 138 to 164 | (QGPGAP) ₁₂ | 72 |
| | | | | Fiber | - | - | - | - |
| | | | | pVII | - | - | - | - |

**Table 2. Recombinant adenoviruses (Plasmodium falciparum circumsporozoite protein)**

| Antigen | Recombinant Adenovirus | Promoter | Transgene | Adenovirus Protein | Insertion Site | Position (a.a.) | Inserted Sequence | Length |
|---|---|---|---|---|---|---|---|---|
| | wt/PfCSP | CMV | PfCSP | Hexon | - | - | - | - |
| | | | | Fiber | - | - | - | - |
| | | | | pVII | - | - | - | - |
| | (NANP)₄-HVR1/PfCSP | CMV | PfCSP | Hexon | HVR1 | from138 to164 | (NANP)₄ | 16 |
| | | | | Fiber | - | - | - | |
| | | | | pVII | - | - | - | |
| | (NANP)₄-Fib/PfCSP | CMV | PfCSP | Hexon | | | | |
| | | | | Fiber | HI Loop | between 543 and 544 | (NANP)₄ | 16 |
| | | | | pVII | - | - | - | |
| | (NANP)₄-HVR1/Fib/PfCSP | CMV | PfCSP | Hexon | HVR1 | from138 to164 | (NANP)₄ | 16 |
| | | | | Fiber | HI Loop | between 543 and 544 | (NANP)₄ | 16 |
| | | | | pVII | - | - | - | |
| | (NANP)₆-HVR1/PfCSP | CMV | PfCSP | Hexon | HVR1 | from138 to164 | (NANP)₆ | 24 |
| | | | | Fiber | - | - | - | |
| | | | | pVII | - | - | - | |
| | (NANP)₈-HVR1/PfCSP | CMV | PfCSP | Hexon | HVR1 | from138 to164 | (NANP)₈ | 32 |
| | | | | Fiber | - | - | - | |
| | | | | pVII | - | - | - | |
| | (NANP)₁₀-HVR1/PfCSP | CMV | PfCSP | Hexon | HVR1 | from 138 to 164 | (NANP)₁₀ | 40 |
| | | | | Fiber | - | - | - | |
| | | | | pVII | - | - | - | |
| | (NANP)₁₂-HVR1/PfCSP | CMV | PfCSP | Hexon | HVR1 | from 138 to 164 | (NANP)₁₂ | 48 |
| | | | | Fiber | - | - | - | |
| | | | | pVII | - | - | - | |
| | (NANP)₁₄-HVR1/PfCSP | CMV | PfCSP | Hexon | HVR1 | from138 to164 | (NANP)₁₄ | 56 |
| | | | | Fiber | - | - | - | |
| | | | | pVII | - | - | - | |
| | (NANP)₁₆-HVR1/PfCSP | CMV | PfCSP | Hexon | HVR1 | from 138 to 164 | (NANP)₁₆ | 64 |
| | | | | Fiber | - | - | - | |
| | | | | pVII | - | - | - | |
| | (NANP)₁₈-HVR1/PfCSP | CMV | PfCSP | Hexon | HVR1 | from 138 to 164 | (NANP)₁₈ | 72 |
| | | | | Fiber | - | - | - | |
| P. falciparum circumsporozoite protein (PfCSP) | | | | pVII | - | - | - | |
| | (NANP)₂₀-HVR1/PfCSP | CMV | PfCSP | Hexon | HVR1 | from138 to164 | (NANP)₂₀ | 80 |
| | | | | Fiber | - | - | - | |
| | | | | pVII | - | - | - | |
| | (NANP)₂₂-HVR1/PfCSP | CMV | PfCSP | Hexon | HVR1 | from 138 to 164 | (NANP)₂₂ | 88 |
| | | | | Fiber | - | - | - | |
| | | | | pVII | - | - | - | |
| | wt/cmv5-PfCSP | CMV5 | PfCSP | Hexon | - | - | - | - |
| | | | | Fiber | - | - | - | - |
| | | | | pVII | - | - | - | - |
| | (NANP)₂₂-HVR1/cmv5-PfCSP | CMV5 | PfCSP | Hexon | HVR1 | from 138 to 164 | (NANP)₂₂ | 88 |
| | | | | Fiber | - | - | - | |
| | | | | pVII | - | - | - | |
| | (NANP)₂₈-HVR1/cmv5-PfCSP | CMV5 | PfCSP | Hexon | HVR1 | from 138 to 164 | (NANP)₁₇(NVDP)₁(NANP)₁₀ | 112 |
| | | | | Fiber | - | - | - | |
| | | | | pVII | - | - | - | |
| | (NANP)₄-HVR1/PfCD4-pVII-1/PfCSP | CMV | PfCSP | Hexon | HVR1 | from138 to164 | (NANP)₄ | 16 |
| | | | | Fiber | - | - | - | |
| | | | | pVII | C-terminus | between 198 and STOP Codon | EYLNKIQNSLSTEWSPCSVT | 20 |
| | (NANP)₄-Fib/PfCD4-pVII-1/PfCSP | CMV | PfCSP | Hexon | - | - | - | - |
| | | | | Fiber | HI Loop | between 543 and 544 | (NANP)₄ | 16 |
| | | | | pVII | C-terminus | between 198 and STOP Codon | EYLNKIQNSLSTEWSPCSVT | 20 |
| | (NANP)₄-HVR1/Fib/PfCD4-pVII-1/PfCSP | CMV | PfCSP | Hexon | HVR1 | from138 to164 | (NANP)₄ | 16 |
| | | | | Fiber | - | - | - | |
| | | | | pVII | C-terminus | between 198 and STOP Codon | EYLNKIQNSLSTEWSPCSVT | 20 |
| | (NANP)₄-HVR1/PfCD4-pVII-2/PfCSP | CMV | PfCSP | Hexon | HVR1 | from138 to 164 | (NANP)₄ | 16 |
| | | | | Fiber | - | - | - | |
| | | | | pVII | Middle | between 92 and 93 Codon | EYLNKIQNSLSTEWSPCSVT | 20 |
| | (NANP)₄-HVR1/PfCD4-pVII-3/PfCSP | CMV | PfCSP | Hexon | HVR1 | from138 to164 | (NANP)₄ | 16 |
| | | | | Fiber | - | | - | |
| | | | | pVII | Middle | between 140 and 141 Codon | EYLNKIQNSLSTEWSPCSVT | 20 |
| | (NANP)₂₂-HVR1/PfCD4-pVII-3/cmv5-PfCSP | CMV5 | PfCSP | Hexon | HVR1 | from138 to 164 | (NANP)₂₂ | 88 |
| | | | | Fiber | - | - | - | |
| | | | | pVII | Middle | between 140 and 141 Codon | EYLNKIQNSLSTEWSPCSVT | 20 |
| | (NANP)₂₈-HVR1/PfCD4-pVII-3/cmv5-PfCSP | CMV5 | PfCSP | Hexon | HVR1 | from138 to164 | (NANP)₁₇(NVDP)₁(NANP)₁₀ | 112 |
| | | | | Fiber | - | - | - | |
| | | | | pVII | Middle | between 140 and 141 Codon | EYLNKIQNSLSTEWSPCSVT | 20 |

The capsid-modified adenovirus genome DNA plasmid was purified, linearized by Pacl digestion, and used for transfection of AD293 cells.

Adenovirus particles were prepared from the transfected AD293 cells by four rounds of freeze/thaw and used for further virus amplification. After the last amplification, adenovirus particles were purified by CsCI gradient centrifugation. The band was then collected and dialyzed against dialysis buffer to remove CsCI. Virus particle (v.p.) was calculated based on O.D. 260 (1 O.D.260=1.25x10¹² v.p./mL) (Bruna-Romero et al. 2003).

During the adenovirus amplification procedure, small differences in adenovirus growth were observed among capsid-modified adenoviruses, demonstrating that adenovirus infectivity and productivity was not adversely affected by the modification.

### Example 2: Plasmodium yoelii circumsporozoite protein-specific immune response

### Validation of Plasmodium voelii recombinant adenoviruses

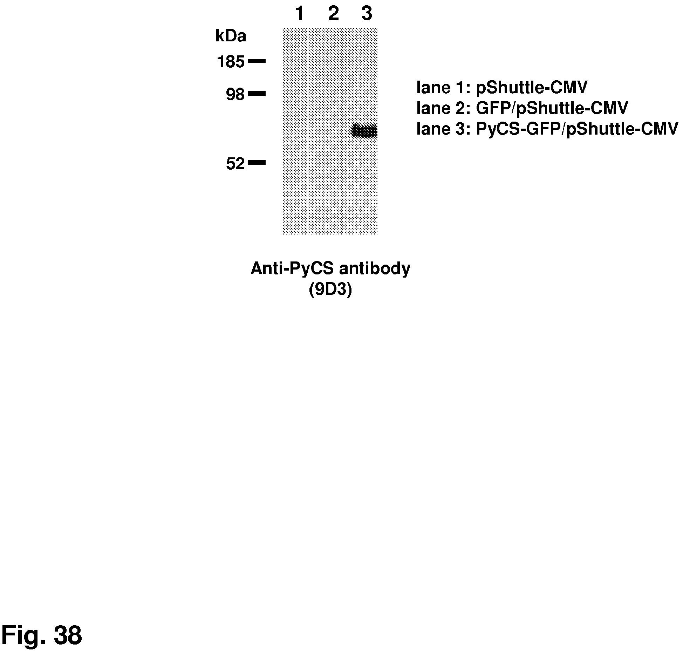

Plasmodium circumsporozoite protein coding adenovirus shuttle vectors were used for transient transfection to confirm Plasmodium circumsporozoite protein expression using AD293 cells (Fig. 38). 24 hours after transfection, cells were lysed in SDS sample buffer followed by SDS PAGE electrophoresis and western blotting with anti-PyCS monoclonal antibody (9D3).

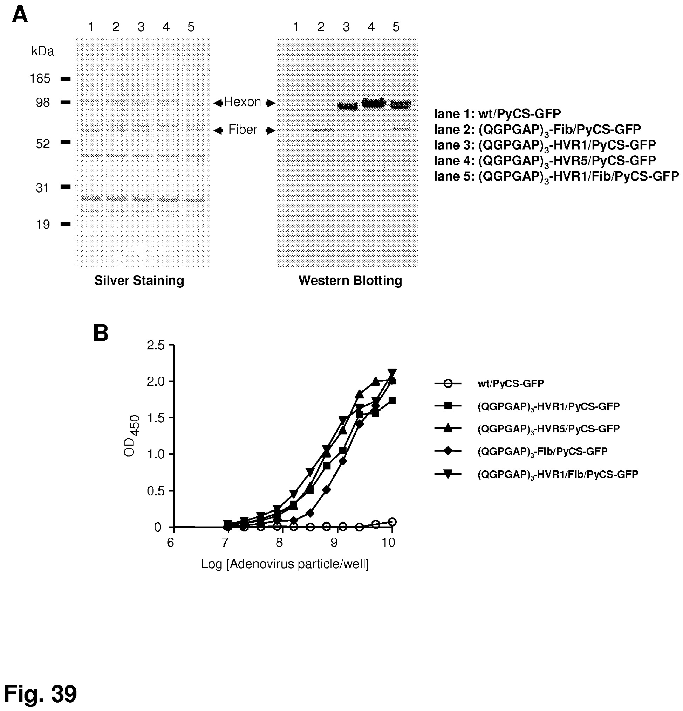

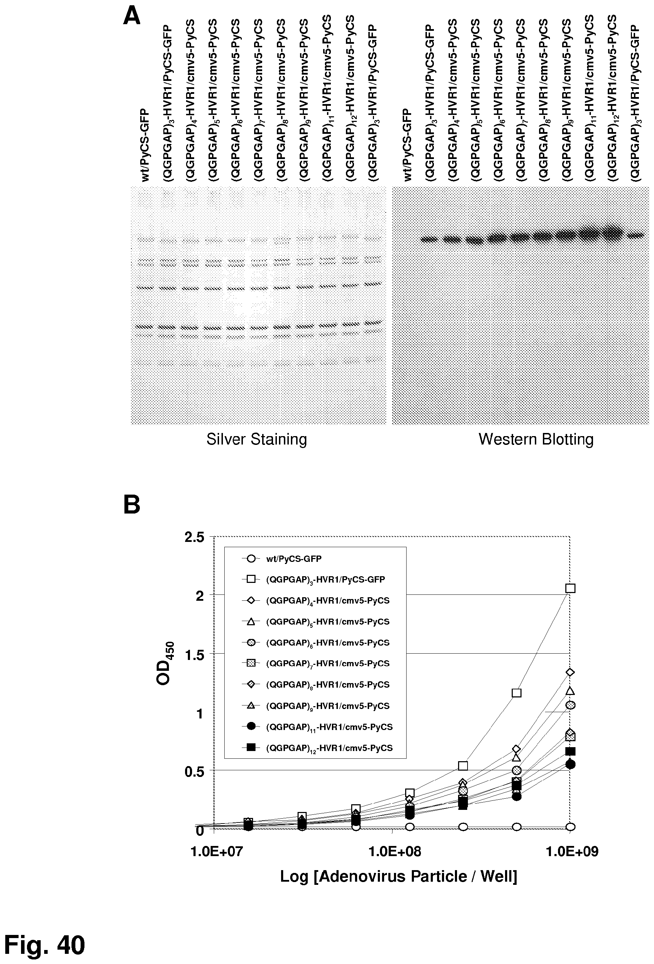

To confirm the epitope insertion into adenovirus capsid proteins, purified recombinant adenoviruses were analyzed by SDS-PAGE (2x10⁹ v.p./lane) and Western blot (1x10⁹ v.p./lane) with anti-sporozoite antibody which recognizes the (QGPGAP)ₙ (SEQ ID NO:59) repeats were done as shown in Fig. 39A and 40A. The intensity of the bands in Fig. 39A correlated with the copy number of the capsid protein in an adenovirus virion: the copy number of Fiber (36 copies per virion) is twenty-times less than Hexon (720 copies per virion). The lower band in lane 4 in Fig. 39A is likely a degraded Hexon. The intensity of the bands in Fig. 40A correlated with the number of (QGPGAP)ₙ (SEQ ID NO:59) repeats inserted into HVR1.

To assess whether the PyCS-B epitope was exposed to the outside of adenovirus virion, serially diluted purified recombinant adenovirus particles were coated onto Enzyme-Linked Immunosorbent Assay (ELISA) plate and detected with anti-PyCS antibody that recognizes (QGPGAP)ₙ (SEQ ID NO:59) repeats. The antibody recognized all the capsid-modified adenoviruses (Fig. 39B and 40B). The results of ELISA assay suggest that the PyCS-B epitope incorporated in capsid proteins were well exposed to the outside of adenovirus virions.

### Plasmodium yoelii circumsporozoite protein-specific immune response after immunization with capsid-modified PyCS adenovirus

Six- to eight-week old female BALB/c mice were purchased from Taconic (Hudson, NY, USA) and maintained under standard conditions in the Laboratory Animal Research Center of The Rockefeller University. For immunization, adenoviruses were diluted in PBS and injected intramuscularly at indicated doses.

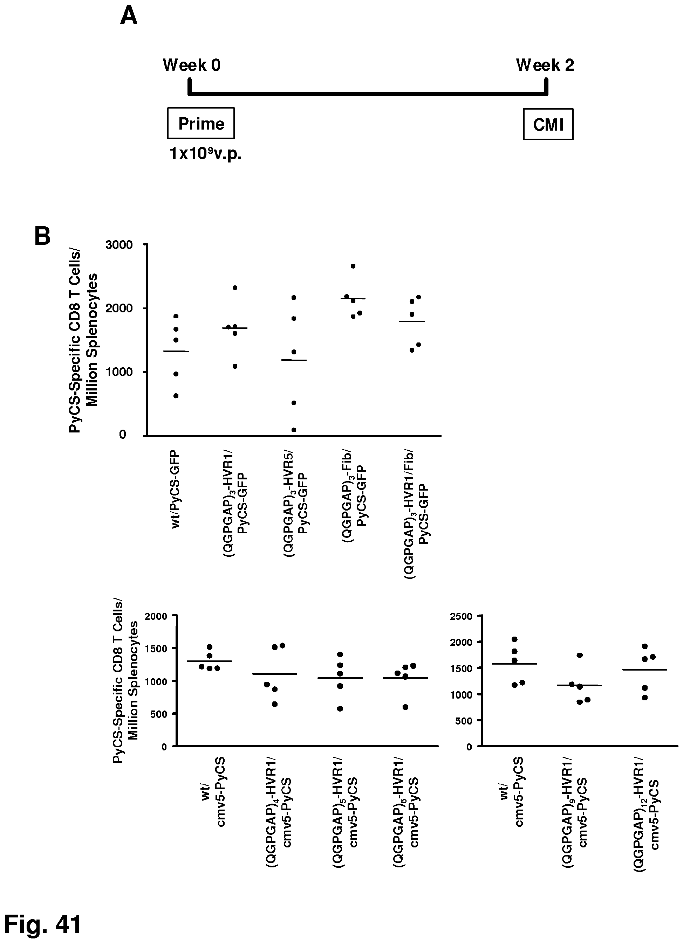

To evaluate the immunogenicity of recombinant adenoviruses after a single immunization, groups of naïve BALB/c mice (five per group) were immunized with 1x10⁹v.p. of various recombinant PyCS adenoviruses intramuscularly and PyCS-specific cell-mediated immune responses (CMI) were measured by ELISPOT 2 weeks after immunization (Fig. 41A).

The number of PyCS-specific, IFN-γ-secreting CD8+ T cells in the spleens of immunized mice were determined by an ELISPOT assay, using a synthetic peptide corresponding to the CD8+ T cell epitope (SYVPSAEQI; SEQ ID NO:66) within the PyCS protein. Briefly, 96 well nitrocellulose plates (Milititer HA, Millipore) were coated overnight with anti-mouse interferon γ mAb, R4. After overnight incubation at room temperature, the wells were washed repeatedly with culture medium and blocked with culture medium for 4hours. 5x10⁵ Splenocytes from immunized mice were added to the ELISPOT wells in the presence or absence of 10 µg/mL CD8+ T cell epitope peptide and incubated 24 hours at 37°C and 5% CO₂. After extensive washing of the plates with PBS containing 0.05% Tween 20 (PBST), biotinylated anti-mouse interferon y mAb, XMG1.2, in PBST were added and incubated overnight at 40°C. After washing with PBST, the plates will be incubated with peroxidase-labeled avidin (eBiosciences). The spots were developed by adding AEC substrate (BD Biosciences).

All of the capsid-modified adenoviruses induced comparable level of CMI to adenoviruses having intact capsid protein at this dose (Fig. 41B).

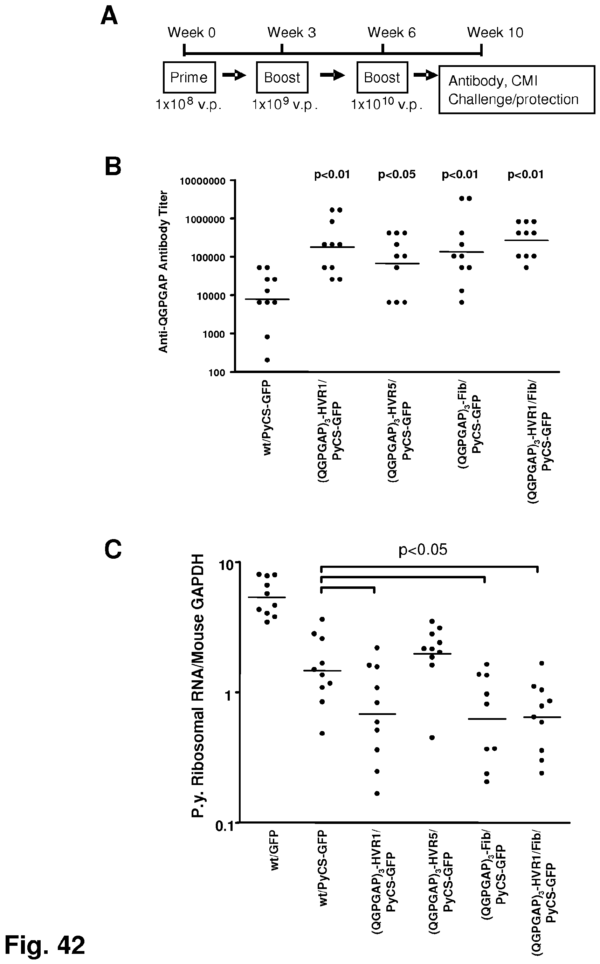

Next, naive BALB/c mice were given multiple doses of recombinant adenoviruses with increasing doses, i.e. 1x10⁸, 1x10⁹, and 1x10¹⁰ v.p., at 3 week intervals, as shown in Fig. 42A. PyCS-specific humoral response was determined by ELISA. Five microliters of blood was collected from tail vein of the immunized mice and diluted in 495 µl of PBS, and then the samples were centrifuged at 5,000 rpm for 5min to prepare diluted plasma samples (x100). Maxisorp ELISA plates were coated with 5 µg/ml CS-specific peptide ((QGPGAP)₃; SEQ ID NO:59, n=3) in 0.1M Sodium Carbonate Buffer (pH 9.5) at 4°C for overnight. Plates were washed and blocked with 1x Diluent for 2 hours at room temperature. The plates were washed again and 100 µl of serially twofold-diluted plasma or serum in 1x Diluent was added to the plates and the plates were incubated for one hour at room temperature. The plates were washed and incubated with 100 µl of HRP-labeled goat anti-mouse IgG antibody. All of the peptides were synthesized by Biosyntheis (Lewisville, TX, USA).

Using this immunization regimen, all capsid-modified adenoviruses induced a significantly higher level of anti-(QGPGAP)₃ antibody response than wt/PyCS-GFP at week 10 (Fig. 42B).

To determine the vaccine efficacy of capsid-modified adenovirus, the immunized mice were challenged with 2x10⁴ infectious *P. yoelii* sporozoites via tail vein injection at week 10. Parasite burden 42 hours after sporozoite challenge was determined by quantifying the amounts of parasite-specific ribosomal RNA in mouse liver and described as a ratio of the absolute copy number of parasite ribosomal RNA to that of mouse GAPDH mRNA. For statistical analysis, the values were log-transformed and then one-way ANOVA followed by a Dunnett's test was employed to determine the differences.

Vaccinations with (QGPGAP)₃-HVR1/PyCS-GFP, (QGPGAP)₃-Fib/PyCS-GFP or (QGPGAP)₃-HVR1/Fib/PyCS-GFP induced a higher level of protection than wt/PyCS-GFP, resulting in a significantly lower parasite burden in the malaria challenged mice (Fig. 42C).

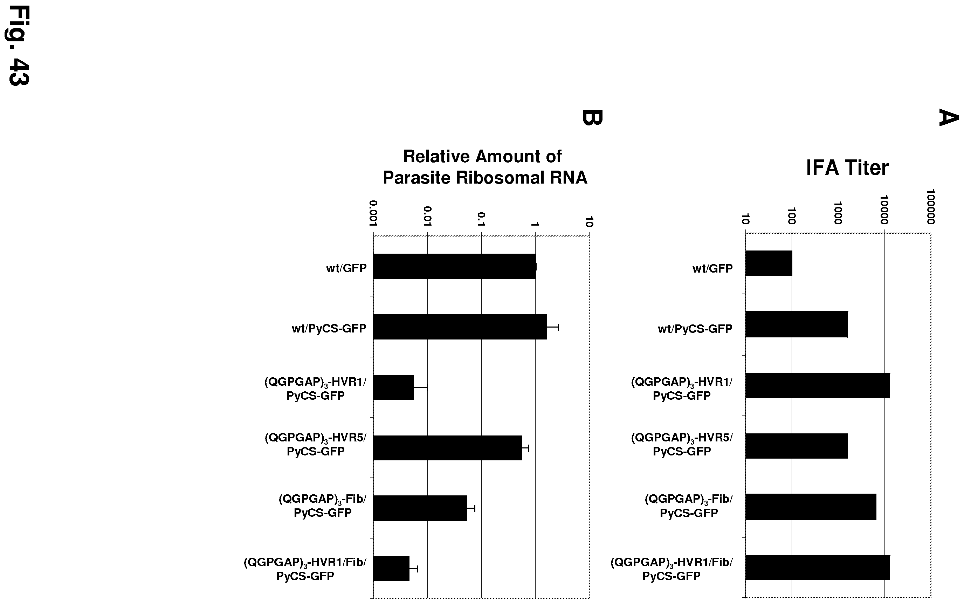

Next, the functionality of PyCS-specific antibody induced by capsid-modified adenoviruses was evaluated. First, to test whether the sera of adenovirus-immunized mice at week 10 (Fig. 42A) could recognize intact sporozoites, an indirect immunofluorescene assay (IFA) was performed. In IFA, air-dried sporozoites on multi-spot glass slides were incubated with 3% Bovine Serum albumin (BSA) in PBS for one hour and then incubated with diluted sera for one hour. After washing, the slides were incubated with fluorescent-labeled secondary antibody for one hour. The slides were washed and IFA titers were determined as the highest dilution producing fluorescence under a fluorescent microscope. Both (QGPGAP)₃-HVR1/PyCS-GFP and (QGPGAP)₃-HVR1/Fib/PyCS-GFP induced a highest IFA titer against sporozoites (Fig. 43A), indicating that the insertion of (QGPGAP)₃ epitope in HVR1 of adenovirus Hexon enabled PyCS adenovirus to elicit a robust antibody response against not only a synthetic peptide, but also a native epitope present in the malaria parasites.

Second, to determine whether mice immunized with capsid-modified adenovirus (Fig. 42A) developed "functional" antibodies that could neutralize the infectivity of sporozoites, an *in vitro* sporozoite neutralization assay was performed.

In the *in vitro* neutralizing assay, *P. yoelii* sporozoites were added to CD81/HepG2 in a 96-well plate in the presence of 30-fold diluted pooled serum from adenovirus-immunized mice. After a two-hour incubation, uninfected sporozoites were washed out with medium and then the cells were cultured for 42 hours. Relative amount of parasite ribosomal RNA to human GAPDH mRNA was measured by real-time PCR (Ophorst et al. 2006).

The pooled serum samples from mice immunized with capsid-modified adenovirus, particularly (QGPGAP)₃-HVR1/PyCS-GFP and (QGPGAP)₃-HVR1/Fib/PyCS-GFP, almost completely inhibited (99%) the sporozoite infectivity *in vitro* (Fig. 43B). It is noted that the degree of inhibition in this assay was inversely correlated with the IFA titers shown in Fig. 43A.

### Protection from blood stage malaria infection

Next it was determined whether immunization with capsid-modified rAd protects mice from developing a blood-stage malaria infection after sporozoite challenge. The experiments were performed twice and in each experiment, 20 BALB/c mice in each group were immunized three times with wt/PyCS-GFP or (QGPGAP)₃-HVR1/PyCS-GFP as shown in Figure 42A and at 4 weeks after the last immunization, the mice were intravenously challenged with 50 *P. yoelii* sporozoites. Giemsa-stained blood smears were analyzed from 3 to 12 days after challenge to detect blood stage malaria parasite infection. In the wt/CS-GFP immunized group, 30 out of 40 mice (75%) were infected whereas 35 out of 40 (87.5%) became infected in the naïve group (Table 3, below). (QGPGAP)₃-HVR1/CS-GFP immunized mice were more protected than wt/CS-GFP; only 15 out of 40 (37.5%) of which became infected, which is consistent with the result of protection experiment measured by parasite burden in liver (Figure 42C).

**Table 3. Detection of blood stage malaria parasite infection after immunization with wt/PyCS-GFP or (QGPGAP)₃-HVR1/PyCS-GFP.**

| Immunization | No. of Mice (Chllenged) | No. of Mice (Infected) | Protection (%) |
|---|---|---|---|
| Experiment 1 | | | |
| None | 20 | 18 | 10 |
| wt/PyCS-GFP | 20 | 14 | 30 |
| (QGPGAP)₃-HVR1/PyCS-GFP | 20 | 10 | 50 |

| | | | |
|---|---|---|---|
| Experiment 2 | | | |
| None | 20 | 17 | 15 |
| wt/PyCS-GFP | 20 | 16 | 20 |
| (QGPGAP)₃-HVR1/PyCS-GFP | 20 | 5 | 75 |

| | | | |
|---|---|---|---|
| Total | | | |
| None | 40 | 35 | 87.5 |
| wt/PyCS-GFP | 40 | 30 | 75 |
| (QGPGAP)₃-HVR1/PyCS-GFP | 40 | 15 | 37.5 |

### Prime-boost immunization 1

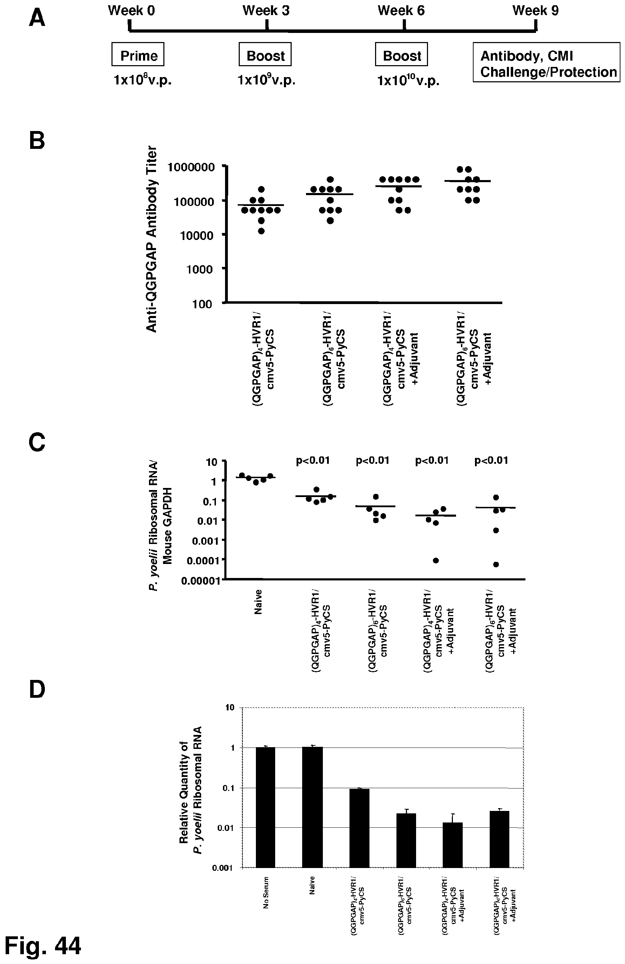

Next, naive BALB/c mice were given "boosts" of HVR1-modified PyCS adenoviruses which have four or six repeats of (QGPGAP)ₙ (SEQ ID NO:59; n=4, 6) with or without the adjuvant at multiple increasing doses (i.e. 1x10⁸, 1x10⁹, and 1x10¹⁰ v.p.) at 3 week intervals, as shown in Fig. 44A. The adjuvant used in this experiment is Sigma Adjuvant System (Sigma-Aldrich) containing 200 µg/mL Saponin (Sigma-Aldrich). A vial of Sigma Adjuvant System (1mL) contains 0.5 mg Monophosphoryl Lipid A (detoxified endotoxin) from *Salmonella minnesota* and 0.5 mg synthetic Trehalose Dicorynomycolate in 2% oil (squalene)-Tween 80 in water. Adenovirus solution was mixed with the equal amount of the adjuvant before the immunization. One hundred microliters of the adenovirus-adjuvant mixture was injected intramuscularly. PyCS-specific humoral and cell-mediated immune responses were measured as described above. A trend was observed that HVR1-modified adenovirus having six repeats induced higher antibody titer than that having four repeats and the use of adjuvant augmented the antibody titer (Fig. 44B). In contrast, there was no effect of the adjuvant on CMI (data not shown).

To determine the vaccine efficacy of HVR1-modified PyCS adenovirus, five mice in each group were challenged with 2x10⁴ infectious P. *yoelii* sporozoites via tail vein injection at week 9. Parasite burden 42 hours after sporozoite challenge was determined as described above. For statistical analysis, the values were log-transformed and then one-way ANOVA followed by a Dunnett's test was employed to determine the differences. There was a trend that HVR1-modified adenovirus having six repeats reduced parasite burden more than that having four repeats and the use of adjuvant augmented the protection (Fig. 44C).

To evaluate the functionality of antibody induced by HVR1-modified PyCS adenoviruses, we performed an *in vitro* sporozoite neutralization assay as described above. Pooled serum samples from mice immunized with HVR1-modified PyCS adenoviruses at week 9 neutralized sporozoite invasion at 50-fold dilution (Fig. 44D).

### Prime-boost immunization 2

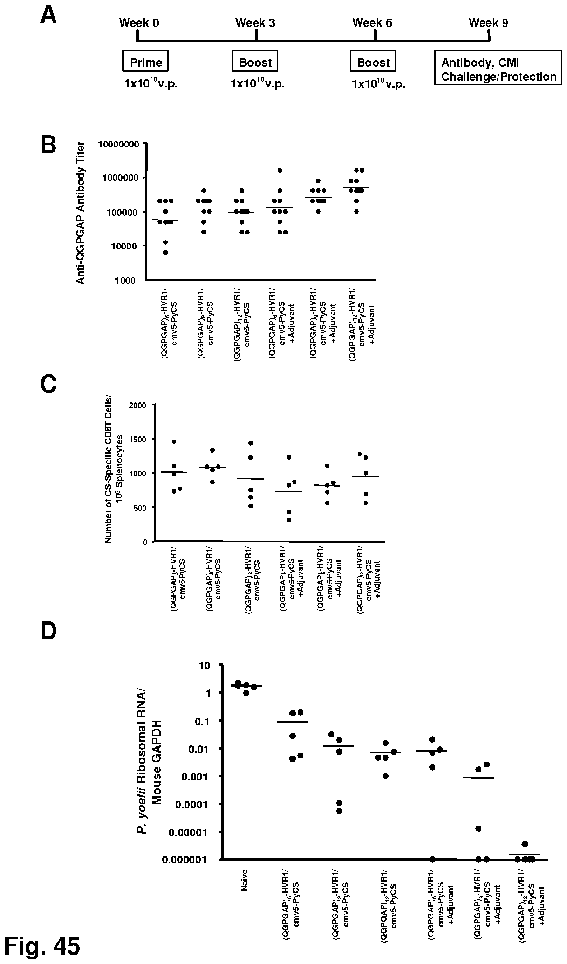

Naive BALB/c mice were given "boosts' of HVR1-modified PyCS adenoviruses which have six, nine, or twelve repeats of (QGPGAP)ₙ (SEQ ID NO: 59; n=6, 9, 12) with or without the adjuvant at three doses of 1x10¹⁰ v.p. at 3 week intervals, as shown in Fig. 45A. The adjuvant used in this experiment is Sigma Adjuvant System (Sigma-Aldrich) containing 200 µg/mL Saponin (Sigma-Aldrich). Adenovirus solution was mixed with the equal amount of the adjuvant before the immunization. PyCS-specific humoral and cell-mediated immune responses were measured as described above. HVR1-modified PyCS adenovirus which has twelve repeats of (QGPGAP)ₙ (SEQ ID NO:59; n=12) with the adjuvant induced the highest antibody titer among the groups at week 9 (Fig. 45B). With respect to PyCS-specific CMI, there was no difference among the groups, indicating that the longer epitope insertion up to twelve does not impair adenovirus infectivity *in vivo* (Fig. 45C). Further, the adjuvant did not affect the ability of adenovirus to induce CMI (Fig. 45C).

To determine the vaccine efficacy of HVR1-modified PyCS adenovirus, five mice in each group were challenged with 2x10⁴ infectious *P. yoelii* sporozoites via tail vein injection at week 9. Parasite burden 42 hours after sporozoite challenge was determined as described above. All of the HVR-1 modified PyCS adenovirus having (QGPGAP)ₙ repeats (SEQ ID NO:59, n=6, 9, 12), with or without adjuvant, showed increased protection. However, HVR1-modified PyCS adenovirus having twelve repeats of (QGPGAP)ₙ (SEQ ID NO:59, n=12) with the adjuvant showed the best protection (Fig. 45D), which was significantly more protective that any other treatment.

### Example 3: Plasmodium falciparum circumsporozoite protein-specific immune response

### Validation of Plasmodium falciparum recombinant adenoviruses

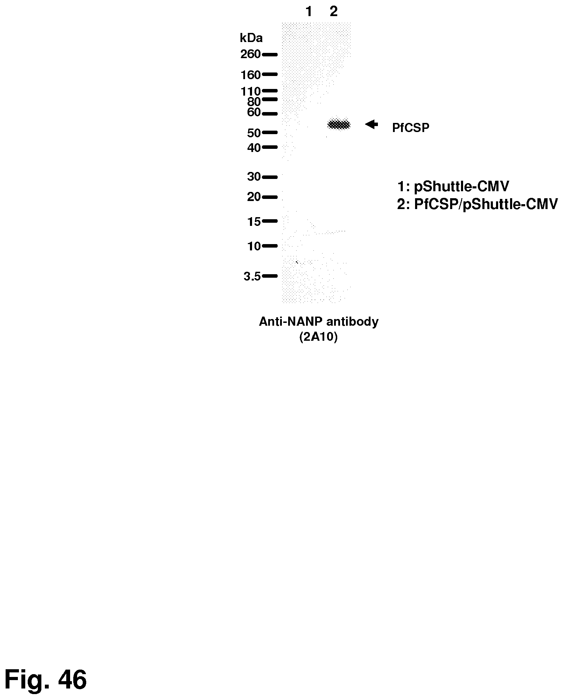

Plasmodium circumsporozoite protein coding adenovirus shuttle vectors were used for transient transfection to confirm Plasmodium circumsporozoite protein expression using AD293 cells (Fig. 46A). 24 hours after transfection, cells were lysed in SDS sample buffer followed by SDS PAGE electrophoresis and western blotting with anti-NANP monoclonal antibody (2A10).

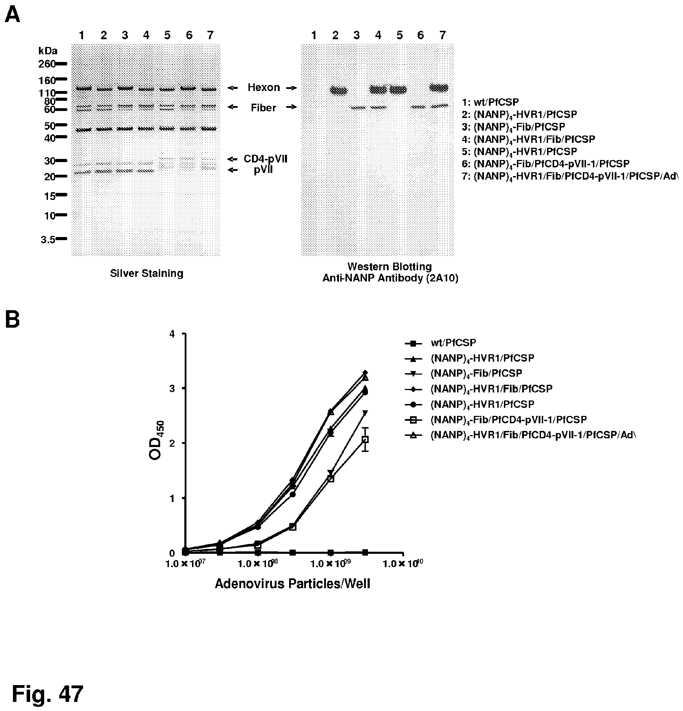

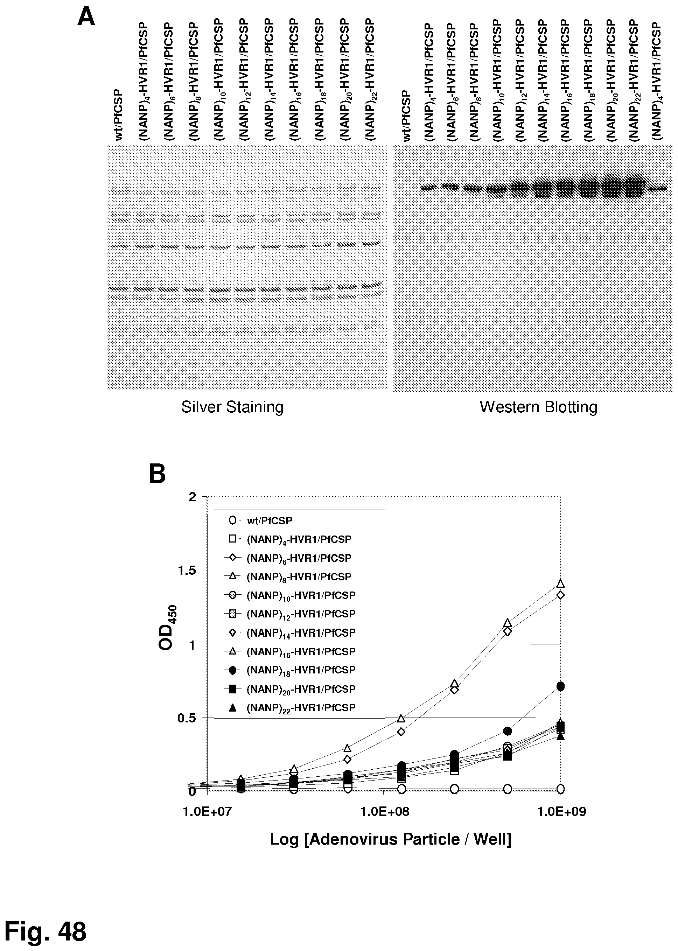

To confirm the epitope insertion into adenovirus capsid proteins, purified recombinant adenoviruses were analyzed by SDS-PAGE (2x10⁹ v.p./lane) and Western blot (1x10⁹ v.p./lane) with anti-sporozoite antibody which recognizes the (NANP)ₙ (SEQ ID NO:60) repeats were done as shown in Fig. 47A and 48A. The intensity of the bands in Fig. 47A correlated with the copy number of the capsid protein in an adenovirus virion: the copy number of Fiber (36 copies per virion) is twenty-times less than Hexon (720 copies per virion). The intensity of the bands in Fig. 48A correlated with the number of NANP repeat HVR1.

To assess whether the PfCSP-B epitope was exposed to the outside of adenovirus virion, serially diluted purified recombinant adenovirus particles were coated onto Enzyme-Linked Immunosorbent Assay (ELISA) plate and detected with anti-PyCS antibody that recognizes (NANP)ₙ (SEQ ID NO:60) repeats. The antibody recognized all the capsid-modified adenoviruses (Fig. 47B and 48B). The results of ELISA assay suggest that the PfCSP-B epitope incorporated in capsid proteins were well exposed to the outside of adenovirus virions.

### Prime-Boost immunization 3

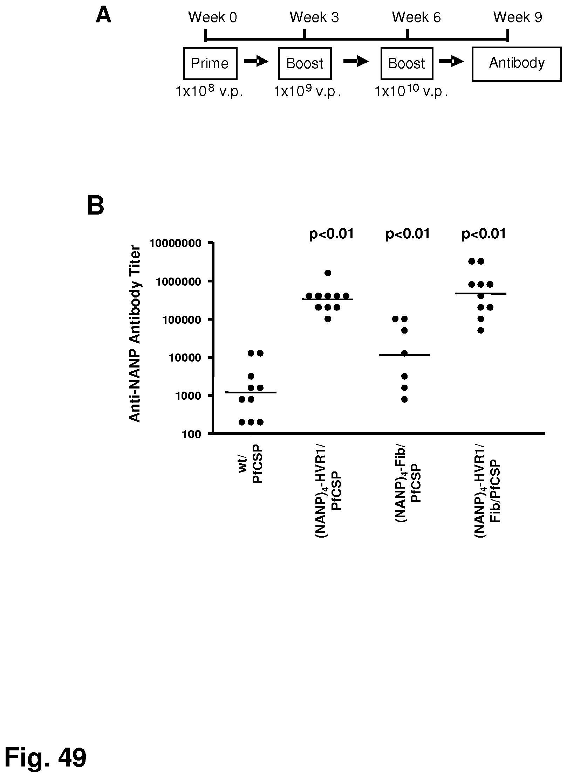

Naïve BALB/c mice were given multiple, increasing doses of recombinant PfCSP adenoviruses (i.e., 1x10⁸, 1x10⁹, and 1x10¹⁰v.p.) at 3 week intervals as shown in Fig 49A. PfCSP-specific humoral response was determined by ELISA as described above using ELISA plates coated with 1 µg/ml (T1B)₄, a CS repeat peptide which contains a (NANP)ₙ repeat sequence (SEQ ID NO:60) (Calvo-Calle et al 2006). For statistical analysis, the values were log-transformed and one-way ANOVA followed by a Dunnett's test was employed to determine the differences between wt/PfCSP and capsid-modified adenoviruses. All capsid-modified adenoviruses induced statistically higher anti-NANP antibody titer than wt/PfCSP.

### Prime-boost immunization 4

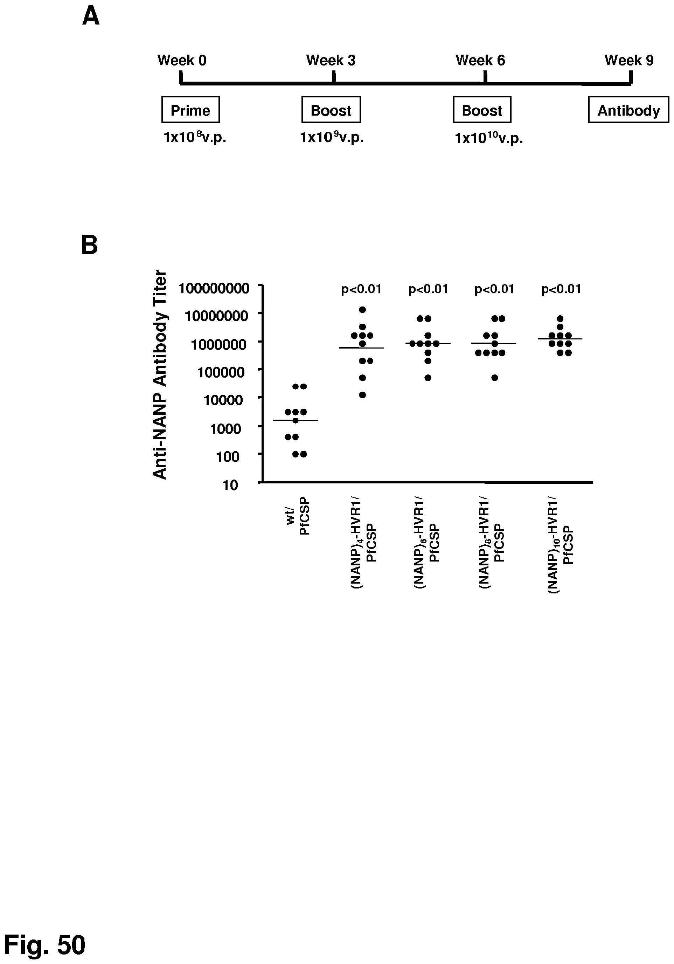

Next, naïve BALB/c mice were given "boosts' of HVR1-modified PfCSP adenoviruses which have four, six, eight, or ten repeats of (NANP)ₙ (SEQ ID NO:60; n=4, 6, 8, 10) with at multiple increasing doses (i.e., 1x10⁸, 1x10⁹, and 1x10¹⁰ v.p.) at 3 week intervals, as shown in Fig. 50A. PfCSP-specific humoral immune responses were measured as described above. All of the HVR1-modified adenoviruses induced significantly higher anti-NANP antibody titer than wt/PfCSP at week 9 (Fig. 50B). For statistical analysis, the values were log-transformed and then one-way ANOVA followed by a Dunnett's test was employed to determine the differences.

### Prime-boost immunization 5

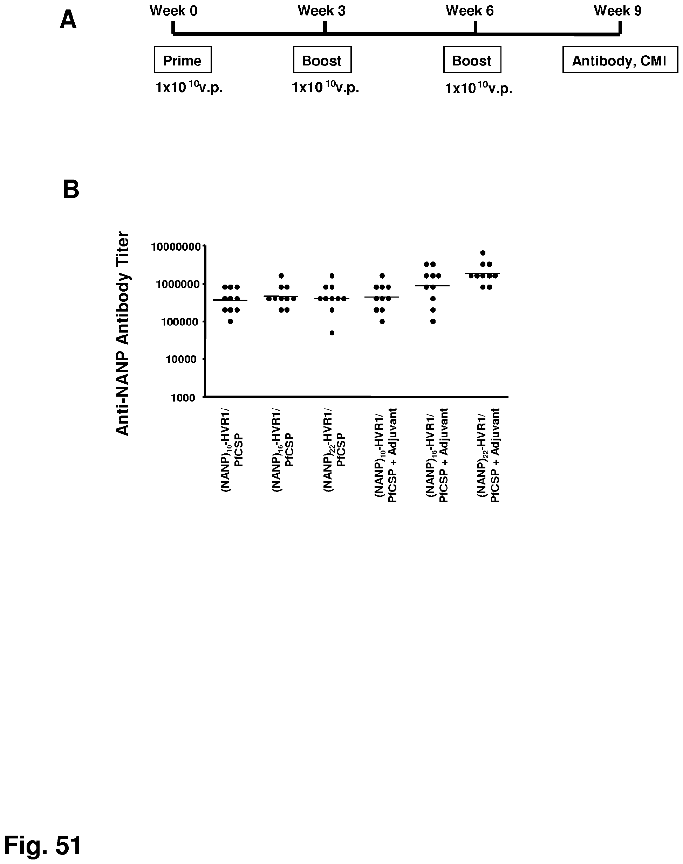

Naive BALB/c mice were given "boosts" of HVR1-modified adenoviruses which have ten, sixteen, or twenty-two repeats of (NANP)ₙ (SEQ ID NO:60; n=10, 16, 22) with or without the adjuvant at three doses of 1x10¹⁰ v.p. at 3 week intervals, as shown in Fig. 51A. The adjuvant used in this experiment is Sigma Adjuvant System (Sigma-Aldrich) containing 200 µg/mL Saponin (Sigma-Aldrich). Adenovirus solution was mixed with the equal amount of the adjuvant before the immunization. PfCSP-specific humoral immune response was measured as described above, and it was determined that HVR1-modified adenoviruses with longer B cell epitope induced higher antibody titer (Fig. 51B).

### Example 4: PyCS CD4 epitope insertion into adenovirus core protein pVII

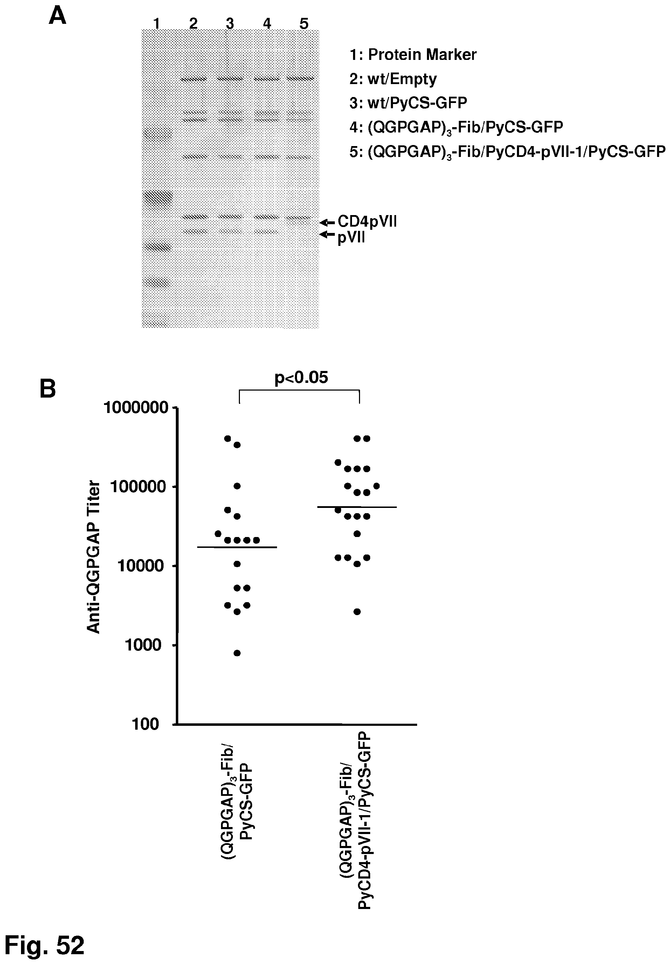

Antigen-specific CD4 T cells are required for antigen-specific B cell development and proliferation. Therefore to determine whether it would be possible to enhance PyCS-specific humoral immune response induced by capsid-modified adenovirus by inserting PyCS CD4 epitope in adenovirus protein, (QGPGAP)₃-Fib/PyCS-GFP that has PyCS CD4 epitope in pVII ((QGPGAP)₆-Fib/CD4-pVII-1/PyCS-GFP) was constructed. pVII is one of the adenovirus core proteins and the copy number per virion is 700-800, which is ideal for efficient CD4 epitope presentation onto MHC class II molecule. As shown in Fig. 52A, the pVII band is shifted by PyCS CD4 epitope insertion into pVII on a SDS-PAGE gel.

To test the effect of PyCS CD4 epitope insertion into pVII, naïve BALB/c mice were immunized with (QGPGAP)₃-Fib/PyCS-GFP or (QGPGAP)₃-Fib/CD4-pVII-1/PyCS-GFP as shown in Fig. 42A and anti-QGPGAP antibody titer was determined by ELISA at week 10. (QGPGAP)₃-Fib/CD4-pVII-1/PyCS-GFP induced significantly higher anti-QGPGAP antibody titer than (QGPGAP)₃-Fib/PyCS-GFP (Fig. 52B), and that indicated PyCS CD4 epitope insertion into pVII augmented humoral immune response induced by capsid-modified adenovirus.

### PfCSP CD4 epitope insertion into adenovirus core protein pVII

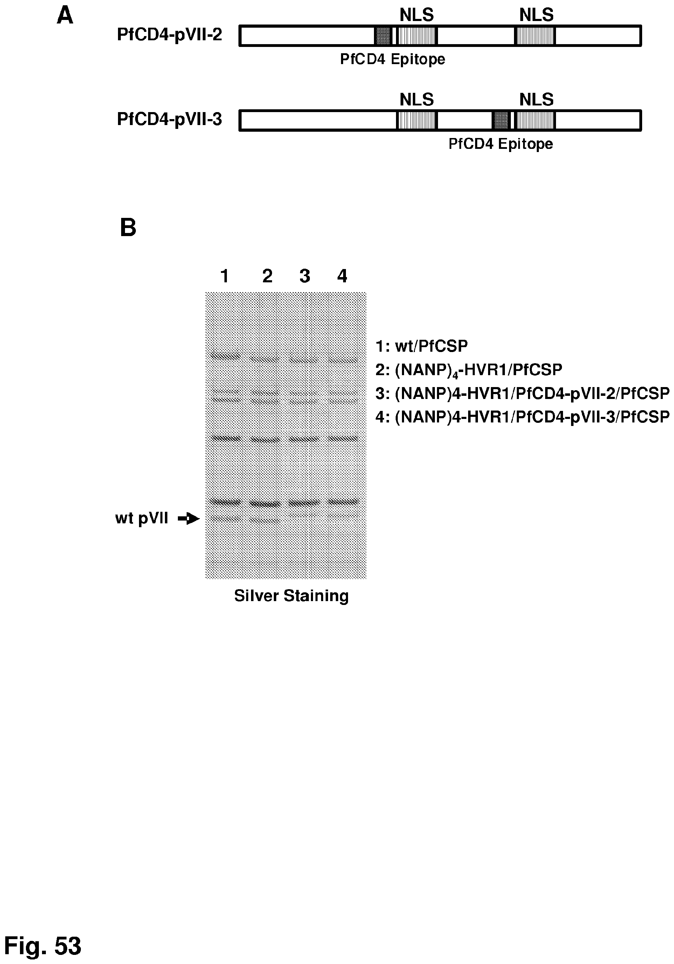

To evaluate the effect of PfCSP CD4+ epitope insertion into different positions in adenovirus core protein pVII on adenovirus-induced immune response, HVR1-modified PfCSP adenoviruses having the PfCSP CD4+ epitope just before the first Nuclear localization Signal (NLS) or between the two NLSs were constructed (Fig.53A).

To confirm the epitope insertion into pVII, purified recombinant adenoviruses were analyzed by SDS-PAGE as described above. As shown in Fig.53B, the pVII bands of (NANP)₄-HVR1/CD4-pVII-2/PfCSP and (NANP)₄-HVR1/CD4-pVII-3/PfCSP were shifted upward because of the epitope insertion.

### PfCSP-specific immune response induced by HVR1and pVII-modified PfCSP adenovirus

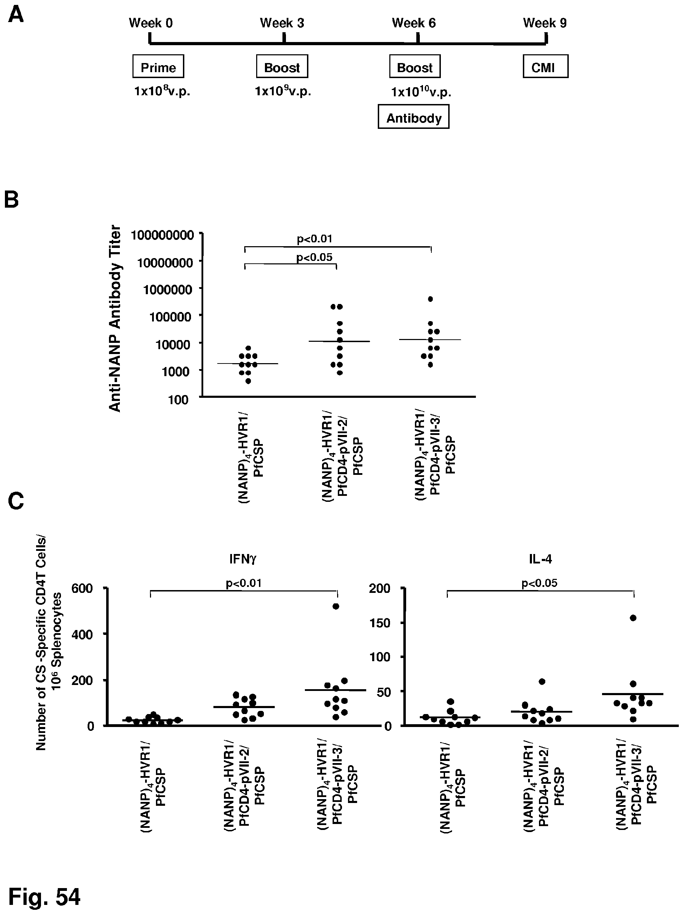

Next, naive BALB/c mice were given "boosts' of HVR1and pVII-modified PfCSP adenoviruses which have four repeats of NANP in HVR1 and the PfCD4+ epitope in pVII with at multiple increasing doses (i.e., 1x10⁸, 1x10⁹, and 1x10¹⁰v.p.) at 3 week intervals, as shown in Fig. 54A. PfCSP-specific humoral immune responses were measured as described above. (NANP)₄-HVR1/CD4-pVII-2/PfCSP and (NANP)₄-HVR1/ CD4-pVII-3/PfCSP induced significantly higher anti-NANP antibody titer than (NANP)₄-HVR1/PfCSP at week 6 (Fig. 54B). In terms of CMI, (NANP)₄-HVR1/CD4-pVII-3/PfCSP induced significantly higher IFNγ and IL-4-secreting PfCSP-specific CD4+ T cells than (NANP)₄-HVR1/PfCSP (Fig. 54C).

### Example 5: Effect of capsid-modification on anti-adenovirus immunity

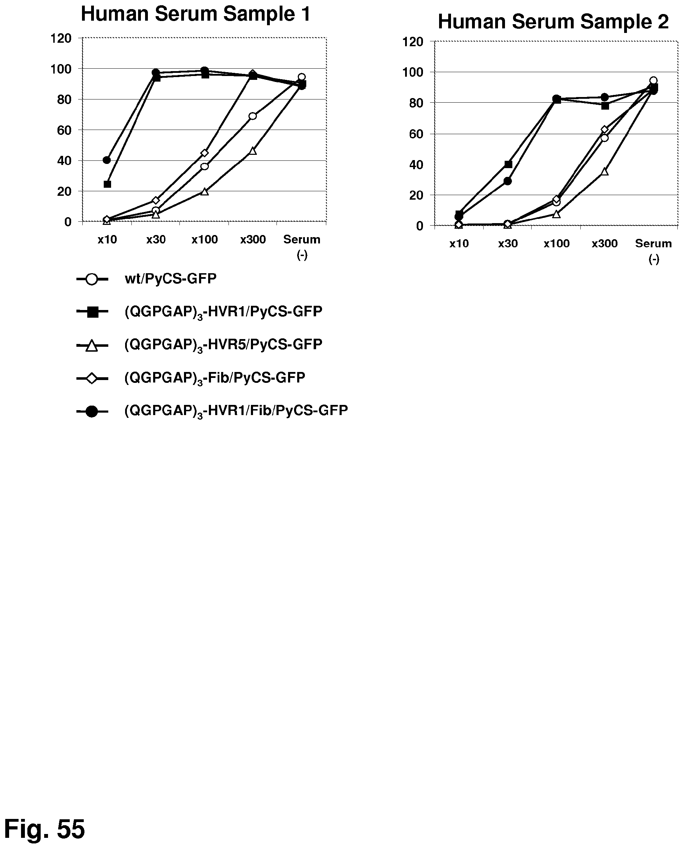

For the *in vitro* adenovirus neutralization experiments, serum was added to the AD293 cells at the indicated dilutions prior to the adenovirus infection. Caucasian serum samples were obtained from Innovative Research (Novi, MI, USA). All flow cytometry data was analyzed with FlowJo v8.8 software (Tree Star, Inc, Ashland, OR, USA). AD293 cells were infected with each capsid-modified adenovirus in the presence of human adenovirus neutralizing serum samples at the indicated dilution followed by measuring GFP expression by flow cytometry. A replacement of HVR1 with the PyCS-B epitope clearly made the adenovirus resilient to anti-adenovirus serotype 5 sera, whereas the modification of HVR5 or Fiber had no effect (Fig. 55).

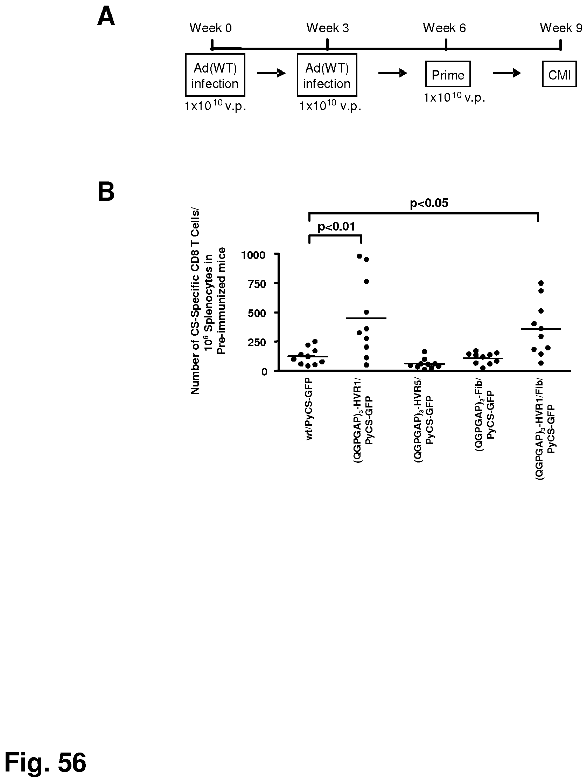

Next, it was determined whether HVR1 is a critical molecule for the neutralization *in vivo.* For this purpose, mice were infected with 1x10¹⁰ v.p. wt/Empty adenovirus twice to mount sufficient pre-existing anti-adenovirus immunity (Fig. 56A) and randomized based on their anti-adenovirus antibody titers, as determined by ELISA. The mice were then given a single immunizing dose of capsid-modified adenovirus or unmodified adenovirus, and the level of PyCS-specific CD8+ T cell response was measured as described above. Only vaccination with (QGPGAP)₃-HVR1/PyCS-GFP or (QGPGAP)₃-HVR1/Fib/PyCS-GFP was able to induce a significantly more potent CS-specific CD8+ T cell response, compared to that induced by other capsid-modified or unmodified adenovirus (Fig. 56B).

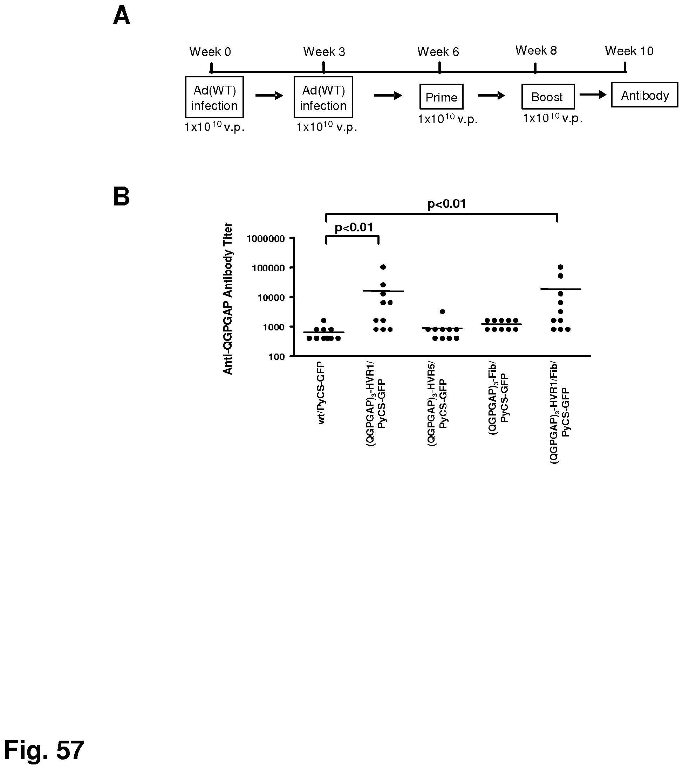

The level of antibody response against (QGPGAP)₃ epitope was also measured, which is expressed on the capsid proteins of rAd, in mice infected with wt/Empty Ad followed by vaccination with capsid-modified rAd (Figure 57A). Only mice vaccinated with (QGPGAP)₃-HVR1/PyCS-GFP and (QGPGAP)₃-HVR1/Fib/PyCS-GFP were able to mount a significantly higher titer of anti-QGPGAP antibody than those vaccinated with wt/PyCS-GFP (Figure 57B).

The examples described above are meant to more fully illustrate the embodiments and are not to be interpreted as limiting the scope of any claimed embodiment. In addition, the references cited within the disclosure, and all references listed below are hereby incorporated by reference in their entirety as if fully set forth herein.

### REFERENCES

Abbink, P., Lemckert, A.A., Ewald, B.A., Lynch, D.M., Denholtz, M., et al. 2007. Comparative seroprevalence and immunogenicity of six rare serotype recombinant adenovirus vaccine vectors from subgroups B and D. J Virol. 81:4654-4663.
Alonso, P.L., Sacarlal, J., Aponte, J.J., Leach, A., Macete, E., et al. 2004. Efficacy of the RTS,S/AS02A vaccine against Plasmodium falciparum infection and disease in young African children: randomised controlled trial. Lancet. 364:1411-1420.
Alonso, P.L., Sacarlal, J., Aponte, J.J., Leach, A., Macete, E., et al. 2005. Duration of protection with RTS,S/AS02A malaria vaccine in prevention of Plasmodium falciparum disease in Mozambican children: single-blind extended follow-up of a randomised controlled trial. Lancet. 366:2012-2018.
Anderson, R.J., Hannan, C.M., Gilbert, S.C., Laidlaw, S.M., Sheu, E.G., et al. 2004. Enhanced CD8+ T cell immune responses and protection elicited against Plasmodium berghei malaria by prime boost immunization regimens using a novel attenuated fowlpox virus. J Immunol. 172:3094-3100.
Athappilly, F.K., Murali, R., Rux, J.J., Cai, Z. & Burnett, R.M. The refined crystal structure of Hexon, the major coat protein of adenovirus type 2, at 2.9 A resolution. Journal of molecular biology 242, 430-455 (1994).
Barrat, F.J., Meeker, T,, Gregorio, J,, Chan, J.H., Uematsu, S., et al. Nucleic acids of mammalian origin can act as endogenous ligands for Toll-like receptors and may promote systemic lupus erythematosus. J Exp Med. 202, 1131-1139 (2005)
Bejon, P., Lusingu, J., Olotu, A., Leach, A., Lievens, M., et al. 2008. Efficacy of RTS,S/AS01E vaccine against malaria in children 5 to 17 months of age. N Engl J Med. 359:2521-2532.
Belousova, N., Krendelchtchikova, V., Curiel, D.T. & Krasnykh, V. Modulation of adenovirus vector tropism via incorporation of polypeptide ligands into the Fiber protein. Journal of virology 76, 8621-8631 (2002).
Bergelson, J.M. et al. Isolation of a common receptor for Coxsackie B viruses and adenoviruses 2 and 5. Science (New York, N.Y 275, 1320-1323 (1997).
Bewley, M.C., Springer, K., Zhang, Y.B., Freimuth, P. & Flanagan, J.M. Structural analysis of the mechanism of adenovirus binding to its human cellular receptor, CAR. Science (New York, N.Y286, 1579-1583 (1999).
Bruña-Romero, O., Schmieg, J., Del Val, M., Buschle, M. & Tsuji, M. The dendritic cell-specific chemokine, dendritic cell-derived CC chemokine 1, enhances protective cell-mediated immunity to murine malaria. J Immunol 170, 3195-3203 (2003).Hong, S.S., Karayan, L., Tournier, J., Curiel, D.T. & Boulanger, P.A. Adenovirus type 5 Fiber knob binds to MHC class I alpha2 domain at the surface of human epithelial and B lymphoblastoid cells. The EMBO journal 16, 2294-2306 (1997).
Bruña-Romero, O., González-Aseguinolaza, G., Hafalla, J.C., Tsuji, M., and Nussenzweig, R.S. 2001. Complete, long-lasting protection against malaria of mice primed and boosted with two distinct viral vectors expressing the same plasmodial antigen. Proc Natl Acad Sci USA. 98:11491-11496.
Bruña-Romero, O., Rocha, C.D., Tsuji, M. & Gazzinelli, R.T. Enhanced protective immunity against malaria by vaccination with a recombinant adenovirus encoding the circumsporozoite protein of Plasmodium lacking the GPI-anchoring motif. Vaccine 22, 3575-3584 (2004).
Calvo-Calle, J.M., Oliveira, G.A., Watta, C.O., Soverow, J., Parra-Lopez, C., et al. 2006. A linear peptide containing minimal T- and B-cell epitopes of Plasmodium falciparum circumsporozoite protein elicits protection against transgenic sporozoite challenge. Infect Immun. 74:6929-6939.
Clyde, D.F., Most, H., McCarthy, V.C., and Vanderberg, J.P. 1973. Immunization of man against sporozite-induced falciparum malaria. Am J Med Sci. 266:169-177.
Chroboczek, J., Ruigrok, R.W. & Cusack, S. Adenovirus Fiber. Current topics in microbiology and immunology 199 (Pt 1), 163-200 (1995).
Chu, Y., Heistad, D., Cybulsky, M.I. & Davidson, B.L. Vascular cell adhesion molecule-1 augments adenovirus-mediated gene transfer. Arterioscler Thromb Vasc Biol 21, 238-242 (2001).
Crawford-Miksza, L. & Schnurr, D.P. Analysis of 15 adenovirus Hexon proteins reveals the location and structure of seven hypervariable regions containing serotype-specific residues. Journal of virology 70, 1836-1844 (1996).
Crompton, J., Toogood, C.I., Wallis, N. & Hay, R.T. Expression of a foreign epitope on the surface of the adenovirus Hexon. The Journal of general virology 75 (Pt 1), 133-139 (1994).
Crystal, R.G. Transfer of genes to humans: early lessons and obstacles to success. Science (New York, N.Y 270, 404-410 (1995).
Douglas, J.T. Adenoviral vectors for gene therapy. Molecular biotechnology 36, 71-80 (2007).
Edelman, R., Hoffman, S.L., Davis, J.R., Beier, M., Sztein, M.B., et al. 1993. Long-term persistence of sterile immunity in a volunteer immunized with X-irradiated Plasmodium falciparum sporozoites. J Infect Dis. 168:1066-1070.
Grillot, D., Valmori, D., Lambert, P.H., Corradin, G., and Del Giudice, G. 1993. Presentation of T cell epitopes assembled as multiple-antigen peptides to murine and human T lymphocytes. Infect Immun. 61:3064-3067.
Graham, F.L. & Prevec, L. Adenovirus-based expression vectors and recombinant vaccines. Biotechnology (Reading, Mass 20, 363-390 (1992).
Gwadz, R.W., Cochrane, A.H., Nussenzweig, V., and Nussenzweig, R.S. 1979. Preliminary studies on vaccination of rhesus monkeys with irradiated sporozoites of Plasmodium knowlesi and characterization of surface antigens of these parasites. Bull World Health Organ.57 Suppl 1:165-173.
Hackett, N.R. et al. Use of quantitative TaqMan real-time PCR to track the time-dependent distribution of gene transfer vectors in vivo. Mol Ther 2, 649-656 (2000).
Harvey, B.G. et al. Airway epithelial CFTR mRNA expression in cystic fibrosis patients after repetitive administration of a recombinant adenovirus. The Journal of clinical investigation 104, 1245-1255 (1999).
Heemskerk, B. et al. Adenovirus-specific CD4+ T cell clones recognizing endogenous antigen inhibit viral replication in vitro through cognate interaction. J Immunol 177, 8851-8859 (2006).
Henry, L.J., Xia, D., Wilke, M.E., Deisenhofer, J. & Gerard, R.D. Characterization of the knob domain of the adenovirus type 5 Fiber protein expressed in Escherichia coli. Journal of virology 68, 5239-5246 (1994).
Hong, S.S., Habib, N.A., Franqueville, L., Jensen, S. & Boulanger, P.A. Identification of adenovirus (ad) penton base neutralizing epitopes by use of sera from patients who had received conditionally replicative ad (addI1520) for treatment of liver tumors. Journal of virology 77, 10366-10375 (2003).
Kester, K.E., Cummings, J.F., Ockenhouse, C.F., Nielsen, R., Hall, B.T., et al. 2008. Phase 2a trial of 0, 1, and 3 month and 0, 7, and 28 day immunization schedules of malaria vaccine RTS,S/AS02 in malaria-naive adults at the Walter Reed Army Institute of Research. Vaccine. 26:2191-2202.
Kirby, I. et al. Mutations in the DG loop of adenovirus type 5 Fiber knob protein abolish highaffinity binding to its cellular receptor CAR. Journal of virology 73, 9508-9514 (1999).
Koizumi, N., Mizuguchi, H., Utoguchi, N., Watanabe, Y. & Hayakawa, T. Generation of Fiber-modified adenovirus vectors containing heterologous peptides in both the HI loop and C terminus of the Fiber knob. The journal of gene medicine 5, 267-276 (2003).
Kozak M. 1987. An analysis of 5'-noncoding sequences from 699 vertebrate messenger RNAs. Nucleic Acids Res. 15:8125-148.
Krause, A., Joh, J.H., Hackett, N.R., Roelvink, P.W., Bruder, J.T., et al. 2006. Epitopes expressed in different adenovirus capsid proteins induce different levels of epitope-specific immunity. J Virol. 80:5523-5530.
Labow, D., Lee, S., Ginsberg, R.J., Crystal, R.G. & Korst, R.J. Adenovirus vector-mediated gene transfer to regional lymph nodes. Human gene therapy 11, 759-769 (2000).
Leen, A.M. et al. Identification of Hexon-specific CD4 and CD8 T cell epitopes for vaccine and immunotherapy. Journal of virology 82, 546-554 (2008).
Leopold, P.L. & Crystal, R.G. Intracellular trafficking of adenovirus: many means to many ends. Advanced drug delivery reviews 59, 810-821 (2007).
Mastrangeli, A. et al. "Sero-switch" adenovirus-mediated in vivo gene transfer: circumvention of anti-adenovirus humoral immune defenses against repeat adenovirus vector administration by changing the adenovirus serotype. Human gene therapy 7, 79-87 (1996).
Mathias, P., Wickham, T., Moore, M. & Nemerow, G. Multiple adenovirus serotypes use alpha v integrins for infection. Journal of virology 68, 6811-6814 (1994).
McConnell, M.J., Danthinne, X., and Imperiale, M.J. 2006. Characterization of a permissive epitope insertion site in adenovirus Hexon. J Virol. 80:5361-5370.
Meier, O. & Greber, U.F. Adenovirus endocytosis. The journal of gene medicine 5, 451-462 (2003).
Miyazawa, N. et al. Fiber swap between adenovirus subgroups B and C alters intracellular trafficking of adenovirus gene transfer vectors. Journal of virology 73, 6056-6065 (1999).
Miyazawa, N., Crystal, R.G. & Leopold, P.L. Adenovirus serotype 7 retention in a late endosomal compartment prior to cytosol escape is modulated by Fiber protein. Journal of virology 75, 1387-1400 (2001).
Mizuguchi, H. & Hayakawa, T. Targeted adenovirus vectors. Human gene therapy 15, 1034-1044 (2004).
Nakano, M.Y., Boucke, K., Suomalainen, M., Stidwill, R.P. & Greber, U.F. The first step of adenovirus type 2 disassembly occurs at the cell surface, independently of endocytosis and escape to the cytosol. Journal of virology 74, 7085-7095 (2000).
Nicklin, S.A. et al. Ablating adenovirus type 5 Fiber-CAR binding and HI loop insertion of the SIGYPLP peptide generate an endothelial cell-selective adenovirus. Mol Ther 4, 534-542 (2001).
Noureddini, S.C. & Curiel, D.T. Genetic targeting strategies for adenovirus. Molecular pharmaceutics 2, 341-347 (2005).
Nussenzweig, R.S., Vanderberg, J., Most, H., and Orton, C. 1967. Protective immunity produced by the injection of x-irradiated sporozoites of plasmodium berghei. Nature. 216:160-162.
Nussenzweig, R.S. & Long, C.A. Malaria vaccines: multiple targets. Science (New York, N. Y 265, 1381-1383 (1994).
Onion, D. et al. The CD4+ T cell response to adenovirus is focused against conserved residues within the Hexon protein. The Journal of general virology 88, 2417-2425 (2007).
Ophorst, O.J., Radosević, K., Havenga, M.J., Pau, M.G., Holterman, L., et al. 2006. Immunogenicity and protection of a recombinant human adenovirus serotype 35-based malaria vaccine against Plasmodium yoelii in mice. Infect Immun. 74:313-320.
Oualikene, W., Gonin, P. & Eloit, M. Short and long term dissemination of deletion mutants of adenovirus in permissive (cotton rat) and non-permissive (mouse) species. The Journal of general virology 75 (Pt 10), 2765-2768 (1994).
Priddy, F.H., Brown, D., Kublin, J., Monahan, K., Wright, D.P., et al. 2008. Safety and immunogenicity of a replication-incompetent adenovirus type 5 HIV-1 clade B gag/pol/nef vaccine in healthy adults. Clin Infect Dis. 46:1769-1781.
Roberts, M.M., White, J.L., Grutter, M.G. & Burnett, R.M. Three-dimensional structure of the adenovirus major coat protein Hexon. Science (New York, N. Y 232, 1148-1151 (1986).
Roberts, D.M., Nanda, A., Havenga, M.J., Abbink, P., Lynch, D.M., et al. 2006. Hexon-chimaeric adenovirus serotype 5 vectors circumvent pre-existing anti-vector immunity. Nature. 441:239-243.
Rodrigues, E.G., Zavala, F., Eichinger, D., Wilson, J.M., and Tsuji, M. 1997. Single immunizing dose of recombinant adenovirus efficiently induces CD8+ T cell-mediated protective immunity against malaria. J Immunol. 158:1268-1274.
Rodrigues, E.G., Zavala, F., Nussenzweig, R.S., Wilson, J.M. & Tsuji, M. Efficient induction of protective anti-malaria immunity by recombinant adenovirus. Vaccine 16, 1812-1817 (1998).
Roelvink, P.W., Mi Lee, G., Einfeld, D.A., Kovesdi, I. & Wickham, T.J. Identification of a conserved receptor-binding site on the Fiber proteins of CAR-recognizing adenoviridae. Science (New York, N.Y 286, 1568-1571 (1999).
Rux, J.J. & Burnett, R.M. Type-specific epitope locations revealed by X-ray crystallographic study of adenovirus type 5 Hexon. Mol Ther 1, 18-30 (2000).
Rux, J.J. & Burnett, R.M. Adenovirus structure. Human gene therapy 15, 1167-1176 (2004).
Roy, S. et al. Use of chimeric adenoviral vectors to assess capsid neutralization determinants. Virology 333, 207-214 (2005).
Silvie, O., Greco, C., Franetich, J.F., Dubart-Kupperschmitt, A., Hannoun, L., et al. 2006. Expression of human CD81 differently affects host cell susceptibility to malaria sporozoites depending on the Plasmodium species. Cell Microbiol. 8:1134-1146.
Sumida, S.M., Truitt, D.M., Lemckert, A.A., Vogels, R., Custers, J.H., et al. 2005. Neutralizing antibodies to adenovirus serotype 5 vaccine vectors are directed primarily against the adenovirus Hexon protein. J Immunol. 174:7179-7185.
Sun, P., Schwenk, R., White, K., Stoute, J.A., Cohen, J., et al. 2003. Protective immunity induced with malaria vaccine, RTS,S, is linked to Plasmodium falciparum circumsporozoite protein-specific CD4+ and CD8+ T cells producing IFN-gamma. J Immunol. 171:6961-6967.
Tao, D., Barba-Spaeth, G., Rai, U., Nussenzweig, V., Rice, C.M., and Nussenzweig, R.S. 2005. Yellow fever 17D as a vaccine vector for microbial CTL epitopes: protection in a rodent malaria model. J Exp Med. 201:201-209**.**
Teramoto, S. et al. Investigation of effects of anesthesia and age on aspiration in mice through LacZ gene transfer by recombinant E1-deleted adenovirus vectors. American journal of respiratory and critical care medicine 158, 1914-1919 (1998).
Top, F.H., Jr., Dudding, B.A., Russell, P.K. & Buescher, E.L. Control of respiratory disease in recruits with types 4 and 7 adenovirus vaccines. American journal of epidemiology 94, 142-146 (1971).
Top, F.H., Jr. Control of adenovirus acute respiratory disease in U.S. Army trainees. Yale J Biol Med 48, 185-195 (1975).
Tsuji, M., Romero, P., Nussenzweig, R.S., and Zavala, F. 1990. CD4+ cytolytic T cell clone confers protection against murine malaria. J Exp Med. 172:1353-1357.
Tsuji, M., Rodrigues, E.G. & Nussenzweig, S. Progress toward a malaria vaccine: efficient induction of protective anti-malaria immunity. Biol Chem 382, 553-570 (2001).
Wickham, T.J., Mathias, P., Cheresh, D.A. & Nemerow, G.R. Integrins alpha v beta 3 and alpha v beta 5 promote adenovirus internalization but not virus attachment. Cell 73, 309-319 (1993).
Wohlfart, C. Neutralization of adenoviruses: kinetics, stoichiometry, and mechanisms. Journal of virology 62, 2321-2328 (1988).
Worgall, S. et al. Modification to the capsid of the adenovirus vector that enhances dendritic cell infection and transgene-specific cellular immune responses. Journal of virology 78, 2572-2580 (2004).
Worgall, S., Krause, A., Rivara, M., Hee, K.K., Vintayen, E.V., et al. 2005. Protection against P. aeruginosa with an adenovirus vector containing an OprF epitope in the capsid. J Clin Invest. 115:1281-1289.
Worgall, S., Krause, A., Qiu, J., Joh, J., Hackett, N.R., and Crystal, R.G. 2007. Protective immunity to pseudomonas aeruginosa induced with a capsid-modified adenovirus expressing P. aeruginosa OprF. J Virol. 81:13801-13808.
Wu, H., Dmitriev, I., Kashentseva, E., Seki, T., Wang, M., et al. 2002. Construction and characterization of adenovirus serotype 5 packaged by serotype 3 Hexon. J Virol. 76:12775-12782.
Wu, H. et al. Identification of sites in adenovirus Hexon for foreign peptide incorporation. Journal of virology 79, 3382-3390 (2005).
Xia, D., Henry, L., Gerard, R.D. & Deisenhofer, J. Structure of the receptor binding domain of adenovirus type 5 Fiber protein. Current topics in microbiology and immunology 199 (Pt 1), 39-46 (1995).
Yang, Y., Li, Q., Ertl, H.C. & Wilson, J.M. Cellular and humoral immune responses to viral antigens create barriers to lung-directed gene therapy with recombinant adenoviruses. Journal of virology 69, 2004-2015 (1995).
Youil, R., Toner, T.J., Su, Q., Chen, M., Tang, A., et al. 2002. Hexon gene switch strategy for the generation of chimeric recombinant adenovirus. Hum Gene Ther. 13: 311-320.

The following section comprises numbered paragraphs which are not claims, but additional statements of the invention.
1. A recombinant adenovirus derived from a recombinant adenovirus plasmid vector, wherein the recombinant adenovirus plasmid vector comprises a nucleotide sequence encoding:
   a *Plasmodium* circumsporozoite protein gene, or antigenic portion thereof, operably linked to a heterologous promoter sequence, and
   one or more modified capsid and/or core protein genes, wherein an immunogenic epitope sequence of *Plasmodium* circumsporozoite has been inserted into or replaces at least part of the one or more capsid and/or core protein genes.
2. **The adenovirus of paragraph 1,** wherein the *Plasmodium* circumsporozoite protein gene further comprises a *Plasmodium falciparum* or a *Plasmodium yoelii* circumsporozoite protein gene.
3. **The adenovirus of paragraph 1,** wherein the *Plasmodium* circumsporozoite protein gene further comprises a codon-optimized *Plasmodium falciparum* or *Plasmodium yoelii* circumsporozoite protein gene.
4. **The adenovirus of paragraph 3,** wherein the codon-optimized protein gene is encoded by SEQ ID NO:1 or SEQ ID NO:2.
5. **The adenovirus of paragraph 1,** wherein the immunogenic epitope sequence further comprises a B cell and/or a T cell epitope sequence of *Plasmodium* circumsporozoite protein gene.
6. **The adenovirus of paragraph 5,** wherein the capsid protein gene further comprises a Hexon hypervariable region (HRV) sequence.
7. **The adenovirus of paragraph 6,** wherein the HRV sequence further comprises an HRV1 or HRV5 sequence and the B cell epitope:
   a) is inserted in the HVR1 or HVR5 sequence; or
   b) replaces a portion of the HVR1 or HRV5 sequence.
8. **The adenovirus of paragraph 7,** wherein the modified capsid protein gene is encoded by a nucleic acid sequence selected from the group consisting of, SEQ ID NO:12, SEQ ID NO:13, SEQID NO:14, SEQID NO:15, SEQID NO:16, SEQ ID NO:17, SEQ ID NO:18, SEQID NO:19, SEQ ID NO:20, SEQID NO:21, SEQ ID NO:22 or SEQ ID NO:23.
9. **The adenovirus of paragraph 6,** wherein the HRV sequence further comprises a HVR1 or HVR5 sequence and a CD4+ or CD8+ T cell epitope:
   a) is inserted in the HVR1 or HVR5 sequence; or
   b) replaces a portion of the HVR1 or HVR5 sequence.
10. **The adenovirus of paragraph 5,** wherein the capsid protein gene further comprises a capsid Fiber protein gene and the B cell epitope sequence is inserted into the Fiber protein gene.
11. **The adenovirus of claim 10,** wherein the modified capsid protein gene is encoded by SEQ ID NO:24 or SEQ ID NO:25.
12. **The adenovirus as in any of paragraphs 5 through 11,** wherein the B cell epitope sequence is a *Plasmodium falciparum* circumsporozoite protein gene B cell epitope sequence.
13. **The adenovirus of paragraph 12,** wherein the B cell epitope sequence is (NANP)ₙ (SEQ ID NO:60) and wherein n is 4 or more.
14. **The adenovirus of paragraph 12,** wherein the B cell epitope sequence is (NANP)₄, (NANP)₆, (NANP)₈, (NANP)₁₀, (NANP)₁₂, (NANP)₁₄, (NANP)₁₆, (NANP)₁₈, (NANP)₂₀, (NANP)₂₂ or (NANP)₂₈.
15. **The adenovirus of paragraph 1,** wherein the immunogenic epitope sequence further comprises a CD4+ T cell epitope sequence of *Plasmodium* circumsporozoite protein gene.
16. **The adenovirus of paragraph 15,** wherein the core protein gene further comprises a pVII protein gene and the CD4+ T cell epitope sequence is inserted into the pVII protein gene.
17. **The adenovirus of paragraph 16,** wherein the modified core protein gene is encoded by SEQ ID NO:26, SEQ ID NO:27, SEQ ID NO:28 or SEQ ID NO:29.
18. **The adenovirus as in any of paragraphs** 15 through 17, wherein the CD4+ T cell epitope sequence is a *Plasmodium falciparum* circumsporozoite protein gene CD4+ T cell epitope sequence.
19. **The adenovirus of paragraph 16,** wherein the CD4+ T cell epitope sequence is EYLNKIQNSLSTEWSPCSVT (SEQ ID NO:62).
20. **The adenovirus of paragraph 1,** wherein the adenovirus is produced from any one of the recombinant adenovirus plasmid vectors selected from the group consisting of an HVR1-modified adenovirus vector, a Fiber-modified adenovirus vector, an HVR1 and Fiber-modified adenovirus vector, a Fiber and pVII-modified adenovirus vector, an HVR1 and pVII-modified adenovirus vector and an HVR1, Fiber and pVII-modified adenovirus vector.
21. A method of inducing a cellular and humoral immune response against a *Plasmodium* circumsporozoite protein in a subject comprising administering to the subject at least one dose of a recombinant adenovirus, wherein said recombinant adenovirus is derived from a recombinant adenovirus plasmid vector, and wherein the recombinant adenovirus plasmid vector comprises a nucleotide sequence encoding:
   a *Plasmodium* circumsporozoite protein, or antigenic portion thereof, operably linked to a heterologous promoter, and
   a modified capsid or core protein, wherein an immunogenic epitope of *Plasmodium* circumsporozoite has been inserted into or replaces at least part of a capsid or core protein.
22. **The method of paragraph 21,** further comprising administering an adjuvant with the recombinant adenovirus.
23. A method of inducing a cellular and humoral immune response against a *Plasmodium* circumsporozoite protein in a subject lacking a pre-existing neutralizing antibody to an adenovirus serotype, comprising administering to the subject:
   a first priming dose of a first recombinant adenovirus, and
   a subsequent boosting dose of a second recombinant adenovirus,
   wherein the first recombinant adenovirus is derived from a recombinant adenovirus plasmid vector, and wherein the recombinant adenovirus plasmid vector comprises a nucleotide sequence encoding a *Plasmodium* circumsporozoite protein, or antigenic portion thereof, operably linked to a heterologous promoter, and
   wherein the second recombinant adenovirus is derived from a recombinant adenovirus plasmid vector, and wherein the recombinant adenovirus plasmid vector comprises a nucleotide sequence encoding a *Plasmodium* circumsporozoite protein, or antigenic portion thereof, operably linked to a heterologous promoter, and a modified capsid or core protein, wherein an immunogenic epitope of *Plasmodium* circumsporozoite has been inserted into or replaces at least part of a capsid or core protein.
24. **The method of paragraph 23,** further comprising administering an adjuvant with the recombinant adenovirus.
25. A pharmaceutical composition comprising a recombinant adenovirus, wherein the recombinant adenovirus is produced from any one of the recombinant adenovirus plasmid vectors selected from the group consisting of an HVR1-modified adenovirus vector, a Fiber-modified adenovirus vector, an HVR1 and Fiber-modified adenovirus vector, a Fiber and pVII-modified adenovirus vector, an HVR1 and pVII-modified adenovirus vector and an HVR1, Fiber and pVII-modified adenovirus vector.
26. A vaccine for malaria infection comprising a recombinant adenovirus, wherein the recombinant adenovirus is produced from any one of the recombinant adenovirus plasmid vectors selected from the group consisting of an HVR1-modified adenovirus vector, a Fiber-modified adenovirus vector, an HVR1 and Fiber-modified adenovirus vector, a Fiber and pVII-modified adenovirus vector, an HVR1 and pVII-modified adenovirus vector and an HVR1, Fiber and pVII-modified adenovirus vector.
27. **The vaccine for malaria infection of paragraph 26,** wherein the vaccine is administered to the subject intramuscularly, intradermally or subcutaneously.
28. **The vaccine for malaria infection of paragraph 26,** wherein the vaccine is administered with an adjuvant to the subject.

## Claims

1. A recombinant adenovirus derived from a recombinant adenovirus plasmid vector, wherein the recombinant adenovirus vector comprises a nucleotide sequence encoding a *Plasmodium* circumsporozoite protein gene, or antigenic portion thereof,
wherein a capsid protein expressed from the recombinant adenovirus vector comprises hexon or hexon hypervariable region (HVR) comprising an immunogenic epitope of *Plasmodium* circumsporozoite; and
wherein the immunogenic epitope sequence comprises a B cell epitope sequence represented by (NANP)ₙ, and n is selected from 12, 14, 16, 18 20 or 22, or a B cell epitope sequence represented by (QGPGAP)ₙ, and n is any one of 3 to 12.

2. The recombinant adenovirus of claim 1, wherein the nucleotide sequence encoding the *Plasmodium* circumsporozoite protein is a nucleotide sequence encoding a codon-optimized *Plasmodium falciparum* circumsporozoite protein.

3. The recombinant adenovirus of claim 2, wherein the nucleotide sequence encoding the codon-optimized *Plasmodium falciparum* circumsporozoite protein is represented by SEQ ID NO:2.

4. The recombinant adenovirus of any one of claims 1-3, wherein the Hexon comprising the immunogenic epitope is encoded by a nucleotide sequence selected from the group consisting of SEQ ID NO:16, SEQ ID NO:17, SEQ ID NO:18, SEQ ID NO:19, SEQ ID NO:20, and SEQ ID NO:21.

5. The recombinant adenovirus of any one of claims 1-4, wherein the immunogenic epitope is a B cell epitope sequence represented by (NANP)ₙ, and n is 16, 18, 20 or 22.

6. A recombinant adenovirus derived from a recombinant adenovirus plasmid vector, wherein the recombinant adenovirus vector comprises a nucleotide sequence encoding a *Plasmodium* circumsporozoite protein gene, or antigenic portion thereof, wherein a core protein expressed from the recombinant adenovirus vector comprises a pVII protein comprising a CD4+ T cell epitope of the *Plasmodium* circumsporozoite protein.

7. The recombinant adenovirus of claim 6, wherein a capsid protein expressed from the recombinant adenovirus vector comprises hexon or hexon hypervariable region (HVR) comprising an immunogenic epitope of *Plasmodium* circumsporozoite; and wherein the immunogenic epitope comprises a B cell epitope represented by (NANP)ₙ and n is 4, 6, 8, 10, 12, 14, 16, 18, 20 or 22, or a B cell epitope represented by (QGPGAP)ₙ, and n is any one of 3 to 12.

8. The recombinant adenovirus of claim 6, wherein the CD4+ T cell epitope is represented by an amino acids sequence represented by EYLNKIQNSLSTEWSPCSVT (SEQ ID NO:62), or YNRNIVNRLLGDALNGKPEEK (SEQ ID NO:61).

9. The recombinant adenovirus of claim 6, wherein the nucleotide sequence encoding the pVII protein comprising a CD4+ T cell epitope is encoded by SEQ ID NO:27, SEQ ID NO:28 or SEQ ID NO:29.

10. The recombinant adenovirus of claim 8, wherein the capsid protein comprises hexon or hexon hypervariable region (HVR) comprising an immunogenic epitope of *Plasmodium* circumsporozoite; and wherein, when the CD4+ T cell epitope comprises an amino acids sequence represented by EYLNKIQNSLSTEWSPCSVT (SEQ ID NO:62), the immunogenic epitope comprises a B cell epitope represented by (NANP)ₙ and n is 4, 6, 8, 10, 12, 14, 16, 18, 20 or 22, or when the CD4+ T cell epitope sequence comprises an amino acid sequence represented by YNRNIVNRLLGDALNGKPEEK (SEQ ID NO: 61), the immunogenic epitope is a B cell epitope represented by (QGPGAP)ₙ, and n is any one of 3 to 12.

11. A pharmaceutical composition comprising the recombinant adenovirus of any one of claims 1 to 10.

12. A vaccine for malaria infection comprising the recombinant adenovirus of any one of claims 1 to 10.

13. The vaccine for malaria infection of claim 12 further comprising an adjuvant.
